# EUROPEAN PATENT SPECIFICATION

(11) **EP 1 802 653 B1**
(45) Date of publication and mention of the grant of the patent: **09.09.2009**
(21) Application number: 05794760.8
(22) Date of filing: 18.10.2005
(51) Int. Cl.: C07K 14/435, C07K 14/705, A61K 38/17, C12Q 1/68, A61K 48/00, C12N 15/62, C12N 15/00

(54) **MAM DOMAIN CONTAINING PROTEIN**
EIN DIE MAM-DOMÄNE ENTHALTENDES PROTEIN
PROTEINE CONTENANT LE DOMAINE MAM

(30) Priority: 18.10.2004 GB 0423126
(43) Date of publication of application: 04.07.2007
(73) Proprietor: ARES TRADING S.A., 1170 Aubonne (CH)
(72) Inventor: FITZGERALD, Stephen Noel, Inpharmatica Limited, London W1T 2NU (GB); FAGAN, Richard Joseph, Inpharmatica Limited, London W1T 2NU (GB); POWER, Christine, F-01710 Thoiry (FR); YORKE-SMITH, Melanie, Golden Lake Garden, No. 938 Sha Ta Bei Road, 510515 Guangzhou, Guangdong (CH)
(74) Representative: Goodfellow, Hugh Robin
(86) International application number: PCT/GB2005/004032
(87) International publication number: WO 2006/043060

(56) References cited:
- WO-A-02/34783
- WO-A-02/097031
- WO-A-20/04044011
- CISMASIU VALERIU B ET AL: "The MAM (meprin/A5-protein/PTPmu) domain is a homophilic binding site promoting the lateral dimerization of receptor-like protein-tyrosine phosphatase mu" JOURNAL OF BIOLOGICAL CHEMISTRY, vol. 279, no. 26, 25 June 2004 (2004-06-25), pages 26922-26931, XP002375952 ISSN: 0021-9258
- BESCO J ET AL: "GENOMIC STRUCTURE AND ALTERNATIVE SPLICING OF MURINE R2B RECEPTOR PROTEIN TYROSINE PHOSPHATASE (PTPKAPPA, MU, RHO AND PCP-2)" BMC GENOMICS, BIOMED CENTRAL, LONDON, GB, vol. 5, no. 14, 11 February 2004 (2004-02-11), XP001182766 ISSN: 1471-2164

## Description

This invention relates to a novel protein, termed INSP 152, herein identified as a secreted protein, in particular, as a MAM domain containing protein and to the use of this protein and nucleic acid sequence from the encoding gene in the diagnosis, prevention and treatment of disease.

### Background

The process of drug discovery is presently undergoing a fundamental revolution as the era of functional genomics comes of age. The term "functional genomics" applies to an approach utilising bioinformatics tools to ascribe function to protein sequences of interest.

Such tools are becoming increasingly necessary as the speed of generation of sequence data is rapidly outpacing the ability of research laboratories to assign functions to these protein sequences.

As bioinformatics tools increase in potency and in accuracy, these tools are rapidly replacing the conventional techniques of biochemical characterisation. Indeed, the advanced bioinformatics tools used in identifying the present invention are now capable of outputting results in which a high degree of confidence can be placed.

Various institutions and commercial organisations are examining sequence data as they become available and significant discoveries are being made on an on-going basis.

However, there remains a continuing need to identify and characterise further genes and the polypeptides that they encode, as targets for research and for drug discovery.

### Introduction

### Secreted Proteins

The ability for cells to make and secrete extracellular proteins is central to many biological processes. Enzymes, growth factors, extracellular matrix proteins and signalling molecules are all secreted by cells. This is through fusion of a secretory vesicle with the plasma membrane. In most cases, but not all, proteins are directed to the endoplasmic reticulum and into secretory vesicles by a signal peptide. Signal peptides are cis-acting sequences that affect the transport of polypeptide chains from the cytoplasm to a membrane bound compartment such as a secretory vesicle. Polypeptides that are targeted to the secretory vesicles are either secreted into the extracellular matrix or are retained in the plasma membrane. The polypeptides that are retained in the plasma membrane will have one or more transmembrane domains. Examples of secreted proteins that play a central role in the functioning of a cell are cytokines, hormones, extracellular matrix proteins (adhesion molecules), proteases, and growth and differentiation factors. Description of some of the properties of these proteins follows.

### MAM domain containing proteins

MAM (meprin, A-5 protein, receptor protein-tyrosine phosphatase µ) domains are comprised of about 160 amino acids. They are present in transmembrane proteins such as the meprins and receptor protein-tyrosine phosphatases, where they appear to function in cell/cell interactions.

Meprins belong to the "astacin family" of metalloendopeptidases and the "metzincin superfamily". They are multidomain, oligomeric proteases that are secreted or highly expressed in mammalian brush-border membranes of the intestine or kidney. The meprins are complex and structurally unique proteases: homo- or heterotetrameric glycoprotein that contain astacin-like catalytic domains and disulfide-bridged dimers. They are capable of degrading proteins such as collagen and gelatin, hormones such as parathyroid hormone, luteinizing hormone-releasing hormone, and melanocyte-stimulating hormone, and small peptides such as bradykinin, angiotensins, and gastrin.

All receptor-like protein tyrosine phosphatases (RPTPs) type II contain a MAM domain. These proteins regulate many important cellular functions including signal transduction, growth, differentiation, cell adhesion and axon guidance. MAM domains have also been shown to be linked to sperm adhesion. The domain may therefore play a role in gamete recognition and/or signalling.

Increasing knowledge of these domains is therefore of extreme importance in increasing the understanding of the underlying pathways that lead to the disease states and associated disease states mentioned above, and in developing more effective gene and/or drug therapies to treat these disorders.

### THE INVENTION

The invention is based on the discovery that the INSP152 polypeptide is a MAM domain containing protein. In particular, the invention is based on the surprising findings that polypeptides of the present invention:
a) show unexpected restricted expression in the small intestine, in lupus and in Crohn's disease biopsy samples derived from ileal biopsies and
b) are potent inhibitors of lymphocytes, preferably of T cells and
c) are CTLA4-Ig-like polypetides.

In one embodiment of the first aspect of the invention, there is provided a polypeptide which:
(i) comprises the amino acid sequence as recited in SEQ ID NO:2 and/or SEQ ID NO: 4; and which acts as an inhibitor of T lymphocytes.

Preferably, the polypeptide according to this first aspect of the invention is selected from the group consisting of:
(i) the amino acid sequence recited in SEQ ID NO: 6;
(ii) the amino acid sequence recited in SEQ ID NO: 8;
(iii) the amino acid sequence recited in SEQ ID NO: 10;
(iv) the amino acid sequence recited in SEQ ID NO: 12;
(v) the amino acid sequence recited in SEQ ID NO: 14;
(vi) the amino acid sequence recited in SEQ ID NO: 16; or
(vii) the amino acid sequence recited in SEQ ID NO: 18.

The polypeptide having the sequence recited in SEQ ID NO: 6 is referred to hereafter as the "cloned INSP152-D1-SV1 polypeptide". The polypeptide having the sequence recited in SEQ ID NO: 12 is referred to hereafter as the "extracellular INSP152-SV1 polypeptide". The polypeptide having the sequence recited in SEQ ID NO: 18 is referred to hereafter as the "INSP152-SV1 polypeptide". The polypeptide having the sequence recited in SEQ ID NO: 20 is referred to hereafter as the "INSP152-D1 fragment polypeptide". The polypeptide having the sequence recited in SEQ ID NO: 22 is referred to hereafter as the "full cloned INSP152-D1-SV1 polypeptide". The polypeptide having the sequence recited in SEQ ID NO: 24 is referred to hereafter as the "extracellular INSP152 polypeptide". The polypeptide having the sequence recited in SEQ ID NO: 26 is referred to hereafter as the "full INSP152 polypeptide". The polypeptide having the sequence recited in SEQ ID NO: 28 is referred to hereafter as the "full INSP152-SVI polypeptide".

A polypeptide according to the first aspect of the invention may comprise a signal peptide and/or a histidine tag. Preferably the histidine tag is found at the C-terminal of the polypeptide. Preferably the histidine tag comprises 1-10 histidine residues (*e*.*g*. 1, 2, 3, 4, 5, 6, 7, 8, 9 or 10 residues). More preferably, the histidine tag comprises 6 histidine residues.

Although the Applicant does not wash to be bound by this theory, it is postulated that the first 19 amino acids of the INSP152 polypeptide form a signal peptide.

The mature INSP152-D1 fragment polypeptide' sequence without this postulated signal sequence is recited in SEQ ID NO: 2. The cloned INSP152-D1-SV1 polypeptides sequence without this postulated signal sequence is recited in SEQ ID NO: 4. The extracellular INSP152 polypeptide sequence without this postulated signal sequence is recited in SEQ ID NO: 8. The extracellular INSP152-SV1 polypeptide sequence without this postulated signal sequence is recited in SEQ ID NO: 10. The INSP152 polypeptide sequences without this postulated signal sequence is recited in SEQ ID NO: 14. The INSP152-SV1 polypeptide sequence without this postulated signal sequence is recited in SEQ ID NO: 16.

The polypeptide having the sequence recited in SEQ ID NO: 2 is referred to hereafter as "mature INSP152-D1 fragment polypeptide". The polypeptide having the sequence recited in SEQ ID NO: 4 is referred to hereafter as "mature cloned INSP152-D1-SV1 polypeptide". The polypeptide having the sequence recited in SEQ ID NO: 8 is referred to hereafter as "mature extracellular INSP152 polypeptide". The polypeptide having the sequence recited in SEQ ID NO: 10 is referred to hereafter as "mature extracellular INSP152-SV1 polypeptide". The polypeptide having the sequence recited in SEQ ID NO: 14 is referred to hereafter as "mature INSP152 polypeptide". The polypeptide having the sequence recited in SEQ ID NO: 16 is referred to hereafter as "mature INSP152-SV1 polypeptide".

The term "INSP152 polypeptides" as used herein includes polypeptides comprising the mature INSP152-D1 fragment polypeptide, the mature cloned INSP152-D1-SV1 polypeptide, the cloned INSP152-D1-SV1 polypeptide, the mature extracellular INSP152 polypeptide, the mature extracellular INSP152-SV1 polypeptide, the extracellular INSP152-SV1 polypeptide, the mature INSP152 polypeptide, the mature INSP152-SV1 polypeptide, the INSP152-SV1 polypeptide, the INSP152-D1 fragment polypeptide, the full cloned INSP152-D1-SV1 polypeptide, the extracellular INSP152 polypeptide, the full INSP152 polypeptide and the full INSP152-SV1 polypeptide.

The term "MAM domain containing protein" refers to a molecule containing at least one MAM domain.

Preferably, the "MAM domain containing protein" may be a molecule containing a MAM domain detected with an e-value lower than 0.1, 0.01, 0.001, 0.0001, 0.0002, 0.00001, 0.000001 or 0.0000001.

Preferably, a polypeptide according to any one of the above-described aspects of the invention functions as a MAM domain containing and/or LDL domain containing polypeptide.

By "functions as a MAM domain containing protein" we refer to polypeptides that comprise amino acid sequence or structural features that can be identified as conserved features within the polypeptides of the MAM domain containing protein family, such that the polypeptide's interaction with protease is maintained intact. In particular, we refer to the presence of cysteine residues in specific positions within the polypeptide that allow the formation of disulphide bonds.

The so-called MAM domain is an approximately 170 amino acid domain that has been recognised in the extracellular region of functionally diverse proteins. These proteins have a modular, receptor-like architecture comprising a signal peptide, an N-terminal extracellular domain, a single transmembrane domain and an intracellular domain. Such proteins include meprin (a cell surface glycoprotein); A5 antigen (a developmentally-regulated cell surface protein); and receptor-like tyrosine protein phosphatase. The MAM domain is thought to have an adhesive function. It contains 4 conserved cysteine residues, which probably form disulphide bridges. Mutations in the meprin MAM domain affect noncovalent associations within meprin oligomers. In receptor tyrosine phosphatase µ-like molecules the MAM domain is important for homophilic cell-cell interactions.

### MAM containing proteins include:

*Meprin*. This cell surface glycoprotein contains a zinc-metalloprotease domain capable of degrading a variety of polypeptides. Meprin is composed of two structurally related subunits (alpha and beta) that form homo- or heterotetramers by the non-covalent association of two disulfide-linked dimers. In both subunits, the MAM domain is located after the catalytic domain. The MAM domain in meprin is involved in oligomerization.

*Neuropilin* (A5 antigen), a calcium-independent cell adhesion molecule that functions during the formation of certain neuronal circuits. The sequence contains 2 CUB domains (2 FA58C domains) and a MAM domain.

*Receptor-like tyrosine protein phosphatases Mu, Kappa and PCP-2* (EC 3.1.3.48). These PTPases have an extracellular region which consists of a MAM domain followed by an Ig-like domain and four fibronectin-type III domains. The family of PTPs consists of soluble and receptor-like PTPs (RPTPs). The extracellular region of PTPµ promotes cell-cell aggregation (the MAM domain of PTPµ is a homophilic binding molecule of the extracellular region). Homophilic binding in the ectodomains of PTPκ and PTPλ strongly suggests involvement of RPTPs in signal transduction through cell-to-cell contact. The MAM domain of PTPµ has homophilic binding properties through four conserved cysteine residues forming two intramolecular disulfide bridges (PTPµ cis (lateral) interactions) (see Cismasiu et al. J Biol Chem. 2004 279(26):26922-31). RPTPs have been involved during development, in axon growth and guidance, neurite outgrowth, as cell adhesion molecules (CAMs) and cell adhesion mediators, cell signaling, in target selection and recognition by retinal axon, and hematopoietic cell differentiation (see Beltran and Bixby, Front Biosci. 2003 8:d87-99.). CAMs are involved in homoestasis, immune response, inflammation, axonogenesis, and embryogenesis. Disruption of CAMs results in the onset of diseases such as Penphigous vulgaris, Leukocyte adhesion deficiency-1 and -2, Glanzmann thrombasthenia, atherosclerosis, diabetic vasculopathy and cancer metastasis.

*Vertebrate enteropeptidase* (EC 3.4.21.9), a type II membrane protein of the intestinal brush border, which activates trypsinogen. It consists at least of a catalytic light chain and a multidomain heavy chain which has 2 LDL receptor class A domains, a MAM domain, a SRCR domain and a CUB domain.

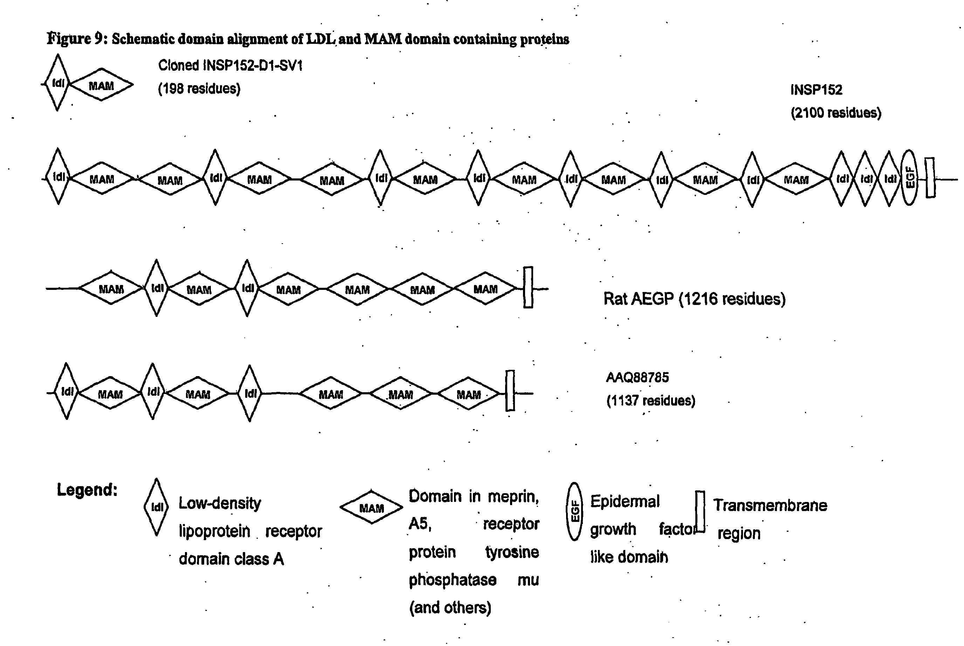

*Apical endosomal glycoprotein* from rat, a protein probably involved in the sorting and selective transport of receptors and ligands across polarized epithelia. This protein contains 6 MAM domains. Figure 9 shows the structural homology between INSP152 and apical endosomal glycoprotein.

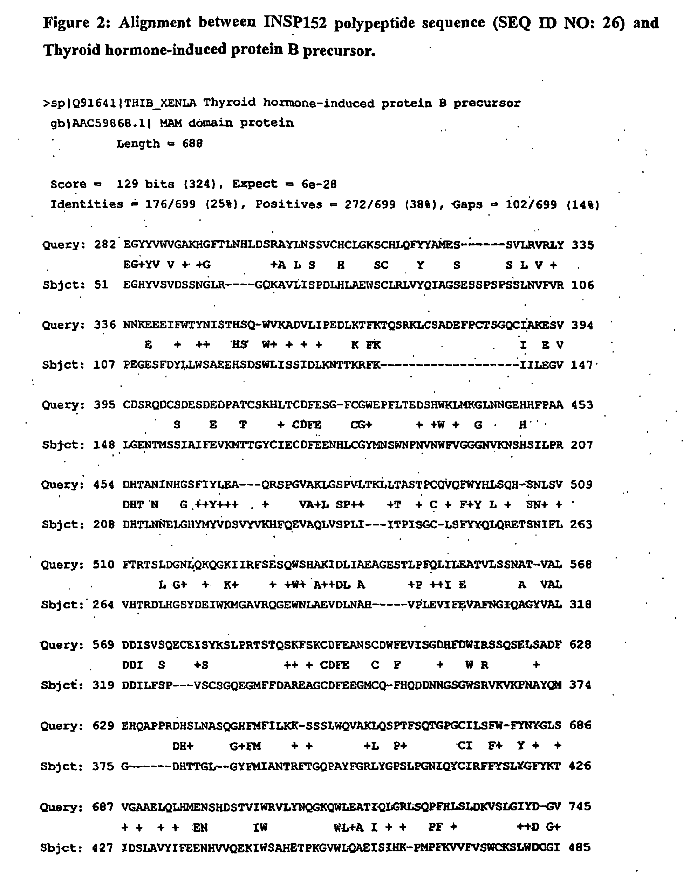

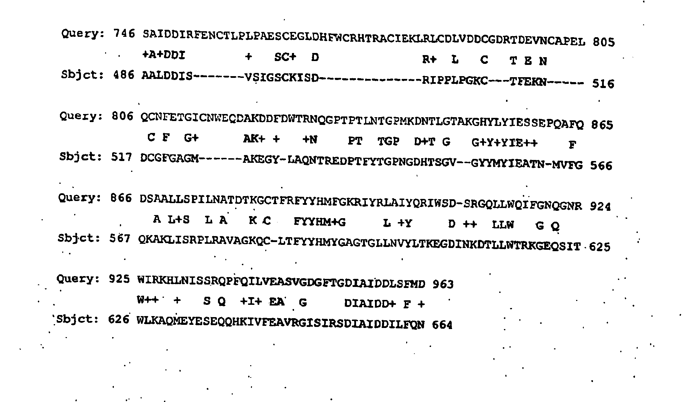

*Xenopus laevis thyroid hormone induced protein B.* This protein contains 4 MAM domains. An amino acid alignment between Xenopus laevis thyroid hormone induced protein B and INSP152 is shown in Figure 2.

*Pig zonadhesin*, a protein that binds in a species-specific manner to' the zona pellucida of the egg.

A polypeptide according to the invention may also function as an LDL domain containing polypeptide. By "functions as an LDL domain containing protein" we refer to polypeptide that comprise amino acid sequence or structural features that can be identified as conserved features within the polypeptides of the LDL domain containing protein family, such that the polypeptide's interaction with its biological protein partners is maintained. In particular, we refer to the presence of cysteine residues in specific positions within the polypeptide that allow the formation of disulphide bonds.

The cysteine-rich repeat in the low-density lipoprotein (LDL) receptor plays a central role in mammalian cholesterol metabolism. The N-terminal type A repeats in LDL receptor bind the lipoproteins. Other homologous domains occur in related receptors, including the very low-density lipoprotein receptor and the LDL receptor-related protein/alpha 2-macroglobulin receptor, and in proteins which are functionally unrelated, such as the C9 component of complement. Mutations in the LDL receptor gene cause familial hypercholesterolemia.

The LDL-receptor class A domain contains 6 disulfide-bound cysteines and a highly conserved cluster of negatively charged amino acids, of which many are clustered on one face of the module. A schematic representation of this domain is shown here: 'C': conserved cysteine involved in a disulfide bond.
'x': any residue.

In LDL-receptors the class A domains form the binding site for LDL and calcium. The acidic resides between the fourth and sixth cysteines are important for high-affinity binding of positively charged sequences in LDLR's ligands. The repeat has been shown to consist of a beta-hairpin structure followed by a series of beta turns. The binding of calcium seems to induce no significant conformational change.

Following these repeats is a 350 residue domain that resembles part of the epidermal growth factor (EGF) precursor (which has been identified in INSP152, see Figure 9).

Similar domains have been found in other extracellular and membrane proteins which are listed below:

*Vertebrate very low density lipoprotein (VLDL) receptor*, which binds and transports VLDL. Its extracellular domain is composed of 8 LDLRA domains; 3 EGF-like domains and 6 LDL-receptor class B domains (LDLRB).

*Vertebrate low-density lipoprotein receptor-related protein 1* (LRP1), which may act as a receptor for the endocytosis of extracellular ligands. LRP1 contains 31 LDLRA domains and 22 EGF-like domains.

*Vertebrate low-density lipoprotein receptor-related protein* 2 (LRP2) (also known as gp330 or megalin). LRP2 contains 36 LDLRA domains and 17 EGF-like domains.

*A LRP-homolog from Caenorhabditis elegans*, which contains 35 LDLRA domains and 17 EGF-like domains.

*Drosophila putative vitellogenin receptor*, with 13 copies of LDLRA domains and 17 EGF-like repeats.

*Complement factor I*, which is responsibly for cleaving the alpha-chains of C4b and C3b. It consists of a FIMAC domain (Factor I/MAC proteins C6/C7), a scavenger receptor-like domain, 2 copies of LDLRA and a C-terminal serine protease domain.

*Complement components C6, C7, C8 and C9.* They contain each one LDLRA domain.

*Perlecan*, a large multidomain basement membrane heparan sulfate proteoglycan composed of 4 LDLRA domains, 3 LamB domains, 12 laminin EGF- like domains, 14-21 IG-like domains, 3 LamG domains, and 4 EGF-like domains. A similar but shorter proteoglycan (UNC52) is found in *Caenorhabditis elegans* which has 3 repeats of LDLRA.

*Invertebrate giant extracellular hemoglobin linker chains*, which allow heme-containing chains to construct giant hemoglobin (1 LDLRA domain).

*G-protein coupled receptor Grl101 of the snail Lymnaea stagnalis*, which might directly transduce signals carried by large extracellular proteins.

*Vertebrate enterokinase* (EC 3.4.21.9), a type II membrane protein of the intestinal brush border, which activates trypsinogen. It consists at least of a catalytic light chain and a multidomain heavy chain which has 2 LDLRA, a MAM domain, a SRCR domain and a CUB domain.

*Human autosomal dominant polycystic kidney disease protein 1* (PKD1), which is involved in adhesive protein-protein and protein-carbohydrate interactions. The potential calcium-binding site of its single LDLRA domain is missing.

*Vertebrate integral membrane protein' DGCR2*/*IDD*, a potential adhesion receptor with 1 LDLRA domain, a C-type lectin and a VWFC domain.

*Drosophila serine protease nudel* (EC 3.4.21.), which is involved in the induction of dorsoventral polarity of the embryo. It has 11 LDLRA domains, 3 of which miss the first disulfide bond (C1-C3).

*Avian subgroup A rous sarcoma virus receptor* (1 copy of LDLRA).

*Bovine Sco-spondin*, which is secreted by the subcommissural organ in embryos and is involved in the modulation of neuronal aggregation. It contains at least 2 EGF-like domains and 3 LDLRA domains.

Preferably, the activity of a polypeptide of the present invention can be confirmed in at least one of the following assays:
a) in the mouse model of inflammatory bowel disease induced by dextran sulphate sodium (DSS) as described in Okayasu et al. A novel method in the induction of reliable experimental acute and chronic ulcerative colitis in mice. Gastroenterology 1990. Vol. 98, pp. 694-702), or
b) in the *in vitro* and *in vivo* assays as well as animal models of inflammatory bowel diseases as reviewed by Borm and Gouma (Drug Discovery Today: Disease Models; Vol. 1, No. 4, 2004, pp. 437-443), or
c) in models of systemic lupus erythematosus as reviewed by Rigby and Vyse (Drug Discovery Today: Disease Models; Vol. 1, No. 4, 2004, pp. 445-449), or
d) in the human MLR assay as described in Example 7.

An "antigenic determinant" of the present invention may be a part of a polypeptide of the present invention, which binds to an antibody-combining site or to a T-cell receptor (TCR). Alternatively, an "antigenic determinant" may be a site on the surface of a polypeptide of the present invention to which a single antibody molecule binds. Generally an antigen has several or many different antigenic determinants and reacts with antibodies of many different specificities. Preferably, the antibody is immunospecific to a polypeptide of the invention. Preferably, the antibody is immunospecific to a polypeptide of the invention, which is not part of a fusion protein. Preferably, the antibody is immunospecific to any one of the INSP152 polypeptides described herein, such as INSP152 or a fragment thereof. Antigenic determinants usually consist of chemically active surface groupings of molecules, such as amino acids or sugar side chains, and can have specific three dimensional structural characteristics, as well as specific charge characteristics. Preferably, the "antigenic determinant" refers to a particular chemical group on a polypeptide of the present invention that is antigenic, *i*.*e*. that elicit a specific immune response.

The polypeptides Q5VYJ5_HUMAN and CAH71834 (AL354695), and their encoding nucleic acid sequences are specifically excluded from the scope of this invention.

In a second aspect, the invention provides a purified nucleic acid molecule which encodes a polypeptide of the first aspect of the invention.

The term "purified nucleic acid molecule" preferably refers to a nucleic acid molecule of the invention that (1) has been separated from at least about 50 percent of proteins, lipids, carbohydrates, or other materials with which it is naturally found when total nucleic acid is isolated from the source cells, (2) is not linked to all or a portion of a polynucleotide to which the "purified nucleic acid molecule" is linked in nature, (3) is operably linked to a polynucleotide which it is not linked to in nature, or (4) does not occur in nature as part of a larger polynucleotide sequence. Preferably, the isolated nucleic acid molecule of the present invention is substantially free from any other contaminating nucleic acid molecule(s) or other contaminants that are found in its natural environment that would interfere with its use in polypeptide production or its therapeutic, diagnostic, prophylactic or research use. In a preferred embodiment, genomic DNA are specifically excluded from the scope of the invention. Preferably, genomic DNA larger than 10 kbp (kilo base pairs), 50 kbp, 100 kbp, 150 kbp, 200 kbp, 250 kbp or 300 kbp are specifically excluded from the scope of the invention. Preferably, the "purified nucleic acid molecule" consists of cDNA only.

Preferably, the purified nucleic acid molecule comprises the nucleic acid sequence as recited in SEQ ID NO: 1 (encoding the mature INSP152-D1 fragment polynucleotide sequence), SEQ ID NO: 3 (encoding the mature cloned INSP152-D1-SV1 polynucleotide sequence), SEQ ID NO: 5 (encoding the cloned INSP152-D1-SV1 polynucleotide sequence), SEQ ID NO: 7 (encoding the mature extracellular INSP152 polynucleotide sequence), SEQ ID NO: 9 (encoding the mature extracellular INSP152-SV1 polynucleotide sequence), SEQ ID NO: 11 (encoding the extracellular INSP152-SV1 polynucleotide sequence), SEQ ID NO: 13 (encoding the mature INSP 152 polynucleotide sequence), SEQ ID NO: 15 (encoding the mature INSP152-SV1 polynucleotide sequence), SEQ ID NO: 17 (encoding the INSP152-SV1 polynucleotide sequence), SEQ ID NO: 19 (encoding the INSP152-D fragment polynucleotide sequence), SEQ ID NO: 21 (encoding the cloned INSP152-D1-SV1 polynucleotide sequence), SEQ ID NO: 23 (encoding the extracellular INSP152 polynucleotide sequence), SEQ ID NO: 25 (encoding the full INSP152 polynucleotide sequence) and/or SEQ ID NO: 27 (encoding the full INSP152-SV1 polynucleotide sequence).

The invention further provides that the purified nucleic acid molecule consists of the nucleic acid sequence as recited in SEQ ID NO: 1 (encoding the mature INSP152-D1 fragment polynucleotide sequence), SEQ ID NO: 3 (encoding the mature cloned INSP 152-D1-SV1 polynucleotide sequence), SEQ ID NO: 5 (encoding the cloned INSP152-D1-SV1 polynucleotide sequence), SEQ ID NO: 7 (encoding the mature extracellular INSP152 polynucleotide sequence), SEQ ID NO: 9 (encoding the mature extracellular INSP152-SV1 polynucleotide sequence), SEQ ID NO: 11 (encoding the extracellular INSP152-SV1 polynucleotide sequence), SEQ ID NO: 13 (encoding the mature INSP152 polynucleotide sequence), SEQ ID NO: 15 (encoding the mature INSP152-SV1 polynucleotide sequence), SEQ ID NO: 17 (encoding the INSP152-SV1 polynucleotide sequence), SEQ ID NO: 19 (encoding the INSP152-D fragment polynucleotide sequence), SEQ ID NO: 21 (encoding the cloned INSP152-D1-SV1 polynucleotide sequence), SEQ ID NO: 23 (encoding the extracellular INSP152 polynucleotide sequence), SEQ ID NO: 25 (encoding the full INSP152 polynucleotide sequence) and/or SEQ ID NO: 27 (encoding the full INSP152-SV1 polynucleotide sequence).

In a third aspect, the disclosure provides a purified nucleic acid molecule which hybridizes under high stringency conditions with a nucleic acid molecule of the second aspect of the invention. High stringency hybridisation conditions are defined as overnight incubation at 42°C in a solution comprising 50% formamide, 5XSSC (150mum NaCl, 15mM trisodium citrate), 50mM sodium phosphate (pH7.6), 5x Denhardts solution, 10% dextran sulphate, and 20 microgram/ml denatured, sheared salmon sperm DNA, followed by washing the filters in 0.1X SSC at approximately 65°C.

In a fourth aspect, the invention provides a vector, such as an expression vector, that contains a nucleic acid molecule of the second or third aspect of the invention.

In a fifth aspect, the invention provides an isolated host cell transformed with a vector of the fourth aspect of the invention.

In a sixth aspect, the invention provides a ligand which binds specifically to protein members of the MAM domain containing protein family of the first aspect of the invention, which is an antibody. Preferably, the ligand inhibits the function of a polypeptide of the first aspect of the invention which is a MAM domain containing protein.

Importantly, the identification of the function of the INSP152 polypeptides allows for the design of screening methods capable of identifying compounds that are effective in the treatment and/or diagnosis of disease. Ligands according to the sixth aspects of the invention may be identified using such methods. These methods are included as aspects of the present invention.

Another aspect of this invention resides in the use of an INSP152 gene or polypeptide as a target for the screening of candidate drug modulators.

A further aspect of this invention resides in methods of screening of compounds for therapy, comprising determining the ability of a compound to bind to an INSP 152 gene or polypeptide.

A further aspect of this invention resides in methods of screening of compounds for therapy, comprising testing for modulation of the activity of an INSP152 gene or polypeptide.

In a seventh aspect, the invention provides a polypeptide of the first aspect of the invention, or a nucleic acid molecule of the second or third aspect of the invention, or a vector of the fourth aspect of the invention, or a host cell of the fifth aspect of the invention, or a ligand of the sixth aspect of the invention, for use in therapy or diagnosis of disease. Such diseases may include inflammatory bowel disease, arthritis, psoriasis, organ transplant rejection, and Crohn's disease. These molecules may also be used in the manufacture of a medicament for the treatment of such diseases.

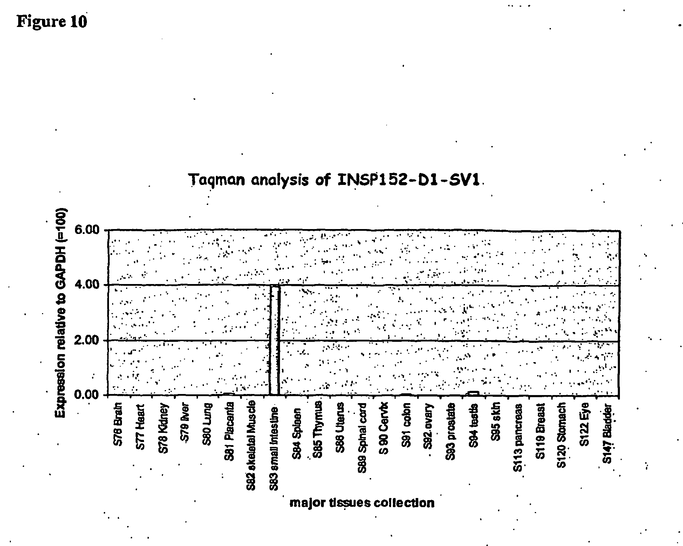

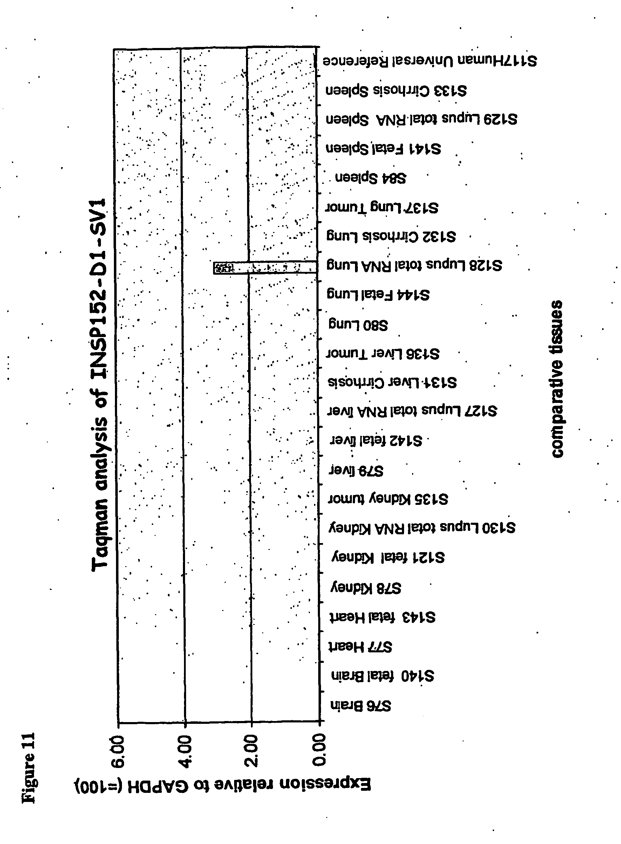

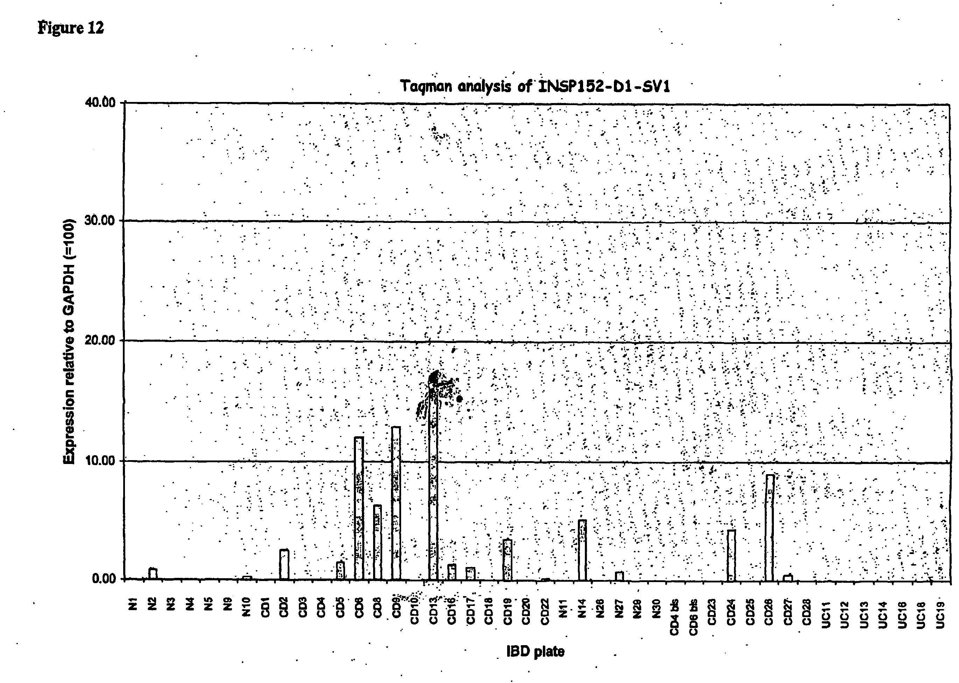

Most particularly, diseases in which MAM domain containing proteins are implicated are selected from, Crohn's disease, inflammatory bowel disease, lupus. As described herein, results of a Taqman analysis show unexpected restricted expression of INSP152 in small intestine (see Table 5 and Figure 10), in a lupus sample (see Table 6 and Figure 11), and in 12/22 Crohn's disease biopsy samples (see Table 7 and Figure 12). This specific pattern of expression leads to the conclusion of the involvement of INSP152, particularly INSP152-D1-SV1, in diseases of the small intestine, including Crohn's disease, inflammatory bowel disease, lupus. These surprising properties characterizing the polynucleotides or the corresponding polypeptides of the present invention make them particularly suitable for the preparation of a drug or pharmaceutical composition. The polynucleotides or the corresponding polypeptides of the present invention therefore display the unexpected finding of a restricted expression in specific tissues.

Additional results (see Figure 13) surprisingly show that the NSP152-D1-SV1 polypeptide is a potent inhibitor of lymphocytes, preferably of T cells. As such, INSP 152-D1-SV1 displays similar functional activity to CTLA4-Ig. We thus infer that polypeptides according to the present invention, particularly INSP152-D1-SV1, are useful for the treatment of CTLA4-Ig related diseases. Such polypeptides are useful on their own, as a component of fusion proteins such as Fc fusion, and/or in combination with another agent.

The polypeptides of the present invention, particularly INSP152-D1-SV1, are also useful in graft versus host disease or in blocking of allograft and xenograft rejection.

In an eighth aspect, the invention provides a method of diagnosing a disease in a patient, comprising assessing the level of expression of a natural gene encoding a polypeptide of the first aspect of the invention or the activity of a polypeptide of the first aspect of the invention in tissue from said patient and comparing said level of expression or activity to a control level, wherein a level that is different to said control level is indicative of disease.

Such a diagnostic method will preferably be carried out *in vitro.* Similar methods may be used for monitoring the therapeutic treatment of disease in a patient, wherein altering the level of expression or activity of a polypeptide or nucleic acid molecule over the period of time towards a control level is indicative of regression of disease.

A preferred method for detecting polypeptides of the first aspect of the invention comprises the steps of: (a) contacting a ligand, such as an antibody, of the sixth aspect of the invention with a biological sample under conditions suitable for the formation of a ligand-polypeptide complex; and (b) detecting said complex.

A number of different such methods according to the eighth aspect of the invention exist, as the skilled reader will be aware, such as methods of nucleic acid hybridization with short probes, point mutation analysis, polymerase chain reaction (PCR) amplification and methods using antibodies to detect aberrant protein levels. Similar methods may be used on a short or long term basis to allow therapeutic treatment of a disease to be monitored in a patient. The invention also provides kits that are useful in these methods for diagnosing disease.

In a ninth aspect, the invention provides for the use of a polypeptide of the first aspect of the invention as a MAM domain containing protein.

In a tenth aspect, the invention provides a pharmaceutical composition comprising a polypeptide of the first aspect of the invention, or a nucleic acid molecule of the second or third aspect of the invention, or a vector of the fourth aspect of the invention, or a host cell of the fifth aspect of the invention, or a ligand of the sixth aspect of the invention, in conjunction with a pharmaceutically-acceptable carrier.

In a eleventh aspect, the present invention provides a polypeptide of the first aspect of the invention, or a nucleic acid molecule of the second or third aspect of the invention, or a vector of the fourth aspect of the invention, or a host cell of the fifth aspect of the invention, or a ligand of the sixth aspect of the invention, for use in the manufacture of a medicament for the diagnosis or treatment of a disease, including, inflammatory bowel disease, arthritis, psoriasis, organ transplant rejection; and Crohn's disease.

In a twelth aspect, the invention provides a method of treating a disease in a patient comprising administering to the patient a polypeptide of the first aspect of the invention, or a nucleic acid molecule of the second or third aspect of the invention, or a vector of the fourth aspect of the invention, or a host cell of the fifth aspect of the invention, or a ligand of the sixth aspect of the invention.

For diseases in which the expression of a natural gene encoding a polypeptide of the first aspect of the invention, or in which the activity of a polypeptide of the first aspect of the invention, is lower in a diseased patient when compared to the level of expression or activity in a healthy patient, the polypeptide, nucleic acid molecule, ligand or compound administered to the patient should be an agonist. Conversely, for diseases in which the expression of the natural gene or activity of the polypeptide is higher in a diseased patient when compared to the level of expression or activity in a healthy patient, the polypeptide, nucleic acid molecule, or ligand administered to the patient should be an antagonist. Examples of such antagonists include antisense nucleic acid molecules, ribozymes and ligands, such as antibodies.

The INSP152 polypeptides are MAM domain containing proteins and thus have roles in many disease states. Antagonists of the INSP152 polypeptides are of particular interest as the provide a way of modulating these disease states.

In a thirteenth aspect, the invention provides transgenic or knockout non-human animals that have been transformed to express higher, lower or absent levels of a polypeptide of the first aspect of the invention. Such transgenic animals are very useful models for the study of disease and may also be used in screening regimes for the identification of compounds that are effective in the treatment or diagnosis of such a disease.

As used herein, "functional equivalent" refers to a protein or nucleic acid molecule that possesses functional or structural characteristics that are substantially similar to a polypeptide or nucleic acid molecule of the present invention. A functional equivalent of a protein may contain modifications depending on the necessity of such modifications for the performance of a specific function. The term "functional equivalent" is intended to include the fragments, mutants, hybrids, variants, analogs, or chemical derivatives of a molecule.

Preferably, the "functional equivalent" may be a protein or nucleic acid molecule that exhibits any one or more of the functional activities of the polypeptides of the present invention.

Preferably, the "functional equivalent" may be a protein or nucleic acid molecule that displays substantially similar activity compared with INSP152 or fragments thereof in a suitable assay for the measurement of biological activity or function. Preferably, the "functional equivalent" may be a protein or nucleic acid molecule that displays identical or higher activity compared with INSP152 or fragments thereof in a suitable assay for the measurement of biological activity or function. Preferably, the "functional equivalent" may be a protein or nucleic acid molecule that displays 50%, 60%, 70%, 80%, 90%, 95%, 98%, 99%, 100% or more activity compared with INSP152 or fragments thereof in a suitable assay for the measurement of biological activity or function.

Preferably, the "functional equivalent" may be a protein or polypeptide capable of exhibiting a substantially similar *in* vivo or *in vitro* activity as the polypeptides of the invention. Preferably, the "functional equivalent" may be a protein or polypeptide capable of interacting with other cellular or extracellular molecules in a manner substantially similar to the way in which the corresponding portion of the polypeptides of the Invention would. For example, a "functional equivalent" would be able, in an immunoassay, to diminish the binding of an antibody to the corresponding peptide (*i*.*e*., the peptide the amino acid sequence of which was modified to achieve the "functional equivalent") of the polypeptide of the invention, or to the polypeptide of the invention itself, where the antibody was raised against the corresponding peptide of the polypeptide of the invention. An equimolar concentration of the functional equivalent will diminish the aforesaid binding of the corresponding peptide by at least about 5%, preferably between about 5% and 10%, more preferably between about 10% and 25%, even more preferably between about 25% and 50%, and most preferably between about 40% and 50%.

For example, functional equivalents can be fully functional or can lack function in one or more activities. Thus, in the present invention, variations can affect the function, for example, of the activities of the polypeptide that reflect its possession of a MAM domain.

A summary of standard techniques and procedures which may be employed in order to utilise the invention is given below. It will be understood that this invention is not limited to the particular methodology, protocols, cell lines, vectors and reagents described. It is also to be understood that the terminology used herein is for the purpose of describing particular embodiments only and it is not intended that this terminology should limit the scope of the present invention. The extent of the invention is limited only by the terms of the appended claims.

Standard abbreviations for nucleotides and amino acids are used in this specification.

The practice of the present invention will employ, unless otherwise indicated, conventional techniques of molecular biology, microbiology, recombinant DNA technology and immunology, which are within the skill of those working in the art.

Such techniques are explained fully in the literature. Examples of particularly suitable texts for consultation include the following: Sambrook Molecular Cloning; A Laboratory Manual, Second Edition (1989); DNA Cloning, Volumes I and II (D.N Glover ed. 1985); Oligonucleotide Synthesis (M.J. Gait ed. 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Immunochemical Methods in Cell and Molecular Biology (Mayor and Walker, eds. 1987, Academic Press, London); Scopes, (1987) Protein Purification: Principles and Practice, Second Edition (Springer Verlag, N.Y.); and Handbook of Experimental Immunology, Volumes I-IV (D.M. Weir and C. C. Blackwell eds. 1986), As used herein, the term "polypeptide" includes any peptide or protein comprising two or more amino acids joined to each other by peptide bonds or modified peptide bonds, *i*.*e*. peptide isosteres. This term refers both to short chains (peptides and oligopeptides) and to longer chains (proteins).

The polypeptide of the present invention may be in the form of a mature protein or may be a pre-, pro- or prepro- protein that can be activated by cleavage of the pre-, pro- or preproportion to produce an active mature polypeptide. In such polypeptides, the pre-, pro- or prepro- sequence may be a leader or secretory sequence or may be a sequence that is employed for purification of the mature polypeptide sequence.

The polypeptide of the first aspect of the invention may form part of a fusion protein. For example, it is often advantageous to include one or more additional amino acid sequences which may contain secretory or leader sequences, pro-sequences, sequences which aid in purification, or sequences that confer higher protein stability, for example during recombinant production. Alternatively or additionally, the mature polypeptide may be fused with another compound, such as a compound to increase the half-life of the polypeptide (for example, polyethylene glycol).

These fusion proteins can be obtained by cloning a polynucleotide encoding a polypeptide in frame to the coding sequences for a heterologous protein sequence. A preferred fusion protein according to the invention comprises INSP152-D1-SV1. INSP152-D1-SV1 is useful on its own, as a component of fusion proteins such as Fc fusion, and/or in combination with another agent.

The term "heterologous", when used herein, is intended to designate any polypeptide other than a human INSP152 polypeptide.

Examples of heterologous sequences, that can be comprised in the soluble fusion proteins either at N- or at C-terminus, are the following: extracellular domains of membrane-bound protein, immunoglobulin constant regions (Fc region), multimerization domains, domains of extracellular proteins, signal sequences, export sequences, or sequences allowing purification by affinity chromatography.

Many of these heterologous sequences are commercially available in expression plasmids since these sequences are commonly included in the fusion proteins in order to provide additional properties without significantly impairing the specific biological activity of the protein fused to them (Terpe K, 2003. Appl Microbiol Biotechnol, 60: 523-33). Examples of such additional properties are a longer lasting half-life in body fluids, the extracellular localization, or an easier purification procedure as allowed by the a stretch of Histidines forming the so-called "histidine tag" (Gentz et al., 1989 Proc Natl Acad Sci USA, 86: 821-4) or by the "HA" tag, an epitope derived from the influenza hemagglutinin protein (Wilson et al., 1994 Cell, 37: 767-78). If needed, the heterologous sequence can be eliminated by a proteolytic cleavage, for example by inserting a proteolytic cleavage site between the protein and the heterologous sequence, and exposing the purified fusion protein to the appropriate protease. These features are of particular importance for the fusion proteins since they facilitate their production and use in the preparation of pharmaceutical compositions. When the fusion protein comprises an immunoglobulin region, the fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gln-Phe-Met (SEQ ID NO: 29) introduced between the sequence of the substances of the invention and the immunoglobulin sequence. The resulting fusion protein has improved properties, such as an extended residence time in body fluids (half-life), increased specific activity, increased expression level, or the purification of the fusion protein is facilitated.

In a preferred embodiment, the protein is fused to the constant region of an Ig molecule. Preferably, it is fused to heavy chain regions, like the CH2 and CH3 domains of human IgG1, for example. Other isoforms of Ig molecules are also suitable for the generation of fusion proteins according to the present invention, such as isoforms IgG2 or IgG4, or other Ig classes, like IgM or IgA, for example. Moieties that possess ADCC activity are typically of the IgG1 or IgG3 isotype. Fusion proteins may be monomeric or multimeric, hetero- or homomultimeric.

In a further preferred embodiment, the functional derivative comprises at least one moiety attached to one or more functional groups, which occur as one or more side chains on the amino acid residues. Preferably, the moiety is a polyethylene (PEG) moiety. PEGylation may be carried out by known methods, such as the ones described in WO99/55377, for example.

Polypeptides may contain amino acids other than the 20 gene-encoded amino acids, modified either by natural processes, such as by post-translational processing or by chemical modification techniques which are well known in the art. Among the known modifications which may commonly be present in polypeptides of the present invention are glycosylation, lipid attachment, sulphation, gamma-carboxylation, for instance of glutamic acid residues, hydroxylation and ADP-ribosylation. Other potential modifications include acetylation, acylation, amidation, covalent attachment of flavin, covalent attachment of a haeme moiety, covalent attachment of a nucleotide or nucleotide derivative, covalent attachment of a lipid derivative, covalent attachment of phosphatidylinositol, cross-linking, cyclization, disulphide bond formation, demethylation, formation of covalent cross-links, formation of cysteine, formation of pyroglutamate, formylation, GPI anchor formation, iodination, methylation, myristoylation, oxidation, proteolytic processing, phosphorylation, prenylation, racemization, selenoylation, transfer-RNA mediated addition of amino acids to proteins such as arginylation, and ubiquitination.

Modifications can occur anywhere in a polypeptide, including the peptide backbone, the amino acid side-chains and the amino or carboxyl termini. In fact, blockage of the amino or carboxyl terminus in a polypeptide, or both, by a covalent modification is common in naturally-occurring and synthetic polypeptides and such modifications may be present in polypeptides of the present invention.

The modifications that occur in a polypeptide often will be a function of how the polypeptide is made. For polypeptides that are made recombinantly, the nature and extent of the modifications in large part will be determined by the post-translational modification capacity of the particular host cell and the modification signals that are present in the amino acid sequence of the polypeptide in question. For instance, glycosylation patterns vary between different types of host cell.

The polypeptides of the present invention can be prepared in any suitable manner. Such polypeptides include isolated naturally-occurring polypeptides (for example purified from cell culture), recombinantly-produced polypeptides (including fusion proteins), synthetically-produced polypeptides or polypeptides that are produced by a combination of these methods.

Nucleic acids according to the invention are preferably 10-6500 nucleotides in length, preferably 1000-6300 nucleotides, preferably 3000-6300, preferably 4000-6250, preferably 5000-6250 nucleotides in length. Polypeptides according to the invention are preferably 10-2500 amino acids in length, preferably 100-2400, preferably 1000-2300, preferably 2000-2250 amino acids in length.

The polypeptides of the present invention or their immunogenic fragments (comprising at least one antigenic determinant) can be used to generate ligands, such as polyclonal or monoclonal antibodies, that are immunospecific for the polypeptides. Such antibodies may be employed to isolate or to identify clones expressing the polypeptides of the invention or to purify the polypeptides by affinity chromatography. The antibodies may also be employed as diagnostic or therapeutic aids, amongst other applications, as will be apparent to the skilled reader.

The term "immunospecific" means that the antibodies have substantially greater affinity for the polypeptides of the invention than their affinity for other related polypeptides in the prior art. As used herein, the term "antibody" refers to intact molecules as well as to fragments thereof, such as Fab, F(ab')2 and Fv, which are capable of binding to the antigenic determinant in question. Such antibodies thus bind to the polypeptides of the first aspect of the invention.

By "substantially greater affinity" we mean that there is a measurable increase in the affinity for a polypeptide of the invention as compared with the affinity for known secreted proteins.

Preferably, the affinity is at least 1.5-fold, 2-fold, 5-fold 10-fold, 100-fold, 10³-fold, 10⁴-fold, 10⁵-fold, 10⁶-fold or greater for a polypeptide of the invention than for known secreted proteins such as MAM domain containing proteins.

Preferably, there is a measurable increase in the affinity for a polypeptide of the invention as compared with known MAM domain containing proteins.

If polyclonal antibodies are desired, a selected mammal, such as a mouse, rabbit, goat or horse, may be immunised with a polypeptide of the first aspect of the invention. The polypeptide used to immunise the animal can be derived by recombinant DNA technology or can be synthesized chemically. If desired, the polypeptide can be conjugated to a carrier protein. Commonly used carriers to which the polypeptides may be chemically coupled include bovine serum albumin, thyroglobulin and keyhole limpet haemocyanin. The coupled polypeptide is then used to immunise the animal. Serum from the immunised animal is collected and treated according to known procedures, for example by immunoaffinity chromatography.

Monoclonal antibodies to the polypeptides of the first aspect of the invention can also be readily produced by one skilled in the art. The general methodology for making monoclonal antibodies using hybridoma technology is well known (see, for example, Kohler, G. and Milstein, C., Nature 256: 495-497 (1975); Kozbor et al., Immunology Today 4: 72 (1983); Cole et al., 77-96 in Monoclonal Antibodies and Cancer Therapy, Alan R. Liss, Inc. (1985).

Panels of monoclonal antibodies produced against the polypeptides of the first aspect of the invention can be screened for various properties, i.e., for isotype, epitope, affinity, etc.

Monoclonal antibodies are particularly useful in purification of the individual polypeptides against which they are directed. Alternatively, genes encoding the monoclonal antibodies of interest may be isolated from hybridomas, for instance by PCR techniques known in the art, and cloned and expressed in appropriate vectors.

Chimeric antibodies, in which non-human variable regions are joined or fused to human constant regions (see, for example, Liu et al., Proc. Nat1. Acad. Sci. USA, 84, 3439 (1987)), may also be of use.

The antibody may be modified to make it less immunogenic in an individual, for example by humanisation (see Jones et al., Nature, 321, 522 (1986); Verhoeyen et al., Science, 239, 1534 (1988); Kabat et al., J. Immunol., 147, 1709 (1991); Queen et al., Proc. Natl. Acad. Sci: USA, 86, 10029 (1989); Gorman et al., Proc. Natl Acad. Sci. USA, 88, 34181 (1991); and Hodgson et al., Bio/Technology, 9, 421 (1991)), The term. "humanised antibody", as used herein, refers to antibody molecules in which the CDR amino acids and selected other amino acids in the variable domains of the heavy and/or light chains of a non-human donor antibody have been substituted in place of the equivalent amino acids in a human antibody. The humanised antibody thus closely resembles a human antibody but has the binding ability of the donor antibody.

In a further alternative, the antibody may be a "bispecific" antibody, that is, an antibody having two different antigen binding domains, each domain being directed against a different epitope.

Phage display technology may be utilised to select genes which encode antibodies with binding activities towards the polypeptides of the invention either from repertoires of PCR amplified V-genes of lymphocytes from humans screened for possessing the relevant antibodies, or from naive libraries (McCafferty, J. et al., (1990), Nature 348, 552-554; Marks, J. et al., (1992) Biotechnology 10, 779-783). The affinity of these antibodies-can also be improved by chain shuffling (Clackson, T. et al., (1991) Nature 352, 624-628).

Antibodies generated by the above techniques, whether polyclonal or monoclonal, have additional utility in that they may be employed as reagents in immunoassays, radioimmunoassays (RIA) or enzyme-linked immunosorbent assays (ELISA). In these applications, the antibodies can be labelled with an analytically-detectable reagent such as a radioisotope, a fluorescent molecule or an enzyme.

Preferred nucleic acid molecules of the second and third aspects of the invention are those which encode a polypeptide sequence as recited in SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26 and SEQ ID NO: 28. These nucleic acid molecules may be used in the methods and applications described herein.

The nucleic acid molecules of the invention also include sequences that are complementary to nucleic acid molecules described above (for example, for antisense or probing purposes).

Nucleic acid molecules of the present invention may be in the form of RNA, such as mRNA, or in the form of DNA, including, for instance cDNA, synthetic DNA or genomic DNA. Such nucleic acid molecules may be obtained by cloning, by chemical synthetic techniques or by a combination thereof. The nucleic acid molecules can be prepared, for example, by chemical synthesis using techniques such as solid phase phosphoramidite chemical synthesis, from genomic or cDNA libraries or by separation from an organism. RNA molecules may generally be generated by the *in vitro* or *in vivo* transcription of DNA sequences.

The nucleic acid molecules may be double-stranded or single-stranded. Single-stranded DNA may be the coding strand, also known as the sense strand, or it may be the non-coding strand, also referred to as the anti-sense strand.

The term "nucleic acid molecule" also includes analogues of DNA and RNA, such as those containing modified backbones, and peptide nucleic acids (PNA). The term "PNA", as used herein, refers to an antisense molecule or an anti-gene agent which comprises an oligonucleotide of at least five nucleotides in length linked to a peptide backbone of amino acid residues, which preferably ends in lysine. The terminal lysine confers solubility to the composition. PNAs may be pegylated to extend their lifespan in a cell, where they preferentially bind complementary single stranded DNA and RNA and stop transcript elongation (Nielsen, P.E. et al. (1993) Anticancer Drug Des. 8:53-63).

A nucleic acid molecule which encodes a polypeptide of this invention may be identical to the coding sequence of one or more of the nucleic acid molecules disclosed herein.

These molecules also may have a different sequence which, as a result of the degeneracy of the genetic code, encodes a polypeptide SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26 or SEQ ID NO: 28.
Such nucleic acid molecules may include, but are not limited to, the coding sequence for the mature polypeptide by itself; the coding sequence for the mature polypeptide and additional coding sequences, such as those encoding a leader or secretory sequence, such as a pro-, pre- or prepro- polypeptide sequence; the coding sequence of the mature polypeptide, with or without the aforementioned additional coding sequences, together with further additional, non-coding sequences, including non-coding 5' and 3' sequences, such as the transcribed, non-translated sequences that play a role in transcription (including termination signals), ribosome binding and mRNA stability. The nucleic acid molecules may also include additional sequences which encode additional amino acids, such as those which provide additional functionalities.

The nucleic acid molecules of the invention can also be engineered, using methods generally known in the art, for a variety of reasons, including modifying the cloning, processing, and/or expression of the gene product (the polypeptide). DNA shuffling by random fragmentation and PCR reassembly of gene fragments and synthetic oligonucleotides are included as techniques which may be used to engineer the nucleotide sequences. Site-directed mutagenesis may be used to insert new restriction sites, alter glycosylation patterns, change codon preference, produce splice variants, introduce mutations and so forth.

Nucleic acid molecules which encode a polypeptide of the first aspect of the invention may be ligated to a heterologous sequence so that the combined nucleic acid molecule encodes a fusion protein. Such combined nucleic acid molecules are included within the second or third aspects of the invention. For example, to screen peptide libraries for inhibitors of the activity of the polypeptide, it may be useful to express, using such a combined nucleic acid molecule, a fusion protein that can be recognised by a commercially-available antibody. A fusion protein may also be engineered to contain a cleavage site located between the sequence of the polypeptide of the invention and the sequence of a heterologous protein so that the polypeptide may be cleaved and purified away from the heterologous protein.

The nucleic acid molecules also include antisense molecules that are partially complementary to nucleic acid molecules encoding polypeptides of the present invention and that therefore hybridize to the encoding nucleic acid molecules (hybridization). Such antisense molecules, such as oligonucleotides, can be designed to recognise, specifically bind to and prevent transcription of a target nucleic acid encoding a polypeptide of the invention, as will be known by those of ordinary skill in the art (see, for example, Cohen, J.S., Trends in Pharm. Sci., 10, 435 (1989), Okano, J. Neurochem. 56, 560 (1991); O'Connor, J. Neurochem 56, 560 (1991); Lee et al., Nucleic Acids Res 6,3073 (1979); Cooney et al., Science 241, 456 (1988); Dervan et al., Science 251, 1360 (1991).

The term "hybridization" as used here refers to the association of two nucleic acid molecules with one another by hydrogen bonding. Typically, one molecule will be fixed to a solid support and the other will be free in solution. Then, the two molecules may be placed in contact with one another under conditions that favour hydrogen bonding. Factors that affect this bonding include: the type and volume of solvent; reaction temperature; time of hybridization; agitation; agents to block the non-specific attachment of the liquid phase molecule to the solid support (Denhardt's reagent or BLOTTO); the concentration of the molecules; use of compounds to increase the rate of association of molecules (dextran sulphate or polyethylene glycol); and the stringency of the washing conditions following hybridization (see Sambrook *et al*. [*supra*]).

The inhibition of hybridization of a completely complementary molecule to a target molecule may be examined using a hybridization assay, as known in the art (see, for example, Sambrook *et al*. [*supra*]). A substantially homologous molecule will then compete for and inhibit the binding of a completely homologous molecule to the target molecule under various conditions of stringency, as taught in Wahl, G.M. and S.L. Berger (1987; Methods Enzymol. 152:399-407) and Kimmel, A.R. (1987; Methods Enzymol. 152:507-511).

"Stringency" refers to conditions in a hybridization reaction that favour the association of very similar molecules over association of molecules that differ. High stringency hybridisation conditions are defined as overnight incubation at 42°C in a solution comprising 50% formamide, 5XSSC (150mM NaCl, 15mM trisodium citrate), 50mM sodium phosphate (pH7.6), 5x Denhardts solution, 10% dextran sulphate, and 20 microgram/ml denatured, sheared salmon sperm DNA, followed by washing the filters in 0.1X SSC at approximately 65°C. Low stringency conditions involve the hybridisation reaction being carried out at 35°C (see Sambrook *et al*. [*supra*]). Preferably, the conditions used for hybridization are those of high stringency.

The invention also provides a process for detecting a nucleic acid molecule of the invention, comprising the steps of: (a) contacting a nucleic probe according to the invention with a biological sample under hybridizing conditions to form duplexes; and (b) detecting any such duplexes that are formed.

As discussed additionally below in connection with assays that may be utilised according to the invention, a nucleic acid molecule as described above may be used as a hybridization probe for RNA, cDNA or genomic DNA, in order to isolate full-length cDNAs and genomic clones encoding the INSP152 polypeptides and to isolate cDNA and genomic clones of homologous or orthologous genes that have a high sequence similarity to the gene encoding this polypeptide.

In this regard, the following techniques, among others known in the art, may be utilised and are discussed below for purposes of illustration. Methods for DNA sequencing and analysis are well known and are generally available in the art and may, indeed, be used to practice many of the embodiments of the invention discussed herein. Such methods may employ such enzymes as the Klenow fragment of DNA polymerase I, Sequenase (US Biochemical Corp; Cleveland, OH), Taq polymerase (Perkin Elmer), thermostable T7 polymerase (Amersham, Chicago, IL), or combinations of polymerases and proof-reading exonucleases such as those found in the ELONGASE Amplification System marketed by Gibco/BRL (Gaithersburg, MD). Preferably, the sequencing process may be automated using machines such as the Hamilton Micro Lab 2200 (Hamilton, Reno, NV), the Peltier Thermal Cycler (PTC200; MJ Research, Watertown, MA) and the ABI Catalyst and 373 and 377 DNA Sequencers (Perkin Elmer).

One method for isolating a nucleic acid molecule encoding a polypeptide with an equivalent function to that of the INSP152 polypeptide is to probe a genomic or cDNA library with a natural or artificially-designed probe using standard procedures that are recognised in the art (see, for example, "Current Protocols in Molecular Biology", Ausubel et al. (eds). Greene Publishing Association and John Wiley Interscience, New York, 1989,1992). Probes comprising at least 15, preferably at least 30, and more preferably at least 50, contiguous bases that correspond to, or are complementary to, nucleic acid sequences from the appropriate encoding gene (SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25 and SEQ ID NO: 27), are particularly useful probes. Such probes may be labelled with an analytically detectable reagent to facilitate their identification. Useful reagents include, but are not limited to, radioisotopes, fluorescent dyes and enzymes that are capable of catalysing the formation of a detectable product. Using these probes, the ordinarily skilled artisan will be capable of isolating complementary copies of genomic DNA, cDNA or RNA polynucleotides encoding proteins of interest from human, mammalian or other animal sources and screening such sources for related sequences, for example, for additional members of the family, type and/or subtype.

In many cases, isolated cDNA sequences will be incomplete, in that the region encoding the polypeptide will be cut short, normally at the 5' end. Several methods are available to obtain full length cDNAs, or to extend short cDNAs. Such sequences may be extended utilising a partial nucleotide sequence and employing various methods known in the art to detect upstream sequences such as promoters and regulatory elements. For example, one method which may be employed is based on the method of Rapid Amplification of cDNA Ends (RACE; see, for example, Frohman et al., PNAS USA 85, 8998-9002, 1988). Recent modifications of this technique, exemplified by the Marathon^{™} technology (Clontech Laboratories Inc.), for example, have significantly simplified the search for longer cDNAs.
A slightly different technique, termed "restriction-site" PCR, uses universal primers to retrieve unknown nucleic acid sequence adjacent a known locus (Sarkar, G. (1993) PCR Methods Applic. 2:318-322). Inverse PCR may also be used to amplify or to extend sequences using divergent primers based on a known region (Triglia, T. et al. (1988) Nucleic Acids Res. 16:8186). Another method which may be used is capture PCR which involves PCR amplification of DNA fragments adjacent a known sequence in human and yeast artificial chromosome DNA (Lagerstrom, M. et al. (1991) PCR Methods Applic., 1, 111-119). Another method which may be used to retrieve unknown sequences is that of Parker, J.D. et al. (1991); Nucleic Acids Res. 19:3055-3060). Additionally, one may use PCR, nested primers, and PromoterFinder^{™} libraries to walk genomic DNA (Clontech, Palo Alto, CA). This process avoids the need to screen libraries and is useful in finding intron/exon junctions.

When screening for full-length cDNAs, it is preferable to use libraries that have been size-selected to include larger cDNAs. Also, random-primed libraries are preferable, in that they will contain more sequences that contain the 5' regions of genes. Use of a randomly primed library may be especially preferable for situations in which an oligo d(T) library does not yield a full-length cDNA. Genomic libraries may be useful for extension of sequence into 5' non-transcribed regulatory regions.

The nucleic acid molecules of the present invention may be used for chromosome localisation. In this technique, a nucleic acid molecule is specifically targeted to, and can hybridize with, a particular location on an individual human chromosome. The mapping of relevant sequences to chromosomes according to the present invention is an important step in the confirmatory correlation of those sequences with the gene-associated disease. Once a sequence has been mapped to a precise chromosomal location, the physical position of the sequence on the chromosomes can be correlated with genetic map data. Such data are found in, for example, V. McKusick, Mendelian Inheritance in Man (available on-line through Johns Hopkins University Welch Medical Library). The relationships between genes and diseases that have been mapped to the same chromosomal region are then identified through linkage analysis (coinheritance of physically adjacent genes). This provides valuable information to investigators searching for disease genes using positional cloning or other gene discovery techniques.

Once the disease or syndrome has been crudely localised by genetic linkage to a particular genomic region, any sequences mapping to that area may represent associated or regulatory genes for further investigation. The nucleic acid molecule may also be used to detect differences in the chromosomal location due to translocation, inversion, etc. among normal, carrier, or affected individuals.

The nucleic acid molecules of the present invention are also valuable for tissue localisation. Such techniques allow the determination of Expression patterns of the polypeptide, in tissues by detection of the mRNAs that encode them. These techniques include *in situ* hybridization techniques and nucleotide amplification techniques, such as PCR. Results from these studies provide an indication of the normal functions of the polypeptide in the organism. In addition, comparative studies of the normal expression pattern of mRNAs with that of mRNAs encoded by as mutant gene provide valuable insights into the role of mutant polypeptides in disease. Such inappropriate expression may be of a temporal, spatial or quantitative nature.

Gene silencing approaches may also be undertaken to down-regulate endogenous expression of a gene encoding a polypeptide of the invention. RNA interference (RNAi) (Elbashir, SM et al., Nature 2001, 411, 494-498) is one method of sequence specific post-transcriptional gene silencing that may be employed. Short dsRNA oligonucleotides are synthesised *in vitro* and introduced into a cell. The sequence specific binding of these dsRNA oligonucleotides triggers the degradation of target mRNA, reducing or ablating target protein expression.

Efficacy of the gene silencing approaches assessed above may be assessed through the measurement of polypeptide expression (for example, by Western blotting), and at the RNA level using TaqMan-based methodologies.

The vectors of the present invention comprise nucleic acid molecules of the invention and may be cloning or expression vectors. The host cells of the invention, which may be transformed, transfected or transduced with the vectors of the invention may be prokaryotic or eukaryotic.

The polypeptides of the invention may be prepared in recombinant form by expression of their encoding nucleic acid molecules in vectors contained within a host cell. Such expression methods are well known to those of skill in the art and many are described in detail by Sambrook *et al*. (*supra*) and Fernandez & Hoeffler (1998, eds. "Gene expression systems. Using nature for the art of expression". Academic Press, San Diego, London, Boston, New York, Sydney, Tokyo, Toronto).

Generally, any system or vector that is suitable to maintain, propagate or express nucleic acid molecules to produce a polypeptide in the required host may be used. The appropriate nucleotide sequence may be inserted into an expression system by any of a variety of well-known and routine techniques, such as, for example, those described in Sambrook *et al*., (*supra*). Generally, the encoding gene can be placed under the control of a control element such as a promoter, ribosome binding site (for bacterial expression) and, optionally, an operator, so that the DNA sequence encoding the desired polypeptide is transcribed into RNA in the transformed host cell.

Examples of suitable expression systems include, for example, chromosomal, episomal and virus-derived systems, including, for example, vectors derived from: bacterial plasmids, bacteriophage, transposons, yeast episomes, insertion elements, yeast chromosomal elements, viruses such as baculoviruses, papova viruses such as SV40, vaccinia viruses, adenoviruses, fowl pox viruses, pseudorabies viruses and retroviruses, or combinations thereof, such as those derived from plasmid and bacteriophage genetic elements, including cosmids and phagemids. Human artificial chromosomes (HACs) may also be employed to deliver larger fragments of DNA than can be contained and expressed in a plasmid. The TOPO vector and pEAK12d-PAC_INSP152-D1-SV1-6HIS-V1 vector are preferred examples of a suitable vectors for use in accordance with the aspects of this invention relating to INSP152.

Particularly suitable expression systems include microorganisms such as bacteria transformed with recombinant bacteriophage, plasmid or cosmid DNA expression vectors; yeast transformed with yeast expression vectors; insect cell systems infected with virus expression vectors (for example, baculovirus); plant cell systems transformed with virus expression vectors (for example, cauliflower mosaic virus, CaMV; tobacco mosaic virus, TMV) or with bacterial expression vectors (for example, Ti or pBR322 plasmids); or animal cell systems. Cell-free translation systems can also be employed to produce the polypeptides of the invention.

Introduction of nucleic acid molecules encoding a polypeptide of the present invention into host cells can be effected by methods described in many standard laboratory manuals, such as Davis *et al*., Basic Methods in Molecular Biology (1986) and Sambrook *et al*., (*supra*). Particularly suitable methods include calcium phosphate transfection, DEAE-dextran mediated transfection, transfection, microinjection, cationic lipid-mediated transfection, electroporation, transduction, scrape loading, ballistic introduction or infection (see Sambrook *et al*., 1989 [*supra*]; Ausubel *et al*., 1991 [*supra*]; Spector, Goldman & Leinwald, 1998). In eukaryotic cells, expression systems may either be transient (for example, episomal) or permanent (chromosomal integration) according to the needs of the system.

The encoding nucleic acid molecule may or may not include a sequence encoding a control sequence, such as a signal peptide or leader sequence, as desired, for example, for secretion of the translated polypeptide into the lumen of the endoplasmic reticulum, into the periplasmic space or into the extracellular environment. These signals may be endogenous to the polypeptide, or they may be heterologous signals. Leader sequences can be removed by the bacterial host in post-translational processing.

In addition to control sequences, it may be desirable to add regulatory sequences that allow for regulation of the expression of the polypeptide relative to the growth of the host cell. Examples of regulatory sequences are those which cause the expression of a gene to be increased or decreased in response to a chemical or physical stimulus, including the presence of a regulatory compound or to various temperature or metabolic conditions. Regulatory sequences are those non-translated regions of the vector, such as enhancers, promoters and 5' and 3' untranslated regions. These interact with host cellular proteins to carry out transcription and translation. Such regulatory sequence may vary in their strength and specificity. Depending on the vector system and host utilised, any number of suitable transcription and translation elements, including constitutive and inducible promoters, may be used. For example, when cloning in bacterial systems, inducible promoters such as the hybrid lacZ promoter of the Bluescript phagemid (Stratagene, LaJolla, CA) or pSportl™ plasmid (Gibco BRL) and the like may be used. The baculovirus polyhedrin promoter may be used in insect cells. Promoters or enhancers derived from the genomes of plant cells (for example, heat shock, RUBISCO and storage protein genes) or from plant viruses (for example, viral promoters or leader sequences) may be cloned into the vector. In mammalian cell systems, promoters from mammalian genes or from mammalian viruses are preferable. If it is necessary to generate a cell line that contains multiple copies of the sequence, vectors based on SV40 or EBV may be used with an appropriate selectable marker.

An expression vector is constructed so that the particular nucleic acid coding sequence is located in the vector with the appropriate regulatory sequences, the positioning and orientation of the coding sequence with respect to the regulatory sequences being such that the coding sequence is transcribed under the "control" of the regulatory sequences, *i*.*e*., RNA polymerase which binds to the DNA molecule at the control sequences transcribes the coding sequence. In some cases it may be necessary to modify the sequence so that it may be attached to the control sequences with the appropriate orientation; i.e., to maintain the reading frame.

The control sequences and other regulatory sequences may be ligated to the nucleic acid coding sequence prior to insertion into a vector. Alternatively, the coding sequence can be cloned directly into an expression vector that already contains the control sequences and an appropriate restriction site.

For long-term, high-yield production of a recombinant polypeptide, stable expression is preferred. For example, cell lines which stably express the polypeptide of interest may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells that successfully express the introduced sequences. Resistant clones of stably transformed cells may be proliferated using tissue culture techniques appropriate to the cell type.

Mammalian cell lines available as hosts for expression are known in the art and include many immortalised cell lines available from the American Type Culture Collection (ATCC) including, but not limited to, Chinese hamster ovary (CHO), HeLa, baby hamster kidney (BHK), monkey kidney (COS), C127, 3T3, BHK, HEK 293, Bowes melanoma and human hepatocellular carcinoma (for example Hep G2) cells and a number of other cell lines.

In the baculovirus system, the materials for baculovirus/insect cell expression systems are commercially available in kit form from, *inter alia*, Invitrogen, San Diego CA (the "MaxBac" kit). These techniques are generally known to those skilled in the art and are described fully in Summers and Smith, Texas Agricultural Experiment Station Bulletin No. 1555 (1987). Particularly suitable host cells for use in this system include insect cells such as Drosophila S2 and Spodoptera Sf9 cells.

There are many plant cell culture and whole plant genetic expression systems known in the art. Examples of suitable plant cellular genetic expression systems include those described in US 5,693,506; US 5,659,122; and US 5,608,143. Additional examples of genetic expression in plant cell culture has been described by Zenk, Phytochemistry 30, 3861-3863 (1991).

In particular, all plants from which protoplasts can be isolated and cultured to give whole regenerated plants can be utilised, so that whole plants are recovered which contain the transferred gene. Practically all plants can be regenerated from cultured cells or tissues, including but not limited to all major species of sugar cane, sugar beet, cotton, fruit and other trees, legumes and vegetables.

Examples of particularly preferred bacterial host cells include *streptococci, staphylococci, E. coli, Streptomyces* and *Bacillus subtilis* cells.

Examples of particularly suitable host cells for fungal expression include yeast cells (for example, *S. cerevisiae*) and *Aspergillus* cells.

Any number of selection systems are known in the art that may be used to recover transformed cell lines. Examples include the herpes simplex virus thymidine kinase (Wigler, M. et al. (1977) Cell 11:223-32) and adenine phosphoribosyltransferase (Lowy, I. et al. (1980) Cell 22:817-23) genes that can be employed in tk⁻ or aprt^{±} cells, respectively.

Also, antimetabolite, antibiotic or herbicide resistance can be used as the basis for selection; for example, dihydrofolate reductase (DHFR) that confers resistance to methotrexate (Wigler, M. et al. (1980) Proc. Natl. Acad. Sci. 77:3567-70); npt, which confers resistance to the aminoglycosides neomycin and G-418 (Colbere-Garapin, F. et al. (1981) J. Mol. Biol. 150:1-14) and als or pat, which confer resistance to chlorsulfuron and phosphinotricin acetyltransferase, respectively. Additional selectable genes have been described, examples of which will be clear to those of skill in the art.

Although the presence or absence of marker gene expression suggests that the gene of interest is also present, its presence and expression may need to be confirmed. For example, if the relevant sequence is inserted within a marker gene sequence, transformed cells containing the appropriate sequences can be identified by the absence of marker gene function. Alternatively, a marker gene can be placed in tandem with a sequence encoding a polypeptide of the invention under the control of a single promoter. Expression of the marker gene in response to induction or selection usually indicates expression of the tandem gene as well.

Alternatively, host cells that contain a nucleic acid sequence encoding a polypeptide of the invention and which express said polypeptide may be identified by a variety of procedures known to those of skill in the art. These procedures include, but are not limited to, DNA-DNA or DNA-RNA hybridizations and protein bioassays, for example, fluorescence activated cell sorting (FACS) or immunoassay techniques (such as the enzyme-linked immunosorbent assay [ELISA] and radioimmunoassay [RIA]), that include membrane, solution, or chip based technologies for the detection and/or quantification of nucleic acid or protein (see Hampton, R. et al. (1990) Serological Methods, a Laboratory Manual, APS Press, St Paul, MN) and Maddox, D.E. et al. (1983) J. Exp. Med, 158, 1211-1216).

A wide variety of labels and conjugation techniques are known by those skilled in the art and may be used in various nucleic acid and amino acid assays. Means for producing labelled hybridization or PCR probes for detecting sequences related to nucleic acid molecules encoding polypeptides of the present invention include oligolabelling, nick translation, end-labelling or PCR amplification using a labelled polynucleotide. Alternatively, the sequences encoding the polypeptide of the invention may be cloned into a vector for the production of an RNA probe. Such vectors are known in the art, are commercially available, and may be used to synthesised RNA probes *in vitro* by addition of an appropriate RNA polymerase such as T7, T3 or SP6 and labelled nucleotides. These procedures may be conducted using a variety of commercially available kits (Pharmacia & Upjohn, (Kalamazoo, MI); Promega (Madison WI); and U.S. Biochemical Corp. (Cleveland, OH)).

Suitable reporter molecules or labels, which may be used for ease of detection, include radionucleides, enzymes and fluorescent, chemiluminescent or chromogenic agents as well as substrates, cofactors, inhibitors, magnetic particles, and the like.

Nucleic acid molecules according to the present invention may also be used to create transgenic animals, particularly rodent animals. Such transgenic, animals form a further aspect of the present invention. This may be done locally by modification of somatic cells, or by germ line therapy to incorporate heritable modifications. Such transgenic animals may be particularly useful in the generation of animal models for drug molecules effective as modulators of the polypeptides of the present invention.

The polypeptide can be recovered and purified from recombinant cell cultures by well-known methods including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. High performance liquid chromatography is particularly useful for purification. Well known techniques for refolding proteins may be employed to regenerate an active conformation when the polypeptide is denatured during isolation and or purification.

Specialised vector constructions may also be used to facilitate purification of proteins, as desired, by joining sequences encoding the polypeptides of the invention to a nucleotide sequence encoding a polypeptide domain that will facilitate purification of soluble proteins. Examples of such purification-facilitating domains include metal chelating peptides such as histidine-tryptophan modules that allow purification on immobilised metals, protein A domains that allow purification on immobilised immunoglobulin, and the domain utilised in the FLAGS extension/affinity purification system (Immunex Corp., Seattle, WA). The inclusion of cleavable linker sequences such as those specific for Factor XA or enterokinase (Invitrogen, San Diego, CA) between the purification domain and the polypeptide of the invention may be used to facilitate purification. One such expression vector provides for expression of a fusion protein containing the polypeptide of the invention fused to several histidine residues preceding a thioredoxin or an enterokinase cleavage site. The histidine residues facilitate purification by IMAC (immobilised metal ion affinity chromatography as described in Porath, J. et al. (1992), Prot. Exp. Purif. 3: 263-281) while the thioredoxin or enterokinase cleavage site provides a means for purifying the polypeptide from the fusion protein. A discussion of vectors which contain fusion proteins is provided in Kroll, D.J. et al. (1993; DNA Cell Biol. 12:441-453).

If the polypeptide is to be expressed for use in screening assays, generally it is preferred that it be produced at the surface of the host cell in which it is expressed. In this event, the host cells may be harvested prior to use in the screening assay, for example using techniques such as fluorescence activated cell sorting (FACS) or immunoaffinity techniques. If the polypeptide is secreted into the medium, the medium can be recovered in order to recover and purify the expressed polypeptide. If polypeptide is produced intracellularly, the cells must first be lysed before the polypeptide is recovered.

The polypeptide of the invention can be used to screen libraries of compounds in any of a variety of drug screening techniques. Such compounds may activate (agonise) or inhibit (antagonise) the level of expression of the gene or the activity of the polypeptide of the invention and form a further aspect of the present invention. Preferred compounds are effective to alter the expression of a natural gene which encodes a polypeptide of the first aspect of the invention or to regulate the activity of a polypeptide of the first aspect of the invention.

Agonist or antagonist compounds may be isolated from, for example, cells, cell-free preparations, chemical libraries or natural product mixtures. These agonists or antagonists may be natural of modified substrates, ligands, enzymes, receptors or structural or functional mimetics. For a suitable review of such screening techniques, see Coligan et al., Current Protocols in Immunology 1(2):Chapter 5 (1991).

Compounds that are most likely to be good antagonists are molecules that bind to the polypeptide of the invention without inducing the biological effects of the polypeptide upon binding to it. Potential antagonists include small organic molecules, peptides, polypeptides and antibodies that bind to the polypeptide of the invention and thereby inhibit or extinguish its activity. In this fashion, binding of the polypeptide to normal cellular binding molecules may be inhibited, such that the normal biological activity of the polypeptide is prevented.

The polypeptide of the invention that is employed in such a screening technique may be free in solution, affixed to a solid support, borne on a cell surface or located intracellularly.

In general, such screening procedures may involve using appropriate cells or cell membranes that express the polypeptide that are contacted with a test compound to observe binding, or stimulation or inhibition of a functional response. The functional response of the cells contacted with the test compound is then compared with control cells that were not contacted with the test compound. Such an assay may assess whether the test compound results in a signal generated by activation of the polypeptide, using an appropriate detection system. Inhibitors of activation are generally assayed in the presence of a known agonist and the effect on activation by the agonist in the presence of the test compound is observed.

Methods for generating detectable signals in the types of assays described herein will be known to those of skill in the art. A particular example is cotransfecting a construct expressing a polypeptide according to the invention, or a fragment that is responsible for binding to target, in fusion with the GAL4 DNA binding domain, into a cell together with a reporter plasmid, an example of which is pFR-Luc (Stratagene Europe, Amsterdam, The Netherlands). This particular plasmid contains a synthetic promoter with five tandem repeats of GAL4 binding sites that control the expression of the luciferase gene. When a potential target or ligand is added to the cells, it will bind the GAL4-polypeptide fusion and induce transcription of the luciferase gene. The level of the luciferase expression can be monitored by its activity using a luminescence reader (see, for example, Lehman et al. JBC 270, 12953, 1995; Pawar et al. JBC, 277, 39243, 2002).

A further preferred method for identifying an agonist or antagonist of a polypeptide of the invention comprises:
(a) contacting a labelled or unlabeled compound with the polypeptide immobilized on any solid support (for example beads, plates, matrix support, chip) and detection of the compound by measuring the label or the presence of the compound itself; or
(b) contacting a cell expressing on the surface thereof the polypeptide, by means of artificially anchoring it to the cell membrane, or by constructing a chimeric receptor being associated with a second component capable of providing a detectable signal in response to the binding of a compound to the polypeptide, with a compound to be screened under conditions to permit binding to the polypeptide; and
(c) determining whether the compound binds to and activates or inhibits the polypeptide by comparing the level of a signal generated from the interaction of the compound with the polypeptide with the level of a signal in the absence of the compound.

For example, a method such as FRET detection of a ligand bound to the polypeptide in the presence of peptide co-activators (Norris et al., Science 285, 744, 1999) might be used.

In further preferred embodiments, the general methods that are described above may further comprise conducting the identification of agonist or antagonist in the presence of labelled or unlabelled ligand for the polypeptide.

In another embodiment of the method for identifying agonist or antagonist of a polypeptide of the present invention comprises:
determining the inhibition of binding of a ligand to the polypeptide of the invention on any solid or cellular surface thereof, in the presence of a candidate compound under conditions
to permit binding to the polypeptide, and determining the amount of ligand bound to the polypeptide. A compound capable of causing reduction of binding of a ligand is considered
to be a competitor which may act as an agonist or antagonist. Preferably the ligand is labelled.

More particularly, a method of screening for a polypeptide antagonist or agonist compound comprises the steps of:
(a) incubating a labelled ligand with a polypeptide according to the invention on any solid support or the cell surface, or a cell membrane containing a polypeptide of the invention.
(b) measuring the amount of labelled ligand bound to the polypeptide on the solid support, whole cell or the cell membrane;
(c) adding a candidate compound to a mixture of labelled ligand and immobilized polypeptide on the solid support, the whole cell or the cell membrane of step (a) and allowing the mixture to attain equilibrium;
(d) measuring the amount of labelled ligand bound to the immobilized polypeptide or the whole cell or the cell membrane after step (c); and
(e) comparing the difference in the labelled ligand bound in step (b) and (d), such that the compound which causes the reduction in binding in step (d) is considered to be an agonist or antagonist.

The polypeptides may be found to modulate a variety of physiological and pathological processes in a dose-dependent manner in the above-described assays. Thus, the "functional equivalents" of the polypeptides of the invention include polypeptides that exhibit any of the same modulatory activities in the above-described assays in a dose-dependent manner. Although the degree of dose-dependent activity need not be identical to that of the polypeptides of the invention, preferably the "functional equivalents" will exhibit substantially similar dose-dependence in a given activity assay compared to the polypeptides of the invention.

In certain of the embodiments described above, simple binding assays may be used, in which the adherence of a test compound to a surface bearing the polypeptide is detected by means of a label directly or indirectly associated with the test compound or in an assay involving competition with a labelled competitor. In another embodiment, competitive drug screening assays may be used, in which neutralising antibodies that are capable of binding the polypeptide specifically compete with a test compound for binding. In this manner, the antibodies can be used to detect the presence of any test compound that possesses specific binding affinity for the polypeptide.

Assays may also be designed to detect the effect of added test compounds on the production of mRNA encoding the polypeptide in cells. For example, an ELISA may be constructed that measures secreted or cell-associated levels of polypeptide using monoclonal or polyclonal antibodies.by standard methods known in the art, and this can be used to search for compounds that may inhibit or enhance the production of the polypeptide from suitably manipulated cells or tissues. The formation of binding complexes between the polypeptide and the compound being tested may then be measured.

Assay methods that are also included within the terms of the present invention are those that involve the use of the genes and polypeptides of the invention in overexpression or ablation assays. Such assays involve the manipulation of levels of these genes/polypeptides in cells and assessment of the impact of this manipulation event on the physiology of the manipulated cells. For example, such experiments reveal details of signaling and metabolic pathways in which the particular genes/polypeptides are implicated, generate information regarding the identities of polypeptides with which the studied polypeptides interact and provide clues as to methods by which related genes and proteins are regulated.

Another technique for drug screening which may be used provides for high throughput screening of compounds having suitable binding affinity to the polypeptide of interest (see International patent application WO84/03564). In this method, large numbers of different small test compounds are synthesised on a solid substrate, which may then be reacted with the polypeptide of the invention and washed. One way of immobilising the polypeptide is to use non-neutralising antibodies. Bound polypeptide may then be detected using methods that are well known in the art. Purified polypeptide can also be coated directly onto plates for use in the aforementioned drug screening techniques.

The polypeptide of the invention may be used to identify membrane-bound or soluble receptors, through standard receptor binding techniques that are known in the art, such as ligand binding and crosslinking assays in which the polypeptide is labelled with a radioactive isotope, is chemically modified, or is fused to a peptide sequence that facilitates its detection or purification, and incubated with a source of the putative receptor (for example, a composition of cells, cell membranes, cell supernatants, tissue extracts, or bodily fluids). The efficacy of binding may be measured using biophysical techniques such as surface plasmon resonance and spectroscopy. Binding assays may be used for the purification and cloning of the receptor, but may also identify agonists and antagonists of the polypeptide, that compete with the binding of the polypeptide to its receptor. Standard methods for conducting screening assays are well understood in the art.

This invention discloses the use of a INSP152 polypeptide for isolating or generating an agonist or stimulator of the INSP152 polypeptide for the treatment of an immune related disorder, wherein said agonist or stimulator is selected from the group consisting of:
1. a specific antibody or fragment thereof including: a) a chimeric, b) a humanized or c) a fully human antibody, as well as;
2. a bispecific or multispecific antibody,
3. a single chain (*e*.*g*. scFv) or
4. single domain antibody, or
5. a peptide- or non-peptide mimetic derived from said antibodies or
6. an antibody-mimetic such as a) an anticalin or b) a fibronectin-based binding molecule (e.g. trinectin or adnectin).

The generation of peptide- or non-peptide mimetics from antibodies is known in the art (Saragovi *et al*., 1991 and Saragovi *et al*., 1992).

Anticalins are also known in the art (Vogt *et al*., 2004). Fibronectin-based binding molecules are described in US6818418 and WO2004029224.

Furthermore, the test compound may be of various origin, nature and composition, such as any small molecule, nucleic acid, lipid, peptide, polypeptide including an antibody such as a chimeric, humanized or fully human antibody or an antibody fragment, peptide- or non-peptide mimetic derived therefrom as well as a bispecific or multispecific antibody, a single chain (*e*.*g*. scFv) or single domain antibody or an antibody-mimetic such as an anticalin or fibronectin-based binding molecule (*e*.*g*. trinectin or adnectin), *etc*., in isolated form or in mixture or combinations.

The invention also includes a screening kit useful in the methods for identifying agonists, antagonists, ligands, receptors, substrates, enzymes, that are described above.

The invention includes the agonists, antagonists, ligands, receptors, substrates and enzymes, and other compounds which modulate the activity or antigenicity of the polypeptide of the invention discovered by the methods that are described above.

As mentioned above, it is envisaged that the various moieties of the invention (*i*.*e*. the polypeptides of the first aspect of the invention, a nucleic acid molecule of the second or third aspect of the invention, a vector of the fourth aspect of the invention, a host cell of the fifth aspect of the invention, a ligand of the sixth aspect of the invention, may be useful in the therapy or diagnosis of diseases. To assess the utility of the moieties of the invention for treating or diagnosing a disease one or more of the following assays may be carried out. Note that although some of the following assays refer to the test compound as being a protein/polypeptide, a person skilled in the art will readily be able to adapt the following assays so that the other moieties of the invention may also be used as the "test compound".

The invention also provides pharmaceutical compositions comprising a polypeptide, nucleic acid, ligand or compound of the invention in combination with a suitable pharmaceutical carrier. These compositions may be suitable as therapeutic or diagnostic reagents, as vaccines, or as other immunogenic compositions, as outlined in detail below.

According to the terminology used herein, a composition containing a polypeptide, nucleic acid, ligand or compound [X] is "substantially free of" impurities [herein, Y] when at least 85% by weight of the total X+Y in the composition is X. Preferably, X comprises at least about 90% by weight of the total of X+Y in the composition, more preferably at least about 95%, 98% or even 99% by weight

The pharmaceutical compositions should preferably comprise a therapeutically effective amount of the polypeptide, nucleic acid molecule, ligand, or compound of the invention. The term "therapeutically effective amount" as used herein refers to an amount of a therapeutic agent needed to treat, ameliorate, or prevent a targeted disease or condition, or to exhibit a detectable therapeutic or preventative effect. For any compound, the therapeutically effective dose can be estimated initially either in cell culture assays, for example, of neoplastic cells, or in animal models, usually mice, rabbits, dogs, or pigs. The animal model may also be used to determine the appropriate concentration range and route of administration. Such information can then be used to determine useful doses and routes for administration in humans.

The precise effective amount for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician. Generally, an effective dose will be from 0.01 mg/kg to 50 mg/kg, preferably 0.05 mg/kg to 10 mg/kg. Compositions may be administered individually to a patient or may be administered in combination with other agents, drugs or hormones.

A pharmaceutical composition may also contain a pharmaceutically acceptable carrier, for administration of a therapeutic agent Such carriers include antibodies and other polypeptides, genes and other therapeutic agents such as liposomes, provided that the carrier does not itself induce the production of antibodies harmful to the individual receiving the composition, and which may be administered without undue toxicity. Suitable carriers may be large, slowly metabolised macromolecules such as proteins, polysaccharides, polylactic acids, polyglycolic acids, polymeric amino acids, amino acid copolymers and inactive virus particles.

Pharmaceutically acceptable salts can be used therein, for example, mineral acid salts such as hydrochlorides, hydrobromides, phosphates, sulphates, and the like; and the salts of organic acids such as acetates, propionates, malonates, benzoates, and the like. A thorough discussion of pharmaceutically acceptable carriers is available in Remington's Pharmaceutical Sciences (Mack Pub. Co., NJ. 1991).

Pharmaceutical compositions according to the present invention may further comprise one or more additional active agent Preferably, the additional active agent is selected from cyclophosphamide (CTX), CLTA4-Ig, rapamycin, mycophenolic acid, methylprednisone, FTY720, an LFA-1 blocking agent, ICOS-Ig, an anti-apoptotic agent, an antagonist of cytolytic T cell function, TACI-Ig or methotrexate.

Pharmaceutically acceptable carriers in therapeutic compositions may additionally contain liquids such as water, saline, glycerol and ethanol. Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient

Once formulated, the compositions of the invention can be administered directly to the subject The subjects to be treated can be animals; in particular, human subjects can be treated.

The pharmaceutical compositions utilised in this invention may be administered by any' number of routes including, but not limited to, oral, intravenous, intramuscular, intraarterial, intramedullary, intrathecal, intraventricular, transdermal or transcutaneous applications (for example, see WO98/20734), subcutaneous, intraperitoneal, intranasal, enteral, topical, sublingual, intravaginal or rectal means. Gene guns or hyposprays may also be used to administer the pharmaceutical compositions of the invention. Typically, the therapeutic compositions may be prepared as injectables, either as liquid solutions or suspensions; solid forms suitable for solution in, or suspension in, liquid vehicles prior to injection may also be prepared.

Direct delivery of the compositions will generally be accomplished by injection, subcutaneously, intraperitoneally, intravenously or intramuscularly, or delivered to the interstitial space of a tissue. The compositions can also be administered into a lesion. Dosage treatment may be a single dose schedule or a multiple dose schedule.

If the activity of the polypeptide of the invention is in excess in a particular disease state, several approaches are available. One approach comprises administering to a subject an inhibitor compound (antagonist) as described above, along with a pharmaceutically acceptable carrier in an amount effective to inhibit the function of the polypeptide, such as by blocking the binding of ligands, substrates, enzymes, receptors, or by inhibiting a second signal, and thereby alleviating the abnormal condition. Preferably, such antagonists are antibodies. Most preferably, such antibodies are chimeric and/or humanised to minimise their immunogenicity, as described previously.

In another approach, soluble forms of the polypeptide that retain binding affinity for the ligand, substrate, enzyme, receptor, in question, may be administered. Typically, the polypeptide may be administered in the form of fragments that retain the relevant portions.

In an alternative approach, expression of the gene encoding the polypeptide can be inhibited using expression blocking techniques, such as the use of antisense nucleic acid molecules (as described above), either internally generated or separately administered. Modifications of gene expression can be obtained by designing complementary sequences or antisense molecules (DNA, RNA, or PNA) to the control, 5' or regulatory regions (signal sequence, promoters, enhancers and introns) of the gene encoding the polypeptide. Similarly, inhibition can be achieved using "triple helix" base-pairing methodology. Triple helix pairing is useful because it causes inhibition of the ability of the double helix to open sufficiently for the binding of polymerases, transcription factors, or regulatory molecules. Recent therapeutic advances using triplex DNA have been described in the literature (Gee, J.E. et al. (1994) In: Huber, B.E. and B.I. Carr, Molecular and Immunologic Approaches, Futura Publishing Co., Mt. Kisco, NY). The complementary sequence or antisense molecule may also be designed to block translation of mRNA by preventing the transcript from binding to ribosomes. Such oligonucleotides may be administered or may be generated *in situ* from expression *in vivo*.

In addition, expression of the polypeptide of the invention may be prevented by using ribozymes specific to its encoding mRNA sequence. Ribozymes are catalytically active RNAs that can be natural or synthetic (see for example Usman, N, et al., Curr. Opin. Struct. Biol (1996) 6(4), 527-33). Synthetic ribozymes can be designed to specifically cleave mRNAs at selected positions thereby preventing translation of the mRNAs into functional polypeptide. Ribozymes may be synthesised with a natural ribose phosphate backbone and natural bases, as normally found in RNA molecules. Alternatively the ribozymes may be synthesised with non-natural backbones, for example, 2'-O-methyl RNA, to provide protection from ribonuclease degradation and may contain modified bases.

RNA molecules may be modified to increase intracellular stability and half-life. Possible modifications include, but are not limited to, the addition of flanking sequences at the 5' and/or 3' ends of the molecule or the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine and butosine, as well as acetyl-, methyl-, thio- and similarly modified forms of adenine, cytidine, guanine, thymine and uridine which are not as easily recognised by endogenous endonucleases.

For treating abnormal conditions related to an under-expression of the polypeptide of the invention and its activity, several approaches are also available. One approach comprises administering to a subject a therapeutically effective amount of a compound that activates the polypeptide, *i*.*e*., an agonist as described above, to alleviate the abnormal condition.

Alternatively, a therapeutic amount of the polypeptide in combination with a suitable pharmaceutical carrier may be administered to restore the relevant physiological balance of polypeptide.

Gene therapy may be employed to effect the endogenous production of the polypeptide by the relevant cells in the subject. Gene therapy is used to treat permanently the inappropriate production of the polypeptide by replacing a defective gene with a corrected therapeutic gene.

Gene therapy can occur *in vivo* or *ex vivo. Ex vivo* gene therapy requires the isolation and purification of patient cells, the introduction of a therapeutic gene and introduction of the genetically altered cells back into the patient. In contrast, *in vivo* gene therapy does not require isolation and purification of a patient's cells.

The therapeutic gene is typically "packaged" for administration to a patient. Gene delivery vehicles may be non-viral, such as liposomes, or replication-deficient viruses, such as adenovirus as described by Berkner, K.L., in Curr. Top. Microbiol. Immunol., 158, 39-66 (1992) or adeno-associated virus (AAV) vectors as described by Muzyczka, N., in Curr. Top. Microbiol. Immunol., 158, 97-129 (1992) and U.S. Patent No. 5,252,479. For example, a nucleic acid molecule encoding a polypeptide of the invention may be engineered for expression in a replication-defective retroviral vector. This expression construct may then be isolated and introduced into a packaging cell transduced with a retroviral plasmid vector containing RNA encoding the polypeptide, such that the packaging cell now produces infectious viral particles containing the gene of interest.

These producer cells may be administered to a subject for engineering cells *in vivo* and expression of the polypeptide *in vivo* (see Chapter 20, Gene Therapy and other Molecular Genetic-based Therapeutic Approaches, (and references cited therein) in Human Molecular Genetics (1996), T Strachan and A P Read, BIOS Scientific Publishers Ltd).

Another approach is the administration of "naked DNA" in which the therapeutic gene is directly injected into the bloodstream or muscle tissue.

In situations in which the polypeptides or nucleic acid molecules of the invention are disease-causing agents, the invention provides that they can be used in vaccines to raise antibodies against the disease causing agent.

Vaccines according to the invention may either be prophylactic (*i*.*e*. to prevent infection) or therapeutic (*i*.*e*. to treat disease after infection). Such vaccines comprise immunising antigen(s), immunogen(s), polypeptide(s), protein(s) or nucleic acid, usually in combination with pharmaceutically-acceptable carriers as described above, which include any carrier that does not itself induce the production of antibodies harmful to the individual receiving the composition. Additionally, these carriers may function as immunostimulating agents ("adjuvants"). Furthermore, the antigen or immunogen may be conjugated to a bacterial toxoid, such as a toxoid from diphtheria, tetanus, cholera, *H pylori*, and other pathogens.

Since polypeptides may be broken down in the stomach, vaccines comprising polypeptides are preferably administered parenterally (for instance, subcutaneous, intramuscular, intravenous, or intradermal injection). Formulations suitable for parenteral administration include aqueous and non-aqueous sterile injection solutions which may contain antioxidants, buffers, bacteriostats and solutes which render the formulation isotonic with the blood of the recipient, and aqueous and non-aqueous sterile suspensions which may include suspending agents or thickening agents.

The vaccine formulations of the invention may be presented in unit-dose or multi-dose containers. For example, sealed ampoules and vials and may be stored in a freeze-dried condition requiring only the addition of the sterile liquid carrier immediately prior to use. The dosage will depend on the specific activity of the vaccine and can be readily determined by routine experimentation.

Genetic delivery of antibodies that bind to polypeptides according to the invention may also be effected, for example, as described in International patent application WO98/55607.

The technology referred to as jet injection (see, for example, www.powderject.com) may also be useful in the formulation of vaccine compositions.

A number of suitable methods for vaccination and vaccine delivery systems are described in International patent application WO00/29428.

This invention also relates to the use of nucleic acid molecules according to the present invention as diagnostic reagents. Detection of a mutated form of the gene characterised by the nucleic acid molecules of the invention which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques.

Nucleic acid molecules for diagnosis may be obtained from a subject's cells, such as from blood, urine, saliva, tissue biopsy or autopsy material. The genomic DNA may be used directly for detection or may be amplified enzymatically by using PCR, ligase chain reaction (LCR), strand displacement amplification (SDA), or other amplification techniques (see Saiki et al., Nature, 324, 163-166 (1986); Bej, et al., Crit. Rev. Biochem. Molec. Biol., 26, 301-334 (1991); Birkenmeyer et al., J. Virol. Meth., 35, 117-126 (1991); Van Brunt, J., Bio/Technology, 8, 291-294 (1990)) prior to analysis.

In one embodiment, this aspect of the invention provides an in vitro method of diagnosing a disease in a patient, comprising assessing the level of expression of a natural gene encoding a polypeptide according to the invention and comparing said level of expression to a control level, wherein a level that is different to said control level is indicative of disease. The method may comprise the steps of:
a)contacting a sample of tissue from the patient with a nucleic acid probe under stringent
conditions that allow the formation of a hybrid complex between a nucleic acid molecule
of the invention and the probe;
b)contacting a control sample with said probe under the same conditions used in step a);
c)and detecting the presence of hybrid complexes in said samples;
wherein detection of levels of the hybrid complex in the patient sample that differ from levels of the hybrid complex in the control sample is indicative of disease.

A further aspect of the invention comprises a diagnostic method comprising the steps of:
a)obtaining a tissue sample from a patient being tested for disease;
b)isolating a nucleic acid molecule according to the invention from said tissue sample; and
c)diagnosing the patient for disease by detecting the presence of a mutation in the nucleic acid molecule which is associated with disease.

To aid the detection of nucleic acid molecules in the above-described methods, an amplification step, for example using PCR, may be included.

Deletions and insertions can be detected by a change in the size of the amplified product in comparison to the normal genotype. Point mutations can be identified by hybridizing amplified DNA to labelled RNA of the invention or alternatively, labelled antisense DNA sequences of the invention. Perfectly-matched sequences can be distinguished from mismatched duplexes by RNase digestion or by assessing differences in melting temperatures. The presence or absence of the mutation in the patient may be detected by contacting DNA with a nucleic acid probe that hybridises to the DNA under stringent conditions to form a hybrid double-stranded molecule, the hybrid double-stranded molecule having an unhybridised portion of the nucleic acid probe strand at any portion corresponding to a mutation associated with disease; and detecting the presence or absence of an unhybridised portion of the probe strand as an indication of the presence or absence of a disease-associated mutation in the corresponding portion of the DNA strand.

Such diagnostics are particularly useful for prenatal and even neonatal testing.

Point mutations and other sequence differences between the reference gene and "mutant" genes can be identified by other well-known techniques, such as direct DNA sequencing or single-strand conformational polymorphism, (see Orita et al., Genomics, 5, 874-879 (1989)). For example, a sequencing primer may be used with double-stranded PCR product or a single-stranded template molecule generated by a modified PCR. The sequence determination is performed by conventional procedures with radiolabelled nucleotides or by automatic sequencing procedures with fluorescent-tags. Cloned DNA segments may also be used as probes to detect specific DNA segments. The sensitivity of this method is greatly enhanced when combined with PCR. Further, point mutations and other sequence variations, such as polymorphisms, can be detected as described above, for example, through the use of allele-specific oligonucleotides for PCR amplification of sequences that differ by single nucleotides.

DNA sequence differences may also be detected by alterations in the electrophoretic mobility of DNA fragments in gels, with or without denaturing agents, or by direct DNA sequencing (for example, Myers et al., Science (1985) 230:1242). Sequence changes at specific locations may also be revealed by nuclease protection assays, such as RNase and S1 protection or the chemical cleavage method (see Cotton et al., Proc. Natl. Acad. Sci. USA (1985) 85: 4397-4401).

In addition to conventional gel electrophoresis and DNA sequencing, mutations such as microdeletions, aneuploidies, translocations, inversions, can also be detected by *in situ* analysis (see, for example, Keller et al., DNA Probes, 2nd Ed., Stockton Press, New York, N.Y., USA (1993)), that is, DNA or RNA sequences in cells can be analysed for mutations without need for their isolation and/or immobilisation onto a membrane. Fluorescence *in situ* hybridization (FISH) is presently the most commonly applied method and numerous reviews of FISH have appeared (see, for example, Trachuck et al., Science, 250, 559-562 ((1990), and Trask et al., Trends, Genet., 7, 149-154 (1991)).

In another embodiment of the invention, an array of oligonucleotide probes comprising a nucleic acid molecule according to the invention can be constructed to conduct efficient screening of genetic variants, mutations and polymorphisms. Array technology methods are well known and have general applicability and can be used to address a variety of questions in molecular genetics including gene expression, genetic linkage, and genetic variability (see for example: M.Chee et al., Science (1996), Vol 274, pp 610-613).

In one embodiment, the array is prepared and used according to the methods described in PCT application WO95/11995 (Chee et al.); Lockhart, D. J. et al. (1996) Nat. Biotech. 14: 1675-1680); and Schena, M. et al. (1996) Proc. Natl. Acad. Sci. 93: 10614-10619). Oligonucleotide pairs may range from two to over one million. The oligomers are synthesized at designated areas on a substrate using a light-directed chemical process. The substrate may be paper, nylon or other type of membrane, filler, chip, glass slide or any other suitable solid support. In another aspect, an oligonucleotide may be synthesized on the surface of the substrate by using a chemical coupling procedure and an ink jet application apparatus, as described in PCT application WO95/25116 (Baldeschweiler et al.). In another aspect, a "gridded" array analogous to a dot (or slot) blot may be used to arrange and link cDNA fragments or oligonucleotides to the surface of a substrate using a vacuum system, thermal, UV, mechanical or chemical bonding procedures. An array, such as those described above, may be produced by hand or by using available devices (slot blot or dot blot apparatus), materials (any suitable solid support), and machines (including robotic instruments), and may contain 8, 24, 96, 384, 1536 or 6144 oligonucleotides, or any other number between two and over one million which lends itself to the efficient use of commercially-available instrumentation.

In addition to the methods discussed above, diseases may be diagnosed by methods comprising determining, from a sample derived from a subject, an abnormally decreased or increased level of polypeptide or mRNA. Decreased or increased expression can be measured at the RNA level using any of the methods well known in the art for the quantitation of polynucleotides, such as, for example, nucleic acid amplification, for instance PCR, RT-PCR, RNase protection, Northern blotting and other hybridization methods.

Assay techniques that can be used to determine levels of a polypeptide of the present invention in a sample derived from a host are well-known to those of skill in the art and are discussed in some detail above (including radioimmunoassays, competitive-binding assays, Western Blot analysis and ELISA assays). This aspect of the invention provides a diagnostic method which comprises the steps of: (a) contacting a ligand as described above with a biological sample under conditions suitable for the formation of a ligand-polypeptide complex; and (b) detecting said complex.

Protocols such as ELISA, RIA, and FACS for measuring polypeptide levels may additionally provide a basis for diagnosing altered or abnormal levels of polypeptide expression. Normal or standard values for polypeptide expression are established by combining body fluids or cell extracts taken from normal mammalian subjects, preferably humans, with antibody to the polypeptide under conditions suitable for complex formation The amount of standard complex formation may be quantified by various methods, such as by photometric means.

Antibodies which specifically bind to a polypeptide of the invention may be used for the diagnosis of conditions or diseases characterised by expression of the polypeptide, or in assays to monitor patients being treated with the polypeptides, nucleic acid molecules, ligands and other compounds of the invention. Antibodies useful for diagnostic purposes may be prepared in the same manner as those described above for therapeutics. Diagnostic assays for the polypeptide include methods that utilise the antibody and a label to detect the polypeptide in human body fluids or extracts of cells or tissues. The antibodies may be used with or without modification, and may be labelled by joining them, either covalently or non-covalently, with a reporter molecule. A wide variety of reporter molecules known in the art may be used, several of which are described above.

Quantities of polypeptide expressed in subject, control and disease samples from biopsied tissues are compared with the standard values. Deviation between standard and subject values establishes the parameters for diagnosing disease. Diagnostic assays may be used to distinguish between absence, presence, and excess expression of polypeptide and to monitor regulation of polypeptide levels during therapeutic intervention. Such assays may also be used to evaluate the efficacy of a particular therapeutic treatment regimen in animal studies, in clinical trials or in monitoring the treatment of an individual patient.

A diagnostic kit of the present invention may comprise:
(a) a nucleic acid molecule of the present invention;
(b) a polypeptide of the present invention; or
(c) a ligand of the present invention.

In one aspect of the invention, a diagnostic kit may comprise a first container containing a nucleic acid probe that hybridises under stringent conditions with a nucleic acid molecule according to the invention; a second container containing primers useful for amplifying the nucleic acid molecule; and instructions for using the probe and primers for facilitating the diagnosis of disease. The kit may further comprise a third container holding an agent for digesting unhybridised RNA.

In an alternative aspect of the invention, a diagnostic kit may comprise an array of nucleic acid molecules, at least one of which may be a nucleic acid molecule according to the invention.

To detect polypeptide according to the invention, a diagnostic kit may comprise one or more antibodies that bind to a polypeptide according to the invention; and a reagent useful for the detection of a binding reaction between the antibody and the polypeptide.

Various aspects and embodiments of the present invention will now be described in more detail by way of example, with particular reference to the INSP152 polypeptides.

### Brief description of the Figures

**Figure 1****:** Top ten BLASTp hits for INSP 152 polypeptide sequence (SEQ ID NO: 26) against NCBI-nr database.
**Figure 2****:** Alignment between INSP152 polypeptide sequence (SEQ ID NO: 26) and thyroid hormone-induced protein B precursor, a MAM domain protein.
**Figure 3****:** Signal peptide prediction (SignalP V2.0) for INSP152 polypeptide sequence (SEQ ID NO: 26).
**Figure 4****:** INSP152 (SEQ ID NO: 80) showing the 9 MAM domains and the transmembrane domain. (The 9 MAM domains predicted at ncbi-CDD are shaded. Predicted transmembrane region is in bold).
**Figure 5****:** DNA and protein sequence of INSP152. The predicted MAM domain 1 is underlined. Position and sense of PCR primers (INSP152-CP1/INSP152-CP2 and INSP152-CP3/INSP152-CP4) are indicated by the arrows.
**Figure 6****:** DNA sequence of the PCR product INSP152-D1-SV1 cloned using primers INSP152-CP3 and INSP152-CP4 and protein translation. Position and sense of PCR primers are indicated by the arrows.
**Figure 7****:** Alignment of the INSP152-D1-SV1 and INSP152-D1 protein sequences.

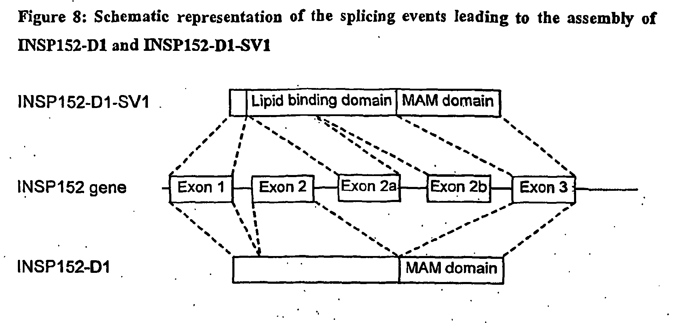

**Figure 8****:** Schematic representation of the splicing events leading to the assembly of INSP152-P1 and INSP152-D1-SV1 protein sequences.
**Figure 9****:** Schematic domain alignment of LDL and MAM domains containing proteins: rat apical endosomal glycoprotein precursor (AEGP, SwissProt Acc. No. Q63191), human UNQ3001 (SwissProt Acc. No. AAQ88785) along with the full length INSP152 and the cloned INSP152-SV1 (INSP152-D1-SV1).
**Figure 10****:** Expression of INSP152 in major human tissues
**Figure 11****:** Expression of INSP152 in comparative human tissues
**Figure 12****:** Expression of INSP152 in diseased human colon and ileum
**Figure 13****:** hMLR proliferation assay results for INSP152-D1-SV1

**TABLE 1**

| **Amino Acid** | **Synonymous Groups** | **More Preferred Synonymous Groups** |
|---|---|---|
| **Ser** | Gly, Ala, Ser, Thr, Pro | Thr, Ser |
| **Arg** | Asn, Lys, Gln, Arg, His | Arg, Lys, His |
| **Leu** | Phe, Ile, Val, Leu, Met | Ile, Val, Leu, Met |
| **Pro** | Gly, Ala, Ser, Thr, Pro | Pro |
| **Thr** | Gly, Ala, Ser, Thr, Pro | Thr, Ser |
| **Ala** | Gly, Thr, Pro, Ala, Ser | Gly, Ala |
| **Val** | Met, Phe, Ile, Leu, Val | Met, Ile, Val, Leu |
| **Gly** | Ala, Thr, Pro, Ser, Gly | Gly, Ala |
| **Ile** | Phe, Ile, Val, Leu, Met | Ile, Val, Leu, Met |
| **Phe** | Trp, Phe,Tyr | Tyr, Phe |
| **Tyr** | Trp, Phe,Tyr | Phe, Tyr |
| **Cys** | Ser, Thr, Cys | Cys |
| **His** | Asn, Lys, Gin, Arg, His | Arg, Lys, His |
| **Gln** | Glu, Asn, Asp, Gln | Asn, Gln |
| **Asn** | Glu, Asn, Asp, Gln | Asn, Gln |
| **Lys** | Asn, Lys, Gln, Arg, His | Arg, Lys, His |
| **Asp** | Glu, Asn, Asp, Gln | Asp, Glu |
| **Glu** | Glu, Asn, Asp, Gln | Asp, Glu |
| **Met** | Phe, Ile, Val, Leu, Met | Ile, Val, Leu, Met |
| **Trp** | Trp, Phe,Tyr | Trp |

**TABLE 2**

| **Amino Acid** | **Synonymous Groups** |
|---|---|
| Ser | D-Ser, Thr, D-Thr, allo-Thr, Met, D-Met, Met(O), D-Met(O), L-Cys, D-Cys |
| Arg | D-Arg, Lys, D-Lys, homo-Arg, D-homo-Arg, Met, Ile, D-.Met, D-Ile, Orn, D-Orn |
| Leu | D-Leu, Val, D-Val, AdaA, AdaG, Leu, D-Leu, Met, D-Met |
| Pro | D-Pro, L-I-thioazolidine-4-carboxylic acid, D-or L-1-oxazolidine-4-carboxylic acid |
| Thr | D-Thr, Ser, D-Ser, allo-Thr, Met,D-Met, Met(O), D-Met(O), Val, D-Val |
| Ala | D-Ala, Gly, Aib, B-Ala, Acp, L-Cys, D-Cys |
| Val | D-Val, Leu, D-Leu, Ile, D-Ile, Met, D-Met, AdaA, AdaG |
| Gly | Ala, D-Ala, Pro, D-Pro, Aib, .beta.-Ala, Acp |
| Ile | D-Ile, Val, D-Val, AdaA, AdaG, Leu, D-Leu, Met, D-Met |
| Phe | D-Phe, Tyr, D-Thr, L-Dopa, His, D-His, Trp, D-Trp, Trans-3,4, or 5-phenylproline, AdaA, AdaG, cis-3,4, or 5-phenylproline, Bpa, D-Bpa |
| Tyr | D-Tyr, Phe, D-Phe, L-Dopa, His, D-His |
| Cys | D-Cys, S--Me--Cys, Met, D-Met, Thr, D-Thr |
| Gln | D-Gln, Asn, D-Asn, Glu, D-Glu, Asp, D-Asp |
| Asn | D-Asn, Asp, D-Asp, Glu, D-Glu, Gln, D-Gln |
| Lys | D-Lys, Arg, D-Arg, homo-Arg, D-homo-Arg, Met, D-Met, Ile, D-Ile, Orn, D-Om |
| Asp | D-Asp, D-Asn, Asn, Glu, D-Glu, Gln, D-Gln |
| Glu | D-Glu, D-Asp, Asp, Asn, D-Asn, Gln, D-Gln |
| Met | D-Met, S--Me--Cys, Ile, D-Ile, Leu, D-Leu, Val, D-Val |

### Examples

### Example 1: INSP152 Protein BLAST Results

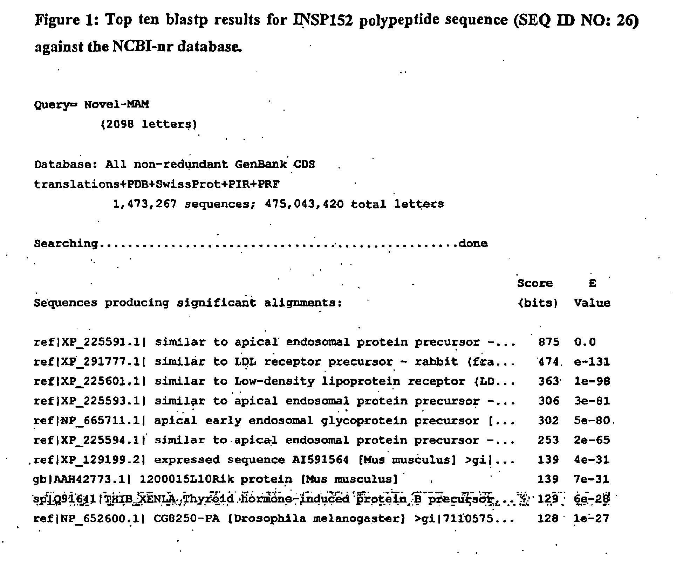

The INSP152 polypeptide sequence (SEQ ID NO: 26) was used as a protein BLAST query sequence against the NCBI non-redundant sequence database. Figure 1 shows the top ten results for the BLAST query, and Figure 2 shows the alignment of thyroid hormone-induced protein B precursor, a MAM domain protein with INSP152 (SEQ ID NO: 26).

### Example 2: INSP152 signal sequence

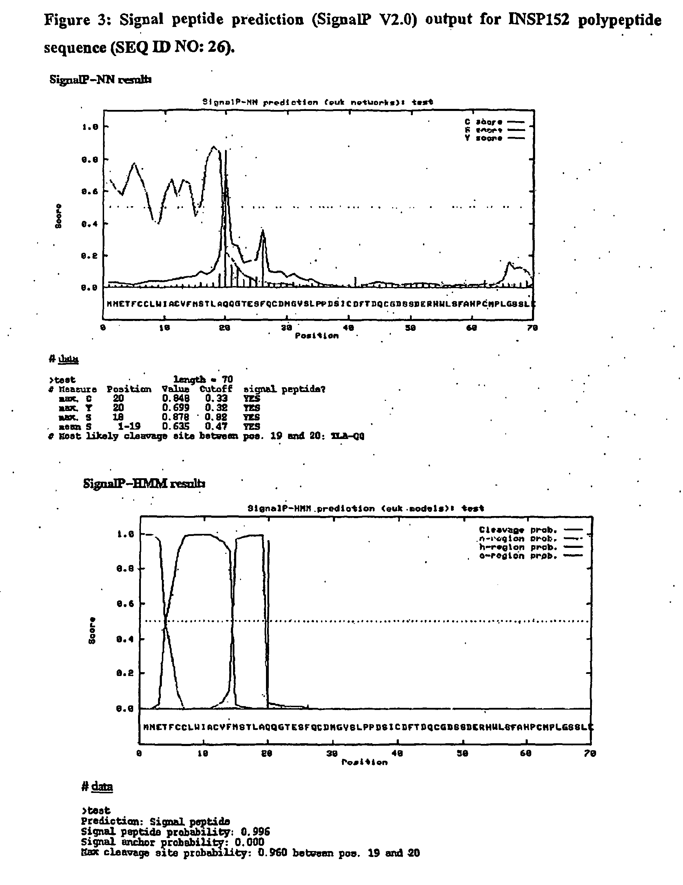

Figure 3 show that INSP152 is predicted to possess a signal peptide at the start of the protein. As the SignalP data in Figure 3 clearly shows, the signal peptide cleavage site is thought to be between residues 19 and 20 of the INSP152 partial polypeptide sequence (Nielsen, H. et al. 1997, Protein Engineering, 10, 1-6; Nielsen, H., and Krogh, A.: Prediction of signal peptides and signal anchors by a hidden Markov model. In Proceedings of the Sixth International Conference on Intelligent Systems for Molecular Biology (ISMB 6), AAAI Press, Menlo Park, California, pp. 122-130 (1998)).

### Example 3: Domain location

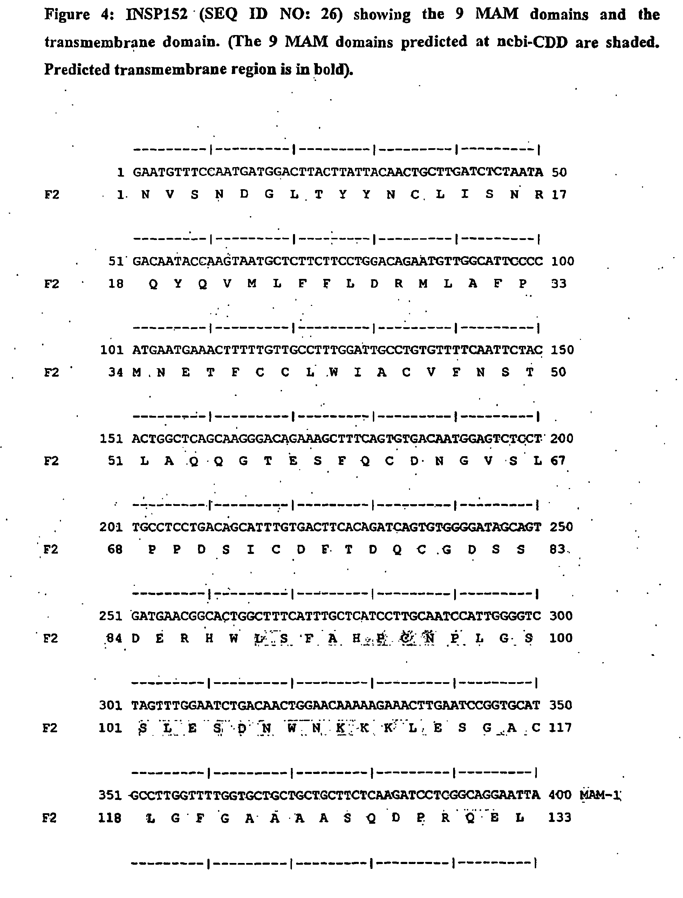

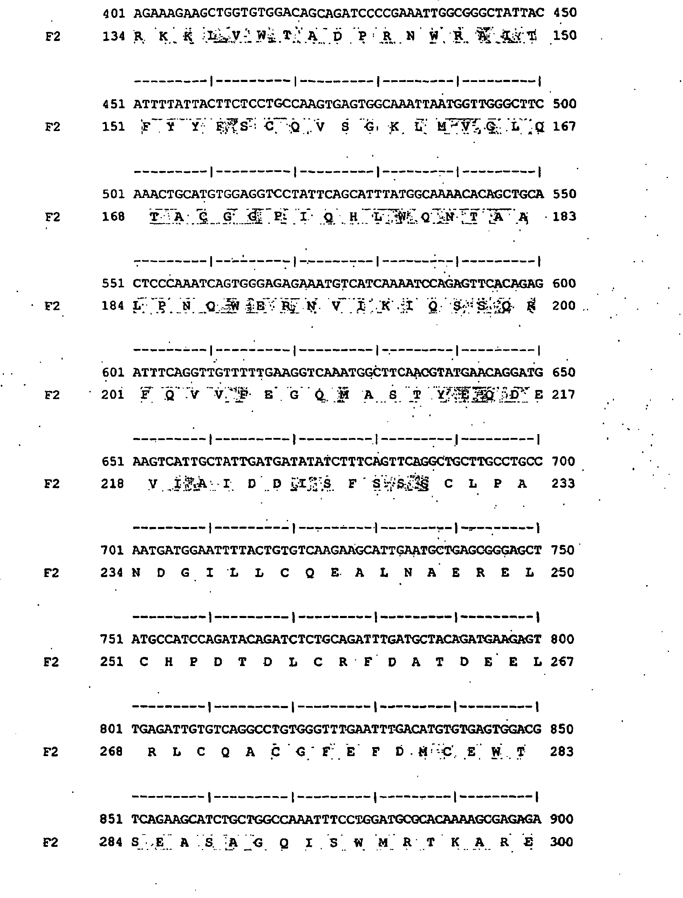

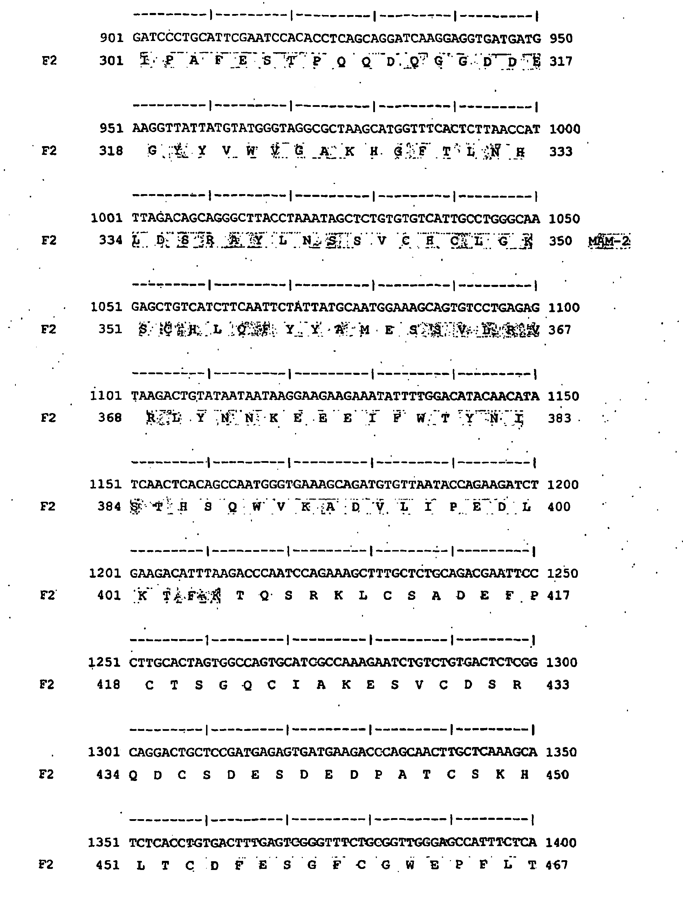

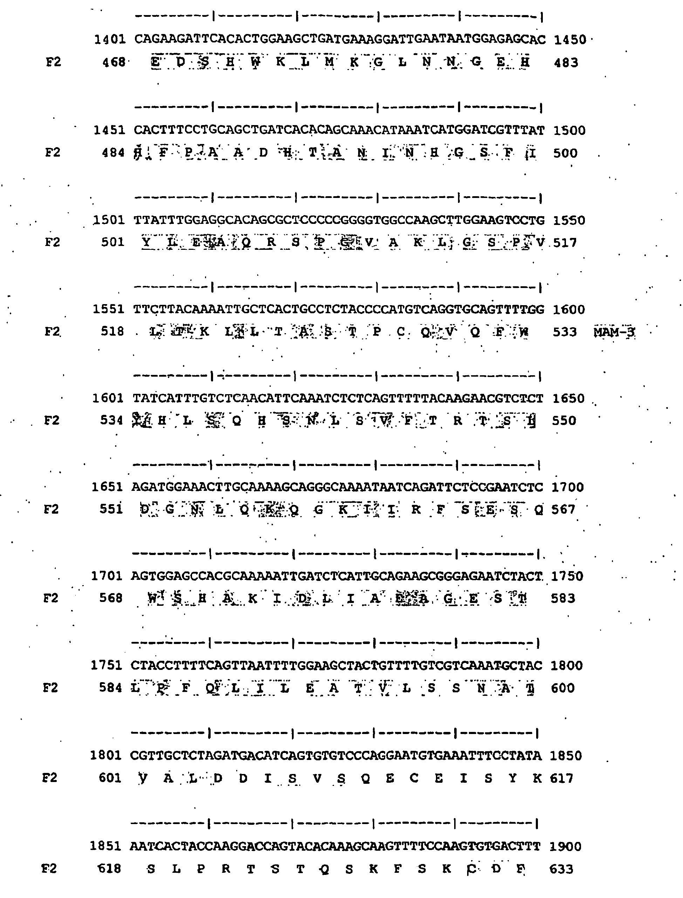

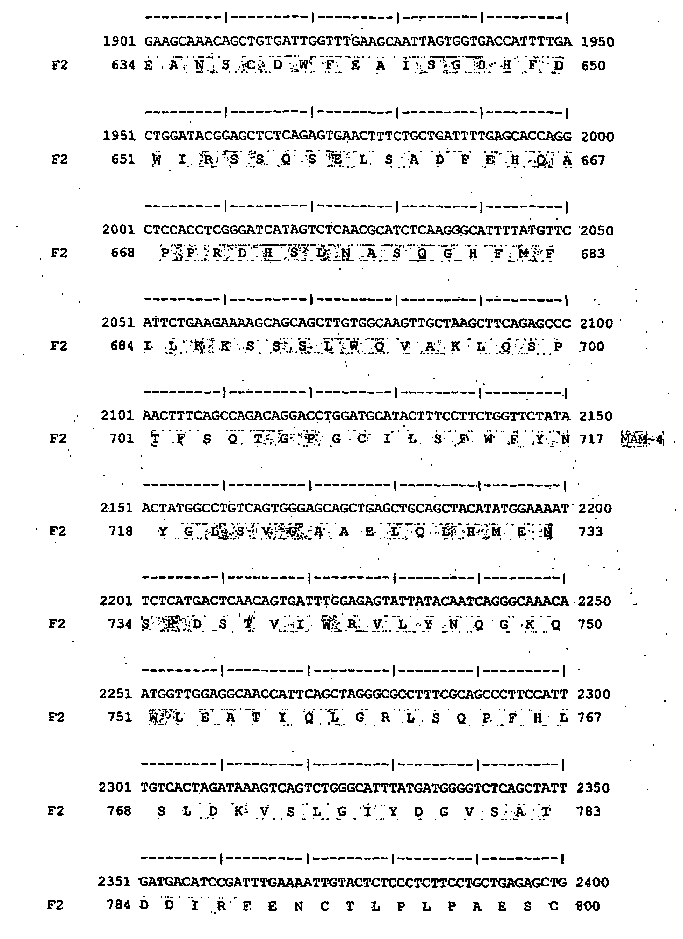

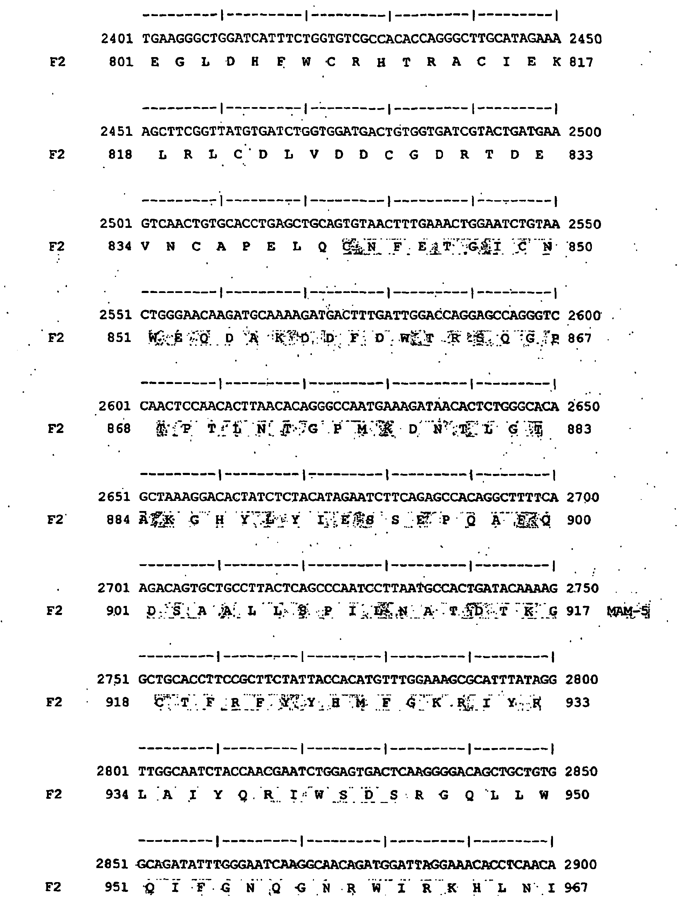

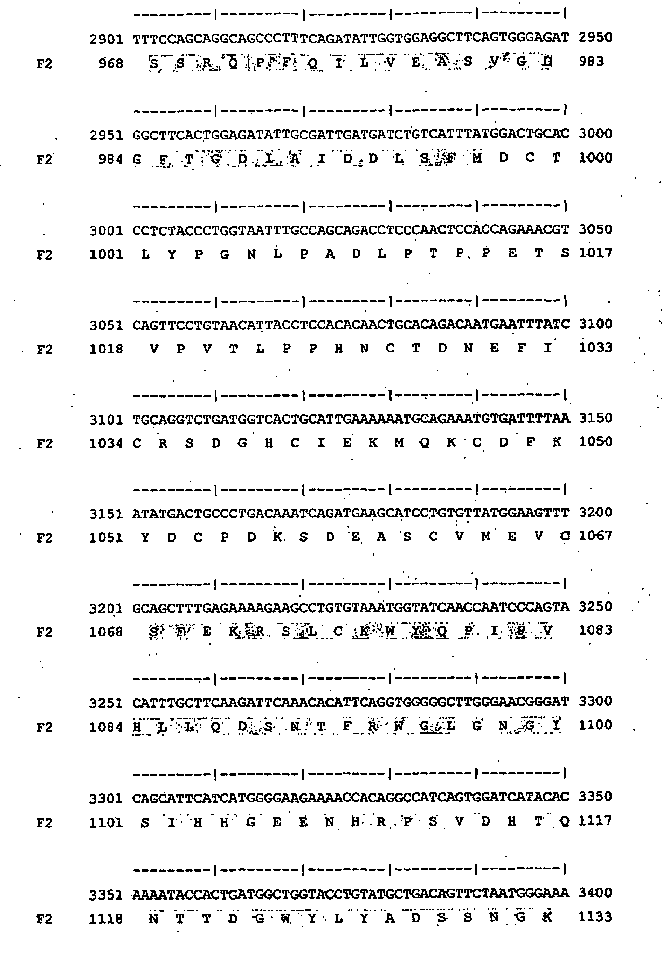

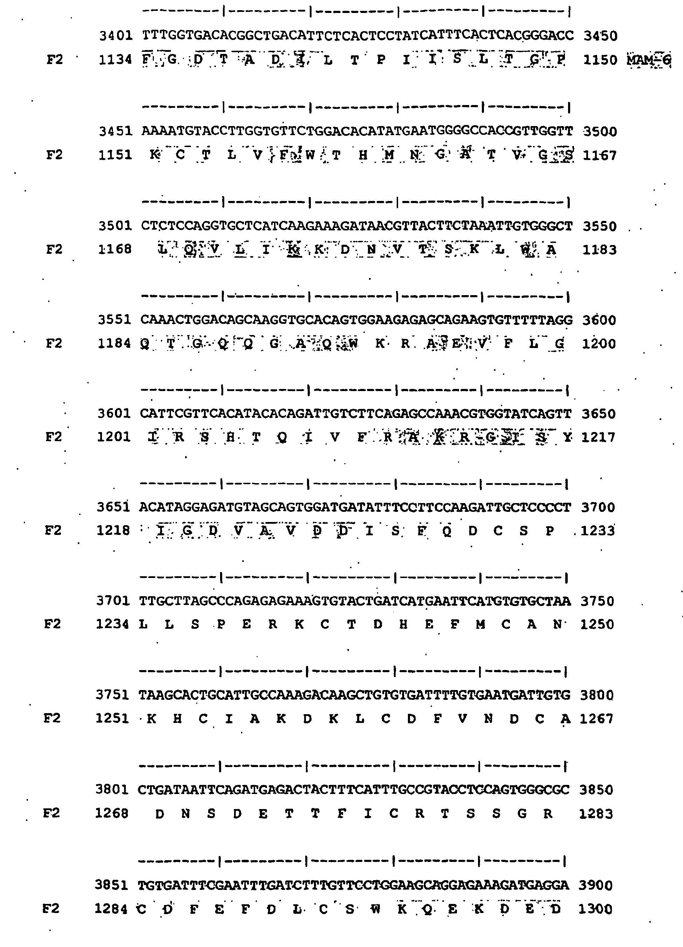

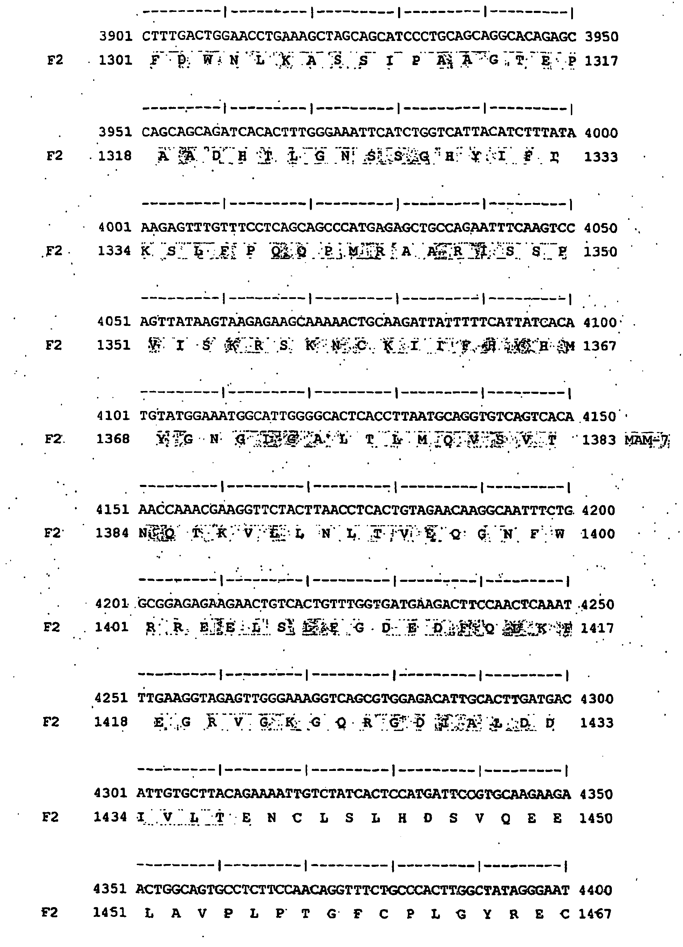

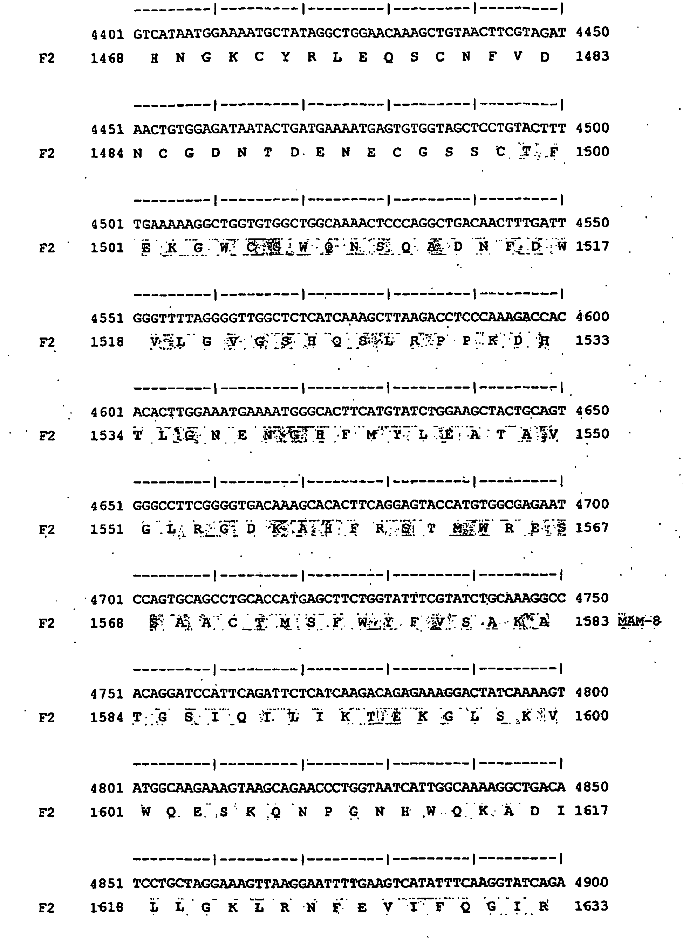

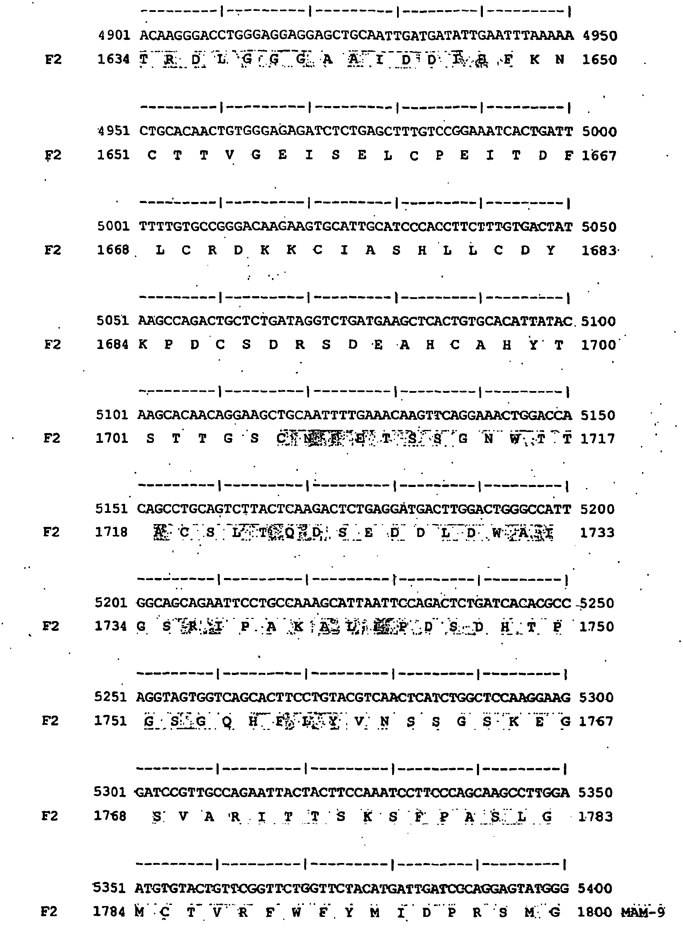

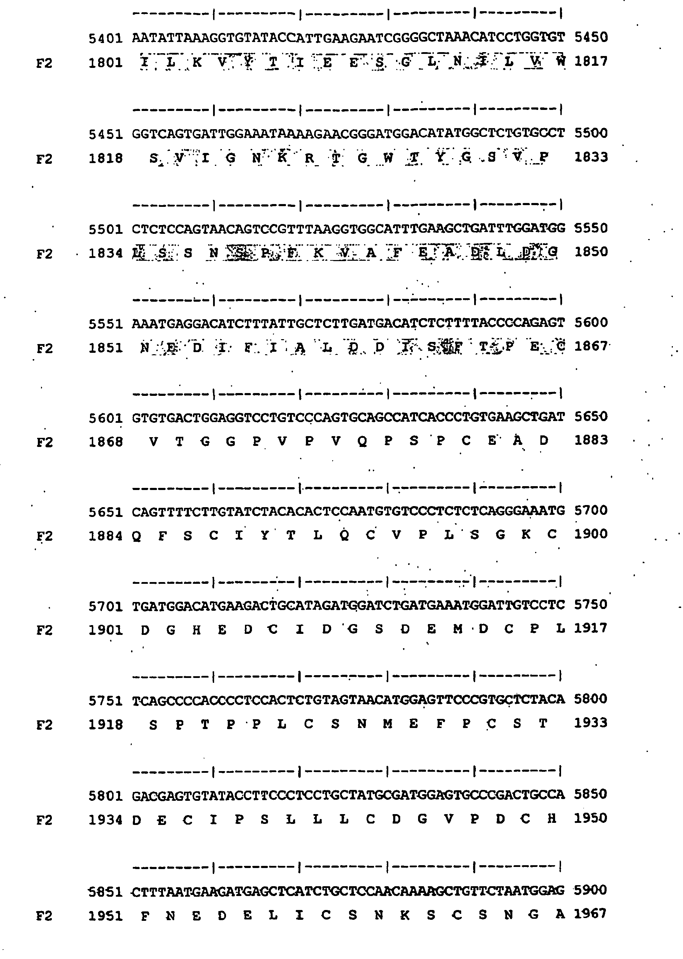

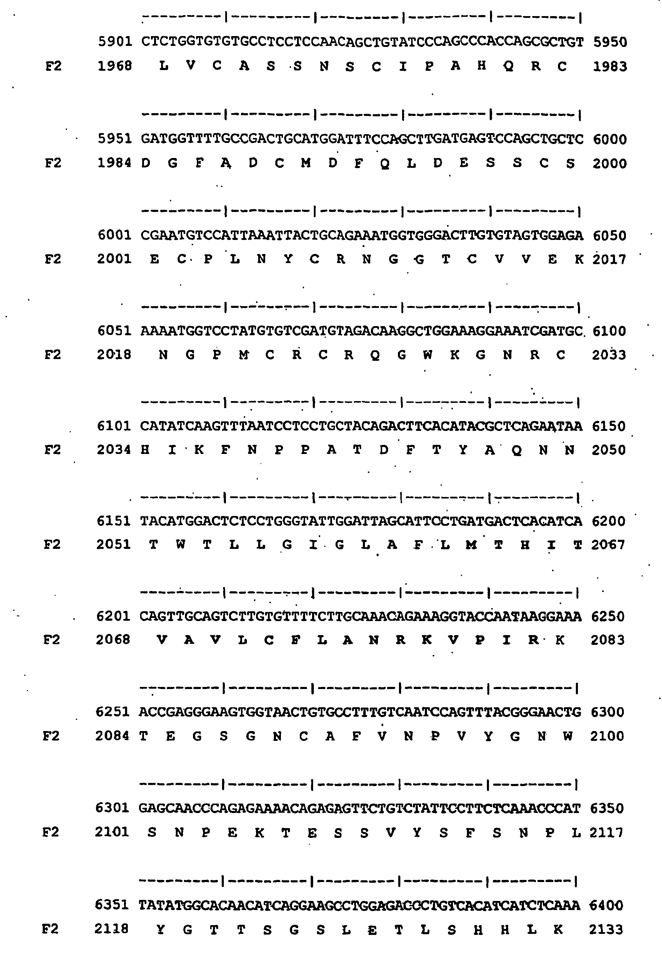

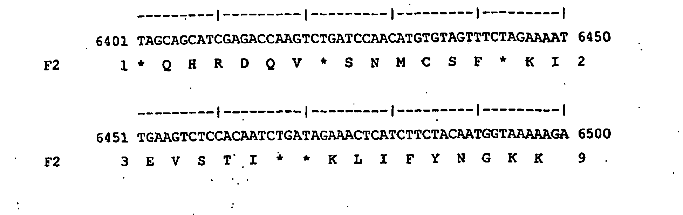

Figure 4 shows the location of the transmembrane domain as predicted by CDD as well as the location of the 9 MAM domains.

### Example 4: Cloning of INSP152-D1-SV1

### 4.1 Preparation of human cDNA templates

First strand cDNA was prepared from a variety of human tissue total RNA samples (Clontech, Stratagene, Ambion, Biochain Institute and in-house preparations) using Superscript II or SuperScript III RNase H⁻ Reverse Transcriptase (Invitrogen) according to the manufacturer's protocol.

For SuperScript II: Oligo (dT)₁₅ primer (1µl at 500µg/ml) (Promega), 2µg human total RNA, 1µ1 10 mM dNTP mix (10mM each of dATP, dGTP, dCTP and dTTP at neutral pH) and sterile distilled water to a final volume of 12µl were combined in a 1.5ml Eppendorf tube, heated to 65°C for 5 min and chilled on ice. The contents were collected by brief centrifugation and 4µl of 5X First-Strand Buffer, 2µl 0.1 M DTT, and 1µl RnaseOUT^{™} Recombinant Ribonuclease Inhibitor (40 units/µl, Invitrogen) were added. The contents of the tube were mixed gently and incubated at 42°C for 2 min, then 1µl (200 units) of SuperScript II™ enzyme was added and mixed gently by pipetting. The mixture was incubated at 42°C for 50 min and then inactivated by heating at 70°C for 15 min. To remove RNA complementary to the cDNA, 1µl (2 units) of *E. coli* RNase H (Invitrogen) was added and the reaction mixture incubated at 37°C for 20 min.

For SuperScript III: 1µl Oligo(dT)₂₀ primer (50µM, Invitrogen), 2µg human total RNA, 1µl 10mM dNTP mix (10mM each of dATP, dGTP, dCTP and dTTP at neutral pH) and sterile distilled water to a final volume of 10µl were combined in a 1.5ml Eppendorf tube, heated to 65°C for 5 min and then chilled on ice. For each RT reaction a cDNA synthesis mix was prepared as follows: 2µl 10X RT buffer, 4µl 25mM MgCl₂, 2µl 0.1M DTT, 1µl RNaseOUT™ (40 U/µl) and 1µl Superscript III™ RT enzyme were combined in a separate tube and then 10µl of this mix added to the tube containing the RNA/primer mixture. The contents of the tube were mixed gently, collected by brief centrifugation, and incubated at 50°C for 50 min. The reaction was terminated by incubating at 80°C for 5 min and the reaction mixture then chilled on ice and collected by brief centrifugation. To remove RNA complementary to the cDNA, 1µl (2 units) of *E. coli* RNase H (Invitrogen) was added and the reaction mixture incubated at 37°C for 20 min.

The final 21µl reaction mix was diluted by adding 179µl sterile water to give a total volume of 200µl. The RNA samples were combined into pools such that each pool contained up to five different cDNA samples. 5µl of each cDNA pool was used as a template for PCR in a 50µl final reaction volume and this consisted of 1µl of each cDNA sample in that pool. This represented approximately 20ng of each individual cDNA template.

### 4.2 INSP152-D1 specific cloning primers for PCR

Two pairs of PCR primers having a length of between 18 and 30 bases were designed for amplifying a 595 bp fragment of the INSP152 predicted cds (INSP152-D1 (Domain 1), from the start of the prediction to the end of the first predicted MAM domain, using Primer Designer Software (Scientific & Educational Software, PO Box 72045, Durham, NC 27722-2045, USA). PCR primers were optimized to have a Tm close to 55±10°C and a GC content of 40-60%. Primers were selected which had high selectivity for the target sequence (INSP152) with little or no none specific priming. The primers were designed to form two nested pairs such that INSP152-CP3/INSP152-CP4 primers were positioned slightly internal to INSP152-CP1/INSP152-CP2 primers.

### 4.3 PCR amplification of INSP152-D1 from human cDNA templates

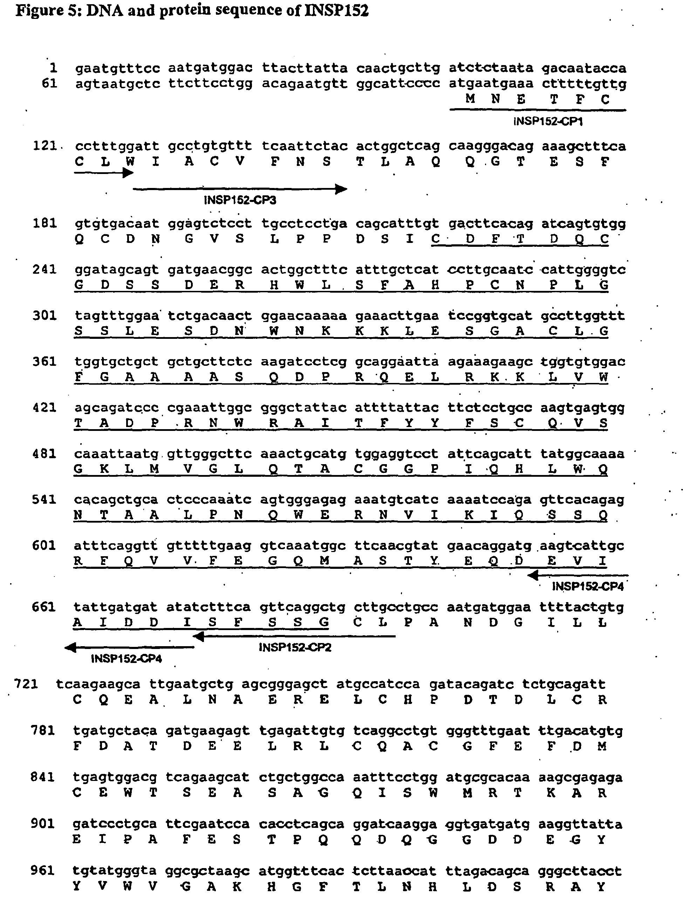

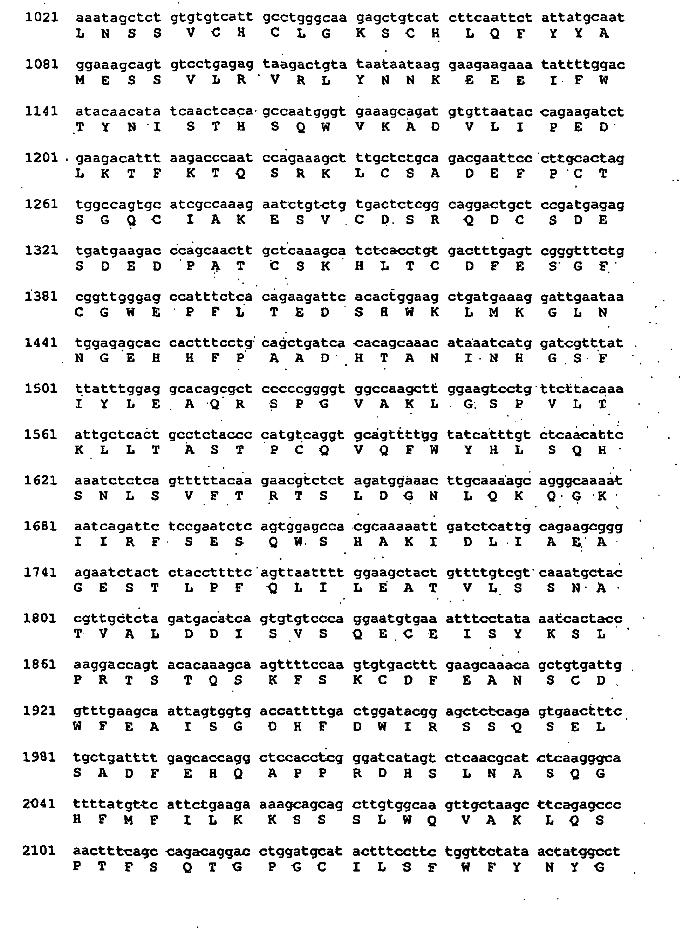

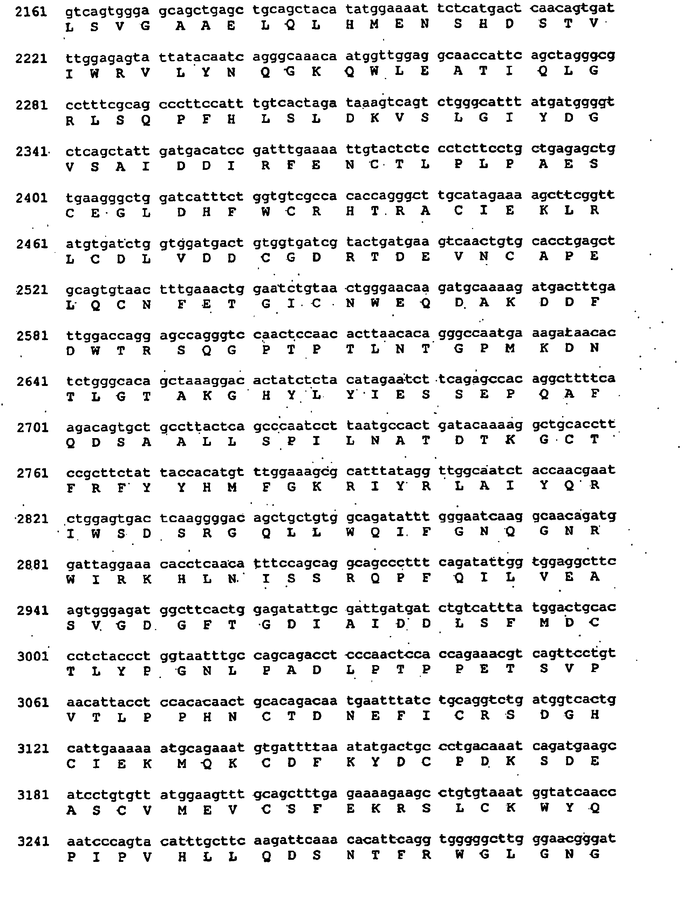

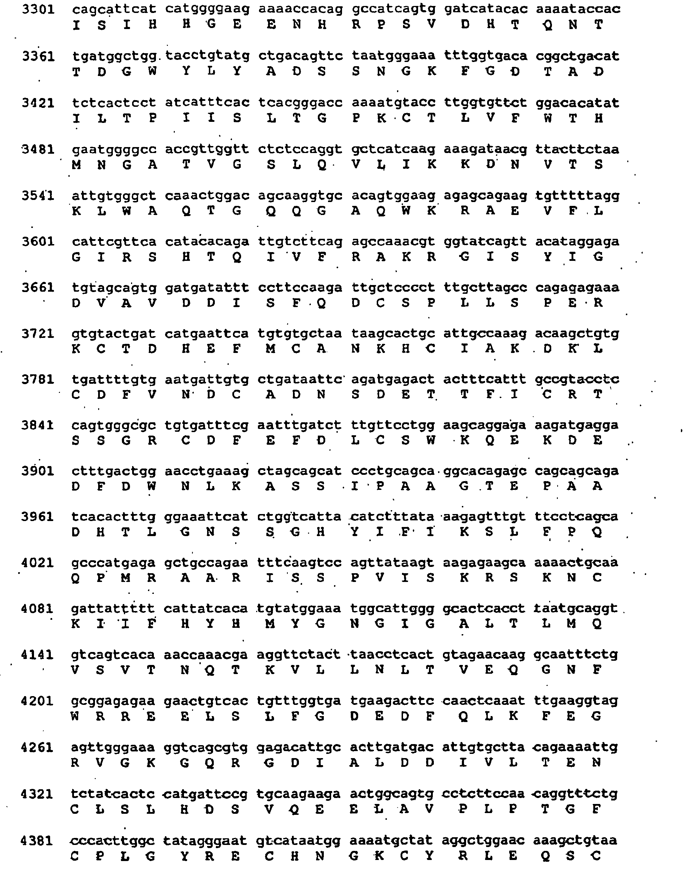

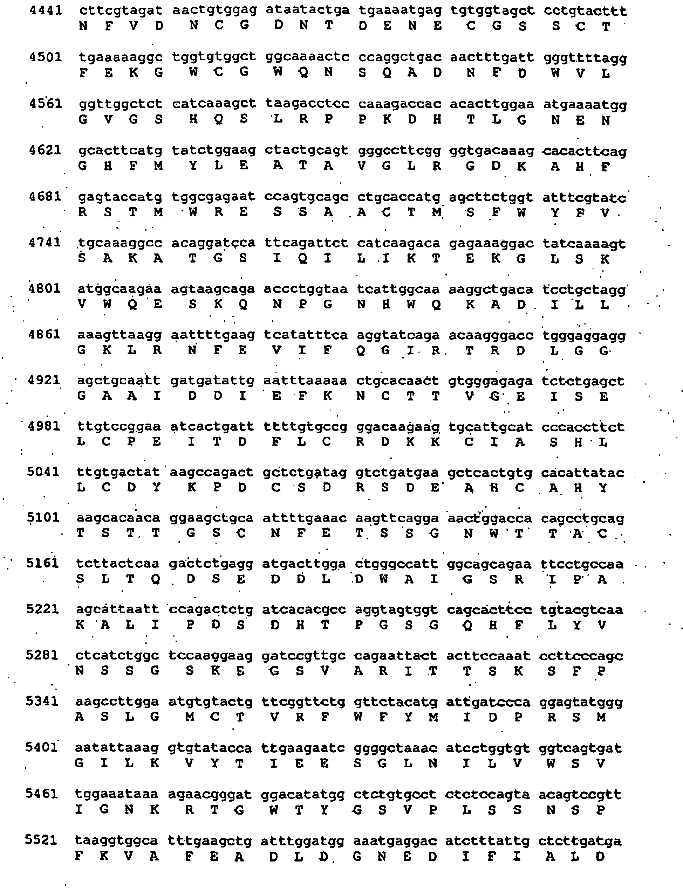

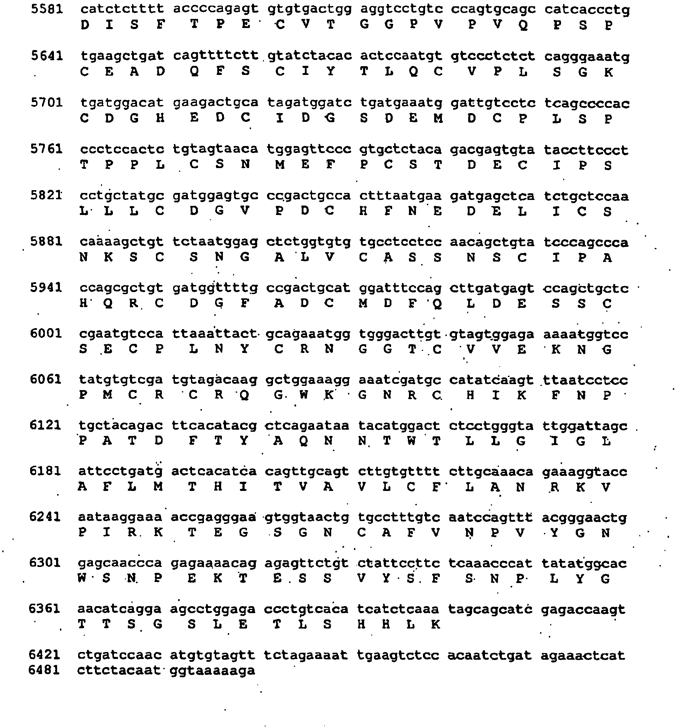

Gene-specific cloning primers INSP152-CP1 and INSP152-CP2 (Table 3, Figure 5) were designed to amplify a cDNA fragment of 595 bp containing the entire INSP152 domain sequence. The primer pair was used with the pools of human cDNA samples described above as PCR templates. This PCR1 was performed in a final volume of 50µl containing 1X AmpliTaq^{™} buffer, 200µM dNTPs, 50 pmoles of INSP152-CP1, 50 pmoles of INSP152-CP2, 2.5 units of AmpliTaq^{™} (Applied Biosystems) and approximately 100ng of pool cDNA using an MJ Research DNA Engine, programmed as follows: 94°C, 2 min; 40 cycles of 94°C, 1 min, 60°C, 1 min, and 72°C, 1 min; followed by 1 cycle at 72°C for 7 min and a holding cycle at 4°C.

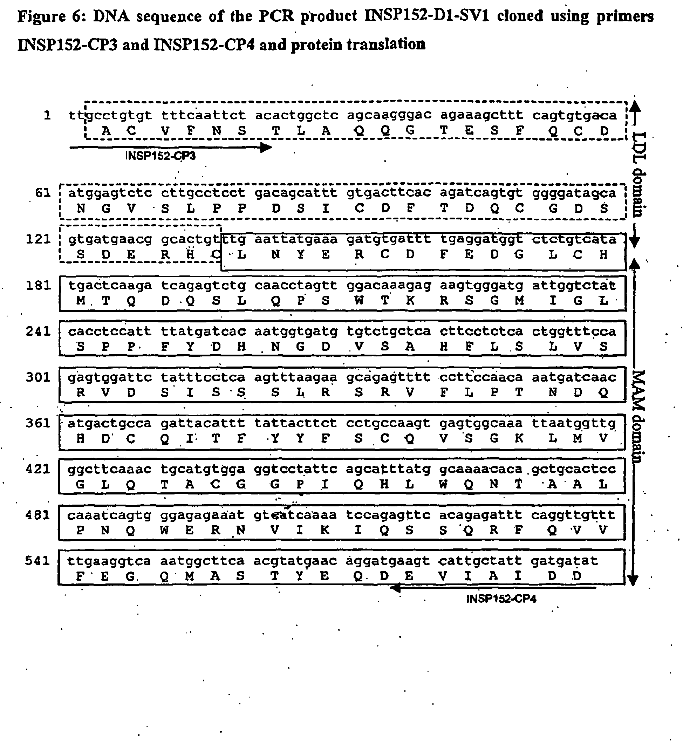

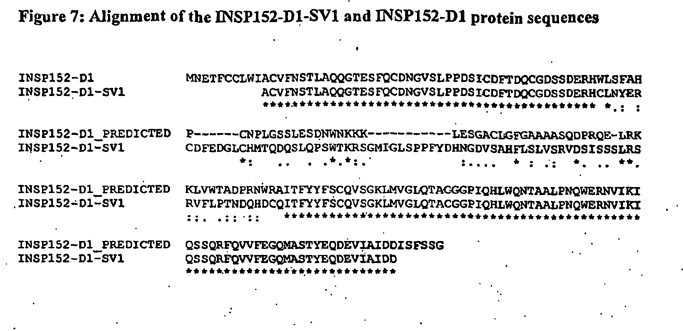

Each PCR1 product was then used as template for PCR2 using amplification primers INSP152-CP3 and INSP152-CP4 (Table 3, Figure 5, Figure 6, Figure 7) designed to amplify a cDNA fragment of 546bp within the INSP152-CP1/INSP152-CP2 product. PCR2 was performed in a final volume of 50µl containing 1X AmpliTaq^{™} buffer, 200µM dNTPs, 50 pmoles of INSP152-CP3, 50 pmoles of INSP152-CP4, 2.5 units of AmphTaq^{™} (Applied Biosystems) and 1µl of PCR1 product. Cycling was performed using an MJ Research DNA Engine, programmed as follows: 94°C, 2 min; 40 cycles of 94°C, 1 min, 57°C, 1 min, and 72°C, 1 min; followed by 1 cycle at 72°C for 7 min and a holding cycle at 4°C.

30µl of each PCR1 and PCR2 amplification product was visualized on a 0.8% agarose gel in 1 X TAE buffer (Invitrogen). Products of the approximately the expected molecular weight were purified from the gel using either the Wizard PCR Preps DNA Purification System (Promega) and eluted in 50µl of water, or the MinElute DNA Purification System (Qiagen) and eluted in 10µl of EB buffer (10mM Tris.Cl, pH 8.5). The purified product were subcloned directly.

### 4.4 Subcloning of PCR Products

The PCR products were subcloned into the topoisomerase I modified cloning vector (pCR4-TOPO) using the TA cloning kit purchased from the Invitrogen Corporation using the conditions specified by the manufacturer. Briefly, 4µl of gel purified PCR product was incubated for 15 min at room temperature with 1µl of TOPO vector and 1µl salt solution. The reaction mixture was then transformed into *E*. *coli* strain TOP10 (Invitrogen) as follows: a 50µl aliquot of One Shot TOP10 cells was thawed on ice and 2µl of TOPO reaction was added. The mixture was incubated for 15 min on ice and then heat shocked by incubation at 42°C for exactly 30 seconds. Samples were returned to ice and 250µl of warm (room temperature) SOC media was added. Samples were incubated with shaking (220 rpm) for 1h at 37°C. The transformation mixture was then plated on L-broth (LB) plates containing ampicillin (100µg/ml) and incubated overnight at 37°C.

### 4.5 Colony PCR

Colonies were inoculated into 50µl sterile water using a sterile toothpick. A 10µl aliquot of the inoculum was then subjected to PCR in a total reaction volume of 20µl containing 1X AmpliTaq^{™} buffer, 200µM dNTPs, 20 pmoles of T7 primer, 20 pmoles of T3 primer, 1 unit of AmpliTaq^{™} (Applied Biosystems) using an MJ Research DNA Engine. The cycling conditions were as follows: 94°C, 2 min; 30 cycles of 94°C, 30 sec, 48°C, 30 sec and 72°C for 1 min. Samples were maintained at 4°C (holding cycle) before further analysis.

PCR products were analyzed on 1% agarose gels in 1 X TAE buffer. Colonies which gave PCR products of approximately the expected molecular weight (595 bp or 546 bp + 105 bp due to the multiple cloning site (MCS)) were grown up overnight at 37°C in 5 ml L-Broth (LB) containing ampicillin (100µg /ml), with shaking at 220 rpm.

### 4.6 Plasmid DNA preparation and sequencing

Miniprep plasmid DNA was prepared from the 5ml cultures using a Biorobot 8000 robotic system (Qiagen) or Wizard Plus SV Minipreps kit (Promega cat. no. 1460) according to the manufacturer's instructions. Plasmid DNA was eluted in 80µl of sterile water. The DNA concentration was measured using an Eppendorf BO photometer or Spectramax 190 photometer (Molecular Devices). Plasmid DNA (200-500 ng) was subjected to DNA sequencing with the T7 and T3 sequencing primers (Table 3) using the BigDye Terminator system (Applied Biosystems cat no. 4390246) according to the manufacturer's instructions. Sequencing reactions were purified using Dye-Ex columns (Qiagen) or Montage SEQ 96 cleanup plates (Millipore cat no. LSKS09624) then analyzed on an Applied Biosystems 3700 sequencer.

**Table 3: INSP152-D1 cloning and sequencing primers**

| **Primer** | **Sequence (5'-3')** |
|---|---|
| INSP152-CP1 | ATG AAT GAA ACT TTT TGT TGC CTT TGG (SEQ ID NO: 30) |
| INSP152-CP2 | GCA AGC AGC CTG AAC TGA AAG A (SEQ ID NO: 31) |
| INSP152-CP3 | ATT GCC TGT GTT TTC AAT TCT AC (SEQ ID NO: 32) |
| INSP152-CP4 | TAT ATC ATC AAT AGC AAT GAC TTC (SEQ ID NO: 33) |
| T7 | TAA TAC GAC TCA CTA TAG G (SEQ ID NO: 34) |
| T3 | ATT AAC CCT CAC TAA AGG (SEQ ID NO: 35) |

| | |
|---|---|
| Underlined sequence = Kozak sequence Bold = Stop codon *Italic* sequence = His tag | |

### Example 5: Expression and purification of INSP152

Further experiments may now be performed to determine the tissue distribution and expression levels of the INSP152 polypeptides *in vivo*, on the basis of the nucleotide and amino acid sequence disclosed herein.

The presence of the transcripts for INSP152 may be investigated by PCR of cDNA from different human tissues. The INSP152 transcripts may be present at very low levels in the samples tested. Therefore, extreme care is needed in the design of experiments to establish the presence of a transcript in various human tissues as a small amount of genomic contamination in the RNA preparation will provide a false positive result. Thus, all RNA should be treated with DNAse prior to use for reverse transcription. In addition, for each tissue a control reaction may be set up in which reverse transcription was not undertaken (a -ve RT control).

For example, 1µg of total RNA from each tissue may be used to generate cDNA using Multiscript reverse transcriptase (ABI) and random hexamer primers. For each tissue, a control reaction is set up in which all the constituents are added except the reverse transcriptase (-ve RT control). PCR reactions are set up for each tissue on the reverse transcribed RNA samples and the minus RT controls. INSP152-specific primers may readily be designed on the basis of the sequence information provided herein. The presence of a product of the correct molecular weight in the reverse transcribed sample together with the absence of a product in the minus RT control may be taken as evidence for the presence of a transcript in that tissue. Any suitable cDNA libraries may be used to screen for the INSP152 transcripts, not only those generated as described above.

The tissue distribution pattern of the INSP152 polypeptides will provide further useful information in relation to the function of those polypeptides.

In addition, further experiments may now be performed using expression vectors. Transfection of mammalian cell lines with such vectors may enable the high level expression of the INSP152 polypeptides and thus enable the continued investigation of the functional characteristics of the INSP152 polypeptides. The following material and methods are an example of those suitable in such experiments:

### Cell Culture

Human Embryonic Kidney 293 cells expressing the Epstein-Barr virus Nuclear Antigen (HEK293-EBNA, Invitrogen) are maintained in suspension in Ex-cell VPRO serum-free medium (seed stock, maintenance medium, JRH). Sixteen to 20 hours prior to transfection (Day-1), cells are seeded in 2x T225 flasks (50ml per flask in DMEM / F12 (1:1) containing 2% FBS seeding medium (JRH) at a density of 2x10⁵ cells/ml). The next day (transfection day 0) transfection takes place using the JetPEITM reagent (2µl/µg of plasmid DNA, PolyPlus-transfection). For each flask, plasmid DNA is co-transfected with GFP (fluorescent reporter gene) DNA. The transfection mix is then added to the 2xT225 flasks and incubated at 37°C (5%CO₂) for 6 days. Confirmation of positive transfection may be carried out by qualitative fluorescence examination at day 1 and day 6 (Axiovert 10 Zeiss).

On day 6 (harvest day), supernatants from the two flasks are pooled and centrifuged (*e*.*g*. 4°C, 400g) and placed into a pot bearing a unique identifier. One aliquot (500µl) is kept for QC of the 6His-tagged protein (internal bioprocessing QC).

Scale-up batches may be produced by following the protocol called "PEI transfection of suspension cells", referenced BP/PEI/HH/02/04, with PolyEthyleneImine from Polysciences as transfection agent.

### Purification process

The culture medium sample containing the recombinant protein with a C-terminal 6His tag is diluted with cold buffer A (50mM NaH₂PO₄; 600mM NaCl; 8.7% (w/v) glycerol, pH 7.5). The sample is filtered then through a sterile filter (Millipore) and kept at 4°C in a sterile square media bottle (Nalgene).

The purification is performed at 4°C on the VISION workstation (Applied Biosystems) connected to an automatic sample loader (Labomatic). The purification procedure is composed of two sequential steps, metal affinity chromatography on a Poms 20 MC (Applied Biosystems) column charged with Ni ions (4.6 x 50 mm, 0.83ml), followed by gel filtration on a Sephadex G-25 medium (Amersham Pharmacia) column (1.0 x 10cm).

For the first chromatography step the metal affinity column is regenerated with 30 column volumes of EDTA solution (100mM EDTA; 1M NaCl; pH 8.0), recharged with Ni ions through washing with 15 column volumes of a 100mM NiSO₄ solution, washed with 10 column volumes of buffer A, followed by 7 column volumes of buffer B (50mM NaH₂PO₄; 600mM NaCl; 8.7 % (w/v) glycerol, 400mM; imidazole, pH 7.5), and finally equilibrated with 15 column volumes of buffer A containing 15mM imidazole. The sample is transferred, by the Labomatic sample loader, into a 200ml sample loop and subsequently charged onto the Ni metal affinity column at a flow rate of 10ml/min. The column is washed with 12 column volumes of buffer A, followed by 28 column volumes of buffer A containing 20mM imidazole. During the 20mM imidazole wash loosely attached contaminating proteins are eluted from the column. The recombinant His-tagged protein is finally eluted with 10 column volumes of buffer B at a flow rate of 2ml/min, and the eluted' protein is collected.

For the second chromatography step, the Sephadex G-25 gel-filtration column is regenerated with 2ml of buffer D (1.137M NaCl; 2.7mM KCl; 1.5mM KH₂PO₄; 8mM Na₂HPO₄; pH 7.2), and subsequently equilibrated with 4 column volumes of buffer C (137mM NaCl; 2.7mM KCI; 1.5mM KH₂PO₄; 8mM Na₂HPO₄; 20% (w/v) glycerol; pH 7.4). The peak fraction eluted from the Ni-column is automatically loaded onto the Sephadex G-25 column through the integrated sample loader on the VISION and the protein is eluted with buffer C at a flow rate of 2 ml/min. The fraction was filtered through a sterile centrifugation filter (Millipore), frozen and stored at -80°C. An aliquot of the sample is analyzed on SDS-PAGE (4-12% NuPAGE gel; Novex) Western blot with anti-His antibodies. The NuPAGE gel may be stained in a 0.1 % Coomassie blue R250 staining solution (30% methanol, 10% acetic acid) at room temperature for 1h and subsequently destained in 20% methanol, 7.5% acetic acid until the background is clear and the protein bands clearly visible.

Following the electrophoresis the proteins are electrotransferred from the gel to a nitrocellulose membrane. The membrane is blocked with 5% milk powder in buffer E (137mM NaCl; 2.7mM KCl; 1.5mM KH₂PO₄; 8mM Na₂HPO₄; 0.1 % Tween 20, pH 7.4) for 1h at room temperature, and subsequently incubated with a mixture of 2 rabbit polyclonal anti-His antibodies (G-18 and H-15, 0.2µg/ml each; Santa Cruz) in 2.5% milk powder in buffer E overnight at 4°C. After a further 1 hour incubation at room temperature, the membrane is washed with buffer E (3 x 10min), and then incubated with a secondary HRP-conjugated anti-rabbit antibody (DAKO, HRP 0399) diluted 1/3000 in buffer E containing 2.5% milk powder for 2 hours at room temperature. After washing with buffer E (3 x 10 minutes), the membrane is developed with the ECL kit (Amersham Pharmacia) for 1 min. The membrane is subsequently exposed to a Hyperfilm (Amersham Pharmacia), the film developed and the western blot image visually analysed.

For samples that showed detectable protein bands by Coomassie staining, the protein concentration may be determined using the BCA protein assay kit (Pierce) with bovine serum albumin as standard.

Furthermore, overexpression or knock-down of the expression of the polypeptides in cell lines may be used to determine the effect on transcriptional activation of the host cell genome. Dimerisation partners, co-activators and co-repressors of the INSP152 polypeptides may be identified by immunoprecipitation combined with Western blotting and immunoprecipitation combined with mass spectroscopy.

### Example 6: Analysis of INSP152 gene expression levels by TaqMan analysis

Total RNA from each sample was reverse transcribed using the Superscript III First-Strand Synthesis System for RT-PCR (Invitrogen, Cat No. 18080-051) in a final reaction volume of 20µl. 2µg of total RNA was combined with 50 ng random hexamer primers, 10mM each of dATP, dGTP, dCTP, and dTTP, and DEPC-treated water in a volume of 10µl. The mixture was incubated at 65°C for 5 min then chilled on ice for 1 min. The following 10µl cDNA synthesis mix was prepared in a separate tube: 2µl 10X RT buffer, 4µl 25mM MgCl₂, 2µl 0.1M DTT, 1µl RnaseOUT™ (40 units/µl), and 1µl SuperScript™ III RT enzyme (200 units/µl). The cDNA synthesis mix was added to the RNA/primer mixture, mixed gently and incubated at 25°C for 10 min then at 50°C for 50 min. The RT enzyme was then inactivated by incubating at 85°C for 5 min. The mixture was chilled on ice and then 1µl of *E. coli* Rnase H (2 units/µl) was added and the mixture incubated at 37°C for 20 min. The mixture was chilled on ice and then diluted 1/250 with sterile water. Dilutions of the reverse transcriptase reaction were then subjected to real time PCR analysis on a TaqMan instrument (PE Biosystems 7700).

PCR primers for human INSP152 and the housekeeping control gene glyceraldehyde 3-phosphate dehydrogenase (GAPDH) were designed using the Primer Express software (PE Biosystems). The PCR primers for INSP152 were designed with the forward primer in exon 4 and the reverse primer in exon 5. These primers would not distinguish between INSP152 from the prediction and the cloned INSP152-SV1-D1. The sequences of the primers are shown in Table 4. The specificity and the optimal primer concentration to use for the TaqMan analysis were determined by testing the INSP152 gene-specific primers on a series of dilutions of plasmid pEAK12d-PAC_INSP152-D1-SV1-6HIS-V1. Potential genomic DNA contamination of the cDNA was excluded by performing PCR reactions using primers specific for GAPDH intronic sequence. The absence of non-specific amplification was controlled by analyzing the PCR products on 4% agarose gels to ensure a single band of the expected molecular weight was produced.

**Table 4: TaqMan PCR primer sequences**

| Primer | Sequence (5'-3') |
|---|---|
| h-INSP152-D1-SV1-519F | GGAGAGAAATGTCATCAAAATCCA (SEQ ID NO: 36) |
| h-INSP152-D1-SV1-584R | GCCATTTGACCTTCAAAAACAAC (SEQ ID NO: 37) |
| hGAPDH-F | CCACCCATGGCAAATTCC (SEQ ID NO: 38) |
| hGAPDH-R | GATGGGATTTCCATTGATGACA (SEQ ID NO: 39) |
| Intron-hGAPDH-F | CCTAGTCCCAGGGCTTTGATT (SEQ ID NO: 40) |
| Intron-hGAPDH-R | CTGTGCTCCCACTCCTGATTT (SEQ ID NO: 41) |

SYBR Green Real-Time PCR reactions were carried out in a reaction volume of 50µl containing 25µl SYBR Green PCR master mix (PE Biosystems) (to which 0.5 units AmpErase Uracil N-Glycosylase (UNG, PE Biosystems) had previously been added), 300nM of each amplification primer, and 5µl of RT-PCR product. Cycling was performed using the ABI PRISM 7700 (TaqMan) Detection System programmed as follows: 1 cycle of 50 °C for 2 min; 1 cycle of 95 °C for 10 min; 40 cycles of 95 °C for 15 sec, 60 °C for 1 min. Each reaction was carried out in duplicate and the results averaged.

The primer-specific regions of the reverse-transcribed cDNA samples were thus amplified and their cycle threshold (Ct) values determined. The Ct value for each cDNA sample was normalized to that of the housekeeping gene GAPDH as follows. The difference in expression level between the GAPDH gene and the INSP152 gene in each cDNA sample was expressed as a difference in Ct value, i.e. Delta (δ) Ct = Ct (GAPDH) - Ct (INSP152). Results for each sample were then expressed as a fold difference in the number of cycles required for detectable INSP152 gene expression relative to that for GAPDH, according to the formula Fold Difference = 2^{(-δCt)}. Finally, the expression level of the INSP152 gene in each cDNA sample was shown relative to the GAPDH gene expression level, where GAPDH expression level = 100%, by dividing 100 by the Fold Difference for INSP152.

### Results

INSP152 primers were tested on a panel of normal and diseased human tissue samples containing 23 major organs; 21 secretory and immune tissues; 43 primary cells and cell lines of non-leukocytic, immune and CNS origin and colon or ileum biopsies from Crohn's disease (CD) (22 samples), ulcerative colitis (UC) (7 samples) and normal colon or ileum (N) respected from patients undergoing surgery for bowel cancer (13 samples). Results are shown in tables 5, 6 and 7 and represented graphically in figures 10, 11 and 12. INSP152 was below the limit of detection in all samples tested except in small intestine (3.94% of GAPDH) (Figure 10), a single lupus lung sample (3.06% of GAPDH) (Figure 11) and in 12/22 Crohn's disease biopsy samples (Figure 12). The 12 positive CD samples were all ileal biopsies.

### Conclusion

Expression results show unexpected restricted expression of INSP152 in small intestine (table 5 and figure 10), in a single lupus sample (table 6 and figure 11), and in 12/22 Crohn's disease biopsy samples (table 7 and figure 12). Except for one normal sample (5.09% of GADPH), all normal colon or ileum biopsies are below 0.8% of GADPH.

This specific pattern of expression leads to the conclusion of the involvement of INSP152, preferably INSP152-D1-SV1, in diseases of the small intestine, preferably Crohn's disease, small bowel disease, inflammatory bowel disease, lupus, celiac disease, Whipple's disease, neoplasias and diarrhea. These surprising properties characterizing the polynucleotides or the corresponding polypeptides of the present invention make them particularly suitable for the preparation of a drug or pharmaceutical composition. The polynucleotides or the corresponding polypeptides of the present invention therefore display the unexpected finding of a restricted expression in specific tissues.

**Table 5: Expression of INSP152 in major human tissues as measured by RT-PCR (TaqMan).**

| **major tissues collection** | **Ct hGAPDH** | **Ct hINSP152 D1-SV1** | **delta ct** | **Fold difference** | **Relative to GAPDH (=100)** |
|---|---|---|---|---|---|
| **S76 Brain** | **20.79** | **37.15** | **-16.36** | **84110.60** | **0.00** |
| **S77 Heart** | **19.96** | **40.00** | **-20.05** | **1081798.22** | **0.00** |
| **S78 Kidney** | **18.58** | **40.00** | **-21.43** | **2815578.61** | **0.00** |
| **S79 liver** | **22.43** | **40.00** | **-17.68** | **196255.00** | **0.00** |
| **S80 Lung** | **20.91** | **40.00** | **-19.10** | **559973.88** | **0.00** |
| **S81 Placenta** | **20.26** | **30.50** | **-10.24** | **1205.15** | **0.08** |
| **S82 skeletal Muscle** | **15.87** | **40.00** | **-24.14** | **18422940.32** | **0.00** |
| **S83 small intestine** | **20.20** | **24.87** | **-4.67** | **25.37** | **3.94** |
| **S84 Spleen** | **20.32** | **37.83** | **-17.52** | **187301.12** | **0.00** |
| **S85 Thymus** | **19.22** | **40.00** | **-20.78** | **1800542.22** | **0.00** |
| **S86 Uterus** | **20.23** | **34.72** | **-14.49** | **23010.42** | **0.00** |
| **S89 Spinal cord** | **19.06** | **40.00** | **-20.95** | **2018706.86** | **0.00** |
| **S 90 Cervix** | **22.12** | **40.00** | **-17.88** | **241221.67** | **0.00** |
| **S91 colon** | **20.15** | **31.83** | **-11.68** | **3281.18** | **0.03** |
| **S92 ovary** | **21.91** | **37.07** | **-15.17** | **36738.37** | **0.00** |
| **S93 prostate** | **19.02** | **40.00** | **-20.98** | **2068279.89** | **0.00** |
| **S94 testis** | **20.21** | **29.71** | **-9.51** | **726.59** | **0.14** |
| **S95 skin** | **23.47** | **40.00** | **-16.54** | **94957.87** | **0.00** |
| **S113 pancreas** | **22.07** | **38.90** | **-16.83** | **116502.39** | **0.00** |
| **S119 Breast** | **20.05** | **38.51** | **-18.46** | **359342.49** | **0.00** |
| **S120 Stomach** | **20.11** | **34.39** | **-14.29** | **19962.43** | **0.01** |
| **S122 Eye** | **21.53** | **40.00** | **-18.48** | **364358.72** | **0.00** |
| **S147 Bladder** | **19.69** | **40.00** | **-20.31** | **1299927.74** | **0.00** |

**Table 6: Expression of INSP152 in comparative human tissues as measured by RT-PCR (TaqMan).**

| **Comparative tissues** | **Ct hGAPDH** | **Ct hINSP152-D1-SV1** | **delta ct** | **Fold difference** | **Relative to GAPDH (=100)** |
|---|---|---|---|---|---|
| **S76 Brain** | **20.36** | **40.00** | **-19.64** | **817013.28** | **0.00** |
| **S140 fetal Brain** | **18.46** | **36.67** | **-18.21** | **303218.87** | **0.00** |
| **S77 Heart** | **20.57** | **40.00** | **-19.43** | **706338.40** | **0.00** |
| **S143 fetal Heart** | **17.82** | **37.55** | **-19.73** | **866595.97** | **0.00** |
| **S78 Kidney** | **20.11** | **40.00** | **-19.89** | **971598.00** | **0.00** |
| **S121 fetal Kidney** | **18.70** | **32.05** | **-13.36** | **10477.45** | **0.01** |
| **S130 Lupus total RNA Kidney** | **21.48** | **40.00** | **-18.52** | **375902.76** | **0.00** |
| **S135 Kidney tumor** | **18.86** | **39.13** | **-20.27** | **1264381.15** | **0.01** |
| **S79 liver** | **22.42** | **40.00** | **-17.59** | **196613.11** | **0.00** |
| **S142 fetal liver** | **18.89** | **40.00** | **-21.12** | **2271163.28** | **0.00** |
| **S127 Lupus total RNA liver** | **23.45** | **36.31** | **-12.87** | **7460.21** | **0.01** |
| **S131 Liver Cirrhosis** | **17.41** | **35.91** | **-18.51** | **372014.67** | **0.00** |
| **S136 Liver Tumor** | **20.66** | **40.00** | **-19.34** | **663620.99** | **0.00** |
| **S80 Lung** | **22.39** | **39.07** | **-16.69** | **105362.36** | **0.00** |
| **S144 Fetal Lung** | **17.42** | **40.00** | **-22.59** | **6291619.53** | **0.00** |
| **S128 Lupus total RNA Lung** | **19.91** | **24.94** | **-5.03** | **32.67** | **3.06** |
| **S132 Cirrhosis Lung** | **16.56** | **40.00** | **-23.45** | **11419530.49** | **0.00** |
| **S137 Lung Tumor** | **21.42** | **40.00** | **-18.59** | **393226.22** | **0.00** |
| **S84 Spleen** | **20.84** | **40.00** | **-19.17** | **587813.92** | **0.00** |
| **S141 Fetal Spleen** | **20.14** | **40.00** | **-19.86** | **951602.81** | **0.00** |
| **S129 Lupus total RNA Spleen** | **21.04** | **40.00** | **-18.96** | **509951.32** | **0.00** |
| **S133 Cirrhosis Spleen** | **20.99** | **40.00** | **-19.01** | **527934.71** | **0.00** |
| **S117Human Universal Reference** | **15.67** | **27.84** | **-12.17** | **4592.30** | **0.02** |

**Table 7: Expression of INSP152 in diseased colon and ileum as measured by RT-PCR (TaqMan).**

| **IBD plate** | **Ct hGAPDH** | **Ct hINSP152 D1-SV1** | **delta ct** | **Fold difference** | **Relative to GAPDH (=100)** |
|---|---|---|---|---|---|
| **N1** | **23.82** | **39.87** | **-16.05** | **67612.39** | **0.00** |
| **N2** | **22.09** | **29.13** | **-7.04** | **131.60** | **0.78** |
| **N3** | **23.66** | **40.00** | **-16.35** | **83240.61** | **0.00** |
| **N4** | **20.63** | **36.43** | **-15.80** | **56855.02** | **0.00** |
| **N6** | **20.44** | **40.00** | **-18.58** | **772941.66** | **0.00** |
| **N9** | **22.46** | **40.00** | **-17.55** | **191236.71** | **0.00** |
| **N10** | **21.62** | **30.10** | **-8.59** | **384.01** | **0.26** |
| **CD1** | **23.41** | **40.00** | **-16.59** | **98647.85** | **0.00** |
| **CD2** | **21.04** | **26.41** | **-5.37** | **41.21** | **2.43** |
| **CD3** | **18.28** | **29.81** | **-11.53** | **2957.17** | **0.03** |
| **CD4** | **20.92** | **40.00** | **-19.09** | **556105.85** | **0.00** |
| **CD5** | **25.31** | **31.47** | **-6.16** | **71.26** | **1.40** |
| **CD8** | **22.54** | **25.59** | **-3.05** | **8.28** | **12.07** |
| **CD8** | **22.06** | **26.08** | **-4.00** | **15.94** | **6.27** |
| **CD9** | **22.24** | **25.19** | **-2.95** | **7.73** | **12.94** |
| **CD10** | **12.35** | **40.00** | **-17.65** | **205674.01** | **0.00** |
| **CD13** | **22.52** | **25.06** | **-2.55** | **5.84** | **17.13** |
| **CD16** | **23.40** | **29.68** | **-6.28** | **77.71** | **1.29** |
| **CD17** | **24.78** | **31.42** | **-6.64** | **99.73** | **1.00** |
| **CD18** | **25.89** | **39.75** | **-13.87** | **14920.41** | **0.01** |
| **CD19** | **21.31** | **26.17** | **-4.87** | **28.14** | **3.43** |
| **CD20** | **21.87** | **40.00** | **-18.13** | **286862.53** | **0.00** |
| **CD22** | **21.26** | **30.82'** | **-9.57** | **757.45** | **0.13** |
| **N11** | **23.67** | **39.15** | **-15.48** | **45544.84** | **0.00** |
| **N14** | **20.86** | **25.15** | **-4.30** | **19.63** | **5.08** |
| **N28** | **20.97** | **40.00** | **-19.03** | **535304.41** | **0.00** |
| **N27** | **21.74** | **28.92** | **-7.19** | **145.51** | **0.69** |
| **N29** | **23.27** | **40.00** | **-16.73** | **108700.57** | **0.00** |
| **N30** | **21.10** | **38.97** | **-17.87** | **239555.43** | **0.00** |
| **CD4 bls** | **21.68** | **38.78** | **-17.11** | **140967.20** | **0.00** |
| **CD6 bis** | **20.71** | **36.08** | **-15.37** | **42347.82** | **0.00** |
| **CD23** | **24.62** | **40.00** | **-15.38** | **42642.37** | **0.00** |
| **CD24** | **21.86** | **26.41** | **-4.56** | **23.51** | **4.25** |
| **CD25** | **21.92** | **38.66** | **-16.75** | **109836.65** | **0.00** |
| **CD26** | **21.96** | **25.45** | **-3.49** | **11.20** | **8.93** |
| **CD27** | **22.69** | **30.48** | **-7.80** | **222.09** | **0.45** |
| **CD28** | **22.11** | **35.28** | **-13.17** | **9184.59** | **0.01** |
| **UC11** | **23.05** | **40.00** | **-18.98** | **127046.75** | **0.00** |
| **UC12** | **21.14** | **40.00** | **-18.87** | **477453.27** | **0.00** |
| **UC13** | **21.16** | **38.65** | **-17.50** | **184722.49** | **0.00** |
| **UC14** | **21.93** | **40.00** | **-18.08** | **276132.28** | **0.00** |
| **UC16** | **22.86** | **39.94** | **-17.08** | **138066.14** | **0.00** |
| **UC18** | **23.05** | **36.15** | **-13.10** | **8779.97** | **0.01** |
| **UC19** | **23.10** | **36.19** | **-13.09** | **8719.32** | **0.01** |

### Example 7: Human Mixed Lymphocyte Reaction (hMLR): proliferation and cytokine secretion.

This cell-based assay addresses antigen-specific T cell and antigen presenting cell functions upon stimulation by PBMC from another donor (alloreactivity). These are crucial cellular responses in inflammatory and autoimmune diseases. This assay allows the identification of compounds that have an effect on lymphocyte proliferation and/or cytokine secretion.

By analysing the cytokine secretion in this assay, it allows us to reveal if a compound is able to drive the polarization of the T lymphocytes to a TH-1 or TH-2 phenotype.

As an example, Multiple Sclerosis is associated with Th-1 cytokine profile (e.g.: γ-IFN, IL-2, TNF-α). With this assay it would be possible to identify compounds, which could inhibit TH-1 pro-inflammatory cytokine production or induced TH-2 anti-inflammatory cytokines (e.g.: IL-4, IL-5, IL-10).

### hMLR Proliferation Assay for dose-response determination - experimental design

10⁵ Stimulated cells (S), 2x10⁵ Responder cells (R) and the diluted protein of interest, i.e. INSP152-D1-SV1 or CTLA4-Ig as a positive control, (starting dilution to have 2% glycerol maximum then seven 1/3 dilutions) were added, in complete medium (DMEM F12, GIBCO, Invitrogen Corporation, supplemented with 10% FCS, CANSERA inactived 50' at 56°C, 10% L-Glutamine GIBCO, Invitrogen Corporation, 10% β-mercaptoethanol, FLUKA and 10% Penicillin/Streptomycin, GIBCO, Invitrogen Corporation) to a 96 well plate, round bottom (COSTAR). Four controls were performed. The positive control consisted in the S and R cells mix, the inhibition control in the S and R cells mix with Cyclosporin A 10⁻⁶ M (ALEXIS) and the last two controls in the S cells alone and R cells alone (negative control).

The cells were put in culture (37°C) for four days. At day 3 1µCi/well of ³H-Thymidine (AMERSHAM) was added. At day 4 the plates were harvested (OptiPhase "HiSafe"2, liquid scintillation cocktail, Pekin Elmer life sciences 1295-004 and Betaplate ^{™} 96-well harvester, Wallac) and the β-radiations counted (1205 Betaplate ® liquid scintillation counter, Wallac). As an alternative, 1µCi/well of ³H-Thymidine (AMERSHAM) was added at day 4 for 6 hours.

**Plate format**

| | | | | | | | | | | | |
|---|---|---|---|---|---|---|---|---|---|---|---|
| | | | | | | | | | | | |
| | S | 1/10 | 1/30 | 1/90 | 1/270 | 1/810 | 1/2430 | 1/7290 | 1/21870 | R | |
| | S | | | | | | | | | R | |
| | S+R | | | | | | | | | R | |
| | S+R | | | | | | | | S+R+CycloA | | |
| | S+R | | | | | | | | S+R+CycloA | | |
| | S+R | | | | | | | | S+R+CycloA | | |
| | | | | | | | | | | | |

### Conclusion

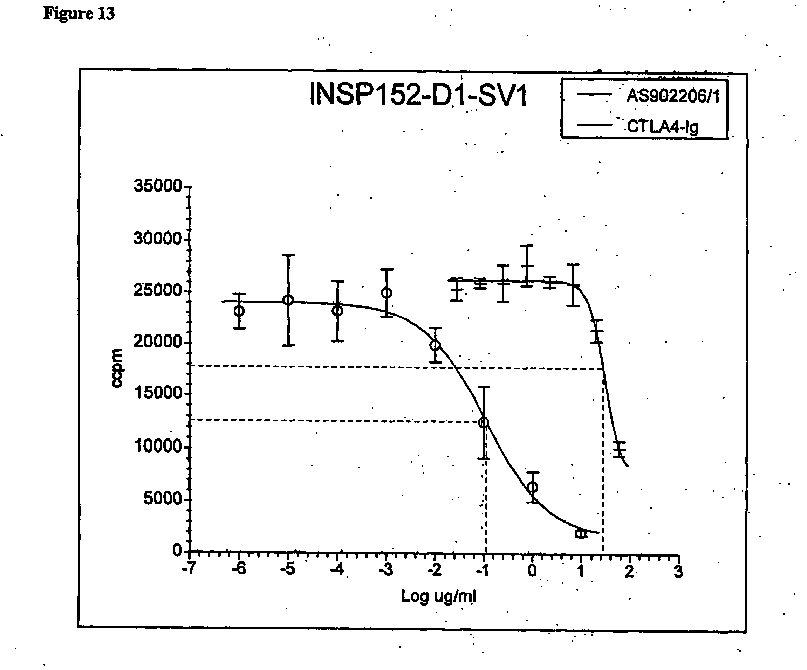

Results (Figure 13) surprisingly show that INSP152-D1-SV1 is a potent inhibitor of lymphocytes, preferably of T cells. As such, INSP152-D1-SV1 displays similar functional activity as CTLA4-Ig. These surprising properties characterizing the polynucleotides or the corresponding polypeptides of the present invention make them particularly suitable for the preparation of a drug or pharmaceutical composition.

CTLA4Ig is a soluble chimeric protein consisting of the extracellular domain of human CTLA4 and a fragment of the Fc portion of human IgG1. It binds to B7-1 and to B7-2 molecules on antigen-presenting cells (APCs) and thereby blocks the CD28-mediated costimulatory signal for T-cell activation and suppresses as well B cell response.

Biologic activity of CTLA4-Ig has been demonstrated in a variety of animal models of transplantation and autoimmunity. CTLA4-Ig has also been implicated or used in many diseases (see for example Davidson et al. Block and tackle: CTLA4Ig takes on lupus. Lupus. 2005;14(3):197-203.). In addition, CTLA4-Ig has been developed as a drug for the treatment of various conditions. For example, Bristol-Myers Squibb developed abatacept for the treatment of multiple sclerosis, psoriasis, rheumatoid arthritis, systemic lupus erythematosus, transplant rejection, graft versus host disease. Repligen Corp developed RG-1046 for the treatment of thrombocytopenic purpura, multiple sclerosis, rheumatoid arthritis and graft versus host disease.

As such, polypeptides of the present invention, preferably INSP152-D1-SV1, are useful for the treatment of CTLA4-Ig related diseases. INSP152-D1-SV1 is useful on its own, as a component of fusion proteins such as Fc fusion, and/or in combination with another agent

### List of INSP152 specific sequences:

<110> ARES TRADING S.A.
   <120> MAM domain containing protein
<130> P038837WO
   <150> GB 0423126.2
   <151> 2004-10-18
   <160> 41
   <170> SeqWin99, version 1.02
   <210> 1
   <211> 531
   <212> DNA
   <213> Homo sapiens
<400> 1
   cagcaaggga cagaaagctt tcagtgtgac aatggagtct ccttgcctcc tgacagcatt    60
   tgtgacttca cagatcagtg tggggatagc agtgatgaac ggcactggct ttcatttgct   120
   catccttgca atccattggg gtctagtttg gaatctgaca actggaacaa aaagaaactt   180
   gaatccggtg catgccttgg ttttggtgct gctgctgctt ctcaagatcc tcggcaggaa   240
   ttaagaaaga agctggtgtg gacagcagat ccccgaaatt ggcgggctat tacattttat   300
   tacttctcct gccaagtgag tggcaaatta atggttgggc ttcaaactgc atgtggaggt   360
   cctattcagc atttatggca aaacacagct gcactcccaa atcagtggga gagaaatgtc   420
   atcaaaatcc agagttcaca gagatttcag gttgtttttg aaggtcaaat ggcttcaacg   480
   tatgaacagg atgaagtcat tgctattgat gatatatctt tcagttcagg c            531
<210> 2
   <211> 177
   <212> PRT
   <213> Homo sapiens
<400> 2
   Gln Gln Gly Thr Glu Ser Phe Gln Cys Asp Asn Gly Val Ser Leu Pro
   1               5                   10                  15
   Pro Asp Ser Ile Cys Asp Phe Thr Asp Gln Cys Gly Asp Ser Ser Asp
               20                  25                  30
   Glu Arg His Trp Leu Ser Phe Ala His Pro Cys Asn Pro Leu Gly Ser
           35                  40                  45
   Ser Leu Glu Ser Asp Asn Trp Asn Lys Lys Lys Leu Glu Ser Gly Ala
       50                  55                  60
   Cys Leu Gly Phe Gly Ala Ala Ala Ala Ser Gln Asp Pro Arg Gln Glu
   65                  70                  75                  80
   Leu Arg Lys Lys Leu Val Trp Thr Ala Asp Pro Arg Asn Trp Arg Ala
                   85                  90                  95
   Ile Thr Phe Tyr Tyr Phe Ser Cys Gln Val Ser Gly Lys Leu Met Val
               100                 105                 110
   Gly Leu Gln Thr Ala Cys Gly Gly Pro Ile Gln His Leu Trp Gln Asn
           115                 120                 125
   Thr Ala Ala Leu Pro Asn Gln Trp Glu Arg Asn Val Ile Lys Ile Gln
       130                 135                 140
   Ser Ser Gln Arg Phe Gln Val Val Phe Glu Gly Gln Met Ala Ser Thr
   145                 150                 155                 160
   Tyr Glu Gln Asp Glu Val Ile Ala Ile Asp Asp Ile Ser Phe Ser Ser
                   165                 170                 175
   Gly
<210> 3
   <211> 569
   <212> DNA
   <213> Homo sapiens
<400> 3
   cagcaaggga cagaaagctt tcagtgtgac aatggagtct ccttgcctcc tgacagcatt    60
   tgtgacttca cagatcagtg tggggatagc agtgatgaac ggcactgttt gaattatgaa   120
   agatgtgatt ttgaggatgg tctctgtcat atgactcaag atcagagtct gcaacctagt   180
   tggacaaaga gaagtgggat gattggtcta tcacctccat tttatgatca caatggtgat   240
   gtgtctgctc acttcctctc actggtttcc agagtggatt ctatttcctc aagtttaaga   300
   agcagagttt tccttccaac aaatgatcaa catgactgcc agattacatt ttattacttc   360
   tcctgccaag tgagtggcaa attaatggtt gggcttcaaa ctgcatgtgg aggtcctatt   420
   cagcatttat ggcaaaacac agctgcactc ccaaatcagt gggagagaaa tgtcatcaaa   480
   atccagagtt cacagagatt tcaggttgtt tttgaaggtc aaatggcttc aacgtatgaa   540
   caggatgaag tcattgctat tgatgatat                                      569
<210> 4
   <211> 189
   <212> PRT
   <213> Homo sapiens
<400> 4
   Gln Gln Gly Thr Glu Ser Phe Gln Cys Asp Asn Gly Val Ser Leu Pro
   1               5                   10                  15
   Pro Asp Ser Ile Cys Asp Phe Thr Asp Gln Cys Gly Asp Ser Ser Asp
               20                  25                  30
   Glu Arg His Cys Leu Asn Tyr Glu Arg Cys Asp Phe Glu Asp Gly Leu
           35                  40                  45
   Cys His Met Thr Gln Asp Gln Ser Leu Gln Pro Ser Trp Thr Lys Arg
       50                  55                  60
   Ser Gly Met Ile Gly Leu Ser Pro Pro Phe Tyr Asp His Asn Gly Asp
   65                  70                  75                  80
   Val Ser Ala His Phe Leu Ser Leu Val Ser Arg Val Asp Ser Ile Ser
                   85                  90                  95
   Ser Ser Leu Arg Ser Arg Val Phe Leu Pro Thr Asn Asp Gln His Asp
               100                 105                 110
   Cys Gln Ile Thr Phe Tyr Tyr Phe Ser Cys Gln Val Ser Gly Lys Leu
           115                 120                 125
   Met Val Gly Leu Gln Thr Ala Cys Gly Gly Pro Ile Gln His Leu Trp
       130                 135                 140
   Gln Asn Thr Ala Ala Leu Pro Asn Gln Trp Glu Arg Asn Val Ile Lys
   145                 150                 155                 160
   Ile Gln Ser Ser Gln Arg Phe Gln Val Val Phe Glu Gly Gln Met Ala
                   165                 170                 175
   Ser Thr Tyr Glu Gln Asp Glu Val Ile Ala Ile Asp Asp
               180                 185
<210> 5
   <211> 598
   <212> DNA
   <213> Homo sapiens
<400> 5
   ttgcctgtgt tttcaattct acactggctc agcaagggac agaaagcttt cagtgtgaca   60
   atggagtctc cttgcctcct gacagcattt gtgacttcac agatcagtgt ggggatagca  120
   gtgatgaacg gcactgtttg aattatgaaa gatgtgattt tgaggatggt ctctgtcata  180
   tgactcaaga tcagagtctg caacctagtt ggacaaagag aagtgggatg attggtctat  240
   cacctccatt ttatgatcac aatggtgatg tgtctgctca cttcctctca ctggtttcca  300
   gagtggattc tatttcctca agtttaagaa gcagagtttt ccttccaaca aatgatcaac  360
   atgactgcca gattacattt tattacttct cctgccaagt gagtggcaaa ttaatggttg  420
   ggcttcaaac tgcatgtgga ggtcctattc agcatttatg gcaaaacaca gctgcactcc  480
   caaatcagtg ggagagaaat gtcatcaaaa tccagagttc acagagattt caggttgttt  540
   ttgaaggtca aatggcttca acgtatgaac aggatgaagt cattgctatt gatgatat    598
<210> 6
   <211> 198
   <212> PRT
   <213> Homo sapiens
<400> 6
   Ala Cys Val Phe Asn Ser Thr Leu Ala Gln Gln Gly Thr Glu Ser Phe
   1               5                   10                  15
   Gln Cys Asp Asn Gly Val Ser Leu Pro Pro Asp Ser Ile Cys Asp Phe
               20                  25                  30
   Thr Asp Gln Cys Gly Asp Ser Ser Asp Glu Arg His Cys Leu Asn Tyr
           35                  40                  45
   Glu Arg Cys Asp Phe Glu Asp Gly Leu Cys His Met Thr Gln Asp Gln
       50                  55                  60
   Ser Leu Gln Pro Ser Trp Thr Lys Arg Ser Gly Met Ile Gly Leu Ser
   65                  70                  75                  80
   Pro Pro Phe Tyr Asp His Asn Gly Asp Val Ser Ala His Phe Leu Ser
                   85                  90                  95
   Leu Val Ser Arg Val Asp Ser Ile Ser Ser Ser Leu Arg Ser Arg Val
               100                 105                 110
   Phe Leu Pro Thr Asn Asp Gln His Asp Cys Gln Ile Thr Phe Tyr Tyr
           115                 120                 125
   Phe Ser Cys Gln Val Ser Gly Lys Leu Met Val Gly Leu Gln Thr Ala
       130                 135                 140
   Cys Gly Gly Pro Ile Gln His Leu Trp Gln Asn Thr Ala Ala Leu Pro
   145                 150                 155                 160
   Asn Gln Trp Glu Arg Asn Val Ile Lys Ile Gln Ser Ser Gln Arg Phe
                   165                 170                 175
   Gln Val Val Phe Glu Gly Gln Met Ala Ser Thr Tyr Glu Gln Asp Glu
               180                 185                 190
   Val Ile Ala Ile Asp Asp
           195
<210> 7
   <211> 5997
   <212> DNA
   <213> Homo sapiens
<400> 7
   cagcaaggga cagaaagctt tcagtgtgac aatggagtct ccttgcctcc tgacagcatt 60
   tgtgacttca cagatcagtg tggggatagc agtgatgaac ggcactggct ttcatttgct 120
   catccttgca atccattggg gtctagtttg gaatctgaca actggaacaa aaagaaactt 180
   gaatccggtg catgccttgg ttttggtgct gctgctgctt ctcaagatcc tcggcaggaa 240
   ttaagaaaga agctggtgtg gacagcagat ccccgaaatt ggcgggctat tacattttat 300
   tacttctcct gccaagtgag tggcaaatta atggttgggc ttcaaactgc atgtggaggt 360
   cctattcagc atttatggca aaacacagct gcactcccaa atcagtggga gagaaatgtc 420
   atcaaaatcc agagttcaca gagatttcag gttgtttttg aaggtcaaat ggcttcaacg 480
   tatgaacagg atgaagtcat tgctattgat gatatatctt tcagttcagg ctgcttgcct 540
   gccaatgatg gaattttact gtgtcaagaa gcattgaatg ctgagcggga gctatgccat 600
   ccagatacag atctctgcag atttgatgct acagatgaag agttgagatt gtgtcaggcc 660
   tgtgggtttg aatttgacat gtgtgagtgg acgtcagaag catctgctgg ccaaatttcc 720
   tggatgcgca caaaagcgag agagatccct gcattcgaat ccacacctca gcaggatcaa 780
   ggaggtgatg atgaaggtta ttatgtatgg gtaggcgcta agcatggttt cactcttaac 840
   catttagaca gcagggctta cctaaatagc tctgtgtgtc attgcctggg caagagctgt 900
   catcttcaat tctattatgc aatggaaagc agtgtcctga gagtaagact gtataataat 960
   aaggaagaag aaatattttg gacatacaac atatcaactc acagccaatg ggtgaaagca 1020
   gatgtgttaa taccagaaga tctgaagaca tttaagaccc aatccagaaa gctttgctct 1080
   gcagacgaat tcccttgcac tagtggccag tgcatcgcca aagaatctgt ctgtgactct 1140
   cggcaggact gctccgatga gagtgatgaa gacccagcaa cttgctcaaa gcatctcacc 1200
   tgtgactttg agtcgggttt ctgcggttgg gagccatttc tcacagaaga ttcacactgg 1260
   aagctgatga aaggattgaa taatggagag caccactttc ctgcagctga tcacacagca 1320
   aacataaatc atggatcgtt tatttatttg gaggcacagc gctcccccgg ggtggccaag 1380
   cttggaagtc ctgttcttac aaaattgctc actgcctcta ccccatgtca ggtgcagttt 1440
   tggtatcatt tgtctcaaca ttcaaatctc tcagttttta caagaacgtc tctagatgga 1500
   aacttgcaaa agcagggcaa aataatcaga ttctccgaat ctcagtggag ccacgcaaaa 1560
   attgatctca ttgcagaagc gggagaatct actctacctt ttcagttaat tttggaagct 1620
   actgttttgt cgtcaaatgc taccgttgct ctagatgaca tcagtgtgtc ccaggaatgt 1680
   gaaatttcct ataaatcact accaaggacc agtacacaaa gcaagttttc caagtgtgac 1740
   tttgaagcaa acagctgtga ttggtttgaa gcaattagtg gtgaccattt tgactggata 1800
   cggagctctc agagtgaact ttctgctgat tttgagcacc aggctccacc tcgggatcat 1860
   agtctcaacg catctcaagg gcattttatg ttcattctga agaaaagcag cagcttgtgg 1920
   caagttgcta agcttcagag cccaactttc agccagacag gacctggatg catactttcc 1980
   ttctggttct ataactatgg cctgtcagtg ggagcagctg agctgcagct acatatggaa 2040
   aattctcatg actcaacagt gatttggaga gtattataca atcagggcaa acaatggttg 2100
   gaggcaacca ttcagctagg gcgcctttcg cagcccttcc atttgtcact agataaagtc 2160
   agtctgggca tttatgatgg ggtctcagct attgatgaca tccgatttga aaattgtact 2220
   ctccctcttc ctgctgagag ctgtgaaggg ctggatcatt tctggtgtcg ccacaccagg 2280
   gcttgcatag aaaagcttcg gttatgtgat ctggtggatg actgtggtga tcgtactgat 2340
   gaagtcaact gtgcacctga gctgcagtgt aactttgaaa ctggaatctg taactgggaa 2400
   caagatgcaa aagatgactt tgattggacc aggagccagg gtccaactcc aacacttaac 2460
   acagggccaa tgaaagataa cactctgggc acagctaaag gacactatct ctacatagaa 2520
   tcttcagagc cacaggcttt tcaagacagt gctgccttac tcagcccaat ccttaatgcc 2580
   actgatacaa aaggctgcac cttccgcttc tattaccaca tgtttggaaa gcgcatttat 2640
   aggttggcaa tctaccaacg aatctggagt gactcaaggg gacagctgct gtggcagata 2700
   tttgggaatc aaggcaacag atggattagg aaacacctca acatttccag caggcagccc 2760
   tttcagatat tggtggaggc ttcagtggga gatggcttca ctggagatat tgcgattgat 2820
   gatctgtcat ttatggactg caccctctac cctggtaatt tgccagcaga cctcccaact 2880
   ccaccagaaa cgtcagttcc tgtaacatta cctccacaca actgcacaga caatgaattt 2940
   atctgcaggt ctgatggtca ctgcattgaa aaaatgcaga aatgtgattt taaatatgac 3000
   tgccctgaca aatcagatga agcatcctgt gttatggaag tttgcagctt tgagaaaaga 3060
   agcctgtgta aatggtatca accaatccca gtacatttgc ttcaagattc aaacacattc 3120
   aggtgggggc ttgggaacgg gatcagcatt catcatgggg aagaaaacca caggccatca 3180
   gtggatcata cacaaaatac cactgatggc tggtacctgt atgctgacag ttctaatggg 3240
   aaatttggtg acacggctga cattctcact cctatcattt cactcacggg accaaaatgt 3300
   accttggtgt tctggacaca tatgaatggg gccaccgttg gttctctcca ggtgctcatc 3360
   aagaaagata acgttacttc taaattgtgg gctcaaactg gacagcaagg tgcacagtgg 3420
   aagagagcag aagtgttttt aggcattcgt tcacatacac agattgtctt cagagccaaa 3480
   cgtggtatca gttacatagg agatgtagca gtggatgata tttccttcca agattgctcc 3540
   cctttgctta gcccagagag aaagtgtact gatcatgaat tcatgtgtgc taataagcac 3600
   tgcattgcca aagacaagct gtgtgatttt gtgaatgatt gtgctgataa ttcagatgag 3660
   actactttca tttgccgtac ctccagtggg cgctgtgatt tcgaatttga tctttgttcc 3720
   tggaagcagg agaaagatga ggactttgac tggaacctga aagctagcag catccctgca 3780
   gcaggcacag agccagcagc agatcacact ttgggaaatt catctggtca ttacatcttt 3840
   ataaagagtt tgtttcctca gcagcccatg agagctgcca gaatttcaag tccagttata 3900
   agtaagagaa gcaaaaactg caagattatt tttcattatc acatgtatgg aaatggcatt 3960
   ggggcactca ccttaatgca ggtgtcagtc acaaaccaaa cgaaggttct acttaacctc 4020
   actgtagaac aaggcaattt ctggcggaga gaagaactgt cactgtttgg tgatgaagac 4080
   ttccaactca aatttgaagg tagagttggg aaaggtcagc gtggagacat tgcacttgat 4140
   gacattgtgc ttacagaaaa ttgtctatca ctccatgatt ccgtgcaaga agaactggca 4200
   gtgcctcttc caacaggttt ctgcccactt ggctataggg aatgtcataa tggaaaatgc 4260
   tataggctgg aacaaagctg taacttcgta gataactgtg gagataatac tgatgaaaat 4320
   gagtgtggta gctcctgtac ttttgaaaaa ggctggtgtg gctggcaaaa ctcccaggct 4380
   gacaactttg attgggtttt aggggttggc tctcatcaaa gcttaagacc tcccaaagac 4440
   cacacacttg gaaatgaaaa tgggcacttc atgtatctgg aagctactgc agtgggcctt 4500
   cggggtgaca aagcacactt caggagtacc atgtggcgag aatccagtgc agcctgcacc 4560
   atgagcttct ggtatttcgt atctgcaaag gccacaggat ccattcagat tctcatcaag 4620
   acagagaaag gactatcaaa agtatggcaa gaaagtaagc agaaccctgg taatcattgg 4680
   caaaaggctg acatcctgct aggaaagtta aggaattttg aagtcatatt tcaaggtatc 4740
   agaacaaggg acctgggagg aggagctgca attgatgata ttgaatttaa aaactgcaca 4800
   actgtgggag agatctctga gctttgtccg gaaatcactg attttttgtg ccgggacaag 4860
   aagtgcattg catcccacct tctttgtgac tataagccag actgctctga taggtctgat 4920
   gaagctcact gtgcacatta tacaagcaca acaggaagct gcaattttga aacaagttca 4980
   ggaaactgga ccacagcctg cagtcttact caagactctg aggatgactt ggactgggcc 5040
   attggcagca gaattcctgc caaagcatta attccagact ctgatcacac gccaggtagt 5100
   ggtcagcact tcctgtacgt caactcatct ggctccaagg aaggatccgt tgccagaatt 5160
   actacttcca aatccttccc agcaagcctt ggaatgtgta ctgttcggtt ctggttctac 5220
   atgattgatc ccaggagtat gggaatatta aaggtgtata ccattgaaga atcggggcta 5280
   aacatcctgg tgtggtcagt gattggaaat aaaagaacgg gatggacata tggctctgtg 5340
   cctctctcca gtaacagtcc gtttaaggtg gcatttgaag ctgatttgga tggaaatgag 5400
   gacatcttta ttgctcttga tgacatctct tttaccccag agtgtgtgac tggaggtcct 5460
   gtcccagtgc agccatcacc ctgtgaagct gatcagtttt cttgtatcta cacactccaa 5520
   tgtgtccctc tctcagggaa atgtgatgga catgaagact gcatagatgg atctgatgaa 5580
   atggattgtc ctctcagccc cacccctcca ctctgtagta acatggagtt cccgtgctct 5640
   acagacgagt gtataccttc cctcctgcta tgcgatggag tgcccgactg ccactttaat 5700
   gaagatgagc tcatctgctc caacaaaagc tgttctaatg gagctctggt gtgtgcctcc 5760
   tccaacagct gtatcccagc ccaccagcgc tgtgatggtt ttgccgactg catggatttc 5820
   cagcttgatg agtccagctg ctccgaatgt ccattaaatt actgcagaaa tggtgggact 5880
   tgtgtagtgg agaaaaatgg tcctatgtgt cgatgtagac aaggctggaa aggaaatcga 5940
   tgccatatca agtttaatcc tcctgctaca gacttcacat acgctcagaa taataca 5997
<210> 8
   <211> 1999
   <212> PRT
   <213> Homo sapiens
<400> 8
   Gln Gln Gly Thr Glu Ser Phe Gln Cys Asp Asn Gly Val Ser Leu Pro
   1               5                   10                  15
   Pro Asp Ser Ile Cys Asp Phe Thr Asp Gln Cys Gly Asp Ser Ser Asp
               20                  25                  30
   Glu Arg His Trp Leu Ser Phe Ala His Pro Cys Asn Pro Leu Gly Ser
           35                  40                  45
   Ser Leu Glu Ser Asp Asn Trp Asn Lys Lys Lys Leu Glu Ser Gly Ala
       50                  55                  60
   Cys Leu Gly Phe Gly Ala Ala Ala Ala Ser Gln Asp Pro Arg Gln Glu
   65                  70                  75                  80
   Leu Arg Lys Lys Leu Val Trp Thr Ala Asp Pro Arg Asn Trp Arg Ala
                   85                  90                  95
   Ile Thr Phe Tyr Tyr Phe Ser Cys Gln Val Ser Gly Lys Leu Met Val
               100                 105                 110
   Gly Leu Gln Thr Ala Cys Gly Gly Pro Ile Gln His Leu Trp Gln Asn
           115                 120                 125
   Thr Ala Ala Leu Pro Asn Gln Trp Glu Arg Asn Val Ile Lys Ile Gln
       130                 135                 140
   Ser Ser Gln Arg Phe Gln Val Val Phe Glu Gly Gln Met Ala Ser Thr
   145                 150                 155                 160
   Tyr Glu Gln Asp Glu Val Ile Ala Ile Asp Asp Ile Ser Phe Ser Ser
                   165                 170                 175
   Gly Cys Leu Pro Ala Asn Asp Gly Ile Leu Leu Cys Gln Glu Ala Leu
               180                 185                 190
   Asn Ala Glu Arg Glu Leu Cys His Pro Asp Thr Asp Leu Cys Arg Phe
           195                 200                 205
   Asp Ala Thr Asp Glu Glu Leu Arg Leu Cys Gln Ala Cys Gly Phe Glu
       210                 215                 220
   Phe Asp Met Cys Glu Trp Thr Ser Glu Ala Ser Ala Gly Gln Ile Ser
   225                 230                 235                 240
   Trp Met Arg Thr Lys Ala Arg Glu Ile Pro Ala Phe Glu Ser Thr Pro
                   245                 250                 255
   Gln Gln Asp Gln Gly Gly Asp Asp Glu Gly Tyr Tyr Val Trp Val Gly
               260                 265                 270
   Ala Lys His Gly Phe Thr Leu Asn His Leu Asp Ser Arg Ala Tyr Leu
           275                 280                 285
   Asn Ser Ser Val Cys His Cys Leu Gly Lys Ser Cys His Leu Gln Phe
       290                 295                 300
   Tyr Tyr Ala Met Glu Ser Ser Val Leu Arg Val Arg Leu Tyr Asn Asn
   305                 310                 315                 320
   Lys Glu Glu Glu Ile Phe Trp Thr Tyr Asn Ile Ser Thr His Ser Gln
                   325                 330                 335
   Trp Val Lys Ala Asp Val Leu Ile Pro Glu Asp Leu Lys Thr Phe Lys
               340                 345                 350
   Thr Gln Ser Arg Lys Leu Cys Ser Ala Asp Glu Phe Pro Cys Thr Ser
           355                 360                 365
   Gly Gln Cys Ile Ala Lys Glu Ser Val Cys Asp Ser Arg Gln Asp Cys
       370                 375                 380
   Ser Asp Glu Ser Asp Glu Asp Pro Ala Thr Cys Ser Lys His Leu Thr
   385                 390                 395                 400
   Cys Asp Phe Glu Ser Gly Phe Cys Gly Trp Glu Pro Phe Leu Thr Glu
                   405                 410                 415
   Asp Ser His Trp Lys Leu Met Lys Gly Leu Asn Asn Gly Glu His His
               420                 425                 430
   Phe Pro Ala Ala Asp His Thr Ala Asn Ile Asn His Gly Ser Phe Ile
           435                 440                 445
   Tyr Leu Glu Ala Gln Arg Ser Pro Gly Val Ala Lys Leu Gly Ser Pro
       450                 455                 460
   Val Leu Thr Lys Leu Leu Thr Ala Ser Thr Pro Cys Gln Val Gln Phe
   465                 470                 475                 480
   Trp Tyr His Leu Ser Gln His Ser Asn Leu Ser Val Phe Thr Arg Thr
                   485                 490                 495
   Ser Leu Asp Gly Asn Leu Gln Lys Gln Gly Lys Ile Ile Arg Phe Ser
               500                 505                 510
   Glu Ser Gln Trp Ser His Ala Lys Ile Asp Leu Ile Ala Glu Ala Gly
           515                 520                 525
   Glu Ser Thr Leu Pro Phe Gln Leu Ile Leu Glu Ala Thr Val Leu Ser
       530                 535                 540
   Ser Asn Ala Thr Val Ala Leu Asp Asp Ile Ser Val Ser Gln Glu Cys
   545                 550                 555                 560
   Glu Ile Ser Tyr Lys Ser Leu Pro Arg Thr Ser Thr Gln Ser Lys Phe
                   565                 570                 575
   Ser Lys Cys Asp Phe Glu Ala Asn Ser Cys Asp Trp Phe Glu Ala Ile
               580                 585                 590
   Ser Gly Asp His Phe Asp Trp Ile Arg Ser Ser Gln Ser Glu Leu Ser
           595                 600                 605
   Ala Asp Phe Glu His Gln Ala Pro Pro Arg Asp His Ser Leu Asn Ala
       610                 615                 620
   Ser Gln Gly His Phe Met Phe Ile Leu Lys Lys Ser Ser Ser Leu Trp
   625                 630                 635                 640
   Gln Val Ala Lys Leu Gln Ser Pro Thr Phe Ser Gln Thr Gly Pro Gly
                   645                 650                 655
   Cys Ile Leu Ser Phe Trp Phe Tyr Asn Tyr Gly Leu Ser Val Gly Ala
   660 665 670
   Ala Glu Leu Gln Leu His Met Glu Asn Ser His Asp Ser Thr Val Ile
   675 680 685
   Trp Arg Val Leu Tyr Asn Gln Gly Lys Gln Trp Leu Glu Ala Thr Ile
   690 695 700
   Gln Leu Gly Arg Leu Ser Gln Pro Phe His Leu Ser Leu Asp Lys Val
   705 710 715 720
   Ser Leu Gly Ile Tyr Asp Gly Val Ser Ala Ile Asp Asp Ile Arg Phe
   725 730 735
   Glu Asn Cys Thr Leu Pro Leu Pro Ala Glu Ser Cys Glu Gly Leu Asp
   740 745 750
   His Phe Trp Cys Arg His Thr Arg Ala Cys Ile Glu Lys Leu Arg Leu
   755 760 765
   Cys Asp Leu Val Asp Asp Cys Gly Asp Arg Thr Asp Glu Val Asn Cys
   770 775 780
   Ala Pro Glu Leu Gln Cys Asn Phe Glu Thr Gly Ile Cys Asn Trp Glu
   785 790 795 800
   Gln Asp Ala Lys Asp Asp Phe Asp Trp Thr Arg Ser Gln Gly Pro Thr
   805 810 815
   Pro Thr Leu Asn Thr Gly Pro Met Lys Asp Asn Thr Leu Gly Thr Ala
   820 825 830
   Lys Gly His Tyr Leu Tyr Ile Glu Ser Ser Glu Pro Gln Ala Phe Gln
   835 840 845
   Asp Ser Ala Ala Leu Leu Ser Pro Ile Leu Asn Ala Thr Asp Thr Lys
   850 855 860
   Gly Cys Thr Phe Arg Phe Tyr Tyr His Met Phe Gly Lys Arg Ile Tyr
   865 870 875 880
   Arg Leu Ala Ile Tyr Gln Arg Ile Trp Ser Asp Ser Arg Gly Gln Leu
   885 890 895
   Leu Trp Gln Ile Phe Gly Asn Gln Gly Asn Arg Trp Ile Arg Lys His
   900 905 910
   Leu Asn Ile Ser Ser Arg Gln Pro Phe Gln Ile Leu Val Glu Ala Ser
   915 920 925
   Val Gly Asp Gly Phe Thr Gly Asp Ile Ala Ile Asp Asp Leu Ser Phe
   930 935 940
   Met Asp Cys Thr Leu Tyr Pro Gly Asn Leu Pro Ala Asp Leu Pro Thr
   945 950 955 960
   Pro Pro Glu Thr Ser Val Pro Val Thr Leu Pro Pro His Asn Cys Thr
   965 970 975
   Asp Asn Glu Phe Ile Cys Arg Ser Asp Gly His Cys Ile Glu Lys Met
   980 985 990
   Gln Lys Cys Asp Phe Lys Tyr Asp Cys Pro Asp Lys Ser Asp Glu Ala
   995 1000 1005
   Ser Cys Val Met Glu Val Cys Ser Phe Glu Lys Arg Ser Leu Cys Lys
   1010 1015 1020
   Trp Tyr Gln Pro Ile Pro Val His Leu Leu Gln Asp Ser Asn Thr Phe
   1025 1030 1035 1040
   Arg Trp Gly Leu Gly Asn Gly Ile Ser Ile His His Gly Glu Glu Asn
   1045 1050 1055
   His Arg Pro Ser Val Asp His Thr Gln Asn Thr Thr Asp Gly Trp Tyr
   1060 1065 1070
   Leu Tyr Ala Asp Ser Ser Asn Gly Lys Phe Gly Asp Thr Ala Asp Ile
   1075 1080 1085
   Leu Thr Pro Ile Ile Ser Leu Thr Gly Pro Lys Cys Thr Leu Val Phe
   1090 1095 1100
   Trp Thr His Met Asn Gly Ala Thr Val Gly Ser Leu Gln Val Leu Ile
   1105 1110 1115 1120
   Lys Lys Asp Asn Val Thr Ser Lys Leu Trp Ala Gln Thr Gly Gln Gln
   1125 1130 1135
   Gly Ala Gln Trp Lys Arg Ala Glu Val Phe Leu Gly Ile Arg Ser His
   1140 1145 1150
   Thr Gln Ile Val Phe Arg Ala Lys Arg Gly Ile Ser Tyr Ile Gly Asp
   1155 1160 1165
   Val Ala Val Asp Asp Ile Ser Phe Gln Asp Cys Ser Pro Leu Leu Ser
   1170 1175 1180
   Pro Glu Arg Lys Cys Thr Asp His Glu Phe Met Cys Ala Asn Lys His
   1185 1190 1195 1200
   Cys Ile Ala Lys Asp Lys Leu Cys Asp Phe Val Asn Asp Cys Ala Asp
   1205 1210 1215
   Asn Ser Asp Glu Thr Thr Phe Ile Cys Arg Thr Ser Ser Gly Arg Cys
   1220 1225 1230
   Asp Phe Glu Phe Asp Leu Cys Ser Trp Lys Gln Glu Lys Asp Glu Asp
   1235 1240 1245
   Phe Asp Trp Asn Leu Lys Ala Ser Ser Ile Pro Ala Ala Gly Thr Glu
   1250 1255 1260
   Pro Ala Ala Asp His Thr Leu Gly Asn Ser Ser Gly His Tyr Ile Phe
   1265 1270 1275 1280
   Ile Lys Ser Leu Phe Pro Gln Gln Pro Met Arg Ala Ala Arg Ile Ser
   1285 1290 1295
   Ser Pro Val Ile Ser Lys Arg Ser Lys Asn Cys Lys Ile Ile Phe His
   1300 1305 1310
   Tyr His Met Tyr Gly Asn Gly Ile Gly Ala Leu Thr Leu Met Gln Val
   1315 1320 1325
   Ser Val Thr Asn Gln Thr Lys Val Leu Leu Asn Leu Thr Val Glu Gln
   1330 1335 1340
   Gly Asn Phe Trp Arg Arg Glu Glu Leu Ser Leu Phe Gly Asp Glu Asp
   1345 1350 1355 1360
   Phe Gln Leu Lys Phe Glu Gly Arg Val Gly Lys Gly Gln Arg Gly Asp
   1365 1370 1375
   Ile Ala Leu Asp Asp Ile Val Leu Thr Glu Asn Cys Leu Ser Leu His
   1380 1385 1390
   Asp Ser Val Gln Glu Glu Leu Ala Val Pro Leu Pro Thr Gly Phe Cys
   1395 1400 1405
   Pro Leu Gly Tyr Arg Glu Cys His Asn Gly Lys Cys Tyr Arg Leu Glu
   1410 1415 1420
   Gln Ser Cys Asn Phe Val Asp Asn Cys Gly Asp Asn Thr Asp Glu Asn
   1425 1430 1435 1440
   Glu Cys Gly Ser Ser Cys Thr Phe Glu Lys Gly Trp Cys Gly Trp Gln
   1445 1450 1455
   Asn Ser Gln Ala Asp Asn Phe Asp Trp Val Leu Gly Val Gly Ser His
   1460 1465 1470
   Gln Ser Leu Arg Pro Pro Lys Asp His Thr Leu Gly Asn Glu Asn Gly
   1475 1480 1485
   His Phe Met Tyr Leu Glu Ala Thr Ala Val Gly Leu Arg Gly Asp Lys
   1490 1495 1500
   Ala His Phe Arg Ser Thr Met Trp Arg Glu Ser Ser Ala Ala Cys Thr
   1505 1510 1515 1520
   Met Ser Phe Trp Tyr Phe Val Ser Ala Lys Ala Thr Gly Ser Ile Gln
   1525 1530 1535
   Ile Leu Ile Lys Thr Glu Lys Gly Leu Ser Lys Val Trp Gln Glu Ser
   1540 1545 1550
   Lys Gln Asn Pro Gly Asn His Trp Gln Lys Ala Asp Ile Leu Leu Gly
   1555 1560 1565
   Lys Leu Arg Asn Phe Glu Val Ile Phe Gln Gly Ile Arg Thr Arg Asp
   1570 1575 1580
   Leu Gly Gly Gly Ala Ala Ile Asp Asp Ile Glu Phe Lys Asn Cys Thr
   1585 1590 1595 1600
   Thr Val Gly Glu Ile Ser Glu Leu Cys Pro Glu Ile Thr Asp Phe Leu
   1605 1610 1615
   Cys Arg Asp Lys Lys Cys Ile Ala Ser His Leu Leu Cys Asp Tyr Lys
   1620 1625 1630
   Pro Asp Cys Ser Asp Arg Ser Asp Glu Ala His Cys Ala His Tyr Thr
   1635 1640 1645
   Ser Thr Thr Gly Ser Cys Asn Phe Glu Thr Ser Ser Gly Asn Trp Thr
   1650 1655 1660
   Thr Ala Cys Ser Leu Thr Gln Asp Ser Glu Asp Asp Leu Asp Trp Ala
   1665 1670 1675 1680
   Ile Gly Ser Arg Ile Pro Ala Lys Ala Leu Ile Pro Asp Ser Asp His
   1685 1690 1695
   Thr Pro Gly Ser Gly Gln His Phe Leu Tyr Val Asn Ser Ser Gly Ser
   1700 1705 1710
   Lys Glu Gly Ser Val Ala Arg Ile Thr Thr Ser Lys Ser Phe Pro Ala
   1715 1720 1725
   Ser Leu Gly Met Cys Thr Val Arg Phe Trp Phe Tyr Met Ile Asp Pro
   1730 1735 1740
   Arg Ser Met Gly Ile Leu Lys Val Tyr Thr Ile Glu Glu Ser Gly Leu
   1745 1750 1755 1760
   Asn Ile Leu Val Trp Ser Val Ile Gly Asn Lys Arg Thr Gly Trp Thr
   1765 1770 1775
   Tyr Gly Ser Val Pro Leu Ser Ser Asn Ser Pro Phe Lys Val Ala Phe
   1780 1785 1790
   Glu Ala Asp Leu Asp Gly Asn Glu Asp Ile Phe Ile Ala Leu Asp Asp
   1795 1800 1805
   Ile Ser Phe Thr Pro Glu Cys Val Thr Gly Gly Pro Val Pro Val Gln
   1810 1815 1820
   Pro Ser Pro Cys Glu Ala Asp Gln Phe Ser Cys Ile Tyr Thr Leu Gln
   1825 1830 1835 1840
   Cys Val Pro Leu Ser Gly Lys Cys Asp Gly His Glu Asp Cys Ile Asp
   1845 1850 1855
   Gly Ser Asp Glu Met Asp Cys Pro Leu Ser Pro Thr Pro Pro Leu Cys
   1860 1865 1870
   Ser Asn Met Glu Phe Pro Cys Ser Thr Asp Glu Cys Ile Pro Ser Leu
   1875 1880 1885
   Leu Leu Cys Asp Gly Val Pro Asp Cys His Phe Asn Glu Asp Glu Leu
   1890 1895 1900
   Ile Cys Ser Asn Lys Ser Cys Ser Asn Gly Ala Leu Val Cys Ala Ser
   1905 1910 1915 1920
   Ser Asn Ser Cys Ile Pro Ala His Gln Arg Cys Asp Gly Phe Ala Asp
   1925 1930 1935
   Cys Met Asp Phe Gln Leu Asp Glu Ser Ser Cys Ser Glu Cys Pro Leu
   1940 1945 1950
   Asn Tyr Cys Arg Asn Gly Gly Thr Cys Val Val Glu Lys Asn Gly Pro
   1955 1960 1965
   Met Cys Arg Cys Arg Gln Gly Trp Lys Gly Asn Arg Cys His Ile Lys
   1970 1975 1980
   Phe Asn Pro Pro Ala Thr Asp Phe Thr Tyr Ala Gln Asn Asn Thr
   1985 1990 1995
<210> 9
   <211> 6051
   <212> DNA
   <213> Homo sapiens
<400> 9
   cagcaaggga cagaaagctt tcagtgtgac aatggagtct ccttgcctcc tgacagcatt   60
   tgtgacttca cagatcagtg tggggatagc agtgatgaac ggcactgttt gaattatgaa  120
   agatgtgatt ttgaggatgg tctctgtcat atgactcaag atcagagtct gcaacctagt  180
   tggacaaaga gaagtgggat gattggtcta tcacctccat tttatgatca caatggtgat  240
   gtgtctgctc acttcctctc actggtttcc agagtggatt ctatttcctc aagtttaaga  300
   agcagagttt tccttccaac aaatgatcaa catgactgcc agattacatt ttattacttc  360
   tcctgccaag tgagtggcaa attaatggtt gggcttcaaa ctgcatgtgg aggtcctatt  420
   cagcatttat ggcaaaacac agctgcactc ccaaatcagt gggagagaaa tgtcatcaaa  480
   atccagagtt cacagagatt tcaggttgtt tttgaaggtc aaatggcttc aacgtatgaa  540
   caggatgaag tcattgctat tgatgatata tctttcagtt caggctgctt gcctgccaat  600
   gatggaattt tactgtgtca agaagcattg aatgctgagc gggagctatg ccatccagat  660
   acagatctct gcagatttga tgctacagat gaagagttga gattgtgtca ggcctgtggg  720
   tttgaatttg acatgtgtga gtggacgtca gaagcatctg ctggccaaat ttcctggatg  780
   cgcacaaaag cgagagagat ccctgcattc gaatccacac ctcagcagga tcaaggaggt  840
   gatgatgaag gttattatgt atgggtaggc gctaagcatg gtttcactct taaccattta  900
   gacagcaggg cttacctaaa tagctctgtg tgtcattgcc tgggcaagag ctgtcatctt  960
   caattctatt atgcaatgga aagcagtgtc ctgagagtaa gactgtataa taataaggaa 1020
   gaagaaatat tttggacata caacatatca actcacagcc aatgggtgaa agcagatgtg 1080
   ttaataccag aagatctgaa gacatttaag acccaatcca gaaagctttg ctctgcagac 1140
   gaattccctt gcactagtgg ccagtgcatc gccaaagaat ctgtctgtga ctctcggcag 1200
   gactgctccg atgagagtga tgaagaccca gcaacttgct caaagcatct cacctgtgac 1260
   tttgagtcgg gtttctgcgg ttgggagcca tttctcacag aagattcaca ctggaagctg 1320
   atgaaaggat tgaataatgg agagcaccac tttcctgcag ctgatcacac agcaaacata 1380
   aatcatggat cgtttattta tttggaggca cagcgctccc ccggggtggc caagcttgga 1440
   agtcctgttc ttacaaaatt gctcactgcc tctaccccat gtcaggtgca gttttggtat 1500
   catttgtctc aacattcaaa tctctcagtt tttacaagaa cgtctctaga tggaaacttg 1560
   caaaagcagg gcaaaataat cagattctcc gaatctcagt ggagccacgc aaaaattgat 1620
   ctcattgcag aagcgggaga atctactcta ccttttcagt taattttgga agctactgtt 1680
   ttgtcgtcaa atgctaccgt tgctctagat gacatcagtg tgtcccagga atgtgaaatt 1740
   tcctataaat cactaccaag gaccagtaca caaagcaagt tttccaagtg tgactttgaa 1800
   gcaaacagct gtgattggtt tgaagcaatt agtggtgacc attttgactg gatacggagc 1860
   tctcagagtg aactttctgc tgattttgag caccaggctc cacctcggga tcatagtctc 1920
   aacgcatctc aagggcattt tatgttcatt ctgaagaaaa gcagcagctt gtggcaagtt 1980
   gctaagcttc agagcccaac tttcagccag acaggacctg gatgcatact ttccttctgg 2040
   ttctataact atggcctgtc agtgggagca gctgagctgc agctacatat ggaaaattct 2100
   catgactcaa cagtgatttg gagagtatta tacaatcagg gcaaacaatg gttggaggca 2160
   accattcagc tagggcgcct ttcgcagccc ttccatttgt cactagataa agtcagtctg 2220
   ggcatttatg atggggtctc agctattgat gacatccgat ttgaaaattg tactctccct 2280
   cttcctgctg agagctgtga agggctggat catttctggt gtcgccacac cagggcttgc 2340
   atagaaaagc ttcggttatg tgatctggtg gatgactgtg gtgatcgtac tgatgaagtc 2400
   aactgtgcac ctgagctgca gtgtaacttt gaaactggaa tctgtaactg ggaacaagat 2460
   gcaaaagatg actttgattg gaccaggagc cagggtccaa ctccaacact taacacaggg 2520
   ccaatgaaag ataacactct gggcacagct aaaggacact atctctacat agaatcttca 2580
   gagccacagg cttttcaaga cagtgctgcc ttactcagcc caatccttaa tgccactgat 2640
   acaaaaggct gcaccttccg cttctattac cacatgtttg gaaagcgcat ttataggttg 2700
   gcaatctacc aacgaatctg gagtgactca aggggacagc tgctgtggca gatatttggg 2760
   aatcaaggca acagatggat taggaaacac ctcaacattt ccagcaggca gccctttcag 2820
   atattggtgg aggcttcagt gggagatggc ttcactggag atattgcgat tgatgatctg 2880
   tcatttatgg actgcaccct ctaccctggt aatttgccag cagacctccc aactccacca 2940
   gaaacgtcag ttcctgtaac attacctcca cacaactgca cagacaatga atttatctgc 3000
   aggtctgatg gtcactgcat tgaaaaaatg cagaaatgtg attttaaata tgactgccct 3060
   gacaaatcag atgaagcatc ctgtgttatg gaagtttgca gctttgagaa aagaagcctg 3120
   tgtaaatggt atcaaccaat cccagtacat ttgcttcaag attcaaacac attcaggtgg 3180
   gggcttggga acgggatcag cattcatcat ggggaagaaa accacaggcc atcagtggat 3240
   catacacaaa ataccactga tggctggtac ctgtatgctg acagttctaa tgggaaattt 3300
   ggtgacacgg ctgacattct cactcctatc atttcactca cgggaccaaa atgtaccttg 3360
   gtgttctgga cacatatgaa tggggccacc gttggttctc tccaggtgct catcaagaaa 3420
   gataacgtta cttctaaatt gtgggctcaa actggacagc aaggtgcaca gtggaagaga 3480
   gcagaagtgt ttttaggcat tcgttcacat acacagattg tcttcagagc caaacgtggt 3540
   atcagttaca taggagatgt agcagtggat gatatttcct tccaagattg ctcccctttg 3600
   cttagcccag agagaaagtg tactgatcat gaattcatgt gtgctaataa gcactgcatt 3660
   gccaaagaca agctgtgtga ttttgtgaat gattgtgctg ataattcaga tgagactact 3720
   ttcatttgcc gtacctccag tgggcgctgt gatttcgaat ttgatctttg ttcctggaag 3780
   caggagaaag atgaggactt tgactggaac ctgaaagcta gcagcatccc tgcagcaggc 3840
   acagagccag cagcagatca cactttggga aattcatctg gtcattacat ctttataaag 3900
   agtttgtttc ctcagcagcc catgagagct gccagaattt caagtccagt tataagtaag 3960
   agaagcaaaa actgcaagat tatttttcat tatcacatgt atggaaatgg cattggggca 4020
   ctcaccttaa tgcaggtgtc agtcacaaac caaacgaagg ttctacttaa cctcactgta 4080
   gaacaaggca atttctggcg gagagaagaa ctgtcactgt ttggtgatga agacttccaa 4140
   ctcaaatttg aaggtagagt tgggaaaggt cagcgtggag acattgcact tgatgacatt 4200
   gtgcttacag aaaattgtct atcactccat gattccgtgc aagaagaact ggcagtgcct 4260
   cttccaacag gtttctgccc acttggctat agggaatgtc ataatggaaa atgctatagg 4320
   ctggaacaaa gctgtaactt cgtagataac tgtggagata atactgatga aaatgagtgt 4380
   ggtagctcct gtactttgta aaaaggctgg tgtggctggc aaaactccca ggctgacaac 4440
   tttgattggg ttttaggggt tggctctcat caaagcttaa gacctcccaa agaccacaca 4500
   cttggaaatg aaaatgggca cttcatgtat ctggaagcta ctgcagtggg ccttcggggt 4560
   gacaaagcac acttcaggag taccatgtgg cgagaatcca gtgcagcctg caccatgagc 4620
   ttctggtatt tcgtatctgc aaaggccaca ggatccattc agattctcat caagacagag 4680
   aaaggactat caaaagtatg gcaagaaagt aagcagaacc ctggtaatca ttggcaaaag 4740
   gctgacatcc tgctaggaaa gttaaggaat tttgaagtca tatttcaagg tatcagaaca 4800
   agggacctgg gaggaggagc tgcaattgat gatattgaat ttaaaaactg cacaactgtg 4860
   ggagagatct ctgagctttg tccggaaatc actgattttt tgtgccggga caagaagtgc 4920
   attgcatccc accttctttg tgactataag ccagactgct ctgataggtc tgatgaagct 4980
   cactgtgcac attatacaag cacaacagga agctgcaatt ttgaaacaag ttcaggaaac 5040
   tggaccacag cctgcagtct tactcaagac tctgaggatg acttggactg ggccattggc 5100
   agcagaattc ctgccaaagc attaattcca gactctgatc acacgccagg tagtggtcag 5160
   cacttcctgt acgtcaactc atctggctcc aaggaaggat ccgttgccag aattactact 5220
   tccaaatcct tcccagcaag ccttggaatg tgtactgttc ggttctggtt ctacatgatt 5280
   gatcccagga gtatgggaat attaaaggtg tataccattg aagaatcggg gctaaacatc 5340
   ctggtgtggt cagtgattgg aaataaaaga acgggatgga catatggctc tgtgcctctc 5400
   tccagtaaca gtccgtttaa ggtggcattt gaagctgatt tggatggaaa tgaggacatc 5460
   tttattgctc ttgatgacat ctcttttacc ccagagtgtg tgactggagg tcctgtccca 5520
   gtgcagccat caccctgtga agctgatcag ttttcttgta tctacacact ccaatgtgtc 5580
   cctctctcag ggaaatgtga tggacatgaa gactgcatag atggatctga tgaaatggat 5640
   tgtcctctca gccccacccc tccactctgt agtaacatgg agttcccgtg ctctacagac 5700
   gagtgtatac cttccctcct gctatgcgat ggagtgcccg actgccactt taatgaagat   5760
   gagctcatct gctccaacaa aagctgttct aatggagctc tggtgtgtgc ctcctccaac   5820
   agctgtatcc cagcccacca gcgctgtgat ggttttgccg actgcatgga tttccagctt   5880
   gatgagtcca gctgctccga atgtccatta aattactgca gaaatggtgg gacttgtgta   5940
   gtggagaaaa atggtcctat gtgtcgatgt agacaaggct ggaaaggaaa tcgatgccat   6000
   atcaagttta atcctcctgc tacagacttc acatacgctc agaataatac a            6051
<210> 10
   <211> 2017
   <212> PRT
   <213> Homo sapiens
<400> 10
   Gln Gln Gly Thr Glu Ser Phe Gln Cys Asp Asn Gly Val Ser Leu Pro
   1               5                   10                  15
   Pro Asp Ser Ile Cys Asp Phe Thr Asp Gln Cys Gly Asp Ser Ser Asp
               20                  25                  30
   Glu Arg His Cys Leu Asn Tyr Glu Arg Cys Asp Phe Glu Asp Gly Leu
           35                  40                  45
   Cys His Met Thr Gln Asp Gln Ser Leu Gln Pro Ser Trp Thr Lys Arg
       50                  55                  60
   Ser Gly Met Ile Gly Leu Ser Pro Pro Phe Tyr Asp His Asn Gly Asp
   65                  70                  75                  80
   Val Ser Ala His Phe Leu Ser Leu Val Ser Arg Val Asp Ser Ile Ser
                   85                  90                  95
   Ser Ser Leu Arg Ser Arg Val Phe Leu Pro Thr Asn Asp Gln His Asp
               100                 105                 110
   Cys Gln Ile Thr Phe Tyr Tyr Phe Ser Cys Gln Val Ser Gly Lys Leu
           115                 120                 125
   Met Val Gly Leu Gln Thr Ala Cys Gly Gly Pro Ile Gln His Leu Trp
       130                 135                 140
   Gln Asn Thr Ala Ala Leu Pro Asn Gln Trp Glu Arg Asn Val Ile Lys
   145                 150                 155                 160
   Ile Gln Ser Ser Gln Arg Phe Gln Val Val Phe Glu Gly Gln Met Ala
                   165                 170                 175
   Ser Thr Tyr Glu Gln Asp Glu Val Ile Ala Ile Asp Asp Ile Ser Phe
               180                 185                 190
   Ser Ser Gly Cys Leu Pro Ala Asn Asp Gly Ile Leu Leu Cys Gln Glu
           195                 200                 205
   Ala Leu Asn Ala Glu Arg Glu Leu Cys His Pro Asp Thr Asp Leu Cys
       210                 215                 220
   Arg Phe Asp Ala Thr Asp Glu Glu Leu Arg Leu Cys Gln Ala Cys Gly
   225                 230                 235                 240
   Phe Glu Phe Asp Met Cys Glu Trp Thr Ser Glu Ala Ser Ala Gly Gln
                   245                 250                 255
   Ile Ser Trp Met Arg Thr Lys Ala Arg Glu Ile Pro Ala Phe Glu Ser
               260                 265                 270
   Thr Pro Gln Gln Asp Gln Gly Gly Asp Asp Glu Gly Tyr Tyr Val Trp
           275                 280                 285
   Val Gly Ala Lys His Gly Phe Thr Leu Asn His Leu Asp Ser Arg Ala
       290                 295                 300
   Tyr Leu Asn Ser Ser Val Cys His Cys Leu Gly Lys Ser Cys His Leu
   305                 310                 315                 320
   Gln Phe Tyr Tyr Ala Met Glu Ser Ser Val Leu Arg Val Arg Leu Tyr
                   325                 330                 335
   Asn Asn Lys Glu Glu Glu Ile Phe Trp Thr Tyr Asn Ile Ser Thr His
               340                 345                 350
   Ser Gln Trp Val Lys Ala Asp Val Leu Ile Pro Glu Asp Leu Lys Thr
           355                 360                 365
   Phe Lys Thr Gln Ser Arg Lys Leu Cys Ser Ala Asp Glu Phe Pro Cys
       370                 375                 380
   Thr Ser Gly Gln Cys Ile Ala Lys Glu Ser Val Cys Asp Ser Arg Gln
   385                 390                 395                 400
   Asp Cys Ser Asp Glu Ser Asp Glu Asp Pro Ala Thr Cys Ser Lys His
                   405                 410                 415
   Leu Thr Cys Asp Phe Glu Ser Gly Phe Cys Gly Trp Glu Pro Phe Leu
               420                 425                 430
   Thr Glu Asp Ser His Trp Lys Leu Met Lys Gly Leu Asn Asn Gly Glu
           435                 440                 445
   His His Phe Pro Ala Ala Asp His Thr Ala Asn Ile Asn His Gly Ser
   450 455 460
   Phe Ile Tyr Leu Glu Ala Gln Arg Ser Pro Gly Val Ala Lys Leu Gly
   465 470 475 480
   Ser Pro Val Leu Thr Lys Leu Leu Thr Ala Ser Thr Pro Cys Gln Val
   485 490 495
   Gln Phe Trp Tyr His Leu Ser Gln His Ser Asn Leu Ser Val Phe Thr
   500 505 510
   Arg Thr Ser Leu Asp Gly Asn Leu Gln Lys Gln Gly Lys Ile Ile Arg
   515 520 525
   Phe Ser Glu Ser Gln Trp Ser His Ala Lys Ile Asp Leu Ile Ala Glu
   530 535 540
   Ala Gly Glu Ser Thr Leu Pro Phe Gln Leu Ile Leu Glu Ala Thr Val
   545 550 555 560
   Leu Ser Ser Asn Ala Thr Val Ala Leu Asp Asp Ile Ser Val Ser Gln
   565 570 575
   Glu Cys Glu Ile Ser Tyr Lys Ser Leu Pro Arg Thr Ser Thr Gln Ser
   580 585 590
   Lys Phe Ser Lys Cys Asp Phe Glu Ala Asn Ser Cys Asp Trp Phe Glu
   595 600 605
   Ala Ile Ser Gly Asp His Phe Asp Trp Ile Arg Ser Ser Gln Ser Glu
   610 615 620
   Leu Ser Ala Asp Phe Glu His Gln Ala Pro Pro Arg Asp His Ser Leu
   625 630 635 640
   Asn Ala Ser Gln Gly His Phe Met Phe Ile Leu Lys Lys Ser Ser Ser
   645 650 655
   Leu Trp Gln Val Ala Lys Leu Gln Ser Pro Thr Phe Ser Gln Thr Gly
   660 665 670
   Pro Gly Cys Ile Leu Ser Phe Trp Phe Tyr Asn Tyr Gly Leu Ser Val
   675 680 685
   Gly Ala Ala Glu Leu Gln Leu His Met Glu Asn Ser His Asp Ser Thr
   690 695 700
   Val Ile Trp Arg Val Leu Tyr Asn Gln Gly Lys Gln Trp Leu Glu Ala
   705 710 715 720
   Thr Ile Gln Leu Gly Arg Leu Ser Gln Pro Phe His Leu Ser Leu Asp
   725 730 735
   Lys Val Ser Leu Gly Ile Tyr Asp Gly Val Ser Ala Ile Asp Asp Ile
   740 745 750
   Arg Phe Glu Asn Cys Thr Leu Pro Leu Pro Ala Glu Ser Cys Glu Gly
   755 760 765
   Leu Asp His Phe Trp Cys Arg His Thr Arg Ala Cys Ile Glu Lys Leu
   770 775 780
   Arg Leu Cys Asp Leu Val Asp Asp Cys Gly Asp Arg Thr Asp Glu Val
   785 790 795 800
   Asn Cys Ala Pro Glu Leu Gln Cys Asn Phe Glu Thr Gly Ile Cys Asn
   805 810 815
   Trp Glu Gln Asp Ala Lys Asp Asp Phe Asp Trp Thr Arg Ser Gln Gly
   820 825 830
   Pro Thr Pro Thr Leu Asn Thr Gly Pro Met Lys Asp Asn Thr Leu Gly
   835 840 845
   Thr Ala Lys Gly His Tyr Leu Tyr Ile Glu Ser Ser Glu Pro Gln Ala
   850 855 860
   Phe Gln Asp Ser Ala Ala Leu Leu Ser Pro Ile Leu Asn Ala Thr Asp
   865 870 875 880
   Thr Lys Gly Cys Thr Phe Arg Phe Tyr Tyr His Met Phe Gly Lys Arg
   885 890 895
   Ile Tyr Arg Leu Ala Ile Tyr Gln Arg Ile Trp Ser Asp Ser Arg Gly
   900 905 910
   Gln Leu Leu Trp Gln Ile Phe Gly Asn Gln Gly Asn Arg Trp Ile Arg
   915 920 925
   Lys His Leu Asn Ile Ser Ser Arg Gln Pro Phe Gln Ile Leu Val Glu
   930 935 940
   Ala Ser Val Gly Asp Gly Phe Thr Gly Asp Ile Ala Ile Asp Asp Leu
   945 950 955 960
   Ser Phe Met Asp Cys Thr Leu Tyr Pro Gly Asn Leu Pro Ala Asp Leu
   965 970 975
   Pro Thr Pro Pro Glu Thr Ser Val Pro Val Thr Leu Pro Pro His Asn
           980                 985                 990
   Cys Thr Asp Asn Glu Phe Ile Cys Arg Ser Asp Gly His Cys Ile Glu
       995                 1000                1005
   Lys Met Gln Lys Cys Asp Phe Lys Tyr Asp Cys Pro Asp Lys Ser Asp
       1010                1015                1020
   Glu Ala Ser Cys Val Met Glu Val Cys Ser Phe Glu Lys Arg Ser Leu
   1025                1030                1035                1040
   Cys Lys Trp Tyr Gln Pro Ile Pro Val His Leu Leu Gln Asp Ser Asn
                   1045                1050                1055
   Thr Phe Arg Trp Gly Leu Gly Asn Gly Ile Ser Ile His His Gly Glu
               1060                1065                1070
   Glu Asn His Arg Pro Ser Val Asp His Thr Gln Asn Thr Thr Asp Gly
           1075                1080                1085
   Trp Tyr Leu Tyr Ala Asp Ser Ser Asn Gly Lys Phe Gly Asp Thr Ala
       1090                1095                1100
   Asp Ile Leu Thr Pro Ile Ile Ser Leu Thr Gly Pro Lys Cys Thr Leu
   1105                1110                1115                1120
   Val Phe Trp Thr His Met Asn Gly Ala Thr Val Gly Ser Leu Gln Val
                   1125                1130                1135
   Leu Ile Lys Lys Asp Asn Val Thr Ser Lys Leu Trp Ala Gln Thr Gly
               1140                1145                1150
   Gln Gln Gly Ala Gln Trp Lys Arg Ala Glu Val Phe Leu Gly Ile Arg
           1155                1160                1165
   Ser His Thr Gln Ile Val Phe Arg Ala Lys Arg Gly Ile Ser Tyr Ile
       1170                1175                1180
   Gly Asp Val Ala Val Asp Asp Ile Ser Phe Gln Asp Cys Ser Pro Leu
   1185                1190                1195                1200
   Leu Ser Pro Glu Arg Lys Cys Thr Asp His Glu Phe Met Cys Ala Asn
                   1205                1210                1215
   Lys His Cys Ile Ala Lys Asp Lys Leu Cys Asp Phe Val Asn Asp Cys
               1220                1225                1230
   Ala Asp Asn Ser Asp Glu Thr Thr Phe Ile Cys Arg Thr Ser Ser Gly
           1235                1240                1245
   Arg Cys Asp Phe Glu Phe Asp Leu Cys Ser Trp Lys Gln Glu Lys Asp
       1250                1255                1260
   Glu Asp Phe Asp Trp Asn Leu Lys Ala Ser Ser Ile Pro Ala Ala Gly
   1265                1270                1275                1280
   Thr Glu Pro Ala Ala Asp His Thr Leu Gly Asn Ser Ser Gly His Tyr
                   1285                1290                1295
   Ile Phe Ile Lys Ser Leu Phe Pro Gln Gln Pro Met Arg Ala Ala Arg
               1300                1305                1310
   Ile Ser Ser Pro Val Ile Ser Lys Arg Ser Lys Asn Cys Lys Ile Ile
           1315                1320                1325
   Phe His Tyr His Met Tyr Gly Asn Gly Ile Gly Ala Leu Thr Leu Met
       1330                1335                1340
   Gln Val Ser Val Thr Asn Gln Thr Lys Val Leu Leu Asn Leu Thr Val
   1345                1350                1355                1360
   Glu Gln Gly Asn Phe Trp Arg Arg Glu Glu Leu Ser Leu Phe Gly Asp
                   1365                1370                1375
   Glu Asp Phe Gln Leu Lys Phe Glu Gly Arg Val Gly Lys Gly Gln Arg
               1380                1385                1390
   Gly Asp Ile Ala Leu Asp Asp Ile Val Leu Thr Glu Asn Cys Leu Ser
           1395                1400                1405
   Leu His Asp Ser Val Gln Glu Glu Leu Ala Val Pro Leu Pro Thr Gly
       1410                1415                1420
   Phe Cys Pro Leu Gly Tyr Arg Glu Cys His Asn Gly Lys Cys Tyr Arg
   1425                1430                1435                1440
   Leu Glu Gln Ser Cys Asn Phe Val Asp Asn Cys Gly Asp Asn Thr Asp
                   1445                1450                1455
   Glu Asn Glu Cys Gly Ser Ser Cys Thr Phe Glu Lys Gly Trp Cys Gly
               1460                1465                1470
   Trp Gln Asn Ser Gln Ala Asp Asn Phe Asp Trp Val Leu Gly Val Gly
           1475                1480                1485
   Ser His Gln Ser Leu Arg Pro Pro Lys Asp His Thr Leu Gly Asn Glu
       1490                1495                1500
   Asn Gly His Phe Met Tyr Leu Glu Ala Thr Ala Val Gly Leu Arg Gly
   1505                1510                1515                1520
   Asp Lys Ala His Phe Arg Ser Thr Met Trp Arg Glu Ser Ser Ala Ala
   1525 1530 1535
   Cys Thr Met Ser Phe Trp Tyr Phe Val Ser Ala Lys Ala Thr Gly Ser
   1540 1545 1550
   Ile Gln Ile Leu Ile Lys Thr Glu Lys Gly Leu Ser Lys Val Trp Gln
   1555 1560 1565
   Glu Ser Lys Gln Asn Pro Gly Asn His Trp Gln Lys Ala Asp Ile Leu
   1570 1575 1580
   Leu Gly Lys Leu Arg Asn Phe Glu Val Ile Phe Gln Gly Ile Arg Thr
   1585 1590 1595 1600
   Arg Asp Leu Gly Gly Gly Ala Ala Ile Asp Asp Ile Glu Phe Lys Asn
   1605 1610 1615
   Cys Thr Thr Val Gly Glu Ile Ser Glu Leu Cys Pro Glu Ile Thr Asp
   1620 1625 1630
   Phe Leu Cys Arg Asp Lys Lys Cys Ile Ala Ser His Leu Leu Cys Asp
   1635 1640 1645
   Tyr Lys Pro Asp Cys Ser Asp Arg Ser Asp Glu Ala His Cys Ala His
   1650 1655 1660
   Tyr Thr Ser Thr Thr Gly Ser Cys Asn Phe Glu Thr Ser Ser Gly Asn
   1665 1670 1675 1680
   Trp Thr Thr Ala Cys Ser Leu Thr Gln Asp Ser Glu Asp Asp Leu Asp
   1685 1690 1695
   Trp Ala Ile Gly Ser Arg Ile Pro Ala Lys Ala Leu Ile Pro Asp Ser
   1700 1705 1710
   Asp His Thr Pro Gly Ser Gly Gln His Phe Leu Tyr Val Asn Ser Ser
   1715 1720 1725
   Gly Ser Lys Glu Gly Ser Val Ala Arg Ile Thr Thr Ser Lys Ser Phe
   1730 1735 1740
   Pro Ala Ser Leu Gly Met Cys Thr Val Arg Phe Trp Phe Tyr Met Ile
   1745 1750 1755 1760
   Asp Pro Arg Ser Met Gly Ile Leu Lys Val Tyr Thr Ile Glu Glu Ser
   1765 1770 1775
   Gly Leu Asn Ile Leu Val Trp Ser Val Ile Gly Asn Lys Arg Thr Gly
   1780 1785 1790
   Trp Thr Tyr Gly Ser Val Pro Leu Ser Ser Asn Ser Pro Phe Lys Val
   1795 1800 1805
   Ala Phe Glu Ala Asp Leu Asp Gly Asn Glu Asp Ile Phe Ile Ala Leu
   1810 1815 1820
   Asp Asp Ile Ser Phe Thr Pro Glu Cys Val Thr Gly Gly Pro Val Pro
   1825 1830 1835 1840
   Val Gln Pro Ser Pro Cys Glu Ala Asp Gln Phe Ser Cys Ile Tyr Thr
   1845 1850 1855
   Leu Gln Cys Val Pro Leu Ser Gly Lys Cys Asp Gly His Glu Asp Cys
   1860 1865 1870
   Ile Asp Gly Ser Asp Glu Met Asp Cys Pro Leu Ser Pro Thr Pro Pro
   1875 1880 1885
   Leu Cys Ser Asn Met Glu Phe Pro Cys Ser Thr Asp Glu Cys Ile Pro
   1890 1895 1900
   Ser Leu Leu Leu Cys Asp Gly Val Pro Asp Cys His Phe Asn Glu Asp
   1905 1910 1915 1920
   Glu Leu Ile Cys Ser Asn Lys Ser Cys Ser Asn Gly Ala Leu Val Cys
   1925 1930 1935
   Ala Ser Ser Asn Ser Cys Ile Pro Ala His Gln Arg Cys Asp Gly Phe
   1940 1945 1950
   Ala Asp Cys Met Asp Phe Gln Leu Asp Glu Ser Ser Cys Ser Glu Cys
   1955 1960 1965
   Pro Leu Asn Tyr Cys Arg Asn Gly Gly Thr Cys Val Val Glu Lys Asn
   1970 1975 1980
   Gly Pro Met Cys Arg Cys Arg Gln Gly Trp Lys Gly Asn Arg Cys His
   1985 1990 1995 2000
   Ile Lys Phe Asn Pro Pro Ala Thr Asp Phe Thr Tyr Ala Gln Asn Asn
   2005 2010 2015
   Thr
<210> 11
   <211> 6326
   <212> DNA
   <213> Homo sapiens
<400> 11
   ttgcctgtgt tttcaattct acactggctc agcaagggac agaaagcttt cagtgtgaca 60
   atggagtctc cttgcctcct gacagcattt gtgacttcac agatcagtgt ggggatagca 120
   gtgatgaacg gcactgtttg aattatgaaa gatgtgattt tgaggatggt ctctgtcata 180
   tgactcaaga tcagagtctg caacctagtt ggacaaagag aagtgggatg attggtctat 240
   cacctccatt ttatgatcac aatggtgatg tgtctgctca cttcctctca ctggtttcca 300
   gagtggattc tatttcctca agtttaagaa gcagagtttt ccttccaaca aatgatcaac 360
   atgactgcca gattacattt tattacttct cctgccaagt gagtggcaaa ttaatggttg 420
   ggcttcaaac tgcatgtgga ggtcctattc agcatttatg gcaaaacaca gctgcactcc 480
   caaatcagtg ggagagaaat gtcatcaaaa tccagagttc acagagattt caggttgttt 540
   ttgaaggtca aatggcttca acgtatgaac aggatgaagt cattgctatt gatgatatat 600
   ctttcagttc aggctgcttg cctgccaatg atggaatttt actgtgtcaa gaagcattga 660
   atgctgagcg ggagctatgc catccagata cagatctctg cagatttgat gctacagatg 720
   aagagttgag attgtgtcag gcctgtgggt ttgaatttga catgtgtgag tggacgtcag 780
   aagcatctgc tggccaaatt tcctggatgc gcacaaaagc gagagagatc cctgcattcg 840
   aatccacacc tcagcaggat caaggaggtg atgatgaagg ttattatgta tgggtaggcg 900
   ctaagcatgg tttcactctt aaccatttag acagcagggc ttacctaaat agctctgtgt 960
   gtcattgcct gggcaagagc tgtcatcttc aattctatta tgcaatggaa agcagtgtcc 1020
   tgagagtaag actgtataat aataaggaag aagaaatatt ttggacatac aacatatcaa 1080
   ctcacagcca atgggtgaaa gcagatgtgt taataccaga agatctgaag acatttaaga 1140
   cccaatccag aaagctttgc tctgcagacg aattcccttg cactagtggc cagtgcatcg 1200
   ccaaagaatc tgtctgtgac tctcggcagg actgctccga tgagagtgat gaagacccag 1260
   caacttgctc aaagcatctc acctgtgact ttgagtcggg tttctgcggt tgggagccat 1320
   ttctcacaga agattcacac tggaagctga tgaaaggatt gaataatgga gagcaccact 1380
   ttcctgcagc tgatcacaca gcaaacataa atcatggatc gtttatttat ttggaggcac 1440
   agcgctcccc cggggtggcc aagcttggaa gtcctgttct tacaaaattg ctcactgcct 1500
   ctaccccatg tcaggtgcag ttttggtatc atttgtctca acattcaaat ctctcagttt 1560
   ttacaagaac gtctctagat ggaaacttgc aaaagcaggg caaaataatc agattctccg 1620
   aatctcagtg gagccacgca aaaattgatc tcattgcaga agcgggagaa tctactctac 1680
   cttttcagtt aattttggaa gctactgttt tgtcgtcaaa tgctaccgtt gctctagatg 1740
   acatcagtgt gtcccaggaa tgtgaaattt cctataaatc actaccaagg accagtacac 1800
   aaagcaagtt ttccaagtgt gactttgaag caaacagctg tgattggttt gaagcaatta 1860
   gtggtgacca ttttgactgg atacggagct ctcagagtga actttctgct gattttgagc 1920
   accaggctcc acctcgggat catagtctca acgcatctca agggcatttt atgttcattc 1980
   tgaagaaaag cagcagcttg tggcaagttg ctaagcttca gagcccaact ttcagccaga 2040
   caggacctgg atgcatactt tccttctggt tctataacta tggcctgtca gtgggagcag 2100
   ctgagctgca gctacatatg gaaaattctc atgactcaac agtgatttgg agagtattat 2160
   acaatcaggg caaacaatgg ttggaggcaa ccattcagct agggcgcctt tcgcagccct 2220
   tccatttgtc actagataaa gtcagtctgg gcatttatga tggggtctca gctattgatg 2280
   acatccgatt tgaaaattgt actctccctc ttcctgctga gagctgtgaa gggctggatc 2340
   atttctggtg tcgccacacc agggcttgca tagaaaagct tcggttatgt gatctggtgg 2400
   atgactgtgg tgatcgtact gatgaagtca actgtgcacc tgagctgcag tgtaactttg 2460
   aaactggaat ctgtaactgg gaacaagatg caaaagatga ctttgattgg accaggagcc 2520
   agggtccaac tccaacactt aacacagggc caatgaaaga taacactctg ggcacagcta 2580
   aaggacacta tctctacata gaatcttcag agccacaggc ttttcaagac agtgctgcct 2640
   tactcagccc aatccttaat gccactgata caaaaggctg caccttccgc ttctattacc 2700
   acatgtttgg aaagcgcatt tataggttgg caatctacca acgaatctgg agtgactcaa 2760
   ggggacagct gctgtggcag atatttggga atcaaggcaa cagatggatt aggaaacacc 2820
   tcaacatttc cagcaggcag ccctttcaga tattggtgga ggcttcagtg ggagatggct 2880
   tcactggaga tattgcgatt gatgatctgt catttatgga ctgcaccctc taccctggta 2940
   atttgccagc agacctccca actccaccag aaacgtcagt tcctgtaaca ttacctccac 3000
   acaactgcac agacaatgaa tttatctgca ggtctgatgg tcactgcatt gaaaaaatgc 3060
   agaaatgtga ttttaaatat gactgccctg acaaatcaga tgaagcatcc tgtgttatgg 3120
   aagtttgcag ctttgagaaa agaagcctgt gtaaatggta tcaaccaatc ccagtacatt 3180
   tgcttcaaga ttcaaacaca ttcaggtggg ggcttgggaa cgggatcagc attcatcatg 3240
   gggaagaaaa ccacaggcca tcagtggatc atacacaaaa taccactgat ggctggtacc 3300
   tgtatgctga cagttctaat gggaaatttg gtgacacggc tgacattctc actcctatca 3360
   tttcactcac gggaccaaaa tgtaccttgg tgttctggac acatatgaat ggggccaccg 3420
   ttggttctct ccaggtgctc atcaagaaag ataacgttac ttctaaattg tgggctcaaa 3480
   ctggacagca aggtgcacag tggaagagag cagaagtgtt tttaggcatt cgttcacata 3540
   cacagattgt cttcagagcc aaacgtggta tcagttacat aggagatgta gcagtggatg 3600
   atatttcctt ccaagattgc tcccctttgc ttagcccaga gagaaagtgt actgatcatg 3660
   aattcatgtg tgctaataag cactgcattg ccaaagacaa gctgtgtgat tttgtgaatg 3720
   attgtgctga taattcagat gagactactt tcatttgccg tacctccagt gggcgctgtg 3780
   atttcgaatt tgatctttgt tcctggaagc aggagaaaga tgaggacttt gactggaacc 3840
   tgaaagctag cagcatccct gcagcaggca cagagccagc agcagatcac actttgggaa 3900
   attcatctgg tcattacatc tttataaaga gtttgtttcc tcagcagccc atgagagctg 3960
   ccagaatttc aagtccagtt ataagtaaga gaagcaaaaa ctgcaagatt atttttcatt 4020
   atcacatgta tggaaatggc attggggcac tcaccttaat gcaggtgtca gtcacaaacc 4080
   aaacgaaggt tctacttaac ctcactgtag aacaaggcaa tttctggcgg agagaagaac 4140
   tgtcactgtt tggtgatgaa gacttccaac tcaaatttga aggtagagtt gggaaaggtc 4200
   agcgtggaga cattgcactt gatgacattg tgcttacaga aaattgtcta tcactccatg 4260
   attccgtgca agaagaactg gcagtgcctc ttccaacagg tttctgccca cttggctata 4320
   gggaatgtca taatggaaaa tgctataggc tggaacaaag ctgtaacttc gtagataact 4380
   gtggagataa tactgatgaa aatgagtgtg gtagctcctg tacttttgaa aaaggctggt 4440
   gtggctggca aaactcccag gctgacaact ttgattgggt tttaggggtt ggctctcatc 4500
   aaagcttaag acctcccaaa gaccacacac ttggaaatga aaatgggcac ttcatgtatc 4560
   tggaagctac tgcagtgggc cttcggggtg acaaagcaca cttcaggagt accatgtggc 4620
   gagaatccag tgcagcctgc accatgagct tctggtattt cgtatctgca aaggccacag 4680
   gatccattca gattctcatc aagacagaga aaggactatc aaaagtatgg caagaaagta 4740
   agcagaaccc tggtaatcat tggcaaaagg ctgacatcct gctaggaaag ttaaggaatt 4800
   ttgaagtcat atttcaaggt atcagaacaa gggacctggg aggaggagct gcaattgatg 4860
   atattgaatt taaaaactgc acaactgtgg gagagatctc tgagctttgt ccggaaatca 4920
   ctgatttttt gtgccgggac aagaagtgca ttgcatccca ccttctttgt gactataagc 4980
   cagactgctc tgataggtct gatgaagctc actgtgcaca ttatacaagc acaacaggaa 5040
   gctgcaattt tgaaacaagt tcaggaaact ggaccacagc ctgcagtctt actcaagact 5100
   ctgaggatga cttggactgg gccattggca gcagaattcc tgccaaagca ttaattccag 5160
   actctgatca cacgccaggt agtggtcagc acttcctgta cgtcaactca tctggctcca 5220
   aggaaggatc cgttgccaga attactactt ccaaatcctt cccagcaagc cttggaatgt 5280
   gtactgttcg gttctggttc tacatgattg atcccaggag tatgggaata ttaaaggtgt 5340
   ataccattga agaatcgggg ctaaacatcc tggtgtggtc agtgattgga aataaaagaa 5400
   cgggatggac atatggctct gtgcctctct ccagtaacag tccgtttaag gtggcatttg 5460
   aagctgattt ggatggaaat gaggacatct ttattgctct tgatgacatc tcttttaccc 5520
   cagagtgtgt gactggaggt cctgtcccag tgcagccatc accctgtgaa gctgatcagt 5580
   tttcttgtat ctacacactc caatgtgtcc ctctctcagg gaaatgtgat ggacatgaag 5640
   actgcataga tggatctgat gaaatggatt gtcctctcag ccccacccct ccactctgta 5700
   gtaacatgga gttcccgtgc tctacagacg agtgtatacc ttccctcctg ctatgcgatg 5760
   gagtgcccga ctgccacttt aatgaagatg agctcatctg ctccaacaaa agctgttcta 5820
   atggagctct ggtgtgtgcc tcctccaaca gctgtatccc agcccaccag cgctgtgatg 5880
   gttttgccga ctgcatggat ttccagcttg atgagtccag ctgctccgaa tgtccattaa 5940
   attactgcag aaatggtggg acttgtgtag tggagaaaaa tggtcctatg tgtcgatgta 6000
   gacaaggctg gaaaggaaat cgatgccata tcaagtttaa tcctcctgct acagacttca 6060
   catacgctca gaataataca tggactctcc tgggtattgg attagcattc ctgatgactc 6120
   acatcacagt tgcagtcttg tgttttcttg caaacagaaa ggtaccaata aggaaaaccg 6180
   agggaagtgg taactgtgcc tttgtcaatc cagtttacgg gaactggagc aacccagaga 6240
   aaacagagag ttctgtctat tccttctcaa acccattata tggcacaaca tcaggaagcc 6300
   tggagaccct gtcacatcat ctcaaa 6326
<210> 12
   <211> 2026
   <212> PRT
   <213> Homo sapiens
<400> 12
   Ala Cys Val Phe Asn Ser Thr Leu Ala Gln Gln Gly Thr Glu Ser Phe
   1               5                   10                  15
   Gln Cys Asp Asn Gly Val Ser Leu Pro Pro Asp Ser Ile Cys Asp Phe
               20                  25                  30
   Thr Asp Gln Cys Gly Asp Ser Ser Asp Glu Arg His Cys Leu Asn Tyr
           35                  40                  45
   Glu Arg Cys Asp Phe Glu Asp Gly Leu Cys His Met Thr Gln Asp Gln
       50                  55                  60
   Ser Leu Gln Pro Ser Trp Thr Lys Arg Ser Gly Met Ile Gly Leu Ser
   65                  70                  75                  80
   Pro Pro Phe Tyr Asp His Asn Gly Asp Val Ser Ala His Phe Leu Ser
                   85                  90                  95
   Leu Val Ser Arg Val Asp Ser Ile Ser Ser Ser Leu Arg Ser Arg Val
               100                 105                 110
   Phe Leu Pro Thr Asn Asp Gln His Asp Cys Gln Ile Thr Phe Tyr Tyr
           115                 120                 125
   Phe Ser Cys Gln Val Ser Gly Lys Leu Met Val Gly Leu Gln Thr Ala
       130                 135                 140
   Cys Gly Gly Pro Ile Gln His Leu Trp Gln Asn Thr Ala Ala Leu Pro
   145                 150                 155                 160
   Asn Gln Trp Glu Arg Asn Val Ile Lys Ile Gln Ser Ser Gln Arg Phe
                       165                 170                 175
   Gln Val Val Phe Glu Gly Gln Met Ala Ser Thr Tyr Glu Gln Asp Glu
                   180                 185                 190
   Val Ile Ala Ile Asp Asp Ile Ser Phe Ser Ser Gly Cys Leu Pro Ala
               195                 200                 205
   Asn Asp Gly Ile Leu Leu Cys Gln Glu Ala Leu Asn Ala Glu Arg Glu
       210                 215                 220
   Leu Cys His Pro Asp Thr Asp Leu Cys Arg Phe Asp Ala Thr Asp Glu
   225                 230                 235                 240
   Glu Leu Arg Leu Cys Gln Ala Cys Gly Phe Glu Phe Asp Met Cys Glu
                   245                 250                 255
   Trp Thr Ser Glu Ala Ser Ala Gly Gln Ile Ser Trp Met Arg Thr Lys
                   260                 265                 270
   Ala Arg Glu Ile Pro Ala Phe Glu Ser Thr Pro Gln Gln Asp Gln Gly
               275                 280                 285
   Gly Asp Asp Glu Gly Tyr Tyr Val Trp Val Gly Ala Lys His Gly Phe
       290                 295                 300
   Thr Leu Asn His Leu Asp Ser Arg Ala Tyr Leu Asn Ser Ser Val Cys
   305                 310                 315                 320
   His Cys Leu Gly Lys Ser Cys His Leu Gln Phe Tyr Tyr Ala Met Glu
                   325                 330                 335
   Ser Ser Val Leu Arg Val Arg Leu Tyr Asn Asn Lys Glu Glu Glu Ile
                   340                 345                 350
   Phe Trp Thr Tyr Asn Ile Ser Thr His Ser Gln Trp Val Lys Ala Asp
               355                 360                 365
   Val Leu Ile Pro Glu Asp Leu Lys Thr Phe Lys Thr Gln Ser Arg Lys
       370                 375                 380
   Leu Cys Ser Ala Asp Glu Phe Pro Cys Thr Ser Gly Gln Cys Ile Ala
   385                 390                 395                 400
   Lys Glu Ser Val Cys Asp Ser Arg Gln Asp Cys Ser Asp Glu Ser Asp
                   405                 410                 415
   Glu Asp Pro Ala Thr Cys Ser Lys His Leu Thr Cys Asp Phe Glu Ser
                   420                 425                 430
   Gly Phe Cys Gly Trp Glu Pro Phe Leu Thr Glu Asp Ser His Trp Lys
               435                 440                 445
   Leu Met Lys Gly Leu Asn Asn Gly Glu His His Phe Pro Ala Ala Asp
       450                 455                 460
   His Thr Ala Asn Ile Asn His Gly Ser Phe Ile Tyr Leu Glu Ala Gln
   465                 470                 475                 480
   Arg Ser Pro Gly Val Ala Lys Leu Gly Ser Pro Val Leu Thr Lys Leu
                   485                 490                 495
   Leu Thr Ala Ser Thr Pro Cys Gln Val Gln Phe Trp Tyr His Leu Ser
                   500                 505                 510
   Gln His Ser Asn Leu Ser Val Phe Thr Arg Thr Ser Leu Asp Gly Asn
               515                 520                 525
   Leu Gln Lys Gln Gly Lys Ile Ile Arg Phe Ser Glu Ser Gln Trp Ser
       530                 535                 540
   His Ala Lys Ile Asp Leu Ile Ala Glu Ala Gly Glu Ser Thr Leu Pro
   545                 550                 555                 560
   Phe Gln Leu Ile Leu Glu Ala Thr Val Leu Ser Ser Asn Ala Thr Val
                   565                 570                 575
   Ala Leu Asp Asp Ile Ser Val Ser Gln Glu Cys Glu Ile Ser Tyr Lys
                   580                 585                 590
   Ser Leu Pro Arg Thr Ser Thr Gln Ser Lys Phe Ser Lys Cys Asp Phe
               595                 600                 605
   Glu Ala Asn Ser Cys Asp Trp Phe Glu Ala Ile Ser Gly Asp His Phe
       610                 615                 620
   Asp Trp Ile Arg Ser Ser Gln Ser Glu Leu Ser Ala Asp Phe Glu His
   625                 630                 635                 640
   Gln Ala Pro Pro Arg Asp His Ser Leu Asn Ala Ser Gln Gly His Phe
                   645                 650                 655
   Met Phe Ile Leu Lys Lys Ser Ser Ser Leu Trp Gln Val Ala Lys Leu
                   660                 665                 670
   Gln Ser Pro Thr Phe Ser Gln Thr Gly Pro Gly Cys Ile Leu Ser Phe
               675                 680                 685
   Trp Phe Tyr Asn Tyr Gly Leu Ser Val Gly Ala Ala Glu Leu Gln Leu
       690                 695                 700
   His Met Glu Asn Ser His Asp Ser Thr Val Ile Trp Arg Val Leu Tyr
   705 710 715 720
   Asn Gln Gly Lys Gln Trp Leu Glu Ala Thr Ile Gln Leu Gly Arg Leu
   725 730 735
   Ser Gln Pro Phe His Leu Ser Leu Asp Lys Val Ser Leu Gly Ile Tyr
   740 745 750
   Asp Gly Val Ser Ala Ile Asp Asp Ile Arg Phe Glu Asn Cys Thr Leu
   755 760 765
   Pro Leu Pro Ala Glu Ser Cys Glu Gly Leu Asp His Phe Trp Cys Arg
   770 775 780
   His Thr Arg Ala Cys Ile Glu Lys Leu Arg Leu Cys Asp Leu Val Asp
   785 790 795 800
   Asp Cys Gly Asp Arg Thr Asp Glu Val Asn Cys Ala Pro Glu Leu Gln
   805 810 815
   Cys Asn Phe Glu Thr Gly Ile Cys Asn Trp Glu Gln Asp Ala Lys Asp
   820 825 830
   Asp Phe Asp Trp Thr Arg Ser Gln Gly Pro Thr Pro Thr Leu Asn Thr
   835 840 845
   Gly Pro Met Lys Asp Asn Thr Leu Gly Thr Ala Lys Gly His Tyr Leu
   850 855 860
   Tyr Ile Glu Ser Ser Glu Pro Gln Ala Phe Gln Asp Ser Ala Ala Leu
   865 870 875 880
   Leu Ser Pro Ile Leu Asn Ala Thr Asp Thr Lys Gly Cys Thr Phe Arg
   885 890 895
   Phe Tyr Tyr His Met Phe Gly Lys Arg Ile Tyr Arg Leu Ala Ile Tyr
   900 905 910
   Gln Arg Ile Trp Ser Asp Ser Arg Gly Gln Leu Leu Trp Gln Ile Phe
   915 920 925
   Gly Asn Gln Gly Asn Arg Trp Ile Arg Lys His Leu Asn Ile Ser Ser
   930 935 940
   Arg Gln Pro Phe Gln Ile Leu Val Glu Ala Ser Val Gly Asp Gly Phe
   945 950 955 960
   Thr Gly Asp Ile Ala Ile Asp Asp Leu Ser Phe Met Asp Cys Thr Leu
   965 970 975
   Tyr Pro Gly Asn Leu Pro Ala Asp Leu Pro Thr Pro Pro Glu Thr Ser
   980 985 990
   Val Pro Val Thr Leu Pro Pro His Asn Cys Thr Asp Asn Glu Phe Ile
   995 1000 1005
   Cys Arg Ser Asp Gly His Cys Ile Glu Lys Met Gln Lys Cys Asp Phe
   1010 1015 1020
   Lys Tyr Asp Cys Pro Asp Lys Ser Asp Glu Ala Ser Cys Val Met Glu
   1025 1030 1035 1040
   Val Cys Ser Phe Glu Lys Arg Ser Leu Cys Lys Trp Tyr Gln Pro Ile
   1045 1050 1055
   Pro Val His Leu Leu Gln Asp Ser Asn Thr Phe Arg Trp Gly Leu Gly
   1060 1065 1070
   Asn Gly Ile Ser Ile His His Gly Glu Glu Asn His Arg Pro Ser Val
   1075 1080 1085
   Asp His Thr Gln Asn Thr Thr Asp Gly Trp Tyr Leu Tyr Ala Asp Ser
   1090 1095 1100
   Ser Asn Gly Lys Phe Gly Asp Thr Ala Asp Ile Leu Thr Pro Ile Ile
   1105 1110 1115 1120
   Ser Leu Thr Gly Pro Lys Cys Thr Leu Val Phe Trp Thr His Met Asn
   1125 1130 1135
   Gly Ala Thr Val Gly Ser Leu Gln Val Leu Ile Lys Lys Asp Asn Val
   1140 1145 1150
   Thr Ser Lys Leu Trp Ala Gln Thr Gly Gln Gln Gly Ala Gln Trp Lys
   1155 1160 1165
   Arg Ala Glu Val Phe Leu Gly Ile Arg Ser His Thr Gln Ile Val Phe
   1170 1175 1180
   Arg Ala Lys Arg Gly Ile Ser Tyr Ile Gly Asp Val Ala Val Asp Asp
   1185 1190 1195 1200
   Ile Ser Phe Gln Asp Cys Ser Pro Leu Leu Ser Pro Glu Arg Lys Cys
   1205 1210 1215
   Thr Asp His Glu Phe Met Cys Ala Asn Lys His Cys Ile Ala Lys Asp
   1220 1225 1230
   Lys Leu Cys Asp Phe Val Asn Asp Cys Ala Asp Asn Ser Asp Glu Thr
   1235 1240 1245
   Thr Phe Ile Cys Arg Thr Ser Ser Gly Arg Cys Asp Phe Glu Phe Asp
   1250 1255 1260
   Leu Cys Ser Trp Lys Gln Glu Lys Asp Glu Asp Phe Asp Trp Asn Leu
   1265 1270 1275 1280
   Lys Ala Ser Ser Ile Pro Ala Ala Gly Thr Glu Pro Ala Ala Asp His
   1285 1290 1295
   Thr Leu Gly Asn Ser Ser Gly His Tyr Ile Phe Ile Lys Ser Leu Phe
   1300 1305 1310
   Pro Gln Gln Pro Met Arg Ala Ala Arg Ile Ser Ser Pro Val Ile Ser
   1315 1320 1325
   Lys Arg Ser Lys Asn Cys Lys Ile Ile Phe His Tyr His Met Tyr Gly
   1330 1335 1340
   Asn Gly Ile Gly Ala Leu Thr Leu Met Gln Val Ser Val Thr Asn Gln
   1345 1350 1355 1360
   Thr Lys Val Leu Leu Asn Leu Thr Val Glu Gln Gly Asn Phe Trp Arg
   1365 1370 1375
   Arg Glu Glu Leu Ser Leu Phe Gly Asp Glu Asp Phe Gln Leu Lys Phe
   1380 1385 1390
   Glu Gly Arg Val Gly Lys Gly Gln Arg Gly Asp Ile Ala Leu Asp Asp
   1395 1400 1405
   Ile Val Leu Thr Glu Asn Cys Leu Ser Leu His Asp Ser Val Gln Glu
   1410 1415 1420
   Glu Leu Ala Val Pro Leu Pro Thr Gly Phe Cys Pro Leu Gly Tyr Arg
   1425 1430 1435 1440
   Glu Cys His Asn Gly Lys Cys Tyr Arg Leu Glu Gln Ser Cys Asn Phe
   1445 1450 1455
   Val Asp Asn Cys Gly Asp Asn Thr Asp Glu Asn Glu Cys Gly Ser Ser
   1460 1465 1470
   Cys Thr Phe Glu Lys Gly Trp Cys Gly Trp Gln Asn Ser Gln Ala Asp
   1475 1480 1485
   Asn Phe Asp Trp Val Leu Gly Val Gly Ser His Gln Ser Leu Arg Pro
   1490 1495 1500
   Pro Lys Asp His Thr Leu Gly Asn Glu Asn Gly His Phe Met Tyr Leu
   1505 1510 1515 1520
   Glu Ala Thr Ala Val Gly Leu Arg Gly Asp Lys Ala His Phe Arg Ser
   1525 1530 1535
   Thr Met Trp Arg Glu Ser Ser Ala Ala Cys Thr Met Ser Phe Trp Tyr
   1540 1545 1550
   Phe Val Ser Ala Lys Ala Thr Gly Ser Ile Gln Ile Leu Ile Lys Thr
   1555 1560 1565
   Glu Lys Gly Leu Ser Lys Val Trp Gln Glu Ser Lys Gln Asn Pro Gly
   1570 1575 1580
   Asn His Trp Gln Lys Ala Asp Ile Leu Leu Gly Lys Leu Arg Asn Phe
   1585 1590 1595 1600
   Glu Val Ile Phe Gln Gly Ile Arg Thr Arg Asp Leu Gly Gly Gly Ala
   1605 1610 1615
   Ala Ile Asp Asp Ile Glu Phe Lys Asn Cys Thr Thr Val Gly Glu Ile
   1620 1625 1630
   Ser Glu Leu Cys Pro Glu Ile Thr Asp Phe Leu Cys Arg Asp Lys Lys
   1635 1640 1645
   Cys Ile Ala Ser His Leu Leu Cys Asp Tyr Lys Pro Asp Cys Ser Asp
   1650 1655 1660
   Arg Ser Asp Glu Ala His Cys Ala His Tyr Thr Ser Thr Thr Gly Ser
   1665 1670 1675 1680
   Cys Asn Phe Glu Thr Ser Ser Gly Asn Trp Thr Thr Ala Cys Ser Leu
   1685 1690 1695
   Thr Gln Asp Ser Glu Asp Asp Leu Asp Trp Ala Ile Gly Ser Arg Ile
   1700 1705 1710
   Pro Ala Lys Ala Leu Ile Pro Asp Ser Asp His Thr Pro Gly Ser Gly
   1715 1720 1725
   Gln His Phe Leu Tyr Val Asn Ser Ser Gly Ser Lys Glu Gly Ser Val
   1730 1735 1740
   Ala Arg Ile Thr Thr Ser Lys Ser Phe Pro Ala Ser Leu Gly Met Cys
   1745 1750 1755 1760
   Thr Val Arg Phe Trp Phe Tyr Met Ile Asp Pro Arg Ser Met Gly Ile
   1765 1770 1775
   Leu Lys Val Tyr Thr Ile Glu Glu Ser Gly Leu Asn Ile Leu Val Trp
   1780 1785 1790
   Ser Val Ile Gly Asn Lys Arg Thr Gly Trp Thr Tyr Gly Ser Val Pro
   1795 1800 1805
   Leu Ser Ser Asn Ser Pro Phe Lys Val Ala Phe Glu Ala Asp Leu Asp
   1810 1815 1820
   Gly Asn Glu Asp Ile Phe Ile Ala Leu Asp Asp Ile Ser Phe Thr Pro
   1825 1830 1835 1840
   Glu Cys Val Thr Gly Gly Pro Val Pro Val Gln Pro Ser Pro Cys Glu
   1845 1850 1855
   Ala Asp Gln Phe Ser Cys Ile Tyr Thr Leu Gln Cys Val Pro Leu Ser
   1860 1865 1870
   Gly Lys Cys Asp Gly His Glu Asp Cys Ile Asp Gly Ser Asp Glu Met
   1875 1880 1885
   Asp Cys Pro Leu Ser Pro Thr Pro Pro Leu Cys Ser Asn Met Glu Phe
   1890 1895 1900
   Pro Cys Ser Thr Asp Glu Cys Ile Pro Ser Leu Leu Leu Cys Asp Gly
   1905 1910 1915 1920
   Val Pro Asp Cys His Phe Asn Glu Asp Glu Leu Ile Cys Ser Asn Lys
   1925 1930 1935
   Ser Cys Ser Asn Gly Ala Leu Val Cys Ala Ser Ser Asn Ser Cys Ile
   1940 1945 1950
   Pro Ala His Gln Arg Cys Asp Gly Phe Ala Asp Cys Met Asp Phe Gln
   1955 1960 1965
   Leu Asp Glu Ser Ser Cys Ser Glu Cys Pro Leu Asn Tyr Cys Arg Asn
   1970 1975 1980
   Gly Gly Thr Cys Val Val Glu Lys Asn Gly Pro Met Cys Arg Cys Arg
   1985 1990 1995 2000
   Gln Gly Trp Lys Gly Asn Arg Cys His Ile Lys Phe Asn Pro Pro Ala
   2005 2010 2015
   Thr Asp Phe Thr Tyr Ala Gln Asn Asn Thr
   2020 2025
<210> 13
   <211> 6243
   <212> DNA
   <213> Homo sapiens
<400> 13
   cagcaaggga cagaaagctt tcagtgtgac aatggagtct ccttgcctcc tgacagcatt   60
   tgtgacttca cagatcagtg tggggatagc agtgatgaac ggcactggct ttcatttgct  120
   catccttgca atccattggg gtctagtttg gaatctgaca actggaacaa aaagaaactt  180
   gaatccggtg catgccttgg ttttggtgct gctgctgctt ctcaagatcc tcggcaggaa  240
   ttaagaaaga agctggtgtg gacagcagat ccccgaaatt ggcgggctat tacattttat  300
   tacttctcct gccaagtgag tggcaaatta atggttgggc ttcaaactgc atgtggaggt  360
   cctattcagc atttatggca aaacacagct gcactcccaa atcagtggga gagaaatgtc  420
   atcaaaatcc agagttcaca gagatttcag gttgtttttg aaggtcaaat ggcttcaacg  480
   tatgaacagg atgaagtcat tgctattgat gatatatctt tcagttcagg ctgcttgcct  540
   gccaatgatg gaattttact gtgtcaagaa gcattgaatg ctgagcggga gctatgccat  600
   ccagatacag atctctgcag atttgatgct acagatgaag agttgagatt gtgtcaggcc  660
   tgtgggtttg aatttgacat gtgtgagtgg acgtcagaag catctgctgg ccaaatttcc  720
   tggatgcgca caaaagcgag agagatccct gcattcgaat ccacacctca gcaggatcaa  780
   ggaggtgatg atgaaggtta ttatgtatgg gtaggcgcta agcatggttt cactcttaac  840
   catttagaca gcagggctta cctaaatagc tctgtgtgtc attgcctggg caagagctgt  900
   catcttcaat tctattatgc aatggaaagc agtgtcctga gagtaagact gtataataat  960
   aaggaagaag aaatattttg gacatacaac atatcaactc acagccaatg ggtgaaagca 1020
   gatgtgttaa taccagaaga tctgaagaca tttaagaccc aatccagaaa gctttgctct 1080
   gcagacgaat tcccttgcac tagtggccag tgcatcgcca aagaatctgt ctgtgactct 1140
   cggcaggact gctccgatga gagtgatgaa gacccagcaa cttgctcaaa gcatctcacc 1200
   tgtgactttg agtcgggttt ctgcggttgg gagccatttc tcacagaaga ttcacactgg 1260
   aagctgatga aaggattgaa taatggagag caccactttc ctgcagctga tcacacagca 1320
   aacataaatc atggatcgtt tatttatttg gaggcacagc gctcccccgg ggtggccaag 1380
   cttggaagtc ctgttcttac aaaattgctc actgcctcta ccccatgtca ggtgcagttt 1440
   tggtatcatt tgtctcaaca ttcaaatctc tcagttttta caagaacgtc tctagatgga 1500
   aacttgcaaa agcagggcaa aataatcaga ttctccgaat ctcagtggag ccacgcaaaa 1560
   attgatctca ttgcagaagc gggagaatct actctacctt ttcagttaat tttggaagct 1620
   actgttttgt cgtcaaatgc taccgttgct ctagatgaca tcagtgtgtc ccaggaatgt 1680
   gaaatttcct ataaatcact accaaggacc agtacacaaa gcaagttttc caagtgtgac 1740
   tttgaagcaa acagctgtga ttggtttgaa gcaattagtg gtgaccattt tgactggata 1800
   cggagctctc agagtgaact ttctgctgat tttgagcacc aggctccacc tcgggatcat   1860
   agtctcaacg catctcaagg gcattttatg ttcattctga agaaaagcag cagcttgtgg 1920
   caagttgcta agcttcagag cccaactttc agccagacag gacctggatg catactttcc 1980
   ttctggttct ataactatgg cctgtcagtg ggagcagctg agctgcagct acatatggaa 2040
   aattctcatg actcaacagt gatttggaga gtattataca atcagggcaa acaatggttg 2100
   gaggcaacca ttcagctagg gcgcctttcg cagcccttcc atttgtcact agataaagtc 2160
   agtctgggca tttatgatgg ggtctcagct attgatgaca tccgatttga aaattgtact 2220
   ctccctcttc ctgctgagag ctgtgaaggg ctggatcatt tctggtgtcg ccacaccagg 2280
   gcttgcatag aaaagcttcg gttatgtgat ctggtggatg actgtggtga tcgtactgat 2340
   gaagtcaact gtgcacctga gctgcagtgt aactttgaaa ctggaatctg taactgggaa 2400
   caagatgcaa aagatgactt tgattggacc aggagccagg gtccaactcc aacacttaac 2460
   acagggccaa tgaaagataa cactctgggc acagctaaag gacactatct ctacatagaa 2520
   tcttcagagc cacaggcttt tcaagacagt gctgccttac tcagcccaat ccttaatgcc 2580
   actgatacaa aaggctgcac cttccgcttc tattaccaca tgtttggaaa gcgcatttat 2640
   aggttggcaa tctaccaacg aatctggagt gactcaaggg gacagctgct gtggcagata 2700
   tttgggaatc aaggcaacag atggattagg aaacacctca acatttccag caggcagccc 2760
   tttcagatat tggtggaggc ttcagtggga gatggcttca ctggagatat tgcgattgat 2820
   gatctgtcat ttatggactg caccctctac cctggtaatt tgccagcaga cctcccaact 2880
   ccaccagaaa cgtcagttcc tgtaacatta cctccacaca actgcacaga caatgaattt 2940
   atctgcaggt ctgatggtca ctgcattgaa aaaatgcaga aatgtgattt taaatatgac 3000
   tgccctgaca aatcagatga agcatcctgt gttatggaag tttgcagctt tgagaaaaga 3060
   agcctgtgta aatggtatca accaatccca gtacatttgc ttcaagattc aaacacattc 3120
   aggtgggggc ttgggaacgg gatcagcatt catcatgggg aagaaaacca caggccatca 3180
   gtggatcata cacaaaatac cactgatggc tggtacctgt atgctgacag ttctaatggg 3240
   aaatttggtg acacggctga cattctcact cctatcattt cactcacggg accaaaatgt 3300
   accttggtgt tctggacaca tatgaatggg gccaccgttg gttctctcca ggtgctcatc 3360
   aagaaagata acgttacttc taaattgtgg gctcaaactg gacagcaagg tgcacagtgg 3420
   aagagagcag aagtgttttt aggcattcgt tcacatacac agattgtctt cagagccaaa 3480
   cgtggtatca gttacatagg agatgtagca gtggatgata tttccttcca agattgctcc 3540
   cctttgctta gcccagagag aaagtgtact gatcatgaat tcatgtgtgc taataagcac 3600
   tgcattgcca aagacaagct gtgtgatttt gtgaatgatt gtgctgataa ttcagatgag 3660
   actactttca tttgccgtac ctccagtggg cgctgtgatt tcgaatttga tctttgttcc 3720
   tggaagcagg agaaagatga ggactttgac tggaacctga aagctagcag catccctgca 3780
   gcaggcacag agccagcagc agatcacact ttgggaaatt catctggtca ttacatcttt 3840
   ataaagagtt tgtttcctca gcagcccatg agagctgcca gaatttcaag tccagttata 3900
   agtaagagaa gcaaaaactg caagattatt tttcattatc acatgtatgg aaatggcatt 3960
   ggggcactca ccttaatgca ggtgtcagtc acaaaccaaa cgaaggttct acttaacctc 4020
   actgtagaac aaggcaattt ctggcggaga gaagaactgt cactgtttgg tgatgaagac 4080
   ttccaactca aatttgaagg tagagttggg aaaggtcagc gtggagacat tgcacttgat 4140
   gacattgtgc ttacagaaaa ttgtctatca ctccatgatt ccgtgcaaga agaactggca 4200
   gtgcctcttc caacaggttt ctgcccactt ggctataggg aatgtcataa tggaaaatgc 4260
   tataggctgg aacaaagctg taacttcgta gataactgtg gagataatac tgatgaaaat 4320
   gagtgtggta gctcctgtac ttttgaaaaa ggctggtgtg gctggcaaaa ctcccaggct 4380
   gacaactttg attgggtttt aggggttggc tctcatcaaa gcttaagacc tcccaaagac 4440
   cacacacttg gaaatgaaaa tgggcacttc atgtatctgg aagctactgc agtgggcctt 4500
   cggggtgaca aagcacactt caggagtacc atgtggcgag aatccagtgc agcctgcacc 4560
   atgagcttct ggtatttcgt atctgcaaag gccacaggat ccattcagat tctcatcaag 4620
   acagagaaag gactatcaaa agtatggcaa gaaagtaagc agaaccctgg taatcattgg 4680
   caaaaggctg acatcctgct aggaaagtta aggaattttg aagtcatatt tcaaggtatc 4740
   agaacaaggg acctgggagg aggagctgca attgatgata ttgaatttaa aaactgcaca 4800
   actgtgggag agatctctga gctttgtccg gaaatcactg attttttgtg ccgggacaag 4860
   aagtgcattg catcccacct tctttgtgac tataagccag actgctctga taggtctgat 4920
   gaagctcact gtgcacatta tacaagcaca acaggaagct gcaattttga aacaagttca 4980
   ggaaactgga ccacagcctg cagtcttact caagactctg aggatgactt ggactgggcc 5040
   attggcagca gaattcctgc caaagcatta attccagact ctgatcacac gccaggtagt 5100
   ggtcagcact tcctgtacgt caactcatct ggctccaagg aaggatccgt tgccagaatt   5160
   actacttcca aatccttccc agcaagcctt ggaatgtgta ctgttcggtt ctggttctac   5220
   atgattgatc ccaggagtat gggaatatta aaggtgtata ccattgaaga atcggggcta 5280
   aacatcctgg tgtggtcagt gattggaaat aaaagaacgg gatggacata tggctctgtg 5340
   cctctctcca gtaacagtcc gtttaaggtg gcatttgaag ctgatttgga tggaaatgag 5400
   gacatcttta ttgctcttga tgacatctct tttaccccag agtgtgtgac tggaggtcct 5460
   gtcccagtgc agccatcacc ctgtgaagct gatcagtttt cttgtatcta cacactccaa 5520
   tgtgtccctc tctcagggaa atgtgatgga catgaagact gcatagatgg atctgatgaa 5580
   atggattgtc ctctcagccc cacccctcca ctctgtagta acatggagtt cccgtgctct 5640
   acagacgagt gtataccttc cctcctgcta tgcgatggag tgcccgactg ccactttaat 5700
   gaagatgagc tcatctgctc caacaaaagc tgttctaatg gagctctggt gtgtgcctcc 5760
   tccaacagct gtatcccagc ccaccagcgc tgtgatggtt ttgccgactg catggatttc 5820
   cagcttgatg agtccagctg ctccgaatgt ccattaaatt actgcagaaa tggtgggact   5880
   tgtgtagtgg agaaaaatgg tcctatgtgt cgatgtagac aaggctggaa aggaaatcga   5940
   tgccatatca agtttaatcc tcctgctaca gacttcacat acgctcagaa taatacatgg   6000
   actctcctgg gtattggatt agcattcctg atgactcaca tcacagttgc agtcttgtgt   6060
   tttcttgcaa acagaaaggt accaataagg aaaaccgagg gaagtggtaa ctgtgccttt   6120
   gtcaatccag tttacgggaa ctggagcaac ccagagaaaa cagagagttc tgtctattcc   6180
   ttctcaaacc cattatatgg cacaacatca ggaagcctgg agaccctgtc acatcatctc   6240
   aaa                                                                  6243
<210> 14
   <211> 2081
   <212> PRT
   <213> Homo sapiens
<400> 14
   Gln Gln Gly Thr Glu Ser Phe Gln Cys Asp Asn Gly Val Ser Leu Pro
   1 5 10 15
   Pro Asp Ser Ile Cys Asp Phe Thr Asp Gln Cys Gly Asp Ser Ser Asp
   20 25 30
   Glu Arg His Trp Leu Ser Phe Ala His Pro Cys Asn Pro Leu Gly Ser
   35 40 45
   Ser Leu Glu Ser Asp Asn Trp Asn Lys Lys Lys Leu Glu Ser Gly Ala
   50 55 60
   Cys Leu Gly Phe Gly Ala Ala Ala Ala Ser Gln Asp Pro Arg Gln Glu
   65 70 75 80
   Leu Arg Lys Lys Leu Val Trp Thr Ala Asp Pro Arg Asn Trp Arg Ala
   85 90 95
   Ile Thr Phe Tyr Tyr Phe Ser Cys Gln Val Ser Gly Lys Leu Met Val
   100 105 110
   Gly Leu Gln Thr Ala Cys Gly Gly Pro Ile Gln His Leu Trp Gln Asn
   115 120 125
   Thr Ala Ala Leu Pro Asn Gln Trp Glu Arg Asn Val Ile Lys Ile Gln
   130 135 140
   Ser Ser Gln Arg Phe Gln Val Val Phe Glu Gly Gln Met Ala Ser Thr
   145 150 155 160
   Tyr Glu Gln Asp Glu Val Ile Ala Ile Asp Asp Ile Ser Phe Ser Ser
   165 170 175
   Gly Cys Leu Pro Ala Asn Asp Gly Ile Leu Leu Cys Gln Glu Ala Leu
   180 185 190
   Asn Ala Glu Arg Glu Leu Cys His Pro Asp Thr Asp Leu Cys Arg Phe
   195 200 205
   Asp Ala Thr Asp Glu Glu Leu Arg Leu Cys Gln Ala Cys Gly Phe Glu
   210 215 220
   Phe Asp Met Cys Glu Trp Thr Ser Glu Ala Ser Ala Gly Gln Ile Ser
   225 230 235 240
   Trp Met Arg Thr Lys Ala Arg Glu Ile Pro Ala Phe Glu Ser Thr Pro
   245 250 255
   Gln Gln Asp Gln Gly Gly Asp Asp Glu Gly Tyr Tyr Val Trp Val Gly
   260 265 270
   Ala Lys His Gly Phe Thr Leu Asn His Leu Asp Ser Arg Ala Tyr Leu
   275 280 285
   Asn Ser Ser Val Cys His Cys Leu Gly Lys Ser Cys His Leu Gln Phe
   290 295 300
   Tyr Tyr Ala Met Glu Ser Ser Val Leu Arg Val Arg Leu Tyr Asn Asn
   305 310 315 320
   Lys Glu Glu Glu Ile Phe Trp Thr Tyr Asn Ile Ser Thr His Ser Gln
   325 330 335
   Trp Val Lys Ala Asp Val Leu Ile Pro Glu Asp Leu Lys Thr Phe Lys
   340 345 350
   Thr Gln Ser Arg Lys Leu Cys Ser Ala Asp Glu Phe Pro Cys Thr Ser
   355 360 365
   Gly Gln Cys Ile Ala Lys Glu Ser Val Cys Asp Ser Arg Gln Asp Cys
   370 375 380
   Ser Asp Glu Ser Asp Glu Asp Pro Ala Thr Cys Ser Lys His Leu Thr
   385 390 395 400
   Cys Asp Phe Glu Ser Gly Phe Cys Gly Trp Glu Pro Phe Leu Thr Glu
   405 410 415
   Asp Ser His Trp Lys Leu Met Lys Gly Leu Asn Asn Gly Glu His His
   420 425 430
   Phe Pro Ala Ala Asp His Thr Ala Asn Ile Asn His Gly Ser Phe Ile
   435 440 445
   Tyr Leu Glu Ala Gln Arg Ser Pro Gly Val Ala Lys Leu Gly Ser Pro
   450 455 460
   Val Leu Thr Lys Leu Leu Thr Ala Ser Thr Pro Cys Gln Val Gln Phe
   465 470 475 480
   Trp Tyr His Leu Ser Gln His Ser Asn Leu Ser Val Phe Thr Arg Thr
   485 490 495
   Ser Leu Asp Gly Asn Leu Gln Lys Gln Gly Lys Ile Ile Arg Phe Ser
   500 505 510
   Glu Ser Gln Trp Ser His Ala Lys Ile Asp Leu Ile Ala Glu Ala Gly
   515 520 525
   Glu Ser Thr Leu Pro Phe Gln Leu Ile Leu Glu Ala Thr Val Leu Ser
   530 535 540
   Ser Asn Ala Thr Val Ala Leu Asp Asp Ile Ser Val Ser Gln Glu Cys
   545 550 555 560
   Glu Ile Ser Tyr Lys Ser Leu Pro Arg Thr Ser Thr Gln Ser Lys Phe
   565 570 575
   Ser Lys Cys Asp Phe Glu Ala Asn Ser Cys Asp Trp Phe Glu Ala Ile
   580 585 590
   Ser Gly Asp His Phe Asp Trp Ile Arg Ser Ser Gln Ser Glu Leu Ser
   595 600 605
   Ala Asp Phe Glu His Gln Ala Pro Pro Arg Asp His Ser Leu Asn Ala
   610 615 620
   Ser Gln Gly His Phe Met Phe Ile Leu Lys Lys Ser Ser Ser Leu Trp
   625 630 635 640
   Gln Val Ala Lys Leu Gln Ser Pro Thr Phe Ser Gln Thr Gly Pro Gly
   645 650 655
   Cys Ile Leu Ser Phe Trp Phe Tyr Asn Tyr Gly Leu Ser Val Gly Ala
   660 665 670
   Ala Glu Leu Gln Leu His Met Glu Asn Ser His Asp Ser Thr Val Ile
   675 680 685
   Trp Arg Val Leu Tyr Asn Gln Gly Lys Gln Trp Leu Glu Ala Thr Ile
   690 695 700
   Gln Leu Gly Arg Leu Ser Gln Pro Phe His Leu Ser Leu Asp Lys Val
   705 710 715 720
   Ser Leu Gly Ile Tyr Asp Gly Val Ser Ala Ile Asp Asp Ile Arg Phe
   725 730 735
   Glu Asn Cys Thr Leu Pro Leu Pro Ala Glu Ser Cys Glu Gly Leu Asp
   740 745 750
   His Phe Trp Cys Arg His Thr Arg Ala Cys Ile Glu Lys Leu Arg Leu
   755 760 765
   Cys Asp Leu Val Asp Asp Cys Gly Asp Arg Thr Asp Glu Val Asn Cys
   770 775 780
   Ala Pro Glu Leu Gln Cys Asn Phe Glu Thr Gly Ile Cys Asn Trp Glu
   785 790 795 800
   Gln Asp Ala Lys Asp Asp Phe Asp Trp Thr Arg Ser Gln Gly Pro Thr
   805 810 815
   Pro Thr Leu Asn Thr Gly Pro Met Lys Asp Asn Thr Leu Gly Thr Ala
   820 825 830
   Lys Gly His Tyr Leu Tyr Ile Glu Ser Ser Glu Pro Gln Ala Phe Gln
   835 840 845
   Asp Ser Ala Ala Leu Leu Ser Pro Ile Leu Asn Ala Thr Asp Thr Lys
   850 855 860
   Gly Cys Thr Phe Arg Phe Tyr Tyr His Met Phe Gly Lys Arg Ile Tyr
   865 870 875 880
   Arg Leu Ala Ile Tyr Gln Arg Ile Trp Ser Asp Ser Arg Gly Gln Leu
   885 890 895
   Leu Trp Gln Ile Phe Gly Asn Gln Gly Asn Arg Trp Ile Arg Lys His
   900 905 910
   Leu Asn Ile Ser Ser Arg Gln Pro Phe Gln Ile Leu Val Glu Ala Ser
   915 920 925
   Val Gly Asp Gly Phe Thr Gly Asp Ile Ala Ile Asp Asp Leu Ser Phe
   930 935 940
   Met Asp Cys Thr Leu Tyr Pro Gly Asn Leu Pro Ala Asp Leu Pro Thr
   945 950 955 960
   Pro Pro Glu Thr Ser Val Pro Val Thr Leu Pro Pro His Asn Cys Thr
                   965                 970                 975
   Asp Asn Glu Phe Ile Cys Arg Ser Asp Gly His Cys Ile Glu Lys Met
               980                 985                 990
   Gln Lys Cys Asp Phe Lys Tyr Asp Cys Pro Asp Lys Ser Asp Glu Ala
           995                 1000                1005
   Ser Cys Val Met Glu Val Cys Ser Phe Glu Lys Arg Ser Leu Cys Lys
       1010                1015                1020
   Trp Tyr Gln Pro Ile Pro Val His Leu Leu Gln Asp Ser Asn Thr Phe
   1025                1030                1035                1040
   Arg Trp Gly Leu Gly Asn Gly Ile Ser Ile His His Gly Glu Glu Asn
                   1045                1050                1055
   His Arg Pro Ser Val Asp His Thr Gln Asn Thr Thr Asp Gly Trp Tyr
               1060                1065                1070
   Leu Tyr Ala Asp Ser Ser Asn Gly Lys Phe Gly Asp Thr Ala Asp Ile
           1075                1080                1085
   Leu Thr Pro Ile Ile Ser Leu Thr Gly Pro Lys Cys Thr Leu Val Phe
       1090                1095                1100
   Trp Thr His Met Asn Gly Ala Thr Val Gly Ser Leu Gln Val Leu Ile
   1105                1110                1115                1120
   Lys Lys Asp Asn Val Thr Ser Lys Leu Trp Ala Gln Thr Gly Gln Gln
                   1125                1130                1135
   Gly Ala Gln Trp Lys Arg Ala Glu Val Phe Leu Gly Ile Arg Ser His
               1140                1145                1150
   Thr Gln Ile Val Phe Arg Ala Lys Arg Gly Ile Ser Tyr Ile Gly Asp
           1155                1160                1165
   Val Ala Val Asp Asp Ile Ser Phe Gln Asp Cys Ser Pro Leu Leu Ser
       1170                1175                1180
   Pro Glu Arg Lys Cys Thr Asp His Glu Phe Met Cys Ala Asn Lys His
   1185                1190                1195                1200
   Cys Ile Ala Lys Asp Lys Leu Cys Asp Phe Val Asn Asp Cys Ala Asp
                   1205                1210                1215
   Asn Ser Asp Glu Thr Thr Phe Ile Cys Arg Thr Ser Ser Gly Arg Cys
               1220                1225                1230
   Asp Phe Glu Phe Asp Leu Cys Ser Trp Lys Gln Glu Lys Asp Glu Asp
           1235                1240                1245
   Phe Asp Trp Asn Leu Lys Ala Ser Ser Ile Pro Ala Ala Gly Thr Glu
       1250                1255                1260
   Pro Ala Ala Asp His Thr Leu Gly Asn Ser Ser Gly His Tyr Ile Phe
   1265                1270                1275                1280
   Ile Lys Ser Leu Phe Pro Gln Gln Pro Met Arg Ala Ala Arg Ile Ser
                   1285                1290                1295
   Ser Pro Val Ile Ser Lys Arg Ser Lys Asn Cys Lys Ile Ile Phe His
               1300                1305                1310
   Tyr His Met Tyr Gly Asn Gly Ile Gly Ala Leu Thr Leu Met Gln Val
           1315                1320                1325
   Ser Val Thr Asn Gln Thr Lys Val Leu Leu Asn Leu Thr Val Glu Gln
       1330                1335                1340
   Gly Asn Phe Trp Arg Arg Glu Glu Leu Ser Leu Phe Gly Asp Glu Asp
   1345                1350                1355                1360
   Phe Gln Leu Lys Phe Glu Gly Arg Val Gly Lys Gly Gln Arg Gly Asp
                   1365                1370                1375
   Ile Ala Leu Asp Asp Ile Val Leu Thr Glu Asn Cys Leu Ser Leu His
               1380                1385                1390
   Asp Ser Val Gln Glu Glu Leu Ala Val Pro Leu Pro Thr Gly Phe Cys
           1395                1400                1405
   Pro Leu Gly Tyr Arg Glu Cys His Asn Gly Lys Cys Tyr Arg Leu Glu
       1410                1415                1420
   Gln Ser Cys Asn Phe Val Asp Asn Cys Gly Asp Asn Thr Asp Glu Asn
   1425                1430                1435                1440
   Glu Cys Gly Ser Ser Cys Thr Phe Glu Lys Gly Trp Cys Gly Trp Gln
                   1445                1450                1455
   Asn Ser Gln Ala Asp Asn Phe Asp Trp Val Leu Gly Val Gly Ser His
               1460                1465                1470
   Gln Ser Leu Arg Pro Pro Lys Asp His Thr Leu Gly Asn Glu Asn Gly
           1475                1480                1485
   His Phe Met Tyr Leu Glu Ala Thr Ala Val Gly Leu Arg Gly Asp Lys
       1490                1495                1500
   Ala His Phe Arg Ser Thr Met Trp Arg Glu Ser Ser Ala Ala Cys Thr
   1505 1510 1515 1520
   Met Ser Phe Trp Tyr Phe Val Ser Ala Lys Ala Thr Gly Ser Ile Gln
   1525 1530 1535
   Ile Leu Ile Lys Thr Glu Lys Gly Leu Ser Lys Val Trp Gln Glu Ser
   1540 1545 1550
   Lys Gln Asn Pro Gly Asn His Trp Gln Lys Ala Asp Ile Leu Leu Gly
   1555 1560 1565
   Lys Leu Arg Asn Phe Glu Val Ile Phe Gln Gly Ile Arg Thr Arg Asp
   1570 1575 1580
   Leu Gly Gly Gly Ala Ala Ile Asp Asp Ile Glu Phe Lys Asn Cys Thr
   1585 1590 1595 1600
   Thr Val Gly Glu Ile Ser Glu Leu Cys Pro Glu Ile Thr Asp Phe Leu
   1605 1610 1615
   Cys Arg Asp Lys Lys Cys Ile Ala Ser His Leu Leu Cys Asp Tyr Lys
   1620 1625 1630
   Pro Asp Cys Ser Asp Arg Ser Asp Glu Ala His Cys Ala His Tyr Thr
   1635 1640 1645
   Ser Thr Thr Gly Ser Cys Asn Phe Glu Thr Ser Ser Gly Asn Trp Thr
   1650 1655 1660
   Thr Ala Cys Ser Leu Thr Gln Asp Ser Glu Asp Asp Leu Asp Trp Ala
   1665 1670 1675 1680
   Ile Gly Ser Arg Ile Pro Ala Lys Ala Leu Ile Pro Asp Ser Asp His
   1685 1690 1695
   Thr Pro Gly Ser Gly Gln His Phe Leu Tyr Val Asn Ser Ser Gly Ser
   1700 1705 1710
   Lys Glu Gly Ser Val Ala Arg Ile Thr Thr Ser Lys Ser Phe Pro Ala
   1715 1720 1725
   Ser Leu Gly Met Cys Thr Val Arg Phe Trp Phe Tyr Met Ile Asp Pro
   1730 1735 1740
   Arg Ser Met Gly Ile Leu Lys Val Tyr Thr Ile Glu Glu Ser Gly Leu
   1745 1750 1755 1760
   Asn Ile Leu Val Trp Ser Val Ile Gly Asn Lys Arg Thr Gly Trp Thr
   1765 1770 1775
   Tyr Gly Ser Val Pro Leu Ser Ser Asn Ser Pro Phe Lys Val Ala Phe
   1780 1785 1790
   Glu Ala Asp Leu Asp Gly Asn Glu Asp Ile Phe Ile Ala Leu Asp Asp
   1795 1800 1805
   Ile Ser Phe Thr Pro Glu Cys Val Thr Gly Gly Pro Val Pro Val Gln
   1810 1815 1820
   Pro Ser Pro Cys Glu Ala Asp Gln Phe Ser Cys Ile Tyr Thr Leu Gln
   1825 1830 1835 1840
   Cys Val Pro Leu Ser Gly Lys Cys Asp Gly His Glu Asp Cys Ile Asp
   1845 1850 1855
   Gly Ser Asp Glu Met Asp Cys Pro Leu Ser Pro Thr Pro Pro Leu Cys
   1860 1865 1870
   Ser Asn Met Glu Phe Pro Cys Ser Thr Asp Glu Cys Ile Pro Ser Leu
   1875 1880 1885
   Leu Leu Cys Asp Gly Val Pro Asp Cys His Phe Asn Glu Asp Glu Leu
   1890 1895 1900
   Ile Cys Ser Asn Lys Ser Cys Ser Asn Gly Ala Leu Val Cys Ala Ser
   1905 1910 1915 1920
   Ser Asn Ser Cys Ile Pro Ala His Gln Arg Cys Asp Gly Phe Ala Asp
   1925 1930 1935
   Cys Met Asp Phe Gln Leu Asp Glu Ser Ser Cys Ser Glu Cys Pro Leu
   1940 1945 1950
   Asn Tyr Cys Arg Asn Gly Gly Thr Cys Val Val Glu Lys Asn Gly Pro
   1955 1960 1965
   Met Cys Arg Cys Arg Gln Gly Trp Lys Gly Asn Arg Cys His Ile Lys
   1970 1975 1980
   Phe Asn Pro Pro Ala Thr Asp Phe Thr Tyr Ala Gln Asn Asn Thr Trp
   1985 1990 1995 2000
   Thr Leu Leu Gly Ile Gly Leu Ala Phe Leu Met Thr His Ile Thr Val
   2005 2010 2015
   Ala Val Leu Cys Phe Leu Ala Asn Arg Lys Val Pro Ile Arg Lys Thr
   2020 2025 2030
   Glu Gly Ser Gly Asn Cys Ala Phe Val Asn Pro Val Tyr Gly Asn Trp
               2035                    2040                    2045
   Ser Asn Pro Glu Lys Thr Glu Ser Ser Val Tyr Ser Phe Ser Asn Pro
           2050                2055                    2060
   Leu Tyr Gly Thr Thr Ser Gly Ser Leu Glu Thr Leu Ser His His Leu
   2065                    2070                    2075                2080
   Lys
<210> 15
   <211> 6297
   <212> DNA
   <213> Homo sapiens
<400> 15
   cagcaaggga cagaaagctt tcagtgtgac aatggagtct ccttgcctcc tgacagcatt    60
   tgtgacttca cagatcagtg tggggatagc agtgatgaac ggcactgttt gaattatgaa   120
   agatgtgatt ttgaggatgg tctctgtcat atgactcaag atcagagtct gcaacctagt   180
   tggacaaaga gaagtgggat gattggtcta tcacctccat tttatgatca caatggtgat   240
   gtgtctgctc acttcctctc actggtttcc agagtggatt ctatttcctc aagtttaaga   300
   agcagagttt tccttccaac aaatgatcaa catgactgcc agattacatt ttattacttc   360
   tcctgccaag tgagtggcaa attaatggtt gggcttcaaa ctgcatgtgg aggtcctatt   420
   cagcatttat ggcaaaacac agctgcactc ccaaatcagt gggagagaaa tgtcatcaaa   480
   atccagagtt cacagagatt tcaggttgtt tttgaaggtc aaatggcttc aacgtatgaa   540
   caggatgaag tcattgctat tgatgatata tctttcagtt caggctgctt gcctgccaat   600
   gatggaattt tactgtgtca agaagcattg aatgctgagc gggagctatg ccatccagat   660
   acagatctct gcagatttga tgctacagat gaagagttga gattgtgtca ggcctgtggg   720
   tttgaatttg acatgtgtga gtggacgtca gaagcatctg ctggccaaat ttcctggatg   780
   cgcacaaaag cgagagagat ccctgcattc gaatccacac ctcagcagga tcaaggaggt   840
   gatgatgaag gttattatgt atgggtaggc gctaagcatg gtttcactct taaccattta   900
   gacagcaggg cttacctaaa tagctctgtg tgtcattgcc tgggcaagag ctgtcatctt   960
   caattctatt atgcaatgga aagcagtgtc ctgagagtaa gactgtataa taataaggaa  1020
   gaagaaatat tttggacata caacatatca actcacagcc aatgggtgaa agcagatgtg  1080
   ttaataccag aagatctgaa gacatttaag acccaatcca gaaagctttg ctctgcagac  1140
   gaattccctt gcactagtgg ccagtgcatc gccaaagaat ctgtctgtga ctctcggcag  1200
   gactgctccg atgagagtga tgaagaccca gcaacttgct caaagcatct cacctgtgac  1260
   tttgagtcgg gtttctgcgg ttgggagcca tttctcacag aagattcaca ctggaagctg  1320
   atgaaaggat tgaataatgg agagcaccac tttcctgcag ctgatcacac agcaaacata  1380
   aatcatggat cgtttattta tttggaggca cagcgctccc ccggggtggc caagcttgga  1440
   agtcctgttc ttacaaaatt gctcactgcc tctaccccat gtcaggtgca gttttggtat  1500
   catttgtctc aacattcaaa tctctcagtt tttacaagaa cgtctctaga tggaaacttg  1560
   caaaagcagg gcaaaataat cagattctcc gaatctcagt ggagccacgc aaaaattgat  1620
   ctcattgcag aagcgggaga atctactcta ccttttcagt taattttgga agctactgtt  1680
   ttgtcgtcaa atgctaccgt tgctctagat gacatcagtg tgtcccagga atgtgaaatt  1740
   tcctataaat cactaccaag gaccagtaca caaagcaagt tttccaagtg tgactttgaa  1800
   gcaaacagct gtgattggtt tgaagcaatt agtggtgacc attttgactg gatacggagc  1860
   tctcagagtg aactttctgc tgattttgag caccaggctc cacctcggga tcatagtctc  1920
   aacgcatctc aagggcattt tatgttcatt ctgaagaaaa gcagcagctt gtggcaagtt  1980
   gctaagcttc agagcccaac tttcagccag acaggacctg gatgcatact ttccttctgg  2040
   ttctataact atggcctgtc agtgggagca gctgagctgc agctacatat ggaaaattct  2100
   catgactcaa cagtgatttg gagagtatta tacaatcagg gcaaacaatg gttggaggca  2160
   accattcagc tagggcgcct ttcgcagccc ttccatttgt cactagataa agtcagtctg  2220
   ggcatttatg atggggtctc agctattgat gacatccgat ttgaaaattg tactctccct  2280
   cttcctgctg agagctgtga agggctggat catttctggt gtcgccacac cagggcttgc  2340
   atagaaaagc ttcggttatg tgatctggtg gatgactgtg gtgatcgtac tgatgaagtc  2400
   aactgtgcac ctgagctgca gtgtaacttt gaaactggaa tctgtaactg ggaacaagat  2460
   gcaaaagatg actttgattg gaccaggagc cagggtccaa ctccaacact taacacaggg  2520
   ccaatgaaag ataacactct gggcacagct aaaggacact atctctacat agaatcttca  2580
   gagccacagg cttttcaaga cagtgctgcc ttactcagcc caatccttaa tgccactgat  2640
   acaaaaggct gcaccttccg cttctattac cacatgtttg gaaagcgcat ttataggttg  2700
   gcaatctacc aacgaatctg gagtgactca aggggacagc tgctgtggca gatatttggg  2760
   aatcaaggca acagatggat taggaaacac ctcaacattt ccagcaggca gccctttcag  2820
   atattggtgg aggcttcagt gggagatggc ttcactggag atattgcgat tgatgatctg  2880
   tcatttatgg actgcaccct ctaccctggt aatttgccag cagacctccc aactccacca  2940
   gaaacgtcag ttcctgtaac attacctcca cacaactgca cagacaatga atttatctgc  3000
   aggtctgatg gtcactgcat tgaaaaaatg cagaaatgtg attttaaata tgactgccct  3060
   gacaaatcag atgaagcatc ctgtgttatg gaagtttgca gctttgagaa aagaagcctg  3120
   tgtaaatggt atcaaccaat cccagtacat ttgcttcaag attcaaacac attcaggtgg  3180
   gggcttggga acgggatcag cattcatcat ggggaagaaa accacaggcc atcagtggat  3240
   catacacaaa ataccactga tggctggtac ctgtatgctg acagttctaa tgggaaattt  3300
   ggtgacacgg ctgacattct cactcctatc atttcactca cgggaccaaa atgtaccttg   3360
   gtgttctgga cacatatgaa tggggccacc gttggttctc tccaggtgct catcaagaaa   3420
   gataacgtta cttctaaatt gtgggctcaa actggacagc aaggtgcaca gtggaagaga   3480
   gcagaagtgt ttttaggcat tcgttcacat acacagattg tcttcagagc caaacgtggt   3540
   atcagttaca taggagatgt agcagtggat gatatttcct tccaagattg ctcccctttg   3600
   cttagcccag agagaaagtg tactgatcat gaattcatgt gtgctaataa gcactgcatt   3660
   gccaaagaca agctgtgtga ttttgtgaat gattgtgctg ataattcaga tgagactact   3720
   ttcatttgcc gtacctccag tgggcgctgt gatttcgaat ttgatctttg ttcctggaag   3780
   caggagaaag atgaggactt tgactggaac ctgaaagcta gcagcatccc tgcagcaggc   3840
   acagagccag cagcagatca cactttggga aattcatctg gtcattacat ctttataaag   3900
   agtttgtttc ctcagcagcc catgagagct gccagaattt caagtccagt tataagtaag   3960
   agaagcaaaa actgcaagat tatttttcat tatcacatgt atggaaatgg cattggggca   4020
   ctcaccttaa tgcaggtgtc agtcacaaac caaacgaagg ttctacttaa cctcactgta   4080
   gaacaaggca atttctggcg gagagaagaa ctgtcactgt ttggtgatga agacttccaa   4140
   ctcaaatttg aaggtagagt tgggaaaggt cagcgtggag acattgcact tgatgacatt   4200
   gtgcttacag aaaattgtct atcactccat gattccgtgc aagaagaact ggcagtgcct   4260
   cttccaacag gtttctgccc acttggctat agggaatgtc ataatggaaa atgctatagg   4320
   ctggaacaaa gctgtaactt cgtagataac tgtggagata atactgatga aaatgagtgt   4380
   ggtagctcct gtacttttga aaaaggctgg tgtggctggc aaaactccca ggctgacaac   4440
   tttgattggg ttttaggggt tggctctcat caaagcttaa gacctcccaa agaccacaca   4500
   cttggaaatg aaaatgggca cttcatgtat ctggaagcta ctgcagtggg ccttcggggt   4560
   gacaaagcac acttcaggag taccatgtgg cgagaatcca gtgcagcctg caccatgagc   4620
   ttctggtatt tcgtatctgc aaaggccaca ggatccattc agattctcat caagacagag   4680
   aaaggactat caaaagtatg gcaagaaagt aagcagaacc ctggtaatca ttggcaaaag   4740
   gctgacatcc tgctaggaaa gttaaggaat tttgaagtca tatttcaagg tatcagaaca   4800
   agggacctgg gaggaggagc tgcaattgat gatattgaat ttaaaaactg cacaactgtg   4860
   ggagagatct ctgagctttg tccggaaatc actgattttt tgtgccggga caagaagtgc   4920
   attgcatccc accttctttg tgactataag ccagactgct ctgataggtc tgatgaagct   4980
   cactgtgcac attatacaag cacaacagga agctgcaatt ttgaaacaag ttcaggaaac   5040
   tggaccacag cctgcagtct tactcaagac tctgaggatg acttggactg ggccattggc   5100
   agcagaattc ctgccaaagc attaattcca gactctgatc acacgccagg tagtggtcag   5160
   cacttcctgt acgtcaactc atctggctcc aaggaaggat ccgttgccag aattactact   5220
   tccaaatcct tcccagcaag ccttggaatg tgtactgttc ggttctggtt ctacatgatt   5280
   gatcccagga gtatgggaat attaaaggtg tataccattg aagaatcggg gctaaacatc   5340
   ctggtgtggt cagtgattgg aaataaaaga acgggatgga catatggctc tgtgcctctc   5400
   tccagtaaca gtccgtttaa ggtggcattt gaagctgatt tggatggaaa tgaggacatc   5460
   tttattgctc ttgatgacat ctcttttacc ccagagtgtg tgactggagg tcctgtccca   5520
   gtgcagccat caccctgtga agctgatcag ttttcttgta tctacacact ccaatgtgtc   5580
   cctctctcag ggaaatgtga tggacatgaa gactgcatag atggatctga tgaaatggat   5640
   tgtcctctca gccccacccc tccactctgt agtaacatgg agttcccgtg ctctacagac   5700
   gagtgtatac cttccctcct gctatgcgat ggagtgcccg actgccactt taatgaagat   5760
   gagctcatct gctccaacaa aagctgttct aatggagctc tggtgtgtgc ctcctccaac   5820
   agctgtatcc cagcccacca gcgctgtgat ggttttgccg actgcatgga tttccagctt   5880
   gatgagtcca gctgctccga atgtccatta aattactgca gaaatggtgg gacttgtgta   5940
   gtggagaaaa atggtcctat gtgtcgatgt agacaaggct ggaaaggaaa tcgatgccat   6000
   atcaagttta atcctcctgc tacagacttc acatacgctc agaataatac atggactctc   6060
   ctgggtattg gattagcatt cctgatgact cacatcacag ttgcagtctt gtgttttctt   6120
   gcaaacagaa aggtaccaat aaggaaaacc gagggaagtg gtaactgtgc ctttgtcaat   6180
   ccagtttacg ggaactggag caacccagag aaaacagaga gttctgtcta ttccttctca   6240
   aacccattat atggcacaac atcaggaagc ctggagaccc tgtcacatca tctcaaa      6297
<210> 16
   <211> 2099
   <212> PRT
   <213> Homo sapiens
<400> 16
   Gln Gln Gly Thr Glu Ser Phe Gln Cys Asp Asn Gly Val Ser Leu Pro
   1               5                   10                  15
   Pro Asp Ser Ile Cys Asp Phe Thr Asp Gln Cys Gly Asp Ser Ser Asp
               20                  25                  30
   Glu Arg His Cys Leu Asn Tyr Glu Arg Cys Asp Phe Glu Asp Gly Leu
           35                  40                  45
   Cys His Met Thr Gln Asp Gln Ser Leu Gln Pro Ser Trp Thr Lys Arg
       50                  55                  60
   Ser Gly Met Ile Gly Leu Ser Pro Pro Phe Tyr Asp His Asn Gly Asp
   65                  70                  75                  80
   Val Ser Ala His Phe Leu Ser Leu Val Ser Arg Val Asp Ser Ile Ser
                   85                  90                  95
   Ser Ser Leu Arg Ser Arg Val Phe Leu Pro Thr Asn Asp Gln His Asp
   100 105 110
   Cys Gln Ile Thr Phe Tyr Tyr Phe Ser Cys Gln Val Ser Gly Lys Leu
   115 120 125
   Met Val Gly Leu Gln Thr Ala Cys Gly Gly Pro Ile Gln His Leu Trp
   130 135 140
   Gln Asn Thr Ala Ala Leu Pro Asn Gln Trp Glu Arg Asn Val Ile Lys
   145 150 155 160
   Ile Gln Ser Ser Gln Arg Phe Gln Val Val Phe Glu Gly Gln Met Ala
   165 170 175
   Ser Thr Tyr Glu Gln Asp Glu Val Ile Ala Ile Asp Asp Ile Ser Phe
   180 185 190
   Ser Ser Gly Cys Leu Pro Ala Asn Asp Gly Ile Leu Leu Cys Gln Glu
   195 200 205
   Ala Leu Asn Ala Glu Arg Glu Leu Cys His Pro Asp Thr Asp Leu Cys
   210 215 220
   Arg Phe Asp Ala Thr Asp Glu Glu Leu Arg Leu Cys Gln Ala Cys Gly
   225 230 235 240
   Phe Glu Phe Asp Met Cys Glu Trp Thr Ser Glu Ala Ser Ala Gly Gln
   245 250 255
   Ile Ser Trp Met Arg Thr Lys Ala Arg Glu Ile Pro Ala Phe Glu Ser
   260 265 270
   Thr Pro Gln Gln Asp Gln Gly Gly Asp Asp Glu Gly Tyr Tyr Val Trp
   275 280 285
   Val Gly Ala Lys His Gly Phe Thr Leu Asn His Leu Asp Ser Arg Ala
   290 295 300
   Tyr Leu Asn Ser Ser Val Cys His Cys Leu Gly Lys Ser Cys His Leu
   305 310 315 320
   Gln Phe Tyr Tyr Ala Met Glu Ser Ser Val Leu Arg Val Arg Leu Tyr
   325 330 335
   Asn Asn Lys Glu Glu Glu Ile Phe Trp Thr Tyr Asn Ile Ser Thr His
   340 345 350
   Ser Gln Trp Val Lys Ala Asp Val Leu Ile Pro Glu Asp Leu Lys Thr
   355 360 365
   Phe Lys Thr Gln Ser Arg Lys Leu Cys Ser Ala Asp Glu Phe Pro Cys
   370 375 380
   Thr Ser Gly Gln Cys Ile Ala Lys Glu Ser Val Cys Asp Ser Arg Gln
   385 390 395 400
   Asp Cys Ser Asp Glu Ser Asp Glu Asp Pro Ala Thr Cys Ser Lys His
   405 410 415
   Leu Thr Cys Asp Phe Glu Ser Gly Phe Cys Gly Trp Glu Pro Phe Leu
   420 425 430
   Thr Glu Asp Ser His Trp Lys Leu Met Lys Gly Leu Asn Asn Gly Glu
   435 440 445
   His His Phe Pro Ala Ala Asp His Thr Ala Asn Ile Asn His Gly Ser
   450 455 460
   Phe Ile Tyr Leu Glu Ala Gln Arg Ser Pro Gly Val Ala Lys Leu Gly
   465 470 475 480
   Ser Pro Val Leu Thr Lys Leu Leu Thr Ala Ser Thr Pro Cys Gln Val
   485 490 495
   Gln Phe Trp Tyr His Leu Ser Gln His Ser Asn Leu Ser Val Phe Thr
   500 505 510
   Arg Thr Ser Leu Asp Gly Asn Leu Gln Lys Gln Gly Lys Ile Ile Arg
   515 520 525
   Phe Ser Glu Ser Gln Trp Ser His Ala Lys Ile Asp Leu Ile Ala Glu
   530 535 540
   Ala Gly Glu Ser Thr Leu Pro Phe Gln Leu Ile Leu Glu Ala Thr Val
   545 550 555 560
   Leu Ser Ser Asn Ala Thr Val Ala Leu Asp Asp Ile Ser Val Ser Gln
   565 570 575
   Glu Cys Glu Ile Ser Tyr Lys Ser Leu Pro Arg Thr Ser Thr Gln Ser
   580 585 590
   Lys Phe Ser Lys Cys Asp Phe Glu Ala Asn Ser Cys Asp Trp Phe Glu
   595 600 605
   Ala Ile Ser Gly Asp His Phe Asp Trp Ile Arg Ser Ser Gln Ser Glu
   610 615 620
   Leu Ser Ala Asp Phe Glu His Gln Ala Pro Pro Arg Asp His Ser Leu
   625 630 635 640
   Asn Ala Ser Gln Gly His Phe Met Phe Ile Leu Lys Lys Ser Ser Ser
   645 650 655
   Leu Trp Gln Val Ala Lys Leu Gln Ser Pro Thr Phe Ser Gln Thr Gly
   660 665 670
   Pro Gly Cys Ile Leu Ser Phe Trp Phe Tyr Asn Tyr Gly Leu Ser Val
   675 680 685
   Gly Ala Ala Glu Leu Gln Leu His Met Glu Asn Ser His Asp Ser Thr
   690 695 700
   Val Ile Trp Arg Val Leu Tyr Asn Gln Gly Lys Gln Trp Leu Glu Ala
   705 710 715 720
   Thr Ile Gln Leu Gly Arg Leu Ser Gln Pro Phe His Leu Ser Leu Asp
   725 730 735
   Lys Val Ser Leu Gly Ile Tyr Asp Gly Val Ser Ala Ile Asp Asp Ile
   740 745 750
   Arg Phe Glu Asn Cys Thr Leu Pro Leu Pro Ala Glu Ser Cys Glu Gly
   755 760 765
   Leu Asp His Phe Trp Cys Arg His Thr Arg Ala Cys Ile Glu Lys Leu
   770 775 780
   Arg Leu Cys Asp Leu Val Asp Asp Cys Gly Asp Arg Thr Asp Glu Val
   785 790 795 800
   Asn Cys Ala Pro Glu Leu Gln Cys Asn Phe Glu Thr Gly Ile Cys Asn
   805 810 815
   Trp Glu Gln Asp Ala Lys Asp Asp Phe Asp Trp Thr Arg Ser Gln Gly
   820 825 830
   Pro Thr Pro Thr Leu Asn Thr Gly Pro Met Lys Asp Asn Thr Leu Gly
   835 840 845
   Thr Ala Lys Gly His Tyr Leu Tyr Ile Glu Ser Ser Glu Pro Gln Ala
   850 855 860
   Phe Gln Asp Ser Ala Ala Leu Leu Ser Pro Ile Leu Asn Ala Thr Asp
   865 870 875 880
   Thr Lys Gly Cys Thr Phe Arg Phe Tyr Tyr His Met Phe Gly Lys Arg
   885 890 895
   Ile Tyr Arg Leu Ala Ile Tyr Gln Arg Ile Trp Ser Asp Ser Arg Gly
   900 905 910
   Gln Leu Leu Trp Gln Ile Phe Gly Asn Gln Gly Asn Arg Trp Ile Arg
   915 920 925
   Lys His Leu Asn Ile Ser Ser Arg Gln Pro Phe Gln Ile Leu Val Glu
   930 935 940
   Ala Ser Val Gly Asp Gly Phe Thr Gly Asp Ile Ala Ile Asp Asp Leu
   945 950 955 960
   Ser Phe Met Asp Cys Thr Leu Tyr Pro Gly Asn Leu Pro Ala Asp Leu
   965 970 975
   Pro Thr Pro Pro Glu Thr Ser Val Pro Val Thr Leu Pro Pro His Asn
   980 985 990
   Cys Thr Asp Asn Glu Phe Ile Cys Arg Ser Asp Gly His Cys Ile Glu
   995 1000 1005
   Lys Met Gln Lys Cys Asp Phe Lys Tyr Asp Cys Pro Asp Lys Ser Asp
   1010 1015 1020
   Glu Ala Ser Cys Val Met Glu Val Cys Ser Phe Glu Lys Arg Ser Leu
   1025 1030 1035 1040
   Cys Lys Trp Tyr Gln Pro Ile Pro Val His Leu Leu Gln Asp Ser Asn
   1045 1050 1055
   Thr Phe Arg Trp Gly Leu Gly Asn Gly Ile Ser Ile His His Gly Glu
   1060 1065 1070
   Glu Asn His Arg Pro Ser Val Asp His Thr Gln Asn Thr Thr Asp Gly
   1075 1080 1085
   Trp Tyr Leu Tyr Ala Asp Ser Ser Asn Gly Lys Phe Gly Asp Thr Ala
   1090 1095 1100
   Asp Ile Leu Thr Pro Ile Ile Ser Leu Thr Gly Pro Lys Cys Thr Leu
   1105 1110 1115 1120
   Val Phe Trp Thr His Met Asn Gly Ala Thr Val Gly Ser Leu Gln Val
   1125 1130 1135
   Leu Ile Lys Lys Asp Asn Val Thr Ser Lys Leu Trp Ala Gln Thr Gly
   1140 1145 1150
   Gln Gln Gly Ala Gln Trp Lys Arg Ala Glu Val Phe Leu Gly Ile Arg
   1155 1160 1165
   Ser His Thr Gln Ile Val Phe Arg Ala Lys Arg Gly Ile Ser Tyr Ile
   1170 1175 1180
   Gly Asp Val Ala Val Asp Asp Ile Ser Phe Gln Asp Cys Ser Pro Leu
   1185 1190 1195 1200
   Leu Ser Pro Glu Arg Lys Cys Thr Asp His Glu Phe Met Cys Ala Asn
   1205 1210 1215
   Lys His Cys Ile Ala Lys Asp Lys Leu Cys Asp Phe Val Asn Asp Cys
   1220 1225 1230
   Ala Asp Asn Ser Asp Glu Thr Thr Phe Ile Cys Arg Thr Ser Ser Gly
   1235 1240 1245
   Arg Cys Asp Phe Glu Phe Asp Leu Cys Ser Trp Lys Gln Glu Lys Asp
   1250 1255 1260
   Glu Asp Phe Asp Trp Asn Leu Lys Ala Ser Ser Ile Pro Ala Ala Gly
   1265 1270 1275 1280
   Thr Glu Pro Ala Ala Asp His Thr Leu Gly Asn Ser Ser Gly His Tyr
   1285 1290 1295
   Ile Phe Ile Lys Ser Leu Phe Pro Gln Gln Pro Met Arg Ala Ala Arg
   1300 1305 1310
   Ile Ser Ser Pro Val Ile Ser Lys Arg Ser Lys Asn Cys Lys Ile Ile
   1315 1320 1325
   Phe His Tyr His Met Tyr Gly Asn Gly Ile Gly Ala Leu Thr Leu Met
   1330 1335 1340
   Gln Val Ser Val Thr Asn Gln Thr Lys Val Leu Leu Asn Leu Thr Val
   1345 1350 1355 1360
   Glu Gln Gly Asn Phe Trp Arg Arg Glu Glu Leu Ser Leu Phe Gly Asp
   1365 1370 1375
   Glu Asp Phe Gln Leu Lys Phe Glu Gly Arg Val Gly Lys Gly Gln Arg
   1380 1385 1390
   Gly Asp Ile Ala Leu Asp Asp Ile Val Leu Thr Glu Asn Cys Leu Ser
   1395 1400 1405
   Leu His Asp Ser Val Gln Glu Glu Leu Ala Val Pro Leu Pro Thr Gly
   1410 1415 1420
   Phe Cys Pro Leu Gly Tyr Arg Glu Cys His Asn Gly Lys Cys Tyr Arg
   1425 1430 1435 1440
   Leu Glu Gln Ser Cys Asn Phe Val Asp Asn Cys Gly Asp Asn Thr Asp
   1445 1450 1455
   Glu Asn Glu Cys Gly Ser Ser Cys Thr Phe Glu Lys Gly Trp Cys Gly
   1460 1465 1470
   Trp Gln Asn Ser Gln Ala Asp Asn Phe Asp Trp Val Leu Gly Val Gly
   1475 1480 1485
   Ser His Gln Ser Leu Arg Pro Pro Lys Asp His Thr Leu Gly Asn Glu
   1490 1495 1500
   Asn Gly His Phe Met Tyr Leu Glu Ala Thr Ala Val Gly Leu Arg Gly
   1505 1510 1515 1520
   Asp Lys Ala His Phe Arg Ser Thr Met Trp Arg Glu Ser Ser Ala Ala
   1525 1530 1535
   Cys Thr Met Ser Phe Trp Tyr Phe Val Ser Ala Lys Ala Thr Gly Ser
   1540 1545 1550
   Ile Gln Ile Leu Ile Lys Thr Glu Lys Gly Leu Ser Lys Val Trp Gln
   1555 1560 1565
   Glu Ser Lys Gln Asn Pro Gly Asn His Trp Gln Lys Ala Asp Ile Leu
   1570 1575 1580
   Leu Gly Lys Leu Arg Asn Phe Glu Val Ile Phe Gln Gly Ile Arg Thr
   1585 1590 1595 1600
   Arg Asp Leu Gly Gly Gly Ala Ala Ile Asp Asp Ile Glu Phe Lys Asn
   1605 1610 1615
   Cys Thr Thr Val Gly Glu Ile Ser Glu Leu Cys Pro Glu Ile Thr Asp
   1620 1625 1630
   Phe Leu Cys Arg Asp Lys Lys Cys Ile Ala Ser His Leu Leu Cys Asp
   1635 1640 1645
   Tyr Lys Pro Asp Cys Ser Asp Arg Ser Asp Glu Ala His Cys Ala His
   1650 1655 1660
   Tyr Thr Ser Thr Thr Gly Ser Cys Asn Phe Glu Thr Ser Ser Gly Asn
   1665 1670 1675 1680
   Trp Thr Thr Ala Cys Ser Leu Thr Gln Asp Ser Glu Asp Asp Leu Asp
   1685 1690 1695
   Trp Ala Ile Gly Ser Arg Ile Pro Ala Lys Ala Leu Ile Pro Asp Ser
   1700 1705 1710
   Asp His Thr Pro Gly Ser Gly Gln His Phe Leu Tyr Val Asn Ser Ser
   1715 1720 1725
   Gly Ser Lys Glu Gly Ser Val Ala Arg Ile Thr Thr Ser Lys Ser Phe
   1730 1735 1740
   Pro Ala Ser Leu Gly Met Cys Thr Val Arg Phe Trp Phe Tyr Met Ile
   1745 1750 1755 1760
   Asp Pro Arg Ser Met Gly Ile Leu Lys Val Tyr Thr Ile Glu Glu Ser
   1765 1770 1775
   Gly Leu Asn Ile Leu Val Trp Ser Val Ile Gly Asn Lys Arg Thr Gly
   1780 1785 1790
   Trp Thr Tyr Gly Ser Val Pro Leu Ser Ser Asn Ser Pro Phe Lys Val
   1795 1800 1805
   Ala Phe Glu Ala Asp Leu Asp Gly Asn Glu Asp Ile Phe Ile Ala Leu
   1810 1815 1820
   Asp Asp Ile Ser Phe Thr Pro Glu Cys Val Thr Gly Gly Pro Val Pro
   1825 1830 1835 1840
   Val Gln Pro Ser Pro Cys Glu Ala Asp Gln Phe Ser Cys Ile Tyr Thr
   1845 1850 1855
   Leu Gln Cys Val Pro Leu Ser Gly Lys Cys Asp Gly His Glu Asp Cys
   1860 1865 1870
   Ile Asp Gly Ser Asp Glu Met Asp Cys Pro Leu Ser Pro Thr Pro Pro
   1875 1880 1885
   Leu Cys Ser Asn Met Glu Phe Pro Cys Ser Thr Asp Glu Cys Ile Pro
   1890 1895 1900
   Ser Leu Leu Leu Cys Asp Gly Val Pro Asp Cys His Phe Asn Glu Asp
   1905 1910 1915 1920
   Glu Leu Ile Cys Ser Asn Lys Ser Cys Ser Asn Gly Ala Leu Val Cys
   1925 1930 1935
   Ala Ser Ser Asn Ser Cys Ile Pro Ala His Gln Arg Cys Asp Gly Phe
   1940 1945 1950
   Ala Asp Cys Met Asp Phe Gln Leu Asp Glu Ser Ser Cys Ser Glu Cys
   1955 1960 1965
   Pro Leu Asn Tyr Cys Arg Asn Gly Gly Thr Cys Val Val Glu Lys Asn
   1970 1975 1980
   Gly Pro Met Cys Arg Cys Arg Gln Gly Trp Lys Gly Asn Arg Cys His
   1985 1990 1995 2000
   Ile Lys Phe Asn Pro Pro Ala Thr Asp Phe Thr Tyr Ala Gln Asn Asn
   2005 2010 2015
   Thr Trp Thr Leu Leu Gly Ile Gly Leu Ala Phe Leu Met Thr His Ile
   2020 2025 2030
   Thr Val Ala Val Leu Cys Phe Leu Ala Asn Arg Lys Val Pro Ile Arg
   2035 2040 2045
   Lys Thr Glu Gly Ser Gly Asn Cys Ala Phe Val Asn Pro Val Tyr Gly
   2050 2055 2060
   Asn Trp Ser Asn Pro Glu Lys Thr Glu Ser Ser Val Tyr Ser Phe Ser
   2065 2070 2075 2080
   Asn Pro Leu Tyr Gly Thr Thr Ser Gly Ser Leu Glu Thr Leu Ser His
   2085 2090 2095
   His Leu Lys
<210> 17
   <211> 6326
   <212> DNA
   <213> Homo sapiens
<400> 17
   ttgcctgtgt tttcaattct acactggctc agcaagggac agaaagcttt cagtgtgaca    60
   atggagtctc cttgcctcct gacagcattt gtgacttcac agatcagtgt ggggatagca   120
   gtgatgaacg gcactgtttg aattatgaaa gatgtgattt tgaggatggt ctctgtcata   180
   tgactcaaga tcagagtctg caacctagtt ggacaaagag aagtgggatg attggtctat   240
   cacctccatt ttatgatcac aatggtgatg tgtctgctca cttcctctca ctggtttcca   300
   gagtggattc tatttcctca agtttaagaa gcagagtttt ccttccaaca aatgatcaac   360
   atgactgcca gattacattt tattacttct cctgccaagt gagtggcaaa ttaatggttg   420
   ggcttcaaac tgcatgtgga ggtcctattc agcatttatg gcaaaacaca gctgcactcc   480
   caaatcagtg ggagagaaat gtcatcaaaa tccagagttc acagagattt caggttgttt   540
   ttgaaggtca aatggcttca acgtatgaac aggatgaagt cattgctatt gatgatatat   600
   ctttcagttc aggctgcttg cctgccaatg atggaatttt actgtgtcaa gaagcattga   660
   atgctgagcg ggagctatgc catccagata cagatctctg cagatttgat gctacagatg 720
   aagagttgag attgtgtcag gcctgtgggt ttgaatttga catgtgtgag tggacgtcag 780
   aagcatctgc tggccaaatt tcctggatgc gcacaaaagc gagagagatc cctgcattcg 840
   aatccacacc tcagcaggat caaggaggtg atgatgaagg ttattatgta tgggtaggcg 900
   ctaagcatgg tttcactctt aaccatttag acagcagggc ttacctaaat agctctgtgt 960
   gtcattgcct gggcaagagc tgtcatcttc aattctatta tgcaatggaa agcagtgtcc 1020
   tgagagtaag actgtataat aataaggaag aagaaatatt ttggacatac aacatatcaa 1080
   ctcacagcca atgggtgaaa gcagatgtgt taataccaga agatctgaag acatttaaga 1140
   cccaatccag aaagctttgc tctgcagacg aattcccttg cactagtggc cagtgcatcg 1200
   ccaaagaatc tgtctgtgac tctcggcagg actgctccga tgagagtgat gaagacccag 1260
   caacttgctc aaagcatctc acctgtgact ttgagtcggg tttctgcggt tgggagccat 1320
   ttctcacaga agattcacac tggaagctga tgaaaggatt gaataatgga gagcaccact 1380
   ttcctgcagc tgatcacaca gcaaacataa atcatggatc gtttatttat ttggaggcac 1440
   agcgctcccc cggggtggcc aagcttggaa gtcctgttct tacaaaattg ctcactgcct 1500
   ctaccccatg tcaggtgcag ttttggtatc atttgtctca acattcaaat ctctcagttt 1560
   ttacaagaac gtctctagat ggaaacttgc aaaagcaggg caaaataatc agattctccg 1620
   aatctcagtg gagccacgca aaaattgatc tcattgcaga agcgggagaa tctactctac 1680
   cttttcagtt aattttggaa gctactgttt tgtcgtcaaa tgctaccgtt gctctagatg 1740
   acatcagtgt gtcccaggaa tgtgaaattt cctataaatc actaccaagg accagtacac 1800
   aaagcaagtt ttccaagtgt gactttgaag caaacagctg tgattggttt gaagcaatta 1860
   gtggtgacca ttttgactgg atacggagct ctcagagtga actttctgct gattttgagc 1920
   accaggctcc acctcgggat catagtctca acgcatctca agggcatttt atgttcattc 1980
   tgaagaaaag cagcagcttg tggcaagttg ctaagcttca gagcccaact ttcagccaga 2040
   caggacctgg atgcatactt tccttctggt tctataacta tggcctgtca gtgggagcag 2100
   ctgagctgca gctacatatg gaaaattctc atgactcaac agtgatttgg agagtattat 2160
   acaatcaggg caaacaatgg ttggaggcaa ccattcagct agggcgcctt tcgcagccct 2220
   tccatttgtc actagataaa gtcagtctgg gcatttatga tggggtctca gctattgatg 2280
   acatccgatt tgaaaattgt actctccctc ttcctgctga gagctgtgaa gggctggatc 2340
   atttctggtg tcgccacacc agggcttgca tagaaaagct tcggttatgt gatctggtgg 2400
   atgactgtgg tgatcgtact gatgaagtca actgtgcacc tgagctgcag tgtaactttg 2460
   aaactggaat ctgtaactgg gaacaagatg caaaagatga ctttgattgg accaggagcc 2520
   agggtccaac tccaacactt aacacagggc caatgaaaga taacactctg ggcacagcta 2580
   aaggacacta tctctacata gaatcttcag agccacaggc ttttcaagac agtgctgcct 2640
   tactcagccc aatccttaat gccactgata caaaaggctg caccttccgc ttctattacc 2700
   acatgtttgg aaagcgcatt tataggttgg caatctacca acgaatctgg agtgactcaa 2760
   ggggacagct gctgtggcag atatttggga atcaaggcaa cagatggatt aggaaacacc 2820
   tcaacatttc cagcaggcag ccctttcaga tattggtgga ggcttcagtg ggagatggct 2880
   tcactggaga tattgcgatt gatgatctgt catttatgga ctgcaccctc taccctggta 2940
   atttgccagc agacctccca actccaccag aaacgtcagt tcctgtaaca ttacctccac 3000
   acaactgcac agacaatgaa tttatctgca ggtctgatgg tcactgcatt gaaaaaatgc 3060
   agaaatgtga ttttaaatat gactgccctg acaaatcaga tgaagcatcc tgtgttatgg 3120
   aagtttgcag ctttgagaaa agaagcctgt gtaaatggta tcaaccaatc ccagtacatt 3180
   tgcttcaaga ttcaaacaca ttcaggtggg ggcttgggaa cgggatcagc attcatcatg 3240
   gggaagaaaa ccacaggcca tcagtggatc atacacaaaa taccactgat ggctggtacc 3300
   tgtatgctga cagttctaat gggaaatttg gtgacacggc tgacattctc actcctatca 3360
   tttcactcac gggaccaaaa tgtaccttgg tgttctggac acatatgaat ggggccaccg 3420
   ttggttctct ccaggtgctc atcaagaaag ataacgttac ttctaaattg tgggctcaaa 3480
   ctggacagca aggtgcacag tggaagagag cagaagtgtt tttaggcatt cgttcacata 3540
   cacagattgt cttcagagcc aaacgtggta tcagttacat aggagatgta gcagtggatg 3600
   atatttcctt ccaagattgc tcccctttgc ttagcccaga gagaaagtgt actgatcatg 3660
   aattcatgtg tgctaataag cactgcattg ccaaagacaa gctgtgtgat tttgtgaatg 3720
   attgtgctga taattcagat gagactactt tcatttgccg tacctccagt gggcgctgtg 3780
   atttcgaatt tgatctttgt tcctggaagc aggagaaaga tgaggacttt gactggaacc 3840
   tgaaagctag cagcatccct gcagcaggca cagagccagc agcagatcac actttgggaa 3900
   attcatctgg tcattacatc tttataaaga gtttgtttcc tcagcagccc atgagagctg 3960
   ccagaatttc aagtccagtt ataagtaaga gaagcaaaaa ctgcaagatt atttttcatt 4020
   atcacatgta tggaaatggc attggggcac tcaccttaat gcaggtgtca gtcacaaacc 4080
   aaacgaaggt tctacttaac ctcactgtag aacaaggcaa tttctggcgg agagaagaac 4140
   tgtcactgtt tggtgatgaa gacttccaac tcaaatttga aggtagagtt gggaaaggtc 4200
   agcgtggaga cattgcactt gatgacattg tgcttacaga aaattgtcta tcactccatg 4260
   attccgtgca agaagaactg gcagtgcctc ttccaacagg tttctgccca cttggctata 4320
   gggaatgtca taatggaaaa tgctataggc tggaacaaag ctgtaacttc gtagataact 4380
   gtggagataa tactgatgaa aatgagtgtg gtagctcctg tacttttgaa aaaggctggt 4440
   gtggctggca aaactcccag gctgacaact ttgattgggt tttaggggtt ggctctcatc 4500
   aaagcttaag acctcccaaa gaccacacac ttggaaatga aaatgggcac ttcatgtatc 4560
   tggaagctac tgcagtgggc cttcggggtg acaaagcaca cttcaggagt accatgtggc 4620
   gagaatccag tgcagcctgc accatgagct tctggtattt cgtatctgca aaggccacag 4680
   gatccattca gattctcatc aagacagaga aaggactatc aaaagtatgg caagaaagta   4740
   agcagaaccc tggtaatcat tggcaaaagg ctgacatcct gctaggaaag ttaaggaatt   4800
   ttgaagtcat atttcaaggt atcagaacaa gggacctggg aggaggagct gcaattgatg   4860
   atattgaatt taaaaactgc acaactgtgg gagagatctc tgagctttgt ccggaaatca   4920
   ctgatttttt gtgccgggac aagaagtgca ttgcatccca ccttctttgt gactataagc   4980
   cagactgctc tgataggtct gatgaagctc actgtgcaca ttatacaagc acaacaggaa   5040
   gctgcaattt tgaaacaagt tcaggaaact ggaccacagc ctgcagtctt actcaagact   5100
   ctgaggatga cttggactgg gccattggca gcagaattcc tgccaaagca ttaattccag   5160
   actctgatca cacgccaggt agtggtcagc acttcctgta cgtcaactca tctggctcca   5220
   aggaaggatc cgttgccaga attactactt ccaaatcctt cccagcaagc cttggaatgt   5280
   gtactgttcg gttctggttc tacatgattg atcccaggag tatgggaata ttaaaggtgt   5340
   ataccattga agaatcgggg ctaaacatcc tggtgtggtc agtgattgga aataaaagaa   5400
   cgggatggac atatggctct gtgcctctct ccagtaacag tccgtttaag gtggcatttg   5460
   aagctgattt ggatggaaat gaggacatct ttattgctct tgatgacatc tcttttaccc   5520
   cagagtgtgt gactggaggt cctgtcccag tgcagccatc accctgtgaa gctgatcagt   5580
   tttcttgtat ctacacactc caatgtgtcc ctctctcagg gaaatgtgat ggacatgaag   5640
   actgcataga tggatctgat gaaatggatt gtcctctcag ccccacccct ccactctgta   5700
   gtaacatgga gttcccgtgc tctacagacg agtgtatacc ttccctcctg ctatgcgatg   5760
   gagtgcccga ctgccacttt aatgaagatg agctcatctg ctccaacaaa agctgttcta   5820
   atggagctct ggtgtgtgcc tcctccaaca gctgtatccc agcccaccag cgctgtgatg   5880
   gttttgccga ctgcatggat ttccagcttg atgagtccag ctgctccgaa tgtccattaa   5940
   attactgcag aaatggtggg acttgtgtag tggagaaaaa tggtcctatg tgtcgatgta   6000
   gacaaggctg gaaaggaaat cgatgccata tcaagtttaa tcctcctgct acagacttca   6060
   catacgctca gaataataca tggactctcc tgggtattgg attagcattc ctgatgactc   6120
   acatcacagt tgcagtcttg tgttttcttg caaacagaaa ggtaccaata aggaaaaccg   6180
   agggaagtgg taactgtgcc tttgtcaatc cagtttacgg gaactggagc aacccagaga   6240
   aaacagagag ttctgtctat tccttctcaa acccattata tggcacaaca tcaggaagcc   6300
   tggagaccct gtcacatcat ctcaaa                                        6326
<210> 18
   <211> 2108
   <212> PRT
   <213> Home sapiens
<400> 18
   Ala Cys Val Phe Asn Ser Thr Leu Ala Gln Gln Gly Thr Glu Ser Phe
   1 5 10 15
   Gln Cys Asp Asn Gly Val Ser Leu Pro Pro Asp Ser Ile Cys Asp Phe
   20 25 30
   Thr Asp Gln Cys Gly Asp Ser Ser Asp Glu Arg His Cys Leu Asn Tyr
   35 40 45
   Glu Arg Cys Asp Phe Glu Asp Gly Leu Cys His Met Thr Gln Asp Gln
   50 55 60
   Ser Leu Gln Pro Ser Trp Thr Lys Arg Ser Gly Met Ile Gly Leu Ser
   65 70 75 80
   Pro Pro Phe Tyr Asp His Asn Gly Asp Val Ser Ala His Phe Leu Ser
   85 90 95
   Leu Val Ser Arg Val Asp Ser Ile Ser Ser Ser Leu Arg Ser Arg Val
   100 105 110
   Phe Leu Pro Thr Asn Asp Gln His Asp Cys Gln Ile Thr Phe Tyr Tyr
   115 120 125
   Phe Ser Cys Gln Val Ser Gly Lys Leu Met Val Gly Leu Gln Thr Ala
   130 135 140
   Cys Gly Gly Pro Ile Gln His Leu Trp Gln Asn Thr Ala Ala Leu Pro
   145 150 155 160
   Asn Gln Trp Glu Arg Asn Val Ile Lys Ile Gln Ser Ser Gln Arg Phe
   165 170 175
   Gln Val Val Phe Glu Gly Gln Met Ala Ser Thr Tyr Glu Gln Asp Glu
   180 185 190
   Val Ile Ala Ile Asp Asp Ile Ser Phe Ser Ser Gly Cys Leu Pro Ala
   195 200 205
   Asn Asp Gly Ile Leu Leu Cys Gln Glu Ala Leu Asn Ala Glu Arg Glu
   210 215 220
   Leu Cys His Pro Asp Thr Asp Leu Cys Arg Phe Asp Ala Thr Asp Glu
   225 230 235 240
   Glu Leu Arg Leu Cys Gln Ala Cys Gly Phe Glu Phe Asp Met Cys Glu
   245 250 255
   Trp Thr Ser Glu Ala Ser Ala Gly Gln Ile Ser Trp Met Arg Thr Lys
   260 265 270
   Ala Arg Glu Ile Pro Ala Phe Glu Ser Thr Pro Gln Gln Asp Gln Gly
   275 280 285
   Gly Asp Asp Glu Gly Tyr Tyr Val Trp Val Gly Ala Lys His Gly Phe
   290 295 300
   Thr Leu Asn His Leu Asp Ser Arg Ala Tyr Leu Asn Ser Ser Val Cys
   305 310 315 320
   His Cys Leu Gly Lys Ser Cys His Leu Gln Phe Tyr Tyr Ala Met Glu
   325 330 335
   Ser Ser Val Leu Arg Val Arg Leu Tyr Asn Asn Lys Glu Glu Glu Ile
   340 345 350
   Phe Trp Thr Tyr Asn Ile Ser Thr His Ser Gln Trp Val Lys Ala Asp
   355 360 365
   Val Leu Ile Pro Glu Asp Leu Lys Thr Phe Lys Thr Gln Ser Arg Lys
   370 375 380
   Leu Cys Ser Ala Asp Glu Phe Pro Cys Thr Ser Gly Gln Cys Ile Ala
   385 390 395 400
   Lys Glu Ser Val Cys Asp Ser Arg Gln Asp Cys Ser Asp Glu Ser Asp
   405 410 415
   Glu Asp Pro Ala Thr Cys Ser Lys His Leu Thr Cys Asp Phe Glu Ser
   420 425 430
   Gly Phe Cys Gly Trp Glu Pro Phe Leu Thr Glu Asp Ser His Trp Lys
   435 440 445
   Leu Met Lys Gly Leu Asn Asn Gly Glu His His Phe Pro Ala Ala Asp
   450 455 460
   His Thr Ala Asn Ile Asn His Gly Ser Phe Ile Tyr Leu Glu Ala Gln
   465 470 475 480
   Arg Ser Pro Gly Val Ala Lys Leu Gly Ser Pro Val Leu Thr Lys Leu
   485 490 495
   Leu Thr Ala Ser Thr Pro Cys Gln Val Gln Phe Trp Tyr His Leu Ser
   500 505 510
   Gln His Ser Asn Leu Ser Val Phe Thr Arg Thr Ser Leu Asp Gly Asn
   515 520 525
   Leu Gln Lys Gln Gly Lys Ile Ile Arg Phe Ser Glu Ser Gln Trp Ser
   530 535 540
   His Ala Lys Ile Asp Leu Ile Ala Glu Ala Gly Glu Ser Thr Leu Pro
   545 550 555 560
   Phe Gln Leu Ile Leu Glu Ala Thr Val Leu Ser Ser Asn Ala Thr Val
   565 570 575
   Ala Leu Asp Asp Ile Ser Val Ser Gln Glu Cys Glu Ile Ser Tyr Lys
   580 585 590
   Ser Leu Pro Arg Thr Ser Thr Gln Ser Lys Phe Ser Lys Cys Asp Phe
   595 600 605
   Glu Ala Asn Ser Cys Asp Trp Phe Glu Ala Ile Ser Gly Asp His Phe
   610 615 620
   Asp Trp Ile Arg Ser Ser Gln Ser Glu Leu Ser Ala Asp Phe Glu His
   625 630 635 640
   Gln Ala Pro Pro Arg Asp His Ser Leu Asn Ala Ser Gln Gly His Phe
   645 650 655
   Met Phe Ile Leu Lys Lys Ser Ser Ser Leu Trp Gln Val Ala Lys Leu
   660 665 670
   Gln Ser Pro Thr Phe Ser Gln Thr Gly Pro Gly Cys Ile Leu Ser Phe
   675 680 685
   Trp Phe Tyr Asn Tyr Gly Leu Ser Val Gly Ala Ala Glu Leu Gln Leu
   690 695 700
   His Met Glu Asn Ser His Asp Ser Thr Val Ile Trp Arg Val Leu Tyr
   705 710 715 720
   Asn Gln Gly Lys Gln Trp Leu Glu Ala Thr Ile Gln Leu Gly Arg Leu
   725 730 735
   Ser Gln Pro Phe His Leu Ser Leu Asp Lys Val Ser Leu Gly Ile Tyr
   740 745 750
   Asp Gly Val Ser Ala Ile Asp Asp Ile Arg Phe Glu Asn Cys Thr Leu
   755 760 765
   Pro Leu Pro Ala Glu Ser Cys Glu Gly Leu Asp His Phe Trp Cys Arg
   770 775 780
   His Thr Arg Ala Cys Ile Glu Lys Leu Arg Leu Cys Asp Leu Val Asp
   785 790 795 800
   Asp Cys Gly Asp Arg Thr Asp Glu Val Asn Cys Ala Pro Glu Leu Gln
   805 810 815
   Cys Asn Phe Glu Thr Gly Ile Cys Asn Trp Glu Gln Asp Ala Lys Asp
   820 825 830
   Asp Phe Asp Trp Thr Arg Ser Gln Gly Pro Thr Pro Thr Leu Asn Thr
   835 840 845
   Gly Pro Met Lys Asp Asn Thr Leu Gly Thr Ala Lys Gly His Tyr Leu
   850 855 860
   Tyr Ile Glu Ser Ser Glu Pro Gln Ala Phe Gln Asp Ser Ala Ala Leu
   865 870 875 880
   Leu Ser Pro Ile Leu Asn Ala Thr Asp Thr Lys Gly Cys Thr Phe Arg
   885 890 895
   Phe Tyr Tyr His Met Phe Gly Lys Arg Ile Tyr Arg Leu Ala Ile Tyr
   900 905 910
   Gln Arg Ile Trp Ser Asp Ser Arg Gly Gln Leu Leu Trp Gln Ile Phe
   915 920 925
   Gly Asn Gln Gly Asn Arg Trp Ile Arg Lys His Leu Asn Ile Ser Ser
   930 935 940
   Arg Gln Pro Phe Gln Ile Leu Val Glu Ala Ser Val Gly Asp Gly Phe
   945 950 955 960
   Thr Gly Asp Ile Ala Ile Asp Asp Leu Ser Phe Met Asp Cys Thr Leu
   965 970 975
   Tyr Pro Gly Asn Leu Pro Ala Asp Leu Pro Thr Pro Pro Glu Thr Ser
   980 985 990
   Val Pro Val Thr Leu Pro Pro His Asn Cys Thr Asp Asn Glu Phe Ile
   995 1000 1005
   Cys Arg Ser Asp Gly His Cys Ile Glu Lys Met Gln Lys Cys Asp Phe
   1010 1015 1020
   Lys Tyr Asp Cys Pro Asp Lys Ser Asp Glu Ala Ser Cys Val Met Glu
   1025 1030 1035 1040
   Val Cys Ser Phe Glu Lys Arg Ser Leu Cys Lys Trp Tyr Gln Pro Ile
   1045 1050 1055
   Pro Val His Leu Leu Gln Asp Ser Asn Thr Phe Arg Trp Gly Leu Gly
   1060 1065 1070
   Asn Gly Ile Ser Ile His His Gly Glu Glu Asn His Arg Pro Ser Val
   1075 1080 1085
   Asp His Thr Gln Asn Thr Thr Asp Gly Trp Tyr Leu Tyr Ala Asp Ser
   1090 1095 1100
   Ser Asn Gly Lys Phe Gly Asp Thr Ala Asp Ile Leu Thr Pro Ile Ile
   1105 1110 1115 1120
   Ser Leu Thr Gly Pro Lys Cys Thr Leu Val Phe Trp Thr His Met Asn
   1125 1130 1135
   Gly Ala Thr Val Gly Ser Leu Gln Val Leu Ile Lys Lys Asp Asn Val
   1140 1145 1150
   Thr Ser Lys Leu Trp Ala Gln Thr Gly Gln Gln Gly Ala Gln Trp Lys
   1155 1160 1165
   Arg Ala Glu Val Phe Leu Gly Ile Arg Ser His Thr Gln Ile Val Phe
   1170 1175 1180
   Arg Ala Lys Arg Gly Ile Ser Tyr Ile Gly Asp Val Ala Val Asp Asp
   1185 1190 1195 1200
   Ile Ser Phe Gln Asp Cys Ser Pro Leu Leu Ser Pro Glu Arg Lys Cys
   1205 1210 1215
   Thr Asp His Glu Phe Met Cys Ala Asn Lys His Cys Ile Ala Lys Asp
   1220 1225 1230
   Lys Leu Cys Asp Phe Val Asn Asp Cys Ala Asp Asn Ser Asp Glu Thr
   1235 1240 1245
   Thr Phe Ile Cys Arg Thr Ser Ser Gly Arg Cys Asp Phe Glu Phe Asp
   1250 1255 1260
   Leu Cys Ser Trp Lys Gln Glu Lys Asp Glu Asp Phe Asp Trp Asn Leu
   1265 1270 1275 1280
   Lys Ala Ser Ser Ile Pro Ala Ala Gly Thr Glu Pro Ala Ala Asp His
   1285 1290 1295
   Thr Leu Gly Asn Ser Ser Gly His Tyr Ile Phe Ile Lys Ser Leu Phe
   1300 1305 1310
   Pro Gln Gln Pro Met Arg Ala Ala Arg Ile Ser Ser Pro Val Ile Ser
   1315 1320 1325
   Lys Arg Ser Lys Asn Cys Lys Ile Ile Phe His Tyr His Met Tyr Gly
   1330 1335 1340
   Asn Gly Ile Gly Ala Leu Thr Leu Met Gln Val Ser Val Thr Asn Gln
   1345 1350 1355 1360
   Thr Lys Val Leu Leu Asn Leu Thr Val Glu Gln Gly Asn Phe Trp Arg
   1365 1370 1375
   Arg Glu Glu Leu Ser Leu Phe Gly Asp Glu Asp Phe Gln Leu Lys Phe
   1380 1385 1390
   Glu Gly Arg Val Gly Lys Gly Gln Arg Gly Asp Ile Ala Leu Asp Asp
   1395 1400 1405
   Ile Val Leu Thr Glu Asn Cys Leu Ser Leu His Asp Ser Val Gln Glu
   1410 1415 1420
   Glu Leu Ala Val Pro Leu Pro Thr Gly Phe Cys Pro Leu Gly Tyr Arg
   1425 1430 1435 1440
   Glu Cys His Asn Gly Lys Cys Tyr Arg Leu Glu Gln Ser Cys Asn Phe
   1445 1450 1455
   Val Asp Asn Cys Gly Asp Asn Thr Asp Glu Asn Glu Cys Gly Ser Ser
   1460 1465 1470
   Cys Thr Phe Glu Lys Gly Trp Cys Gly Trp Gln Asn Ser Gln Ala Asp
   1475 1480 1485
   Asn Phe Asp Trp Val Leu Gly Val Gly Ser His Gln Ser Leu Arg Pro
   1490 1495 1500
   Pro Lys Asp His Thr Leu Gly Asn Glu Asn Gly His Phe Met Tyr Leu
   1505 1510 1515 1520
   Glu Ala Thr Ala Val Gly Leu Arg Gly Asp Lys Ala His Phe Arg Ser
   1525 1530 1535
   Thr Met Trp Arg Glu Ser Ser Ala Ala Cys Thr Met Ser Phe Trp Tyr
   1540 1545 1550
   Phe Val Ser Ala Lys Ala Thr Gly Ser Ile Gln Ile Leu Ile Lys Thr
   1555 1560 1565
   Glu Lys Gly Leu Ser Lys Val Trp Gln Glu Ser Lys Gln Asn Pro Gly
   1570 1575 1580
   Asn His Trp Gln Lys Ala Asp Ile Leu Leu Gly Lys Leu Arg Asn Phe
   1585 1590 1595 1600
   Glu Val Ile Phe Gln Gly Ile Arg Thr Arg Asp Leu Gly Gly Gly Ala
   1605 1610 1615
   Ala Ile Asp Asp Ile Glu Phe Lys Asn Cys Thr Thr Val Gly Glu Ile
   1620 1625 1630
   Ser Glu Leu Cys Pro Glu Ile Thr Asp Phe Leu Cys Arg Asp Lys Lys
   1635 1640 1645
   Cys Ile Ala Ser His Leu Leu Cys Asp Tyr Lys Pro Asp Cys Ser Asp
   1650 1655 1660
   Arg Ser Asp Glu Ala His Cys Ala His Tyr Thr Ser Thr Thr Gly Ser
   1665 1670 1675 1680
   Cys Asn Phe Glu Thr Ser Ser Gly Asn Trp Thr Thr Ala Cys Ser Leu
   1685 1690 1695
   Thr Gln Asp Ser Glu Asp Asp Leu Asp Trp Ala Ile Gly Ser Arg Ile
   1700 1705 1710
   Pro Ala Lys Ala Leu Ile Pro Asp Ser Asp His Thr Pro Gly Ser Gly
   1715 1720 1725
   Gln His Phe Leu Tyr Val Asn Ser Ser Gly Ser Lys Glu Gly Ser Val
   1730 1735 1740
   Ala Arg Ile Thr Thr Ser Lys Ser Phe Pro Ala Ser Leu Gly Met Cys
   1745 1750 1755 1760
   Thr Val Arg Phe Trp Phe Tyr Met Ile Asp Pro Arg Ser Met Gly Ile
   1765 1770 1775
   Leu Lys Val Tyr Thr Ile Glu Glu Ser Gly Leu Asn Ile Leu Val Trp
   1780 1785 1790
   Ser Val Ile Gly Asn Lys Arg Thr Gly Trp Thr Tyr Gly Ser Val Pro
   1795 1800 1805
   Leu Ser Ser Asn Ser Pro Phe Lys Val Ala Phe Glu Ala Asp Leu Asp
   1810 1815 1820
   Gly Asn Glu Asp Ile Phe Ile Ala Leu Asp Asp Ile Ser Phe Thr Pro
   1825 1830 1835 1840
   Glu Cys Val Thr Gly Gly Pro Val Pro Val Gln Pro Ser Pro Cys Glu
   1845 1850 1855
   Ala Asp Gln Phe Ser Cys Ile Tyr Thr Leu Gln Cys Val Pro Leu Ser
   1860 1865 1870
   Gly Lys Cys Asp Gly His Glu Asp Cys Ile Asp Gly Ser Asp Glu Met
                   1875                1880                1885
   Asp Cys Pro Leu Ser Pro Thr Pro Pro Leu Cys Ser Asn Met Glu Phe
       1890                1895                1900
   Pro Cys Ser Thr Asp Glu Cys Ile Pro Ser Leu Leu Leu Cys Asp Gly
   1905                1910                1915                1920
   Val Pro Asp Cys His Phe Asn Glu Asp Glu Leu Ile Cys Ser Asn Lys
                   1925                1930                1935
   Ser Cys Ser Asn Gly Ala Leu Val Cys Ala Ser Ser Asn Ser Cys Ile
               1940                1945                1950
   Pro Ala His Gln Arg Cys Asp Gly Phe Ala Asp Cys Met Asp Phe Gln
           1955                1960                1965
   Leu Asp Glu Ser Ser Cys Ser Glu Cys Pro Leu Asn Tyr Cys Arg Asn
       1970                1975                1980
   Gly Gly Thr Cys Val Val Glu Lys Asn Gly Pro Met Cys Arg Cys Arg
   1985                1990                1995                2000
   Gln Gly Trp Lys Gly Asn Arg Cys His Ile Lys Phe Asn Pro Pro Ala
                   2005                2010                2015
   Thr Asp Phe Thr Tyr Ala Gln Asn Asn Thr Trp Thr Leu Leu Gly Ile
               2020                2025                2030
   Gly Leu Ala Phe Leu Met Thr His Ile Thr Val Ala Val Leu Cys Phe
           2035                2040                2045
   Leu Ala Asn Arg Lys Val Pro Ile Arg Lys Thr Glu Gly Ser Gly Asn
       2050                2055                2060
   Cys Ala Phe Val Asn Pro Val Tyr Gly Asn Trp Ser Asn Pro Glu Lys
   2065                2070                2075                2080
   Thr Glu Ser Ser Val Tyr Ser Phe Ser Asn Pro Leu Tyr Gly Thr Thr
                   2085                2090                2095
   Ser Gly Ser Leu Glu Thr Leu Ser His His Leu Lys
               2100                2105
<210> 19
   <211> 588
   <212> DNA
   <213> Homo sapiens
<400> 19
   atgaatgaaa ctttttgttg cctttggatt gcctgtgttt tcaattctac actggctcag    60
   caagggacag aaagctttca gtgtgacaat ggagtctcct tgcctcctga cagcatttgt   120
   gacttcacag atcagtgtgg ggatagcagt gatgaacggc actggctttc atttgctcat   180
   ccttgcaatc cattggggtc tagtttggaa tctgacaact ggaacaaaaa gaaacttgaa   240
   tccggtgcat gccttggttt tggtgctgct gctgcttctc aagatcctcg gcaggaatta   300
   agaaagaagc tggtgtggac agcagatccc cgaaattggc gggctattac attttattac    360
   ttctcctgcc aagtgagtgg caaattaatg gttgggcttc aaactgcatg tggaggtcct   420
   attcagcatt tatggcaaaa cacagctgca ctcccaaatc agtgggagag aaatgtcatc   480
   aaaatccaga gttcacagag atttcaggtt gtttttgaag gtcaaatggc ttcaacgtat   540
   gaacaggatg aagtcattgc tattgatgat atatctttca gttcaggc                588
<210> 20
   <211> 196
   <212> PRT
   <213> Homo sapiens
<400> 20
   Met Asn Glu Thr Phe Cys Cys Leu Trp Ile Ala Cys Val Phe Asn Ser
   1               5                   10                  15
   Thr Leu Ala Gln Gln Gly Thr Glu Ser Phe Gln Cys Asp Asn Gly Val
               20                  25                  30
   Ser Leu Pro Pro Asp Ser Ile Cys Asp Phe Thr Asp Gln Cys Gly Asp
           35                  40                  45
   Ser Ser Asp Glu Arg His Trp Leu Ser Phe Ala His Pro Cys Asn Pro
       50                  55                  60
   Leu Gly Ser Ser Leu Glu Ser Asp Asn Trp Asn Lys Lys Lys Leu Glu
   65                  70                  75                  80
   Ser Gly Ala Cys Leu Gly Phe Gly Ala Ala Ala Ala Ser Gln Asp Pro
                   85                  90                  95
   Arg Gln Glu Leu Arg Lys Lys Leu Val Trp Thr Ala Asp Pro Arg Asn
               100                 105                 110
   Trp Arg Ala Ile Thr Phe Tyr Tyr Phe Ser Cys Gln Val Ser Gly Lys
           115                 120                 125
   Leu Met Val Gly Leu Gln Thr Ala Cys Gly Gly Pro Ile Gln His Leu
       130                 135                 140
   Trp Gln Asn Thr Ala Ala Leu Pro Asn Gln Trp Glu Arg Asn Val Ile
   145                 150                 155                 160
   Lys Ile Gln Ser Ser Gln Arg Phe Gln Val Val Phe Glu Gly Gln Met
                   165                 170                 175
   Ala Ser Thr Tyr Glu Gln Asp Glu Val Ile Ala Ile Asp Asp Ile Ser
               180                 185                 190
   Phe Ser Ser Gly
           195
<210> 21
   <211> 626
   <212> DNA
   <213> Homo sapiens
<400> 21
   atgaatgaaa ctttttgttg cctttggatt gcctgtgttt tcaattctac actggctcag   60
   caagggacag aaagctttca gtgtgacaat ggagtctcct tgcctcctga cagcatttgt   120
   gacttcacag atcagtgtgg ggatagcagt gatgaacggc actgtttgaa ttatgaaaga   180
   tgtgattttg aggatggtct ctgtcatatg actcaagatc agagtctgca acctagttgg   240
   acaaagagaa gtgggatgat tggtctatca cctccatttt atgatcacaa tggtgatgtg   300
   tctgctcact tcctctcact ggtttccaga gtggattcta tttcctcaag tttaagaagc   360
   agagttttcc ttccaacaaa tgatcaacat gactgccaga ttacatttta ttacttctcc   420
   tgccaagtga gtggcaaatt aatggttggg cttcaaactg catgtggagg tcctattcag   480
   catttatggc aaaacacagc tgcactccca aatcagtggg agagaaatgt catcaaaatc   540
   cagagttcac agagatttca ggttgttttt gaaggtcaaa tggcttcaac gtatgaacag   600
   gatgaagtca ttgctattga tgatat                                         626
<210> 22
   <211> 208
   <212> PRT
   <213> Homo sapiens
<900> 22
<210> 23
   <211> 6054
   <212> DNA
   <213> Homo sapiens
<400> 23
   atgaatgaaa ctttttgttg cctttggatt gcctgtgttt tcaattctac actggctcag 60
   caagggacag aaagctttca gtgtgacaat ggagtctcct tgcctcctga cagcatttgt 120
   gacttcacag atcagtgtgg ggatagcagt gatgaacggc actggctttc atttgctcat 180
   ccttgcaatc cattggggtc tagtttggaa tctgacaact ggaacaaaaa gaaacttgaa 240
   tccggtgcat gccttggttt tggtgctgct gctgcttctc aagatcctcg gcaggaatta 300
   agaaagaagc tggtgtggac agcagatccc cgaaattggc gggctattac attttattac 360
   ttctcctgcc aagtgagtgg caaattaatg gttgggcttc aaactgcatg tggaggtcct 420
   attcagcatt tatggcaaaa cacagctgca ctcccaaatc agtgggagag aaatgtcatc 480
   aaaatccaga gttcacagag atttcaggtt gtttttgaag gtcaaatggc ttcaacgtat 540
   gaacaggatg aagtcattgc tattgatgat atatctttca gttcaggctg cttgcctgcc 600
   aatgatggaa ttttactgtg tcaagaagca ttgaatgctg agcgggagct atgccatcca 660
   gatacagatc tctgcagatt tgatgctaca gatgaagagt tgagattgtg tcaggcctgt 720
   gggtttgaat ttgacatgtg tgagtggacg tcagaagcat ctgctggcca aatttcctgg 780
   atgcgcacaa aagcgagaga gatccctgca ttcgaatcca cacctcagca ggatcaagga 840
   ggtgatgatg aaggttatta tgtatgggta ggcgctaagc atggtttcac tcttaaccat 900
   ttagacagca gggcttacct aaatagctct gtgtgtcatt gcctgggcaa gagctgtcat 960
   cttcaattct attatgcaat ggaaagcagt gtcctgagag taagactgta taataataag 1020
   gaagaagaaa tattttggac atacaacata tcaactcaca gccaatgggt gaaagcagat 1080
   gtgttaatac cagaagatct gaagacattt aagacccaat ccagaaagct ttgctctgca 1140
   gacgaattcc cttgcactag tggccagtgc atcgccaaag aatctgtctg tgactctcgg 1200
   caggactgct ccgatgagag tgatgaagac ccagcaactt gctcaaagca tctcacctgt 1260
   gactttgagt cgggtttctg cggttgggag ccatttctca cagaagattc acactggaag 1320
   ctgatgaaag gattgaataa tggagagcac cactttcctg cagctgatca cacagcaaac 1380
   ataaatcatg gatcgtttat ttatttggag gcacagcgct cccccggggt ggccaagctt 1440
   ggaagtcctg ttcttacaaa attgctcact gcctctaccc catgtcaggt gcagttttgg 1500
   tatcatttgt ctcaacattc aaatctctca gtttttacaa gaacgtctct agatggaaac 1560
   ttgcaaaagc agggcaaaat aatcagattc tccgaatctc agtggagcca cgcaaaaatt 1620
   gatctcattg cagaagcggg agaatctact ctaccttttc agttaatttt ggaagctact 1680
   gttttgtcgt caaatgctac cgttgctcta gatgacatca gtgtgtccca ggaatgtgaa 1740
   atttcctata aatcactacc aaggaccagt acacaaagca agttttccaa gtgtgacttt 1800
   gaagcaaaca gctgtgattg gtttgaagca attagtggtg accattttga ctggatacgg 1860
   agctctcaga gtgaactttc tgctgatttt gagcaccagg ctccacctcg ggatcatagt 1920
   ctcaacgcat ctcaagggca ttttatgttc attctgaaga aaagcagcag cttgtggcaa 1980
   gttgctaagc ttcagagccc aactttcagc cagacaggac ctggatgcat actttccttc 2040
   tggttctata actatggcct gtcagtggga gcagctgagc tgcagctaca tatggaaaat 2100
   tctcatgact caacagtgat ttggagagta ttatacaatc agggcaaaca atggttggag 2160
   gcaaccattc agctagggcg cctttcgcag cccttccatt tgtcactaga taaagtcagt 2220
   ctgggcattt atgatggggt ctcagctatt gatgacatcc gatttgaaaa ttgtactctc 2280
   cctcttcctg ctgagagctg tgaagggctg gatcatttct ggtgtcgcca caccagggct 2340
   tgcatagaaa agcttcggtt atgtgatctg gtggatgact gtggtgatcg tactgatgaa 2400
   gtcaactgtg cacctgagct gcagtgtaac tttgaaactg gaatctgtaa ctgggaacaa 2460
   gatgcaaaag atgactttga ttggaccagg agccagggtc caactccaac acttaacaca 2520
   gggccaatga aagataacac tctgggcaca gctaaaggac actatctcta catagaatct 2580
   tcagagccac aggcttttca agacagtgct gccttactca gcccaatcct taatgccact 2640
   gatacaaaag gctgcacctt ccgcttctat taccacatgt ttggaaagcg catttatagg 2700
   ttggcaatct accaacgaat ctggagtgac tcaaggggac agctgctgtg gcagatattt 2760
   gggaatcaag gcaacagatg gattaggaaa cacctcaaca tttccagcag gcagcccttt 2820
   cagatattgg tggaggcttc agtgggagat ggcttcactg gagatattgc gattgatgat 2880
   ctgtcattta tggactgcac cctctaccct ggtaatttgc cagcagacct cccaactcca 2940
   ccagaaacgt cagttcctgt aacattacct ccacacaact gcacagacaa tgaatttatc 3000
   tgcaggtctg atggtcactg cattgaaaaa atgcagaaat gtgattttaa atatgactgc 3060
   cctgacaaat cagatgaagc atcctgtgtt atggaagttt gcagctttga gaaaagaagc 3120
   ctgtgtaaat ggtatcaacc aatcccagta catttgcttc aagattcaaa cacattcagg 3180
   tgggggcttg ggaacgggat cagcattcat catggggaag aaaaccacag gccatcagtg 3240
   gatcatacac aaaataccac tgatggctgg tacctgtatg ctgacagttc taatgggaaa 3300
   tttggtgaca cggctgacat tctcactcct atcatttcac tcacgggacc aaaatgtacc 3360
   ttggtgttct ggacacatat gaatggggcc accgttggtt ctctccaggt gctcatcaag 3420
   aaagataacg ttacttctaa attgtgggct caaactggac agcaaggtgc acagtggaag 3480
   agagcagaag tgtttttagg cattcgttca catacacaga ttgtcttcag agccaaacgt 3540
   ggtatcagtt acataggaga tgtagcagtg gatgatattt ccttccaaga ttgctcccct 3600
   ttgcttagcc cagagagaaa gtgtactgat catgaattca tgtgtgctaa taagcactgc 3660
   attgccaaag acaagctgtg tgattttgtg aatgattgtg ctgataattc agatgagact 3720
   actttcattt gccgtacctc cagtgggcgc tgtgatttcg aatttgatct ttgttcctgg 3780
   aagcaggaga aagatgagga ctttgactgg aacctgaaag ctagcagcat ccctgcagca 3840
   ggcacagagc cagcagcaga tcacactttg ggaaattcat ctggtcatta catctttata 3900
   aagagtttgt ttcctcagca gcccatgaga gctgccagaa tttcaagtcc agttataagt 3960
   aagagaagca aaaactgcaa gattattttt cattatcaca tgtatggaaa tggcattggg 4020
   gcactcacct taatgcaggt gtcagtcaca aaccaaacga aggttctact taacctcact 4080
   gtagaacaag gcaatttctg gcggagagaa gaactgtcac tgtttggtga tgaagacttc 4140
   caactcaaat ttgaaggtag agttgggaaa ggtcagcgtg gagacattgc acttgatgac 4200
   attgtgctta cagaaaattg tctatcactc catgattccg tgcaagaaga actggcagtg 4260
   cctcttccaa caggtttctg cccacttggc tatagggaat gtcataatgg aaaatgctat 4320
   aggctggaac aaagctgtaa cttcgtagat aactgtggag ataatactga tgaaaatgag 4380
   tgtggtagct cctgtacttt tgaaaaaggc tggtgtggct ggcaaaactc ccaggctgac   4440
   aactttgatt gggttttagg ggttggctct catcaaagct taagacctcc caaagaccac   4500
   acacttggaa atgaaaatgg gcacttcatg tatctggaag ctactgcagt gggccttcgg   4560
   ggtgacaaag cacacttcag gagtaccatg tggcgagaat ccagtgcagc ctgcaccatg   4620
   agcttctggt atttcgtatc tgcaaaggcc acaggatcca ttcagattct catcaagaca   4680
   gagaaaggac tatcaaaagt atggcaagaa agtaagcaga accctggtaa tcattggcaa   4740
   aaggctgaca tcctgctagg aaagttaagg aattttgaag tcatatttca aggtatcaga   4800
   acaagggacc tgggaggagg agctgcaatt gatgatattg aatttaaaaa ctgcacaact   4860
   gtgggagaga tctctgagct ttgtccggaa atcactgatt ttttgtgccg ggacaagaag   4920
   tgcattgcat cccaccttct ttgtgactat aagccagact gctctgatag gtctgatgaa   4980
   gctcactgtg cacattatac aagcacaaca ggaagctgca attttgaaac aagttcagga   5040
   aactggacca cagcctgcag tcttactcaa gactctgagg atgacttgga ctgggccatt   5100
   ggcagcagaa ttcctgccaa agcattaatt ccagactctg atcacacgcc aggtagtggt   5160
   cagcacttcc tgtacgtcaa ctcatctggc tccaaggaag gatccgttgc cagaattact   5220
   acttccaaat ccttcccagc aagccttgga atgtgtactg ttcggttctg gttctacatg   5280
   attgatccca ggagtatggg aatattaaag gtgtatacca ttgaagaatc ggggctaaac   5340
   atcctggtgt ggtcagtgat tggaaataaa agaacgggat ggacatatgg ctctgtgcct   5400
   ctctccagta acagtccgtt taaggtggca tttgaagctg atttggatgg aaatgaggac   5460
   atctttattg ctcttgatga catctctttt accccagagt gtgtgactgg aggtcctgtc   5520
   ccagtgcagc catcaccctg tgaagctgat cagttttctt gtatctacac actccaatgt   5580
   gtccctctct cagggaaatg tgatggacat gaagactgca tagatggatc tgatgaaatg   5640
   gattgtcctc tcagccccac ccctccactc tgtagtaaca tggagttccc gtgctctaca   5700
   gacgagtgta taccttccct cctgctatgc gatggagtgc ccgactgcca ctttaatgaa   5760
   gatgagctca tctgctccaa caaaagctgt tctaatggag ctctggtgtg tgcctcctcc   5820
   aacagctgta tcccagccca ccagcgctgt gatggttttg ccgactgcat ggatttccag   5880
   cttgatgagt ccagctgctc cgaatgtcca ttaaattact gcagaaatgg tgggacttgt   5940
   gtagtggaga aaaatggtcc tatgtgtcga tgtagacaag gctggaaagg aaatcgatgc   6000
   catatcaagt ttaatcctcc tgctacagac ttcacatacg ctcagaataa taca         6054
<210> 24
   <211> 2018
   <212> PRT
   <213> Homo sapiens
<400> 24
   Met Asn Glu Thr Phe Cys Cys Leu Trp Ile Ala Cys Val Phe Asn Ser
   1               5                   10                  15
   Thr Leu Ala Gln Gln Gly Thr Glu Ser Phe Gln Cys Asp Asn Gly Val
               20                  25                  30
   Ser Leu Pro Pro Asp Ser Ile Cys Asp Phe Thr Asp Gln Cys Gly Asp
           35                  40                  45
   Ser Ser Asp Glu Arg His Trp Leu Ser Phe Ala His Pro Cys Asn Pro
       50                  55                  60
   Leu Gly Ser Ser Leu Glu Ser Asp Asn Trp Asn Lys Lys Lys Leu Glu
   65                  70                  75                  80
   Ser Gly Ala Cys Leu Gly Phe Gly Ala Ala Ala Ala Ser Gln Asp Pro
                   85                  90                  95
   Arg Gln Glu Leu Arg Lys Lys Leu Val Trp Thr Ala Asp Pro Arg Asn
               100                 105                 110
   Trp Arg Ala Ile Thr Phe Tyr Tyr Phe Ser Cys Gln Val Ser Gly Lys
           115                 120                 125
   Leu Met Val Gly Leu Gln Thr Ala Cys Gly Gly Pro Ile Gln His Leu
       130                 135                 140
   Trp Gln Asn Thr Ala Ala Leu Pro Asn Gln Trp Glu Arg Asn Val Ile
   145                 150                 155                 160
   Lys Ile Gln Ser Ser Gln Arg Phe Gln Val Val Phe Glu Gly Gln Met
                   165                 170                 175
   Ala Ser Thr Tyr Glu Gln Asp Glu Val Ile Ala Ile Asp Asp Ile Ser
               180                 185                 190
   Phe Ser Ser Gly Cys Leu Pro Ala Asn Asp Gly Ile Leu Leu Cys Gln
           195                 200                 205
   Glu Ala Leu Asn Ala Glu Arg Glu Leu Cys His Pro Asp Thr Asp Leu
       210                 215                 220
   Cys Arg Phe Asp Ala Thr Asp Glu Glu Leu Arg Leu Cys Gln Ala Cys
   225                 230                 235                 240
   Gly Phe Glu Phe Asp Met Cys Glu Trp Thr Ser Glu Ala Ser Ala Gly
                   245                 250                 255
   Gln Ile Ser Trp Met Arg Thr Lys Ala Arg Glu Ile Pro Ala Phe Glu
               260                 265                 270
   Ser Thr Pro Gln Gln Asp Gln Gly Gly Asp Asp Glu Gly Tyr Tyr Val
   275 280 285
   Trp Val Gly Ala Lys His Gly Phe Thr Leu Asn His Leu Asp Ser Arg
   290 295 300
   Ala Tyr Leu Asn Ser Ser Val Cys His Cys Leu Gly Lys Ser Cys His
   305 310 315 320
   Leu Gln Phe Tyr Tyr Ala Met Glu Ser Ser Val Leu Arg Val Arg Leu
   325 330 335
   Tyr Asn Asn Lys Glu Glu Glu Ile Phe Trp Thr Tyr Asn Ile Ser Thr
   340 345 350
   His Ser Gln Trp Val Lys Ala Asp Val Leu Ile Pro Glu Asp Leu Lys
   355 360 365
   Thr Phe Lys Thr Gln Ser Arg Lys Leu Cys Ser Ala Asp Glu Phe Pro
   370 375 380
   Cys Thr Ser Gly Gln Cys Ile Ala Lys Glu Ser Val Cys Asp Ser Arg
   385 390 395 400
   Gln Asp Cys Ser Asp Glu Ser Asp Glu Asp Pro Ala Thr Cys Ser Lys
   405 410 415
   His Leu Thr Cys Asp Phe Glu Ser Gly Phe Cys Gly Trp Glu Pro Phe
   420 425 430
   Leu Thr Glu Asp Ser His Trp Lys Leu Met Lys Gly Leu Asn Asn Gly
   435 440 445
   Glu His His Phe Pro Ala Ala Asp His Thr Ala Asn Ile Asn His Gly
   450 455 460
   Ser Phe Ile Tyr Leu Glu Ala Gln Arg Ser Pro Gly Val Ala Lys Leu
   465 470 475 480
   Gly Ser Pro Val Leu Thr Lys Leu Leu Thr Ala Ser Thr Pro Cys Gln
   485 490 495
   Val Gln Phe Trp Tyr His Leu Ser Gln His Ser Asn Leu Ser Val Phe
   500 505 510
   Thr Arg Thr Ser Leu Asp Gly Asn Leu Gln Lys Gln Gly Lys Ile Ile
   515 520 525
   Arg Phe Ser Glu Ser Gln Trp Ser His Ala Lys Ile Asp Leu Ile Ala
   530 535 540
   Glu Ala Gly Glu Ser Thr Leu Pro Phe Gln Leu Ile Leu Glu Ala Thr
   545 550 555 560
   Val Leu Ser Ser Asn Ala Thr Val Ala Leu Asp Asp Ile Ser Val Ser
   565 570 575
   Gln Glu Cys Glu Ile Ser Tyr Lys Ser Leu Pro Arg Thr Ser Thr Gln
   580 585 590
   Ser Lys Phe Ser Lys Cys Asp Phe Glu Ala Asn Ser Cys Asp Trp Phe
   595 600 605
   Glu Ala Ile Ser Gly Asp His Phe Asp Trp Ile Arg Ser Ser Gln Ser
   610 615 620
   Glu Leu Ser Ala Asp Phe Glu His Gln Ala Pro Pro Arg Asp His Ser
   625 630 635 640
   Leu Asn Ala Ser Gln Gly His Phe Met Phe Ile Leu Lys Lys Ser Ser
   645 650 655
   Ser Leu Trp Gln Val Ala Lys Leu Gln Ser Pro Thr Phe Ser Gln Thr
   660 665 670
   Gly Pro Gly Cys Ile Leu Ser Phe Trp Phe Tyr Asn Tyr Gly Leu Ser
   675 680 685
   Val Gly Ala Ala Glu Leu Gln Leu His Met Glu Asn Ser His Asp Ser
   690 695 700
   Thr Val Ile Trp Arg Val Leu Tyr Asn Gln Gly Lys Gln Trp Leu Glu
   705 710 715 720
   Ala Thr Ile Gln Leu Gly Arg Leu Ser Gln Pro Phe His Leu Ser Leu
   725 730 735
   Asp Lys Val Ser Leu Gly Ile Tyr Asp Gly Val Ser Ala Ile Asp Asp
   740 745 750
   Ile Arg Phe Glu Asn Cys Thr Leu Pro Leu Pro Ala Glu Ser Cys Glu
   755 760 765
   Gly Leu Asp His Phe Trp Cys Arg His Thr Arg Ala Cys Ile Glu Lys
   770 775 780
   Leu Arg Leu Cys Asp Leu Val Asp Asp Cys Gly Asp Arg Thr Asp Glu
   785 790 795 800
   Val Asn Cys Ala Pro Glu Leu Gln Cys Asn Phe Glu Thr Gly Ile Cys
   805 810 815
   Asn Trp Glu Gln Asp Ala Lys Asp Asp Phe Asp Trp Thr Arg Ser Gln
   820 825 830
   Gly Pro Thr Pro Thr Leu Asn Thr Gly Pro Met Lys Asp Asn Thr Leu
   835 840 845
   Gly Thr Ala Lys Gly His Tyr Leu Tyr Ile Glu Ser Ser Glu Pro Gln
   850 855 860
   Ala Phe Gln Asp Ser Ala Ala Leu Leu Ser Pro Ile Leu Asn Ala Thr
   865 870 875 880
   Asp Thr Lys Gly Cys Thr Phe Arg Phe Tyr Tyr His Met Phe Gly Lys
   885 890 895
   Arg Ile Tyr Arg Leu Ala Ile Tyr Gln Arg Ile Trp Ser Asp Ser Arg
   900 905 910
   Gly Gln Leu Leu Trp Gln Ile Phe Gly Asn Gln Gly Asn Arg Trp Ile
   915 920 925
   Arg Lys His Leu Asn Ile Ser Ser Arg Gln Pro Phe Gln Ile Leu Val
   930 935 940
   Glu Ala Ser Val Gly Asp Gly Phe Thr Gly Asp Ile Ala Ile Asp Asp
   945 950 955 960
   Leu Ser Phe Met Asp Cys Thr Leu Tyr Pro Gly Asn Leu Pro Ala Asp
   965 970 975
   Leu Pro Thr Pro Pro Glu Thr Ser Val Pro Val Thr Leu Pro Pro His
   980 985 990
   Asn Cys Thr Asp Asn Glu Phe Ile Cys Arg Ser Asp Gly His Cys Ile
   995 1000 1005
   Glu Lys Met Gln Lys Cys Asp Phe Lys Tyr Asp Cys Pro Asp Lys Ser
   1010 1015 1020
   Asp Glu Ala Ser Cys Val Met Glu Val Cys Ser Phe Glu Lys Arg Ser
   1025 1030 1035 1040
   Leu Cys Lys Trp Tyr Gln Pro Ile Pro Val His Leu Leu Gln Asp Ser
   1045 1050 1055
   Asn Thr Phe Arg Trp Gly Leu Gly Asn Gly Ile Ser Ile His His Gly
   1060 1065 1070
   Glu Glu Asn His Arg Pro Ser Val Asp His Thr Gln Asn Thr Thr Asp
   1075 1080 1085
   Gly Trp Tyr Leu Tyr Ala Asp Ser Ser Asn Gly Lys Phe Gly Asp Thr
   1090 1095 1100
   Ala Asp Ile Leu Thr Pro Ile Ile Ser Leu Thr Gly Pro Lys Cys Thr
   1105 1110 1115 1120
   Leu Val Phe Trp Thr His Met Asn Gly Ala Thr Val Gly Ser Leu Gln
   1125 1130 1135
   Val Leu Ile Lys Lys Asp Asn Val Thr Ser Lys Leu Trp Ala Gln Thr
   1140 1145 1150
   Gly Gln Gln Gly Ala Gln Trp Lys Arg Ala Glu Val Phe Leu Gly Ile
   1155 1160 1165
   Arg Ser His Thr Gln Ile Val Phe Arg Ala Lys Arg Gly Ile Ser Tyr
   1170 1175 1180
   Ile Gly Asp Val Ala Val Asp Asp Ile Ser Phe Gln Asp Cys Ser Pro
   1185 1190 1195 1200
   Leu Leu Ser Pro Glu Arg Lys Cys Thr Asp His Glu Phe Met Cys Ala
   1205 1210 1215
   Asn Lys His Cys Ile Ala Lys Asp Lys Leu Cys Asp Phe Val Asn Asp
   1220 1225 1230
   Cys Ala Asp Asn Ser Asp Glu Thr Thr Phe Ile Cys Arg Thr Ser Ser
   1235 1240 1245
   Gly Arg Cys Asp Phe Glu Phe Asp Leu Cys Ser Trp Lys Gln Glu Lys
   1250 1255 1260
   Asp Glu Asp Phe Asp Trp Asn Leu Lys Ala Ser Ser Ile Pro Ala Ala
   1265 1270 1275 1280
   Gly Thr Glu Pro Ala Ala Asp His Thr Leu Gly Asn Ser Ser Gly His
   1285 1290 1295
   Tyr Ile Phe Ile Lys Ser Leu Phe Pro Gln Gln Pro Met Arg Ala Ala
   1300 1305 1310
   Arg Ile Ser Ser Pro Val Ile Ser Lys Arg Ser Lys Asn Cys Lys Ile
   1315 1320 1325
   Ile Phe His Tyr His Met Tyr Gly Asn Gly Ile Gly Ala Leu Thr Leu
   1330 1335 1340
   Met Gln Val Ser Val Thr Asn Gln Thr Lys Val Leu Leu Asn Leu Thr
   1345 1350 1355 1360
   Val Glu Gln Gly Asn Phe Trp Arg Arg Glu Glu Leu Ser Leu Phe Gly
   1365 1370 1375
   Asp Glu Asp Phe Gln Leu Lys Phe Glu Gly Arg Val Gly Lys Gly Gln
   1380 1385 1390
   Arg Gly Asp Ile Ala Leu Asp Asp Ile Val Leu Thr Glu Asn Cys Leu
   1395 1400 1405
   Ser Leu His Asp Ser Val Gln Glu Glu Leu Ala Val Pro Leu Pro Thr
   1410 1415 1420
   Gly Phe Cys Pro Leu Gly Tyr Arg Glu Cys His Asn Gly Lys Cys Tyr
   1425 1430 1435 1440
   Arg Leu Glu Gln Ser Cys Asn Phe Val Asp Asn Cys Gly Asp Asn Thr
   1445 1450 1455
   Asp Glu Asn Glu Cys Gly Ser Ser Cys Thr Phe Glu Lys Gly Trp Cys
   1460 1465 1470
   Gly Trp Gln Asn Ser Gln Ala Asp Asn Phe Asp Trp Val Leu Gly Val
   1475 1480 1485
   Gly Ser His Gln Ser Leu Arg Pro Pro Lys Asp His Thr Leu Gly Asn
   1490 1495 1500
   Glu Asn Gly His Phe Met Tyr Leu Glu Ala Thr Ala Val Gly Leu Arg
   1505 1510 1515 1520
   Gly Asp Lys Ala His Phe Arg Ser Thr Met Trp Arg Glu Ser Ser Ala
   1525 1530 1535
   Ala Cys Thr Met Ser Phe Trp Tyr Phe Val Ser Ala Lys Ala Thr Gly
   1540 1545 1550
   Ser Ile Gln Ile Leu Ile Lys Thr Glu Lys Gly Leu Ser Lys Val Trp
   1555 1560 1565
   Gln Glu Ser Lys Gln Asn Pro Gly Asn His Trp Gln Lys Ala Asp Ile
   1570 1575 1580
   Leu Leu Gly Lys Leu Arg Asn Phe Glu Val Ile Phe Gln Gly Ile Arg
   1585 1590 1595 1600
   Thr Arg Asp Leu Gly Gly Gly Ala Ala Ile Asp Asp Ile Glu Phe Lys
   1605 1610 1615
   Asn Cys Thr Thr Val Gly Glu Ile Ser Glu Leu Cys Pro Glu Ile Thr
   1620 1625 1630
   Asp Phe Leu Cys Arg Asp Lys Lys Cys Ile Ala Ser His Leu Leu Cys
   1635 1640 1645
   Asp Tyr Lys Pro Asp Cys Ser Asp Arg Ser Asp Glu Ala His Cys Ala
   1650 1655 1660
   His Tyr Thr Ser Thr Thr Gly Ser Cys Asn Phe Glu Thr Ser Ser Gly
   1665 1670 1675 1680
   Asn Trp Thr Thr Ala Cys Ser Leu Thr Gln Asp Ser Glu Asp Asp Leu
   1685 1690 1695
   Asp Trp Ala Ile Gly Ser Arg Ile Pro Ala Lys Ala Leu Ile Pro Asp
   1700 1705 1710
   Ser Asp His Thr Pro Gly Ser Gly Gln His Phe Leu Tyr Val Asn Ser
   1715 1720 1725
   Ser Gly Ser Lys Glu Gly Ser Val Ala Arg Ile Thr Thr Ser Lys Ser
   1730 1735 1740
   Phe Pro Ala Ser Leu Gly Met Cys Thr Val Arg Phe Trp Phe Tyr Met
   1745 1750 1755 1760
   Ile Asp Pro Arg Ser Met Gly Ile Leu Lys Val Tyr Thr Ile Glu Glu
   1765 1770 1775
   Ser Gly Leu Asn Ile Leu Val Trp Ser Val Ile Gly Asn Lys Arg Thr
   1780 1785 1790
   Gly Trp Thr Tyr Gly Ser Val Pro Leu Ser Ser Asn Ser Pro Phe Lys
   1795 1800 1805
   Val Ala Phe Glu Ala Asp Leu Asp Gly Asn Glu Asp Ile Phe Ile Ala
   1810 1815 1820
   Leu Asp Asp Ile Ser Phe Thr Pro Glu Cys Val Thr Gly Gly Pro Val
   1825 1830 1835 1840
   Pro Val Gln Pro Ser Pro Cys Glu Ala Asp Gln Phe Ser Cys Ile Tyr
   1845 1850 1855
   Thr Leu Gln Cys Val Pro Leu Ser Gly Lys Cys Asp Gly His Glu Asp
   1860 1865 1870
   Cys Ile Asp Gly Ser Asp Glu Met Asp Cys Pro Leu Ser Pro Thr Pro
               1875                1880                1885
   Pro Leu Cys Ser Asn Met Glu Phe Pro Cys Ser Thr Asp Glu Cys Ile
       1890                1895                1900
   Pro Ser Leu Leu Leu Cys Asp Gly Val Pro Asp Cys His Phe Asn Glu
   1905                1910                1915                1920
   Asp Glu Leu Ile Cys Ser Asn Lys Ser Cys Ser Asn Gly Ala Leu Val
                   1925                1930                1935
   Cys Ala Ser Ser Asn Ser Cys Ile Pro Ala His Gln Arg Cys Asp Gly
               1940                1945                1950
   Phe Ala Asp Cys Met Asp Phe Gln Leu Asp Glu Ser Ser Cys Ser Glu
           1955                1960                1965
   Cys Pro Leu Asn Tyr Cys Arg Asn Gly Gly Thr Cys Val Val Glu Lys
       1970                1975                1980
   Asn Gly Pro Met Cys Arg Cys Arg Gln Gly Trp Lys Gly Asn Arg Cys
   1985                1990                1995                2000
   His Ile Lys Phe Asn Pro Pro Ala Thr Asp Phe Thr Tyr Ala Gln Asn
                   2005                2010                2015
   Asn Thr
<210> 25
   <211> 6300
   <212> DNA
   <213> Homo sapiens
<400> 25
   atgaatgaaa ctttttgttg cctttggatt gcctgtgttt tcaattctac actggctcag    60
   caagggacag aaagctttca gtgtgacaat ggagtctcct tgcctcctga cagcatttgt   120
   gacttcacag atcagtgtgg ggatagcagt gatgaacggc actggctttc atttgctcat   180
   ccttgcaatc cattggggtc tagtttggaa tctgacaact ggaacaaaaa gaaacttgaa   240
   tccggtgcat gccttggttt tggtgctgct gctgcttctc aagatcctcg gcaggaatta   300
   agaaagaagc tggtgtggac agcagatccc cgaaattggc gggctattac attttattac   360
   ttctcctgcc aagtgagtgg caaattaatg gttgggcttc aaactgcatg tggaggtcct   420
   attcagcatt tatggcaaaa cacagctgca ctcccaaatc agtgggagag aaatgtcatc   480
   aaaatccaga gttcacagag atttcaggtt gtttttgaag gtcaaatggc ttcaacgtat   540
   gaacaggatg aagtcattgc tattgatgat atatctttca gttcaggctg cttgcctgcc   600
   aatgatggaa ttttactgtg tcaagaagca ttgaatgctg agcgggagct atgccatcca   660
   gatacagatc tctgcagatt tgatgctaca gatgaagagt tgagattgtg tcaggcctgt   720
   gggtttgaat ttgacatgtg tgagtggacg tcagaagcat ctgctggcca aatttcctgg   780
   atgcgcacaa aagcgagaga gatccctgca ttcgaatcca cacctcagca ggatcaagga   840
   ggtgatgatg aaggttatta tgtatgggta ggcgctaagc atggtttcac tcttaaccat   900
   ttagacagca gggcttacct aaatagctct gtgtgtcatt gcctgggcaa gagctgtcat   960
   cttcaattct attatgcaat ggaaagcagt gtcctgagag taagactgta taataataag  1020
   gaagaagaaa tattttggac atacaacata tcaactcaca gccaatgggt gaaagcagat  1080
   gtgttaatac cagaagatct gaagacattt aagacccaat ccagaaagct ttgctctgca  1140
   gacgaattcc cttgcactag tggccagtgc atcgccaaag aatctgtctg tgactctcgg  1200
   caggactgct ccgatgagag tgatgaagac ccagcaactt gctcaaagca tctcacctgt  1260
   gactttgagt cgggtttctg cggttgggag ccatttctca cagaagattc acactggaag  1320
   ctgatgaaag gattgaataa tggagagcac cactttcctg cagctgatca cacagcaaac  1380
   ataaatcatg gatcgtttat ttatttggag gcacagcgct cccccggggt ggccaagctt  1440
   ggaagtcctg ttcttacaaa attgctcact gcctctaccc catgtcaggt gcagttttgg  1500
   tatcatttgt ctcaacattc aaatctctca gtttttacaa gaacgtctct agatggaaac  1560
   ttgcaaaagc agggcaaaat aatcagattc tccgaatctc agtggagcca cgcaaaaatt  1620
   gatctcattg cagaagcggg agaatctact ctaccttttc agttaatttt ggaagctact  1680
   gttttgtcgt caaatgctac cgttgctcta gatgacatca gtgtgtccca ggaatgtgaa  1740
   atttcctata aatcactacc aaggaccagt acacaaagca agttttccaa gtgtgacttt  1800
   gaagcaaaca gctgtgattg gtttgaagca attagtggtg accattttga ctggatacgg  1860
   agctctcaga gtgaactttc tgctgatttt gagcaccagg ctccacctcg ggatcatagt  1920
   ctcaacgcat ctcaagggca ttttatgttc attctgaaga aaagcagcag cttgtggcaa  1980
   gttgctaagc ttcagagccc aactttcagc cagacaggac ctggatgcat actttccttc  2040
   tggttctata actatggcct gtcagtggga gcagctgagc tgcagctaca tatggaaaat  2100
   tctcatgact caacagtgat ttggagagta ttatacaatc agggcaaaca atggttggag  2160
   gcaaccattc agctagggcg cctttcgcag cccttccatt tgtcactaga taaagtcagt  2220
   ctgggcattt atgatggggt ctcagctatt gatgacatcc gatttgaaaa ttgtactctc  2280
   cctcttcctg ctgagagctg tgaagggctg gatcatttct ggtgtcgcca caccagggct  2340
   tgcatagaaa agcttcggtt atgtgatctg gtggatgact gtggtgatcg tactgatgaa  2400
   gtcaactgtg cacctgagct gcagtgtaac tttgaaactg gaatctgtaa ctgggaacaa  2460
   gatgcaaaag atgactttga ttggaccagg agccagggtc caactccaac acttaacaca  2520
   gggccaatga aagataacac tctgggcaca gctaaaggac actatctcta catagaatct  2580
   tcagagccac aggcttttca agacagtgct gccttactca gcccaatcct taatgccact 2640
   gatacaaaag gctgcacctt ccgcttctat taccacatgt ttggaaagcg catttatagg 2700
   ttggcaatct accaacgaat ctggagtgac tcaaggggac agctgctgtg gcagatattt 2760
   gggaatcaag gcaacagatg gattaggaaa cacctcaaca tttccagcag gcagcccttt 2820
   cagatattgg tggaggcttc agtgggagat ggcttcactg gagatattgc gattgatgat 2880
   ctgtcattta tggactgcac cctctaccct ggtaatttgc cagcagacct cccaactcca 2940
   ccagaaacgt cagttcctgt aacattacct ccacacaact gcacagacaa tgaatttatc 3000
   tgcaggtctg atggtcactg cattgaaaaa atgcagaaat gtgattttaa atatgactgc 3060
   cctgacaaat cagatgaagc atcctgtgtt atggaagttt gcagctttga gaaaagaagc 3120
   ctgtgtaaat ggtatcaacc aatcccagta catttgcttc aagattcaaa cacattcagg 3180
   tgggggcttg ggaacgggat cagcattcat catggggaag aaaaccacag gccatcagtg 3240
   gatcatacac aaaataccac tgatggctgg tacctgtatg ctgacagttc taatgggaaa 3300
   tttggtgaca cggctgacat tctcactcct atcatttcac tcacgggacc aaaatgtacc 3360
   ttggtgttct ggacacatat gaatggggcc accgttggtt ctctccaggt gctcatcaag 3420
   aaagataacg ttacttctaa attgtgggct caaactggac agcaaggtgc acagtggaag 3480
   agagcagaag tgtttttagg cattcgttca catacacaga ttgtcttcag agccaaacgt 3540
   ggtatcagtt acataggaga tgtagcagtg gatgatattt ccttccaaga ttgctcccct 3600
   ttgcttagcc cagagagaaa gtgtactgat catgaattca tgtgtgctaa taagcactgc 3660
   attgccaaag acaagctgtg tgattttgtg aatgattgtg ctgataattc agatgagact 3720
   actttcattt gccgtacctc cagtgggcgc tgtgatttcg aatttgatct ttgttcctgg 3780
   aagcaggaga aagatgagga ctttgactgg aacctgaaag ctagcagcat ccctgcagca 3840
   ggcacagagc cagcagcaga tcacactttg ggaaattcat ctggtcatta catctttata 3900
   aagagtttgt ttcctcagca gcccatgaga gctgccagaa tttcaagtcc agttataagt 3960
   aagagaagca aaaactgcaa gattattttt cattatcaca tgtatggaaa tggcattggg 4020
   gcactcacct taatgcaggt gtcagtcaca aaccaaacga aggttctact taacctcact 4080
   gtagaacaag gcaatttctg gcggagagaa gaactgtcac tgtttggtga tgaagacttc 4140
   caactcaaat ttgaaggtag agttgggaaa ggtcagcgtg gagacattgc acttgatgac 4200
   attgtgctta cagaaaattg tctatcactc catgattccg tgcaagaaga actggcagtg 4260
   cctcttccaa caggtttctg cccacttggc tatagggaat gtcataatgg aaaatgctat 4320
   aggctggaac aaagctgtaa cttcgtagat aactgtggag ataatactga tgaaaatgag 4380
   tgtggtagct cctgtacttt tgaaaaaggc tggtgtggct ggcaaaactc ccaggctgac 4440
   aactttgatt gggttttagg ggttggctct catcaaagct taagacctcc caaagaccac 4500
   acacttggaa atgaaaatgg gcacttcatg tatctggaag ctactgcagt gggccttcgg 4560
   ggtgacaaag cacacttcag gagtaccatg tggcgagaat ccagtgcagc ctgcaccatg 4620
   agcttctggt atttcgtatc tgcaaaggcc acaggatcca ttcagattct catcaagaca 4680
   gagaaaggac tatcaaaagt atggcaagaa agtaagcaga accctggtaa tcattggcaa 4740
   aaggctgaca tcctgctagg aaagttaagg aattttgaag tcatatttca aggtatcaga 4800
   acaagggacc tgggaggagg agctgcaatt gatgatattg aatttaaaaa ctgcacaact 4860
   gtgggagaga tctctgagct ttgtccggaa atcactgatt ttttgtgccg ggacaagaag 4920
   tgcattgcat cccaccttct ttgtgactat aagccagact gctctgatag gtctgatgaa 4980
   gctcactgtg cacattatac aagcacaaca ggaagctgca attttgaaac aagttcagga 5040
   aactggacca cagcctgcag tcttactcaa gactctgagg atgacttgga ctgggccatt 5100
   ggcagcagaa ttcctgccaa agcattaatt ccagactctg atcacacgcc aggtagtggt 5160
   cagcacttcc tgtacgtcaa ctcatctggc tccaaggaag gatccgttgc cagaattact 5220
   acttccaaat ccttcccagc aagccttgga atgtgtactg ttcggttctg gttctacatg 5280
   attgatccca ggagtatggg aatattaaag gtgtatacca ttgaagaatc ggggctaaac 5340
   atcctggtgt ggtcagtgat tggaaataaa agaacgggat ggacatatgg ctctgtgcct 5400
   ctctccagta acagtccgtt taaggtggca tttgaagctg atttggatgg aaatgaggac 5460
   atctttattg ctcttgatga catctctttt accccagagt gtgtgactgg aggtcctgtc 5520
   ccagtgcagc catcaccctg tgaagctgat cagttttctt gtatctacac actccaatgt 5580
   gtccctctct cagggaaatg tgatggacat gaagactgca tagatggatc tgatgaaatg 5640
   gattgtcctc tcagccccac ccctccactc tgtagtaaca tggagttccc gtgctctaca 5700
   gacgagtgta taccttccct cctgctatgc gatggagtgc ccgactgcca ctttaatgaa 5760
   gatgagctca tctgctccaa caaaagctgt tctaatggag ctctggtgtg tgcctcctcc 5820
   aacagctgta tcccagccca ccagcgctgt gatggttttg ccgactgcat ggatttccag 5880
   cttgatgagt ccagctgctc cgaatgtcca ttaaattact gcagaaatgg tgggacttgt 5940
   gtagtggaga aaaatggtcc tatgtgtcga tgtagacaag gctggaaagg aaatcgatgc 6000
   catatcaagt ttaatcctcc tgctacagac ttcacatacg ctcagaataa tacatggact 6060
   ctcctgggta ttggattagc attcctgatg actcacatca cagttgcagt cttgtgtttt 6120
   cttgcaaaca gaaaggtacc aataaggaaa accgagggaa gtggtaactg tgcctttgtc 6180
   aatccagttt acgggaactg gagcaaccca gagaaaacag agagttctgt ctattccttc 6240
   tcaaacccat tatatggcac aacatcagga agcctggaga ccctgtcaca tcatctcaaa 6300
<210> 26
   <211> 2100
   <212> PRT
   <213> Homo sapiens
<400> 26
   Met Asn Glu Thr Phe Cys Cys Leu Trp Ile Ala Cys Val Phe Asn Ser
   1 5 10 15
   Thr Leu Ala Gln Gln Gly Thr Glu Ser Phe Gln Cys Asp Asn Gly Val
   20 25 30
   Ser Leu Pro Pro Asp Ser Ile Cys Asp Phe Thr Asp Gln Cys Gly Asp
   35 40 45
   Ser Ser Asp Glu Arg His Trp Leu Ser Phe Ala His Pro Cys Asn Pro
   50 55 60
   Leu Gly Ser Ser Leu Glu Ser Asp Asn Trp Asn Lys Lys Lys Leu Glu
   65 70 75 80
   Ser Gly Ala Cys Leu Gly Phe Gly Ala Ala Ala Ala Ser Gln Asp Pro
   85 90 95
   Arg Gln Glu Leu Arg Lys Lys Leu Val Trp Thr Ala Asp Pro Arg Asn
   100 105 110
   Trp Arg Ala Ile Thr Phe Tyr Tyr Phe Ser Cys Gln Val Ser Gly Lys
   115 120 125
   Leu Met Val Gly Leu Gln Thr Ala Cys Gly Gly Pro Ile Gln His Leu
   130 135 140
   Trp Gln Asn Thr Ala Ala Leu Pro Asn Gln Trp Glu Arg Asn Val Ile
   145 150 155 160
   Lys Ile Gln Ser Ser Gln Arg Phe Gln Val Val Phe Glu Gly Gln Met
   165 170 175
   Ala Ser Thr Tyr Glu Gln Asp Glu Val Ile Ala Ile Asp Asp Ile Ser
   180 185 190
   Phe Ser Ser Gly Cys Leu Pro Ala Asn Asp Gly Ile Leu Leu Cys Gln
   195 200 205
   Glu Ala Leu Asn Ala Glu Arg Glu Leu Cys His Pro Asp Thr Asp Leu
   210 215 220
   Cys Arg Phe Asp Ala Thr Asp Glu Glu Leu Arg Leu Cys Gln Ala Cys
   225 230 235 240
   Gly Phe Glu Phe Asp Met Cys Glu Trp Thr Ser Glu Ala Ser Ala Gly
   245 250 255
   Gln Ile Ser Trp Met Arg Thr Lys Ala Arg Glu Ile Pro Ala Phe Glu
   260 265 270
   Ser Thr Pro Gln Gln Asp Gln Gly Gly Asp Asp Glu Gly Tyr Tyr Val
   275 280 285
   Trp Val Gly Ala Lys His Gly Phe Thr Leu Asn His Leu Asp Ser Arg
   290 295 300
   Ala Tyr Leu Asn Ser Ser Val Cys His Cys Leu Gly Lys Ser Cys His
   305 310 315 320
   Leu Gln Phe Tyr Tyr Ala Met Glu Ser Ser Val Leu Arg Val Arg Leu
   325 330 335
   Tyr Asn Asn Lys Glu Glu Glu Ile Phe Trp Thr Tyr Asn Ile Ser Thr
   340 345 350
   His Ser Gln Trp Val Lys Ala Asp Val Leu Ile Pro Glu Asp Leu Lys
   355 360 365
   Thr Phe Lys Thr Gln Ser Arg Lys Leu Cys Ser Ala Asp Glu Phe Pro
   370 375 380
   Cys Thr Ser Gly Gln Cys Ile Ala Lys Glu Ser Val Cys Asp Ser Arg
   385 390 395 400
   Gln Asp Cys Ser Asp Glu Ser Asp Glu Asp Pro Ala Thr Cys Ser Lys
   405 410 415
   His Leu Thr Cys Asp Phe Glu Ser Gly Phe Cys Gly Trp Glu Pro Phe
   420 425 430
   Leu Thr Glu Asp Ser His Trp Lys Leu Met Lys Gly Leu Asn Asn Gly
   435 440 445
   Glu His His Phe Pro Ala Ala Asp His Thr Ala Asn Ile Asn His Gly
   450 455 460
   Ser Phe Ile Tyr Leu Glu Ala Gln Arg Ser Pro Gly Val Ala Lys Leu
   465 470 475 480
   Gly Ser Pro Val Leu Thr Lys Leu Leu Thr Ala Ser Thr Pro Cys Gln
   485 490 495
   Val Gln Phe Trp Tyr His Leu Ser Gln His Ser Asn Leu Ser Val Phe
   500 505 510
   Thr Arg Thr Ser Leu Asp Gly Asn Leu Gln Lys Gln Gly Lys Ile Ile
   515 520 525
   Arg Phe Ser Glu Ser Gln Trp Ser His Ala Lys Ile Asp Leu Ile Ala
       530                 535                 540
   Glu Ala Gly Glu Ser Thr Leu Pro Phe Gln Leu Ile Leu Glu Ala Thr
   545                 550                 555                 560
   Val Leu Ser Ser Asn Ala Thr Val Ala Leu Asp Asp Ile Ser Val Ser
                   565                 570                 575
   Gln Glu Cys Glu Ile Ser Tyr Lys Ser Leu Pro Arg Thr Ser Thr Gln
               580                 585                 590
   Ser Lys Phe Ser Lys Cys Asp Phe Glu Ala Asn Ser Cys Asp Trp Phe
           595                 600                 605
   Glu Ala Ile Ser Gly Asp His Phe Asp Trp Ile Arg Ser Ser Gln Ser
       610                 615                 620
   Glu Leu Ser Ala Asp Phe Glu His Gln Ala Pro Pro Arg Asp His Ser
   625                 630                 635                 640
   Leu Asn Ala Ser Gln Gly His Phe Met Phe Ile Leu Lys Lys Ser Ser
                   645                 650                 655
   Ser Leu Trp Gln Val Ala Lys Leu Gln Ser Pro Thr Phe Ser Gln Thr
               660                 665                 670
   Gly Pro Gly Cys Ile Leu Ser Phe Trp Phe Tyr Asn Tyr Gly Leu Ser
           675                 680                 685
   Val Gly Ala Ala Glu Leu Gln Leu His Met Glu Asn Ser His Asp Ser
       690                 695                 700
   Thr Val Ile Trp Arg Val Leu Tyr Asn Gln Gly Lys Gln Trp Leu Glu
   705                 710                 715                 720
   Ala Thr Ile Gln Leu Gly Arg Leu Ser Gln Pro Phe His Leu Ser Leu
                   725                 730                 735
   Asp Lys Val Ser Leu Gly Ile Tyr Asp Gly Val Ser Ala Ile Asp Asp
               740                 745                 750
   Ile Arg Phe Glu Asn Cys Thr Leu Pro Leu Pro Ala Glu Ser Cys Glu
           755                 760                 765
   Gly Leu Asp His Phe Trp Cys Arg His Thr Arg Ala Cys Ile Glu Lys
       770                 775                 780
   Leu Arg Leu Cys Asp Leu Val Asp Asp Cys Gly Asp Arg Thr Asp Glu
   785                 790                 795                 800
   Val Asn Cys Ala Pro Glu Leu Gln Cys Asn Phe Glu Thr Gly Ile Cys
                   805                 810                 815
   Asn Trp Glu Gln Asp Ala Lys Asp Asp Phe Asp Trp Thr Arg Ser Gln
               820                 825                 830
   Gly Pro Thr Pro Thr Leu Asn Thr Gly Pro Met Lys Asp Asn Thr Leu
           835                 840                 845
   Gly Thr Ala Lys Gly His Tyr Leu Tyr Ile Glu Ser Ser Glu Pro Gln
       850                 855                 860
   Ala Phe Gln Asp Ser Ala Ala Leu Leu Ser Pro Ile Leu Asn Ala Thr
   865                 870                 875                 880
   Asp Thr Lys Gly Cys Thr Phe Arg Phe Tyr Tyr His Met Phe Gly Lys
                   885                 890                 895
   Arg Ile Tyr Arg Leu Ala Ile Tyr Gln Arg Ile Trp Ser Asp Ser Arg
               900                 905                 910
   Gly Gln Leu Leu Trp Gln Ile Phe Gly Asn Gln Gly Asn Arg Trp Ile
           915                 920                 925
   Arg Lys His Leu Asn Ile Ser Ser Arg Gln Pro Phe Gln Ile Leu Val
       930                 935                 940
   Glu Ala Ser Val Gly Asp Gly Phe Thr Gly Asp Ile Ala Ile Asp Asp
   945                 950                 955                 960
   Leu Ser Phe Met Asp Cys Thr Leu Tyr Pro Gly Asn Leu Pro Ala Asp
                   965                 970                 975
   Leu Pro Thr Pro Pro Glu Thr Ser Val Pro Val Thr Leu Pro Pro His
               980                 985                 990
   Asn Cys Thr Asp Asn Glu Phe Ile Cys Arg Ser Asp Gly His Cys Ile
           995                 1000                1005
   Glu Lys Met Gln Lys Cys Asp Phe Lys Tyr Asp Cys Pro Asp Lys Ser
       1010                1015                1020
   Asp Glu Ala Ser Cys Val Met Glu Val Cys Ser Phe Glu Lys Arg Ser
   1025                1030                1035                1040
   Leu Cys Lys Trp Tyr Gln Pro Ile Pro Val His Leu Leu Gln Asp Ser
                   1045                1050                1055
   Asn Thr Phe Arg Trp Gly Leu Gly Asn Gly Ile Ser Ile His His Gly
               1060                1065                1070
   Glu Glu Asn His Arg Pro Ser Val Asp His Thr Gln Asn Thr Thr Asp
   1075 1080 1085
   Gly Trp Tyr Leu Tyr Ala Asp Ser Ser Asn Gly Lys Phe Gly Asp Thr
   1090 1095 1100
   Ala Asp Ile Leu Thr Pro Ile Ile Ser Leu Thr Gly Pro Lys Cys Thr
   1105 1110 1115 1120
   Leu Val Phe Trp Thr His Met Asn Gly Ala Thr Val Gly Ser Leu Gln
   1125 1130 1135
   Val Leu Ile Lys Lys Asp Asn Val Thr Ser Lys Leu Trp Ala Gln Thr
   1140 1145 1150
   Gly Gln Gln Gly Ala Gln Trp Lys Arg Ala Glu Val Phe Leu Gly Ile
   1155 1160 1165
   Arg Ser His Thr Gln Ile Val Phe Arg Ala Lys Arg Gly Ile Ser Tyr
   1170 1175 1180
   Ile Gly Asp Val Ala Val Asp Asp Ile Ser Phe Gln Asp Cys Ser Pro
   1185 1190 1195 1200
   Leu Leu Ser Pro Glu Arg Lys Cys Thr Asp His Glu Phe Met Cys Ala
   1205 1210 1215
   Asn Lys His Cys Ile Ala Lys Asp Lys Leu Cys Asp Phe Val Asn Asp
   1220 1225 1230
   Cys Ala Asp Asn Ser Asp Glu Thr Thr Phe Ile Cys Arg Thr Ser Ser
   1235 1240 1245
   Gly Arg Cys Asp Phe Glu Phe Asp Leu Cys Ser Trp Lys Gln Glu Lys
   1250 1255 1260
   Asp Glu Asp Phe Asp Trp Asn Leu Lys Ala Ser Ser Ile Pro Ala Ala
   1265 1270 1275 1280
   Gly Thr Glu Pro Ala Ala Asp His Thr Leu Gly Asn Ser Ser Gly His
   1285 1290 1295
   Tyr Ile Phe Ile Lys Ser Leu Phe Pro Gln Gln Pro Met Arg Ala Ala
   1300 1305 1310
   Arg Ile Ser Ser Pro Val Ile Ser Lys Arg Ser Lys Asn Cys Lys Ile
   1315 1320 1325
   Ile Phe His Tyr His Met Tyr Gly Asn Gly Ile Gly Ala Leu Thr Leu
   1330 1335 1340
   Met Gln Val Ser Val Thr Asn Gln Thr Lys Val Leu Leu Asn Leu Thr
   1345 1350 1355 1360
   Val Glu Gln Gly Asn Phe Trp Arg Arg Glu Glu Leu Ser Leu Phe Gly
   1365 1370 1375
   Asp Glu Asp Phe Gln Leu Lys Phe Glu Gly Arg Val Gly Lys Gly Gln
   1380 1385 1390
   Arg Gly Asp Ile Ala Leu Asp Asp Ile Val Leu Thr Glu Asn Cys Leu
   1395 1400 1405
   Ser Leu His Asp Ser Val Gln Glu Glu Leu Ala Val Pro Leu Pro Thr
   1410 1415 1420
   Gly Phe Cys Pro Leu Gly Tyr Arg Glu Cys His Asn Gly Lys Cys Tyr
   1425 1430 1435 1440
   Arg Leu Glu Gln Ser Cys Asn Phe Val Asp Asn Cys Gly Asp Asn Thr
   1445 1450 1455
   Asp Glu Asn Glu Cys Gly Ser Ser Cys Thr Phe Glu Lys Gly Trp Cys
   1460 1465 1470
   Gly Trp Gln Asn Ser Gln Ala Asp Asn Phe Asp Trp Val Leu Gly Val
   1475 1480 1485
   Gly Ser His Gln Ser Leu Arg Pro Pro Lys Asp His Thr Leu Gly Asn
   1490 1495 1500
   Glu Asn Gly His Phe Met Tyr Leu Glu Ala Thr Ala Val Gly Leu Arg
   1505 1510 1515 1520
   Gly Asp Lys Ala His Phe Arg Ser Thr Met Trp Arg Glu Ser Ser Ala
   1525 1530 1535
   Ala Cys Thr Met Ser Phe Trp Tyr Phe Val Ser Ala Lys Ala Thr Gly
   1540 1545 1550
   Ser Ile Gln Ile Leu Ile Lys Thr Glu Lys Gly Leu Ser Lys Val Trp
   1555 1560 1565
   Gln Glu Ser Lys Gln Asn Pro Gly Asn His Trp Gln Lys Ala Asp Ile
   1570 1575 1580
   Leu Leu Gly Lys Leu Arg Asn Phe Glu Val Ile Phe Gln Gly Ile Arg
   1585 1590 1595 1600
   Thr Arg Asp Leu Gly Gly Gly Ala Ala Ile Asp Asp Ile Glu Phe Lys
   1605 1610 1615
   Asn Cys Thr Thr Val Gly Glu Ile Ser Glu Leu Cys Pro Glu Ile Thr
   1620 1625 1630
   Asp Phe Leu Cys Arg Asp Lys Lys Cys Ile Ala Ser His Leu Leu Cys
   1635 1640 1645
   Asp Tyr Lys Pro Asp Cys Ser Asp Arg Ser Asp Glu Ala His Cys Ala
   1650 1655 1660
   His Tyr Thr Ser Thr Thr Gly Ser Cys Asn Phe Glu Thr Ser Ser Gly
   1665 1670 1675 1680
   Asn Trp Thr Thr Ala Cys Ser Leu Thr Gln Asp Ser Glu Asp Asp Leu
   1685 1690 1695
   Asp Trp Ala Ile Gly Ser Arg Ile Pro Ala Lys Ala Leu Ile Pro Asp
   1700 1705 1710
   Ser Asp His Thr Pro Gly Ser Gly Gln His Phe Leu Tyr Val Asn Ser
   1715 1720 1725
   Ser Gly Ser Lys Glu Gly Ser Val Ala Arg Ile Thr Thr Ser Lys Ser
   1730 1735 1740
   Phe Pro Ala Ser Leu Gly Met Cys Thr Val Arg Phe Trp Phe Tyr Met
   1745 1750 1755 1760
   Ile Asp Pro Arg Ser Met Gly Ile Leu Lys Val Tyr Thr Ile Glu Glu
   1765 1770 1775
   Ser Gly Leu Asn Ile Leu Val Trp Ser Val Ile Gly Asn Lys Arg Thr
   1780 1785 1790
   Gly Trp Thr Tyr Gly Ser Val Pro Leu Ser Ser Asn Ser Pro Phe Lys
   1795 1800 1805
   Val Ala Phe Glu Ala Asp Leu Asp Gly Asn Glu Asp Ile Phe Ile Ala
   1810 1815 1820
   Leu Asp Asp Ile Ser Phe Thr Pro Glu Cys Val Thr Gly Gly Pro Val
   1825 1830 1835 1840
   Pro Val Gln Pro Ser Pro Cys Glu Ala Asp Gln Phe Ser Cys Ile Tyr
   1845 1850 1855
   Thr Leu Gln Cys Val Pro Leu Ser Gly Lys Cys Asp Gly His Glu Asp
   1860 1865 1870
   Cys Ile Asp Gly Ser Asp Glu Met Asp Cys Pro Leu Ser Pro Thr Pro
   1875 1880 1885
   Pro Leu Cys Ser Asn Met Glu Phe Pro Cys Ser Thr Asp Glu Cys Ile
   1890 1895 1900
   Pro Ser Leu Leu Leu Cys Asp Gly Val Pro Asp Cys His Phe Asn Glu
   1905 1910 1915 1920
   Asp Glu Leu Ile Cys Ser Asn Lys Ser Cys Ser Asn Gly Ala Leu Val
   1925 1930 1935
   Cys Ala Ser Ser Asn Ser Cys Ile Pro Ala His Gln Arg Cys Asp Gly
   1940 1945 1950
   Phe Ala Asp Cys Met Asp Phe Gln Leu Asp Glu Ser Ser Cys Ser Glu
   1955 1960 1965
   Cys Pro Leu Asn Tyr Cys Arg Asn Gly Gly Thr Cys Val Val Glu Lys
   1970 1975 1980
   Asn Gly Pro Met Cys Arg Cys Arg Gln Gly Trp Lys Gly Asn Arg Cys
   1985 1990 1995 2000
   His Ile Lys Phe Asn Pro Pro Ala Thr Asp Phe Thr Tyr Ala Gln Asn
   2005 2010 2015
   Asn Thr Trp Thr Leu Leu Gly Ile Gly Leu Ala Phe Leu Met Thr His
   2020 2025 2030
   Ile Thr Val Ala Val Leu Cys Phe Leu Ala Asn Arg Lys Val Pro Ile
   2035 2040 2045
   Arg Lys Thr Glu Gly Ser Gly Asn Cys Ala Phe Val Asn Pro Val Tyr
   2050 2055 2060
   Gly Asn Trp Ser Asn Pro Glu Lys Thr Glu Ser Ser Val Tyr Ser Phe
   2065 2070 2075 2080
   Ser Asn Pro Leu Tyr Gly Thr Thr Ser Gly Ser Leu Glu Thr Leu Ser
   2085 2090 2095
   His His Leu Lys
   2100
<210> 27
   <211> 6354
   <212> DNA
   <213> Homo sapiens
<400> 27
   atgaatgaaa ctttttgttg cctttggatt gcctgtgttt tcaattctac actggctcag 60
   caagggacag aaagctttca gtgtgacaat ggagtctcct tgcctcctga cagcatttgt 120
   gacttcacag atcagtgtgg ggatagcagt gatgaacggc actgtttgaa ttatgaaaga 180
   tgtgattttg aggatggtct ctgtcatatg actcaagatc agagtctgca acctagttgg 240
   acaaagagaa gtgggatgat tggtctatca cctccatttt atgatcacaa tggtgatgtg 300
   tctgctcact tcctctcact ggtttccaga gtggattcta tttcctcaag tttaagaagc 360
   agagttttcc ttccaacaaa tgatcaacat gactgccaga ttacatttta ttacttctcc 420
   tgccaagtga gtggcaaatt aatggttggg cttcaaactg catgtggagg tcctattcag 480
   catttatggc aaaacacagc tgcactccca aatcagtggg agagaaatgt catcaaaatc 540
   cagagttcac agagatttca ggttgttttt gaaggtcaaa tggcttcaac gtatgaacag 600
   gatgaagtca ttgctattga tgatatatct ttcagttcag gctgcttgcc tgccaatgat 660
   ggaattttac tgtgtcaaga agcattgaat gctgagcggg agctatgcca tccagataca 720
   gatctctgca gatttgatgc tacagatgaa gagttgagat tgtgtcaggc ctgtgggttt 780
   gaatttgaca tgtgtgagtg gacgtcagaa gcatctgctg gccaaatttc ctggatgcgc 840
   acaaaagcga gagagatccc tgcattcgaa tccacacctc agcaggatca aggaggtgat 900
   gatgaaggtt attatgtatg ggtaggcgct aagcatggtt tcactcttaa ccatttagac 960
   agcagggctt acctaaatag ctctgtgtgt cattgcctgg gcaagagctg tcatcttcaa 1020
   ttctattatg caatggaaag cagtgtcctg agagtaagac tgtataataa taaggaagaa 1080
   gaaatatttt ggacatacaa catatcaact cacagccaat gggtgaaagc agatgtgtta 1140
   ataccagaag atctgaagac atttaagacc caatccagaa agctttgctc tgcagacgaa 1200
   ttcccttgca ctagtggcca gtgcatcgcc aaagaatctg tctgtgactc tcggcaggac 1260
   tgctccgatg agagtgatga agacccagca acttgctcaa agcatctcac ctgtgacttt 1320
   gagtcgggtt tctgcggttg ggagccattt ctcacagaag attcacactg gaagctgatg 1380
   aaaggattga ataatggaga gcaccacttt cctgcagctg atcacacagc aaacataaat 1440
   catggatcgt ttatttattt ggaggcacag cgctcccccg ggtggccaag gcttggaagt 1500
   cctgttctta caaaattgct cactgcctct accccatgtc aggtgcagtt ttggtatcat 1560
   ttgtctcaac attcaaatct ctcagttttt acaagaacgt ctctagatgg aaacttgcaa 1620
   aagcagggca aaataatcag attctccgaa tctcagtgga gccacgcaaa aattgatctc 1680
   attgcagaag cgggagaatc tactctacct tttcagttaa ttttggaagc tactgttttg 1740
   tcgtcaaatg ctaccgttgc tctagatgac atcagtgtgt cccaggaatg tgaaatttcc 1800
   tataaatcac taccaaggac cagtacacaa agcaagtttt ccaagtgtga ctttgaagca 1860
   aacagctgtg attggtttga agcaattagt ggtgaccatt ttgactggat acggagctct 1920
   cagagtgaac tttctgctga ttttgagcac caggctccac ctcgggatca tagtctcaac 1980
   gcatctcaag ggcattttat gttcattctg aagaaaagca gcagcttgtg gcaagttgct 2040
   aagcttcaga gcccaacttt cagccagaca ggacctggat gcatactttc cttctggttc 2100
   tataactatg gcctgtcagt gggagcagct gagctgcagc tacatatgga aaattctcat 2160
   gactcaacag tgatttggag agtattatac aatcagggca aacaatggtt ggaggcaacc 2220
   attcagctag ggcgcctttc gcagcccttc catttgtcac tagataaagt cagtctgggc 2280
   atttatgatg gggtctcagc tattgatgac atccgatttg aaaattgtac tctccctctt 2340
   cctgctgaga gctgtgaagg gctggatcat ttctggtgtc gccacaccag ggcttgcata 2400
   gaaaagcttc ggttatgtga tctggtggat gactgtggtg atcgtactga tgaagtcaac 2460
   tgtgcacctg agctgcagtg taactttgaa actggaatct gtaactggga acaagatgca 2520
   aaagatgact ttgattggac caggagccag ggtccaactc caacacttaa cacagggcca 2580
   atgaaagata acactctggg cacagctaaa ggacactatc tctacataga atcttcagag 2640
   ccacaggctt ttcaagacag tgctgcctta ctcagcccaa tccttaatgc cactgataca 2700
   aaaggctgca ccttccgctt ctattaccac atgtttggaa agcgcattta taggttggca 2760
   atctaccaac gaatctggag tgactcaagg ggacagctgc tgtggcagat atttgggaat 2820
   caaggcaaca gatggattag gaaacacctc aacatttcca gcaggcagcc ctttcagata 2880
   ttggtggagg cttcagtggg agatggcttc actggagata ttgcgattga tgatctgtca 2940
   tttatggact gcaccctcta ccctggtaat ttgccagcag acctcccaac tccaccagaa 3000
   acgtcagttc ctgtaacatt acctccacac aactgcacag acaatgaatt tatctgcagg 3060
   tctgatggtc actgcattga aaaaatgcag aaatgtgatt ttaaatatga ctgccctgac 3120
   aaatcagatg aagcatcctg tgttatggaa gtttgcagct ttgagaaaag aagcctgtgt 3180
   aaatggtatc aaccaatccc agtacatttg cttcaagatt caaacacatt caggtggggg 3240
   cttgggaacg ggatcagcat tcatcatggg gaagaaaacc acaggccatc agtggatcat 3300
   acacaaaata ccactgatgg ctggtacctg tatgctgaca gttctaatgg gaaatttggt 3360
   gacacggctg acattctcac tcctatcatt tcactcacgg gaccaaaatg taccttggtg 3420
   ttctggacac atatgaatgg ggccaccgtt ggttctctcc aggtgctcat caagaaagat 3480
   aacgttactt ctaaattgtg ggctcaaact ggacagcaag gtgcacagtg gaagagagca 3540
   gaagtgtttt taggcattcg ttcacataca cagattgtct tcagagccaa acgtggtatc 3600
   agttacatag gagatgtagc agtggatgat atttccttcc aagattgctc ccctttgctt 3660
   agcccagaga gaaagtgtac tgatcatgaa ttcatgtgtg ctaataagca ctgcattgcc 3720
   aaagacaagc tgtgtgattt tgtgaatgat tgtgctgata attcagatga gactactttc 3780
   atttgccgta cctccagtgg gcgctgtgat ttcgaatttg atctttgttc ctggaagcag 3840
   gagaaagatg aggactttga ctggaacctg aaagctagca gcatccctgc agcaggcaca 3900
   gagccagcag cagatcacac tttgggaaat tcatctggtc attacatctt tataaagagt 3960
   ttgtttcctc agcagcccat gagagctgcc agaatttcaa gtccagttat aagtaagaga    4020
   agcaaaaact gcaagattat ttttcattat cacatgtatg gaaatggcat tggggcactc    4080
   accttaatgc aggtgtcagt cacaaaccaa acgaaggttc tacttaacct cactgtagaa    4140
   caaggcaatt tctggcggag agaagaactg tcactgtttg gtgatgaaga cttccaactc    4200
   aaatttgaag gtagagttgg gaaaggtcag cgtggagaca ttgcacttga tgacattgtg    4260
   cttacagaaa attgtctatc actccatgat tccgtgcaag aagaactggc agtgcctctt    4320
   ccaacaggtt tctgcccact tggctatagg gaatgtcata atggaaaatg ctataggctg    4380
   gaacaaagct gtaacttcgt agataactgt ggagataata ctgatgaaaa tgagtgtggt    4440
   agctcctgta cttttgaaaa aggctggtgt ggctggcaaa actcccaggc tgacaacttt    4500
   gattgggttt taggggttgg ctctcatcaa agcttaagac ctcccaaaga ccacacactt    4560
   ggaaatgaaa atgggcactt catgtatctg gaagctactg cagtgggcct tcggggtgac    4620
   aaagcacact tcaggagtac catgtggcga gaatccagtg cagcctgcac catgagcttc    4680
   tggtatttcg tatctgcaaa ggccacagga tccattcaga ttctcatcaa gacagagaaa    4740
   ggactatcaa aagtatggca agaaagtaag cagaaccctg gtaatcattg gcaaaaggct    4800
   gacatcctgc taggaaagtt aaggaatttt gaagtcatat ttcaaggtat cagaacaagg    4860
   gacctgggag gaggagctgc aattgatgat attgaattta aaaactgcac aactgtggga    4920
   gagatctctg agctttgtcc ggaaatcact gattttttgt gccgggacaa gaagtgcatt    4980
   gcatcccacc ttctttgtga ctataagcca gactgctctg ataggtctga tgaagctcac    5040
   tgtgcacatt atacaagcac aacaggaagc tgcaattttg aaacaagttc aggaaactgg    5100
   accacagcct gcagtcttac tcaagactct gaggatgact tggactgggc cattggcagc    5160
   agaattcctg ccaaagcatt aattccagac tctgatcaca cgccaggtag tggtcagcac    5220
   ttcctgtacg tcaactcatc tggctccaag gaaggatccg ttgccagaat tactacttcc    5280
   aaatccttcc cagcaagcct tggaatgtgt actgttcggt tctggttcta catgattgat    5340
   cccaggagta tgggaatatt aaaggtgtat accattgaag aatcggggct aaacatcctg    5400
   gtgtggtcag tgattggaaa taaaagaacg ggatggacat atggctctgt gcctctctcc    5460
   agtaacagtc cgtttaaggt ggcatttgaa gctgatttgg atggaaatga ggacatcttt    5520
   attgctcttg atgacatctc ttttacccca gagtgtgtga ctggaggtcc tgtcccagtg    5580
   cagccatcac cctgtgaagc tgatcagttt tcttgtatct acacactcca atgtgtccct    5640
   ctctcaggga aatgtgatgg acatgaagac tgcatagatg gatctgatga aatggattgt    5700
   cctctcagcc ccacccctcc actctgtagt aacatggagt tcccgtgctc tacagacgag    5760
   tgtatacctt ccctcctgct atgcgatgga gtgcccgact gccactttaa tgaagatgag    5820
   ctcatctgct ccaacaaaag ctgttctaat ggagctctgg tgtgtgcctc ctccaacagc    5880
   tgtatcccag cccaccagcg ctgtgatggt tttgccgact gcatggattt ccagcttgat    5940
   gagtccagct gctccgaatg tccattaaat tactgcagaa atggtgggac ttgtgtagtg    6000
   gagaaaaatg gtcctatgtg tcgatgtaga caaggctgga aaggaaatcg atgccatatc    6060
   aagtttaatc ctcctgctac agacttcaca tacgctcaga ataatacatg gactctcctg    6120
   ggtattggat tagcattcct gatgactcac atcacagttg cagtcttgtg ttttcttgca    6180
   aacagaaagg taccaataag gaaaaccgag ggaagtggta actgtgcctt tgtcaatcca    6240
   gtttacggga actggagcaa cccagagaaa acagagagtt ctgtctattc cttctcaaac    6300
   ccattatatg gcacaacatc aggaagcctg gagaccctgt cacatcatct caaa          6354
<210> 28
   <211> 2118
   <212> PRT
   <213> Homo sapiens
<400> 28
   Met Asn Glu Thr Phe Cys Cys Leu Trp Ile Ala Cys Val Phe Asn Ser
   1               5                   10                  15
   Thr Leu Ala Gln Gln Gly Thr Glu Ser Phe Gln Cys Asp Asn Gly Val
               20                  25                  30
   Ser Leu Pro Pro Asp Ser Ile Cys Asp Phe Thr Asp Gln Cys Gly Asp
           35                  40                  45
   Ser Ser Asp Glu Arg His Cys Leu Asn Tyr Glu Arg Cys Asp Phe Glu
       50                  55                  60
   Asp Gly Leu Cys His Met Thr Gln Asp Gln Ser Leu Gln Pro Ser Trp
   65                  70                  75                  80
   Thr Lys Arg Ser Gly Met Ile Gly Leu Ser Pro Pro Phe Tyr Asp His
                   85                  90                  95
   Asn Gly Asp Val Ser Ala His Phe Leu Ser Leu Val Ser Arg Val Asp
               100                 105                 110
   Ser Ile Ser Ser Ser Leu Arg Ser Arg Val Phe Leu Pro Thr Asn Asp
           115                 120                 125
   Gln His Asp Cys Gln Ile Thr Phe Tyr Tyr Phe Ser Cys Gln Val Ser
       130                 135                 140
   Gly Lys Leu Met Val Gly Leu Gln Thr Ala Cys Gly Gly Pro Ile Gln
   145                 150                 155                 160
   His Leu Trp Gln Asn Thr Ala Ala Leu Pro Asn Gln Trp Glu Arg Asn
                   165                 170                 175
   Val Ile Lys Ile Gln Ser Ser Gln Arg Phe Gln Val Val Phe Glu Gly
   180 185 190
   Gln Met Ala Ser Thr Tyr Glu Gln Asp Glu Val Ile Ala Ile Asp Asp
   195 200 205
   Ile Ser Phe Ser Ser Gly Cys Leu Pro Ala Asn Asp Gly Ile Leu Leu
   210 215 220
   Cys Gln Glu Ala Leu Asn Ala Glu Arg Glu Leu Cys His Pro Asp Thr
   225 230 235 240
   Asp Leu Cys Arg Phe Asp Ala Thr Asp Glu Glu Leu Arg Leu Cys Gln
   245 250 255
   Ala Cys Gly Phe Glu Phe Asp Met Cys Glu Trp Thr Ser Glu Ala Ser
   260 265 270
   Ala Gly Gln Ile Ser Trp Met Arg Thr Lys Ala Arg Glu Ile Pro Ala
   275 280 285
   Phe Glu Ser Thr Pro Gln Gln Asp Gln Gly Gly Asp Asp Glu Gly Tyr
   290 295 300
   Tyr Val Trp Val Gly Ala Lys His Gly Phe Thr Leu Asn His Leu Asp
   305 310 315 320
   Ser Arg Ala Tyr Leu Asn Ser Ser Val Cys His Cys Leu Gly Lys Ser
   325 330 335
   Cys His Leu Gln Phe Tyr Tyr Ala Met Glu Ser Ser Val Leu Arg Val
   340 345 350
   Arg Leu Tyr Asn Asn Lys Glu Glu Glu Ile Phe Trp Thr Tyr Asn Ile
   355 360 365
   Ser Thr His Ser Gln Trp Val Lys Ala Asp Val Leu Ile Pro Glu Asp
   370 375 380
   Leu Lys Thr Phe Lys Thr Gln Ser Arg Lys Leu Cys Ser Ala Asp Glu
   385 390 395 400
   Phe Pro Cys Thr Ser Gly Gln Cys Ile Ala Lys Glu Ser Val Cys Asp
   405 410 415
   Ser Arg Gln Asp Cys Ser Asp Glu Ser Asp Glu Asp Pro Ala Thr Cys
   420 425 430
   Ser Lys His Leu Thr Cys Asp Phe Glu Ser Gly Phe Cys Gly Trp Glu
   435 440 445
   Pro Phe Leu Thr Glu Asp Ser His Trp Lys Leu Met Lys Gly Leu Asn
   450 455 460
   Asn Gly Glu His His Phe Pro Ala Ala Asp His Thr Ala Asn Ile Asn
   465 470 475 480
   His Gly Ser Phe Ile Tyr Leu Glu Ala Gln Arg Ser Pro Gly Val Ala
   485 490 495
   Lys Leu Gly Ser Pro Val Leu Thr Lys Leu Leu Thr Ala Ser Thr Pro
   500 505 510
   Cys Gln Val Gln Phe Trp Tyr His Leu Ser Gln His Ser Asn Leu Ser
   515 520 525
   Val Phe Thr Arg Thr Ser Leu Asp Gly Asn Leu Gln Lys Gln Gly Lys
   530 535 540
   Ile Ile Arg Phe Ser Glu Ser Gln Trp Ser His Ala Lys Ile Asp Leu
   545 550 555 560
   Ile Ala Glu Ala Gly Glu Ser Thr Leu Pro Phe Gln Leu Ile Leu Glu
   565 570 575
   Ala Thr Val Leu Ser Ser Asn Ala Thr Val Ala Leu Asp Asp Ile Ser
   580 585 590
   Val Ser Gln Glu Cys Glu Ile Ser Tyr Lys Ser Leu Pro Arg Thr Ser
   595 600 605
   Thr Gln Ser Lys Phe Ser Lys Cys Asp Phe Glu Ala Asn Ser Cys Asp
   610 615 620
   Trp Phe Glu Ala Ile Ser Gly Asp His Phe Asp Trp Ile Arg Ser Ser
   625 630 635 640
   Gln Ser Glu Leu Ser Ala Asp Phe Glu His Gln Ala Pro Pro Arg Asp
   645 650 655
   His Ser Leu Asn Ala Ser Gln Gly His Phe Met Phe Ile Leu Lys Lys
   660 665 670
   Ser Ser Ser Leu Trp Gln Val Ala Lys Leu Gln Ser Pro Thr Phe Ser
   675 680 685
   Gln Thr Gly Pro Gly Cys Ile Leu Ser Phe Trp Phe Tyr Asn Tyr Gly
   690 695 700
   Leu Ser Val Gly Ala Ala Glu Leu Gln Leu His Met Glu Asn Ser His
   705 710 715 720
   Asp Ser Thr Val Ile Trp Arg Val Leu Tyr Asn Gln Gly Lys Gln Trp
   725 730 735
   Leu Glu Ala Thr Ile Gln Leu Gly Arg Leu Ser Gln Pro Phe His Leu
   740 745 750
   Ser Leu Asp Lys Val Ser Leu Gly Ile Tyr Asp Gly Val Ser Ala Ile
   755 760 765
   Asp Asp Ile Arg Phe Glu Asn Cys Thr Leu Pro Leu Pro Ala Glu Ser
   770 775 780
   Cys Glu Gly Leu Asp His Phe Trp Cys Arg His Thr Arg Ala Cys Ile
   785 790 795 800
   Glu Lys Leu Arg Leu Cys Asp Leu Val Asp Asp Cys Gly Asp Arg Thr
   805 810 815
   Asp Glu Val Asn Cys Ala Pro Glu Leu Gln Cys Asn Phe Glu Thr Gly
   820 825 830
   Ile Cys Asn Trp Glu Gln Asp Ala Lys Asp Asp Phe Asp Trp Thr Arg
   835 840 845
   Ser Gln Gly Pro Thr Pro Thr Leu Asn Thr Gly Pro Met Lys Asp Asn
   850 855 860
   Thr Leu Gly Thr Ala Lys Gly His Tyr Leu Tyr Ile Glu Ser Ser Glu
   865 870 875 880
   Pro Gln Ala Phe Gln Asp Ser Ala Ala Leu Leu Ser Pro Ile Leu Asn
   885 890 895
   Ala Thr Asp Thr Lys Gly Cys Thr Phe Arg Phe Tyr Tyr His Met Phe
   900 905 910
   Gly Lys Arg Ile Tyr Arg Leu Ala Ile Tyr Gln Arg Ile Trp Ser Asp
   915 920 925
   Ser Arg Gly Gln Leu Leu Trp Gln Ile Phe Gly Asn Gln Gly Asn Arg
   930 935 940
   Trp Ile Arg Lys His Leu Asn Ile Ser Ser Arg Gln Pro Phe Gln Ile
   945 950 955 960
   Leu Val Glu Ala Ser Val Gly Asp Gly Phe Thr Gly Asp Ile Ala Ile
   965 970 975
   Asp Asp Leu Ser Phe Met Asp Cys Thr Leu Tyr Pro Gly Asn Leu Pro
   980 985 990
   Ala Asp Leu Pro Thr Pro Pro Glu Thr Ser Val Pro Val Thr Leu Pro
   995 1000 1005
   Pro His Asn Cys Thr Asp Asn Glu Phe Ile Cys Arg Ser Asp Gly His
   1010 1015 1020
   Cys Ile Glu Lys Met Gln Lys Cys Asp Phe Lys Tyr Asp Cys Pro Asp
   1025 1030 1035 1040
   Lys Ser Asp Glu Ala Ser Cys Val Met Glu Val Cys Ser Phe Glu Lys
   1045 1050 1055
   Arg Ser Leu Cys Lys Trp Tyr Gln Pro Ile Pro Val His Leu Leu Gln
   1060 1065 1070
   Asp Ser Asn Thr Phe Arg Trp Gly Leu Gly Asn Gly Ile Ser Ile His
   1075 1080 1085
   His Gly Glu Glu Asn His Arg Pro Ser Val Asp His Thr Gln Asn Thr
   1090 1095 1100
   Thr Asp Gly Trp Tyr Leu Tyr Ala Asp Ser Ser Asn Gly Lys Phe Gly
   1105 1110 1115 1120
   Asp Thr Ala Asp Ile Leu Thr Pro Ile Ile Ser Leu Thr Gly Pro Lys
   1125 1130 1135
   Cys Thr Leu Val Phe Trp Thr His Met Asn Gly Ala Thr Val Gly Ser
   1140 1145 1150
   Leu Gln Val Leu Ile Lys Lys Asp Asn Val Thr Ser Lys Leu Trp Ala
   1155 1160 1165
   Gln Thr Gly Gln Gln Gly Ala Gln Trp Lys Arg Ala Glu Val Phe Leu
   1170 1175 1180
   Gly Ile Arg Ser His Thr Gln Ile Val Phe Arg Ala Lys Arg Gly Ile
   1185 1190 1195 1200
   Ser Tyr Ile Gly Asp Val Ala Val Asp Asp Ile Ser Phe Gln Asp Cys
   1205 1210 1215
   Ser Pro Leu Leu Ser Pro Glu Arg Lys Cys Thr Asp His Glu Phe Met
   1220 1225 1230
   Cys Ala Asn Lys His Cys Ile Ala Lys Asp Lys Leu Cys Asp Phe Val
   1235 1240 1245
   Asn Asp Cys Ala Asp Asn Ser Asp Glu Thr Thr Phe Ile Cys Arg Thr
   1250 1255 1260
   Ser Ser Gly Arg Cys Asp Phe Glu Phe Asp Leu Cys Ser Trp Lys Gln
   1265 1270 1275 1280
   Glu Lys Asp Glu Asp Phe Asp Trp Asn Leu Lys Ala Ser Ser Ile Pro
   1285 1290 1295
   Ala Ala Gly Thr Glu Pro Ala Ala Asp His Thr Leu Gly Asn Ser Ser
   1300 1305 1310
   Gly His Tyr Ile Phe Ile Lys Ser Leu Phe Pro Gln Gln Pro Met Arg
   1315 1320 1325
   Ala Ala Arg Ile Ser Ser Pro Val Ile Ser Lys Arg Ser Lys Asn Cys
   1330 1335 1340
   Lys Ile Ile Phe His Tyr His Met Tyr Gly Asn Gly Ile Gly Ala Leu
   1345 1350 1355 1360
   Thr Leu Met Gln Val Ser Val Thr Asn Gln Thr Lys Val Leu Leu Asn
   1365 1370 1375
   Leu Thr Val Glu Gln Gly Asn Phe Trp Arg Arg Glu Glu Leu Ser Leu
   1380 1385 1390
   Phe Gly Asp Glu Asp Phe Gln Leu Lys Phe Glu Gly Arg Val Gly Lys
   1395 1400 1405
   Gly Gln Arg Gly Asp Ile Ala Leu Asp Asp Ile Val Leu Thr Glu Asn
   1410 1415 1420
   Cys Leu Ser Leu His Asp Ser Val Gln Glu Glu Leu Ala Val Pro Leu
   1425 1430 1435 1440
   Pro Thr Gly Phe Cys Pro Leu Gly Tyr Arg Glu Cys His Asn Gly Lys
   1445 1450 1455
   Cys Tyr Arg Leu Glu Gln Ser Cys Asn Phe Val Asp Asn Cys Gly Asp
   1460 1465 1470
   Asn Thr Asp Glu Asn Glu Cys Gly Ser Ser Cys Thr Phe Glu Lys Gly
   1475 1480 1485
   Trp Cys Gly Trp Gln Asn Ser Gln Ala Asp Asn Phe Asp Trp Val Leu
   1490 1495 1500
   Gly Val Gly Ser His Gln Ser Leu Arg Pro Pro Lys Asp His Thr Leu
   1505 1510 1515 1520
   Gly Asn Glu Asn Gly His Phe Met Tyr Leu Glu Ala Thr Ala Val Gly
   1525 1530 1535
   Leu Arg Gly Asp Lys Ala His Phe Arg Ser Thr Met Trp Arg Glu Ser
   1540 1545 1550
   Ser Ala Ala Cys Thr Met Ser Phe Trp Tyr Phe Val Ser Ala Lys Ala
   1555 1560 1565
   Thr Gly Ser Ile Gln Ile Leu Ile Lys Thr Glu Lys Gly Leu Ser Lys
   1570 1575 1580
   Val Trp Gln Glu Ser Lys Gln Asn Pro Gly Asn His Trp Gln Lys Ala
   1585 1590 1595 1600
   Asp Ile Leu Leu Gly Lys Leu Arg Asn Phe Glu Val Ile Phe Gln Gly
   1605 1610 1615
   Ile Arg Thr Arg Asp Leu Gly Gly Gly Ala Ala Ile Asp Asp Ile Glu
   1620 1625 1630
   Phe Lys Asn Cys Thr Thr Val Gly Glu Ile Ser Glu Leu Cys Pro Glu
   1635 1640 1645
   Ile Thr Asp Phe Leu Cys Arg Asp Lys Lys Cys Ile Ala Ser His Leu
   1650 1655 1660
   Leu Cys Asp Tyr Lys Pro Asp Cys Ser Asp Arg Ser Asp Glu Ala His
   1665 1670 1675 1680
   Cys Ala His Tyr Thr Ser Thr Thr Gly Ser Cys Asn Phe Glu Thr Ser
   1685 1690 1695
   Ser Gly Asn Trp Thr Thr Ala Cys Ser Leu Thr Gln Asp Ser Glu Asp
   1700 1705 1710
   Asp Leu Asp Trp Ala Ile Gly Ser Arg Ile Pro Ala Lys Ala Leu Ile
   1715 1720 1725
   Pro Asp Ser Asp His Thr Pro Gly Ser Gly Gln His Phe Leu Tyr Val
   1730 1735 1740
   Asn Ser Ser Gly Ser Lys Glu Gly Ser Val Ala Arg Ile Thr Thr Ser
   1745 1750 1755 1760
   Lys Ser Phe Pro Ala Ser Leu Gly Met Cys Thr Val Arg Phe Trp Phe
   1765 1770 1775
   Tyr Met Ile Asp Pro Arg Ser Met Gly Ile Leu Lys Val Tyr Thr Ile
               1780                1785                1790
   Glu Glu Ser Gly Leu Asn Ile Leu Val Trp Ser Val Ile Gly Asn Lys
           1795                1800                1805
   Arg Thr Gly Trp Thr Tyr Gly Ser Val Pro Leu Ser Ser Asn Ser Pro
       1810                1815                1820
   Phe Lys Val Ala Phe Glu Ala Asp Leu Asp Gly Asn Glu Asp Ile Phe
   1825                1830                1835                1840
   Ile Ala Leu Asp Asp Ile Ser Phe Thr Pro Glu Cys Val Thr Gly Gly
                   1845                1850                1855
   Pro Val Pro Val Gln Pro Ser Pro Cys Glu Ala Asp Gln Phe Ser Cys
               1860                1865                1870
   Ile Tyr Thr Leu Gln Cys Val Pro Leu Ser Gly Lys Cys Asp Gly His
           1875                1880                1885
   Glu Asp Cys Ile Asp Gly Ser Asp Glu Met Asp Cys Pro Leu Ser Pro
       1890                1895                1900
   Thr Pro Pro Leu Cys Ser Asn Met Glu Phe Pro Cys Ser Thr Asp Glu
   1905                1910                1915                1920
   Cys Ile Pro Ser Leu Leu Leu Cys Asp Gly Val Pro Asp Cys His Phe
                   1925                1930                1935
   Asn Glu Asp Glu Leu Ile Cys Ser Asn Lys Ser Cys Ser Asn Gly Ala
               1940                1945                1950
   Leu Val Cys Ala Ser Ser Asn Ser Cys Ile Pro Ala His Gln Arg Cys
           1955                1960                1965
   Asp Gly Phe Ala Asp Cys Met Asp Phe Gln Leu Asp Glu Ser Ser Cys
       1970                1975                1980
   Ser Glu Cys Pro Leu Asn Tyr Cys Arg Asn Gly Gly Thr Cys Val Val
   1985                1990                1995                2000
   Glu Lys Asn Gly Pro Met Cys Arg Cys Arg Gln Gly Trp Lys Gly Asn
                   2005                2010                2015
   Arg Cys His Ile Lys Phe Asn Pro Pro Ala Thr Asp Phe Thr Tyr Ala
               2020                2025                2030
   Gln Asn Asn Thr Trp Thr Leu Leu Gly Ile Gly Leu Ala Phe Leu Met
           2035                2040                2045
   Thr His Ile Thr Val Ala Val Leu Cys Phe Leu Ala Asn Arg Lys Val
       2050                2055                2060
   Pro Ile Arg Lys Thr Glu Gly Ser Gly Asn Cys Ala Phe Val Asn Pro
   2065                2070                2075                2080
   Val Tyr Gly Asn Trp Ser Asn Pro Glu Lys Thr Glu Ser Ser Val Tyr
                   2085                2090                2095
   Ser Phe Ser Asn Pro Leu Tyr Gly Thr Thr Ser Gly Ser Leu Glu Thr
               2100                2105                2110
   Leu Ser His His Leu Lys
           2115
<210> 29
   <211> 13
   <212> PRT
   <213> Artificial Sequence
   <220>
   <223> Fusion protein linker sequence
<400> 29
   Glu Phe Gly Ala Gly Leu Val Leu Gly Gly Gln Phe Met
   1               5                   10
<210> 30
   <211> 27
   <212> DNA
   <213> Artificial Sequence
   <220>
   <223> Primer INSP152-CP1
<400> 30
   atgaatgaaa ctttttgttg cctttgg 27
<210> 31
   <211> 22
   <212> DNA
   <213> Artificial Sequence
   <220>
   <223> Primer INSP152-CP2
<400> 31
   gcaagcagcc tgaactgaaa ga 22
<210> 32
   <211> 23
   <212> DNA
   <213> Artificial Sequence
   <220>
   <223> Primer INSP152-CP3
<400> 32
   attgcctgtg ttttcaattc tac 23
<210> 33
   <211> 24
   <212> DNA
   <213> Artificial Sequence
   <220>
   <223> Primer INSP152-CP4
<400> 33
   tatatcatca atagcaatga cttc 24
<210> 34
   <211> 19
   <212> DNA
   <213> Artificial Sequence
   <220>
   <223> Primer T7
<400> 34
   taatacgact cactatagg 19
<210> 35
   <211> 18
   <212> DNA
   <213> Artificial Sequence
   <220>
   <223> Primer T3
<400> 35
   attaaccctc actaaagg 18
<210> 36
   <211> 24
   <212> DNA
   <213> Artificial Sequence
   <220>
   <223> Primer h-INSP152-D1-SV1-519F
<400> 36
   ggagagaaat gtcatcaaaa tcca 24
<210> 37
   <211> 23
   <212> DNA
   <213> Artificial Sequence
   <220>
   <223> Primer h-INSP152-D1-SV1-584R
<400> 37
   gccatttgac cttcaaaaac aac 23
<210> 38
   <211> 18
   <212> DNA
   <213> Artificial Sequence
   <220>
   <223> Primer hGAPDH-F
<400> 38
   ccacccatgg caaattcc 18
<210> 39
   <211> 22
   <212> DNA
   <213> Artificial Sequence
   <220>
   <223> Primer hGAPDH-R
<400> 39
   gatgggattt ccattgatga ca 22
<210> 40
   <211> 21
   <212> DNA
   <213> Artificial Sequence
   <220>
   <223> Primer Intron-hGAPDH-F
<400> 40
   cctagtccca gggctttgat t 21
<210> 41
   <211> 21
   <212> DNA
   <213> Artificial Sequence
   <220>
   <223> Primer Intron-hGAPDH-R
<400> 41
   ctgtgctccc actcctgatt t 21

## Claims

1. A polypeptide, which comprises the amino acid sequence as recited in SEQ ID NO:2 and/or SEQ ID NO: 4, and which acts as an inhibitor of T lymphocytes.

2. A polypeptide according to claim 1, wherein the polypeptide is selected from the group consisting of the amino acid sequence recited in SEQ ID NO: 6; SEQ ID NO: 8; SEQ ID NO: 10; SEQ ID NO: 12; SEQ ID NO: 14; SEQ ID NO: 16; and/or SEQ ID NO: 18.

3. The polypeptide of claim I or claim 2, wherein said polypeptide comprises a histidine tag.

4. The polypeptide of any preceding claim, wherein said polypeptide comprises a signal peptide.

5. The polypeptide of claim 4, whose sequence is recited in SEQ ID NO: 20, SEQ ID NO: 22, SEQ ID NO: 24, SEQ ID NO: 26 and/or SEQ ID NO: 28.

6. A fusion protein comprising a polypeptide according to any previous claim.

7. A fusion protein according to claim 6, comprising SEQ ID NO: 4, SEQ ID NO: 6 or SEQ ID NO: 22.

8. A purified nucleic acid molecule which encodes a polypeptide according to any one of the preceding claims.

9. A purified nucleic acid molecule according to claim 8, which comprises or consists of the nucleic acid sequence as recited in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25 and/or SEQ ID NO: 27.

10. A purified nucleic acid molecule according to claim 8 or claim 9 which consists of the nucleic acid sequence as recited in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25 and/or SEQ ID NO: 27.

11. A vector comprising a nucleic acid molecule as recited in any one of claims 8 to 10.

12. An isolated host cell transformed with a vector according to claim 11.

13. A ligand which binds specifically to the MAM domain containing protein according to any one of claims 1 to 7 and which is an antibody.

14. A polypeptide according to any one of claims 1 to 7, a nucleic acid molecule according to any one of claims 8 to 10, a vector according to claim 11, a host cell according to claim 12, or a ligand according to claim 13, for use in therapy or diagnosis of disease.

15. An *in vitro* method of diagnosing a disease in a patient, comprising assessing the level of expression of a natural gene encoding a polypeptide according to any one of claims 1 to 7, or assessing the activity of a polypeptide according to any one of claims 1 to 7, in tissue from said patient and comparing said level of expression or activity to a control level, wherein a level that is different to said control level is indicative of disease.

16. A method according to claim 15, which comprises the steps of: (a) contacting a ligand according to claim 13 with a biological sample under conditions suitable for the formation of a ligand-polypeptide complex; and (b) detecting said complex.

17. A method according to claim 15, comprising the steps of:
a) contacting a sample of tissue from the patient with a nucleic acid probe under stringent conditions that allow the formation of a hybrid complex between a nucleic acid molecule according to any one of claims 8 to 10 and the probe;
b) contacting a control sample with said probe under the same conditions used in step a); and
c) detecting the presence of hybrid complexes in said samples; wherein detection of levels of the hybrid complex in the patient sample that differ from levels of the hybrid complex in the control sample is indicative of disease.

18. A method according to claim 15, comprising:
a)contacting a sample of nucleic acid from tissue of the patient with a nucleic acid primer under stringent conditions that allow the formation of a hybrid complex between a nucleic acid molecule according to any one of claims 8 to 10 and the primer;
b)contacting a control sample with said primer under the same conditions used in step a); and
c)amplifying the sampled nucleic acid; and
d)detecting the level of amplified nucleic acid from both patient and control samples; wherein detection of levels of the amplified nucleic acid in the patient sample that differ significantly from levels of the amplified nucleic acid in the control sample is indicative of disease.

19. A method according to any one of claims 15 to 18, wherein said disease includes, inflammatory bowel disease, arthritis, psoriasis, organ transplant rejection; and Crohn's disease,

20. A method according to any one of claims 15 to 18, wherein said disease is lupus.

21. Use of a polypeptide according to any one of claims 1 to 7 as a MAM domain containing protein and/or LDL domain containing protein.

22. A pharmaceutical composition comprising a polypeptide according to any one of claims 1 to 7, a nucleic acid molecule according to any one of claims 8 to 10, a vector according to claim 11, an isolated host cell according to claim 12, or a ligand according to claim 13.

23. A pharmaceutical composition according to claim 22, further comprising one or more of cyclophosphamide (CTX), CTLA4-Ig rapamycin, mycophenolic acid, methylprednisone, FTY720, an LFA-1 blocking agent, ICOS-Ig, an anti-apoptotic agent, an antagonist of cytolytic T cell function, TACI-Ig or methotrexate.

24. A vaccine composition comprising a polypeptide according to any one of claims 1 to 7 or a nucleic acid molecule according to any one of claims 8 to 10.

25. A polypeptide according to any one of claims 1 to 7, a nucleic acid molecule according to any one of claims 8 to 10, a vector according to claim 11, a host cell according to claim 12, a ligand according to claim 13, or a pharmaceutical composition according to claim 22 or claim 23, for use in the manufacture of a medicament for the treatment of inflammatory bowel disease, arthritis, psoriasis, organ transplant rejection; and Crohn's disease.

26. A polypeptide according to any one of claims 1 to 7, a nucleic acid molecule according to any one of claims 8 to 10, a vector according to claim 11, a host cell according to claim 12 a ligand according to claim 13, or a pharmaceutical composition according to claim 22 or claim 23, for use in the manufacture of a medicament for the treatment of lupus.

27. A method of monitoring the therapeutic treatment of disease in a patient, comprising monitoring over a period of time the level of expression or activity of a polypeptide according to any one of claims 1 to 7, or the level of expression of a nucleic acid molecule according to any one of claims 8 to 10 in tissue from said patient, wherein altering said level of expression or activity over the period of time towards a control level is indicative of regression of said disease.

28. A method for the identification of a compound that is effective in the treatment and/or diagnosis of disease, comprising contacting a polypeptide according to any one of claims 1 to 7, or a nucleic acid molecule according to any one of claims 8 to 10 with one or more compounds suspected of possessing binding affinity for said polypeptide or nucleic acid molecule, and selecting a compound that binds specifically to said nucleic acid molecule or polypeptide.

29. A kit useful for diagnosing disease comprising a first container containing a nucleic acid probe that hybridises under stringent conditions with a nucleic acid molecule according to any one of claims 8 to 10; a second container containing primers useful for amplifying said nucleic acid molecule; and instructions for using the probe and primers for facilitating the diagnosis of disease.

30. The kit of claim 29, further comprising a third container holding an agent for digesting unhybridised RNA.

31. A kit comprising an array of nucleic acid molecules, at least one of which is a nucleic acid molecule according to any one of claims 8 to 10.

32. A kit comprising one or more antibodies that bind to a polypeptide as recited in any one of claims 1 to 7; and a reagent useful for the detection of a binding reaction between said antibody and said polypeptide.

33. A transgenic or knockout non-human animal that has been transformed to express higher, lower or absent levels of a polypeptide according to any one of claims I to 7.

34. A method for screening for a compound effective to treat disease, by contacting a non-human transgenic animal according to claim 33 with a candidate compound and determining the effect of the compound on the disease of the animal.

35. Method of selecting biologically active compounds comprising:
(i) contacting a candidate compound with recombinant host cells according to claim 12;
(ii) selecting compounds that bind the expressed polypeptide according to any one of claims 1-7, at the surface of said cells and/or that modulate the activity of the said polypeptide.

## Patentansprüche

1. Polypeptid, das die in SEQ ID NO:2 und/oder SEQ ID NO: 4 genannte Aminosäuresequenz(en) umfaßt und das als ein Inhibitor von T-Lymphozyten wirkt.

2. Polypeptid nach Anspruch 1, wobei das Polypeptid ausgewählt ist aus der Gruppe, bestehend aus den in SEQ ID NO: 6, SEQ ID NO: 8, SEQ ID NO: 10, SEQ ID NO: 12, SEQ ID NO: 14, SEQ ID NO: 16 und/oder SEQ ID NO: 18 genannten Aminosäuresequenzen.

3. Polypeptid nach Anspruch 1 oder Anspruch 2, wobei das Polypeptid ein Histidin-Tag umfaßt.

4. Polypeptid nach einem der vorhergehenden Ansprüche, wobei das Polypeptid ein Signalpeptid umfaßt.

5. Polypeptid nach Anspruch 4, dessen Sequenz in SEQ ID NO: 20, SEQ ID NO: 22, SEQ ID NO: 24, SEQ ID NO: 26 und/oder SEQ ID NO: 28 genannt ist.

6. Fusionsprotein, umfassend ein Polypeptid nach einem der vorherigen Ansprüche.

7. Fusionsprotein nach Anspruch 6, umfassend SEQ ID NO: 4, SEQ ID NO: 6 oder SEQ ID NO: 22.

8. Gereinigtes Nukleinsäuremolekül, das ein Polypeptid nach einem der vorhergehenden Ansprüche kodiert.

9. Gereinigtes Nukleinsäuremolekül nach Anspruch 8, das die in SEQ ID NO: 1, SEO ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25 und/oder SEQ ID NO: 27 genannte(n) Nukleinsäuresequenz(en) umfaßt oder daraus besteht.

10. Gereinigtes Nukleinsäuremolekül nach Anspruch 8 oder Anspruch 9, das aus der in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15,. SEQ ID NO:17, SEQ ID NO: 19, SEQ ID NO: 21, SEQ ID NO: 23, SEQ ID NO: 25 und/oder SEQ ID NO: 27 genannte(n) Nukleinsäuresequenz(en) besteht.

11. Vektor, umfassend ein Nukleinsäuremolekül wie in einem der Ansprüche 8 bis 10 genannt.

12. Isolierte Wirtszelle, die mit einem Vektor nach Anspruch 11 transformiert ist.

13. Ligand, der spezifisch an das MAM-Domäne-enthaltende Protein nach einem der Ansprüche 1 bis 7 bindet und der ein Antikörper ist.

14. Polypeptid nach einem der Ansprüche 1 bis 7, Nukleinsäuremolekül nach einem der Ansprüche 8 bis 10, Vektor nach Anspruch 11, Wirtszelle nach Anspruch 12 oder Ligand nach Anspruch 13, zur Verwendung in der Therapie oder Diagnose einer Krankheit.

15. *In vitro* Verfahren zur Diagnose einer Krankheit in einem Patienten, umfassend das Beurteilen des Expressionslevels eines natürlichen Gens, das ein Polypeptid nach einem der Ansprüche 1 bis 7 kodiert, oder das Beurteilen der Aktivität eines Polypeptids nach einem der Ansprüche 1 bis 7 in Gewebe von dem Patienten und das Vergleichen des Expressionslevels oder der Aktivität mit einem Kontroll-Level, wobei ein Level, der verschieden von dem Kontroll-Level ist, eine Krankheit anzeigt.

16. Verfahren nach Anspruch 15, das die Schritte umfaßt:
(a) Inkontaktbringen eines Liganden nach Anspruch 13 mit einer biologischen Probe unter Bedingungen, die für die Bildung eines Ligand-Polypeptid-Komplexes geeignet sind, und
(b) Nachweisen des Komplexes.

17. Verfahren nach Anspruch 15, umfassend die Schritte:
(a) Inkontaktbringen einer Gewebeprobe von dem Patienten mit einer Nukleinsäuresonde unter stringenten Bedingungen, welche die Bildung eines Hybridkomplexes zwischen einem Nukleinsäuremolekül nach einem der Ansprüche 8 bis 10 und der Sonde erlauben,
(b) Inkontaktbringen einer Kontrollprobe mit der Sonde unter den gleichen Bedingungen wie in Schritt (a) verwendet, und
(c) Nachweisen der Anwesenheit von Hybridkomplexen in der Probe, wobei der Nachweis von Leveln des Hybridkomplexes in der Patientenprobe, die von Leveln des Hybridkomplexes in der Kontrollprobe verschieden sind, eine Krankheit anzeigen.

18. Verfahren nach Anspruch 15, umfassend:
(a) Inkontaktbringen einer Nukleinsäureprobe aus Gewebe des Patienten mit einem Nukleinsäureprimer unter stringenten Bedingungen, welche die Bildung eines Hybridkomplexes zwischen einem Nukleinsäuremolekül nach einem der Ansprüche 8 bis 10 und dem Primer erlauben,
(b) Inkontaktbringen einer Kontrollprobe mit dem Primer unter den gleichen Bedingungen wie in Schritt (a) verwendet, und
(c) Amplifizieren der als Probe genommenen Nukleinsäure, und
(d) Nachweisen des Levels amplifizierter Nukleinsäure von sowohl den Patienten- und den Kontrollproben, wobei der Nachweis von Leveln amplifizierter Nukleinsäure in der Patientenprobe, die signifikant von Leveln amplifizierter Nukleinsäure in der Kontrollprobe verschieden sind, eine Krankheit anzeigen.

19. Verfahren nach einem der Ansprüche 15 bis 18, wobei die Krankheit chronisch entzündliche Darmerkrankung, Arthritis, Psoriasis, Organtransplantatabstoßung und Morbus Crohn einschließt.

20. Verfahren nach einem der Ansprüche 15 bis 18, wobei die Krankheit Lupus ist.

21. Verwendung eines Polypeptids nach einem der Ansprüche 1 bis 7 als ein MAM-Domäne-enthaltendes Protein und/oder LDL-Domäne-enthaltendes Protein.

22. Pharmazeutische Zusammensetzung, umfassend ein Polypeptid nach einem der Ansprüche 1 bis 7, ein Nukleinsäuremolekül nach einem der Ansprüche 8 bis 10, einen Vektor nach Anspruch 11, eine isolierte Wirtszelle nach Anspruch 12 oder einen Liganden nach Anspruch 13.

23. Pharmazeutische Zusammensetzung nach Anspruch 22, weiter umfassend eines oder mehrere von Cyclophosphamid (CTX), CTLA4-Ig, Rapamycin, Mycophenolsäure, Methylprednison, FTY720, einem LFA-1 blockierenden Mittel, ICOS-Ig, einem anti-apoptotischen Mittel, einem Antagonisten zytolytischer T-Zellfunktion, TACI-Ig oder Methotrexat.

24. Impfstoffzusammensetzung, umfassend ein Polypeptid nach einem der Ansprüche 1 bis 7 oder ein Nukleinsäuremolekül nach einem der Ansprüche 8 bis 10.

25. Polypeptid nach einem der Ansprüche 1 bis 7, Nukleinsäuremolekül nach einem der Ansprüche 8 bis 10, Vektor nach Anspruch 11, Wirtszelle nach Anspruch 12, Ligand nach Anspruch 13 oder pharmazeutische Zusammensetzung nach Anspruch 22 oder Anspruch 23 zur Verwendung in der Herstellung eines Medikaments für die Behandlung von chronisch entzündlicher Darmerkrankung, Arthritis, Psoriasis, Organtransplantatabstoßung und Morbus Crohn.

26. Polypeptid nach einem der Ansprüche 1 bis 7, Nukleinsäuremolekül nach einem der Ansprüche 8 bis 10, Vektor nach Anspruch 11, Wirtszelle nach Anspruch 12, Ligand nach Anspruch 13 oder pharmazeutische Zusammensetzung nach Anspruch 22 oder Anspruch 23 zur Verwendung in der Herstellung eines Medikaments für die Behandlung von Lupus.

27. Verfahren zur Überwachung der therapeutischen Behandlung einer Krankheit in einem Patienten, umfassend das Überwachen des Expressionslevels oder der Aktivität eines Polypeptids nach einem der Ansprüche 1 bis 7 oder des Expressionslevels eines Nukleinsäuremoleküls nach einem der Ansprüche 8 bis 10 in Gewebe von dem Patienten über einen Zeitraum hinweg, wobei ein Verändern des Expressionslevels oder der Aktivität über den Zeitraum hinweg in Richtung eines Kontroll-Levels einen Rückgang der Krankheit anzeigt.

28. Verfahren zur Identifizierung einer Verbindung, die wirksam in der Behandlung und/oder Diagnose einer Krankheit ist, umfassend das Inkontaktbringen eines Polypeptids nach einem der Ansprüche 1 bis 7 oder eines Nukleinsäuremoleküls nach einem der Ansprüche 8 bis 10 mit einer oder mehreren Verbindungen, die im Verdacht stehen, Bindungsaffinität zu dem Polypeptid oder Nukleinsäuremolekül aufzuweisen, und das Auswählen einer Verbindung, die spezifisch an das Nukleinsäuremolekül oder Polypeptid bindet.

29. Kit, das nützlich für die Diagnose einer Krankheit ist, umfassend einen ersten Behälter, enthaltend eine Nukleinsäuresonde, die unter stringenten Bedingungen mit einem Nukleinsäuremolekül nach einem der Ansprüche 8 bis 10 hybridisiert, einen zweiten Behälter, enthaltend Primer, die nützlich für die Amplifikation des Nukleinsäuremoleküls sind, und Anweisungen zur Verwendung der Sonde und der Primer, um die Diagnose einer Krankheit zu ermöglichen.

30. Kit nach Anspruch 29, weiter umfassend einen dritten Behälter, der ein Mittel zum Verdauen von unhybridisierter RNA enthält.

31. Kit, umfassend ein Array von Nukleinsäuremolekülen, von denen mindestens eines ein Nukleinsäuremolekül nach einem der Ansprüche 8 bis 10 ist.

32. Kit, umfassend einen oder mehrere Antikörper, die an ein Polypeptid wie in einem der Ansprüche 1 bis 7 genannt binden, und ein Reagenz, das nützlich für den Nachweis einer Bindungsreaktion zwischen dem Antikörper und dem Polypeptid ist.

33. Nicht-humanes transgenes oder Knock-out Tier, das transformiert wurde, um höhere, niedrigere, oder keine Level eines Polypeptids nach einem der Ansprüche 1 bis 7 zu exprimieren.

34. Verfahren zum Screening einer Verbindung, die wirksam für die Behandlung einer Krankheit ist, durch Inkontaktbringen eines nicht-menschlichen, transgenen Tieres nach Anspruch 33 mit einer Kandidatenverbindung und Bestimmen der Wirkung der Verbindung auf die Krankheit des Tieres.

35. Verfahren zum Auswählen biologisch aktiver Verbindungen, umfassend:
(i) Inkontaktbringen einer Kandidatenverbindung mit rekombinanten Wirtszellen nach Anspruch 12,
(ii) Auswählen von Verbindungen, die an das exprimierte Polypeptid nach einem der Ansprüche 1 bis 7 an der Oberfläche der Zellen binden und/oder welche die Aktivität des Polypeptids modulieren.

## Revendications

1. Polypeptide, qui comprend la séquence d'acides aminés telle qu'indiquée dans SÉQ ID No.: 2 et/ou SÉQ ID No.: 4, et qui agit comme un inhibiteur de lymphocytes T.

2. Polypeptide selon la revendication 1, dans lequel le polypeptide est choisi dans le groupe constitué par la séquence d'acides aminés indiquée dans SÉQ ID No.: 6 ; SÉQ ID No.: 8 ; SÉQ ID No.: 10 ; SÉQ ID No.: 12 ; SÉQ ID No.: 14 ; SÉQ ID No.: 16 ; et/ou SÉQ ID No.: 18.

3. Polypeptide selon la revendication 1 ou la revendication 2, dans lequel ledit polypeptide comprend une étiquette histidine.

4. Polypeptide selon l'une quelconque des revendications précédentes, dans lequel ledit polypeptide comprend un peptide signal.

5. Polypeptide selon la revendication 4, dont la séquence est indiquée dans SÉQ ID No.: 20, SÉQ ID No.: 22, SÉQ ID No.: 24, SÉQ ID No.: 26 et/ou SÉQ ID No.: 28.

6. Protéine de fusion comprenant un polypeptide selon l'une quelconque des revendications précédentes.

7. Protéine de fusion selon la revendication 6, comprenant SÉQ ID No.: 4, SÉQ ID No.: 6 ou SÉQ ID No.: 22.

8. Molécule d'acide nucléique purifiée qui code pour un polypeptide selon l'une quelconque des revendications précédentes.

9. Molécule d'acide nucléique purifiée selon la revendication 8, qui comprend ou est constituée par la séquence d'acide nucléique telle qu'indiquée dans SÉQ ID No.: 1, SÉQ ID No.: 3, SÉQ ID No.: 5, SÉQ ID No.: 7, SÉQ ID No.: 9, SÉQ ID No.: 11, SÉQ ID No.: 13, SÉQ ID No.: 15, SÉQ ID No.: 17, SÉQ ID No.: 19, SÉQ ID No.: 21, SÉQ ID No.: 23, SÉQ ID No.: 25 et/ou SÉQ ID No.: 27.

10. Molécule d'acide nucléique purifiée selon la revendication 8 ou la revendication 9, qui est constituée par la séquence d'acide nucléique telle qu'indiquée dans SÉQ ID No.: 1, SÉQ ID No.: 3, SÉQ ID No.: 5, SÉQ ID No.: 7, SÉQ ID No.: 9, SÉQ ID No.: 11, SÉQ ID No.: 13, SÉQ ID No.: 15, SÉQ ID No.: 17, SÉQ ID No.: 19, SÉQ ID No.: 21, SÉQ ID No.: 23, SÉQ ID No.: 25 et/ou SÉQ ID No.: 27.

11. Vecteur comprenant une molécule d'acide nucléique selon l'une quelconque des revendications 8 à 10.

12. Cellule-hôte isolée transformée avec un vecteur selon la revendication 11.

13. Ligand qui se lie spécifiquement à une protéine contenant un domaine MAM selon l'une quelconque des revendications 1 à 7 et qui est un anticorps.

14. Polypeptide selon l'une quelconque des revendications 1 à 7, molécule d'acide nucléique selon l'une quelconque des revendications 8 à 10, vecteur selon la revendication 11, cellule-hôte selon la revendication 12, ou ligand selon la revendication 13, utilisable pour traiter ou diagnostiquer une maladie.

15. Procédé in vitro pour diagnostiquer une maladie chez un patient, comprenant l'évaluation du niveau d'expression d'un gène naturel codant pour un polypeptide selon l'une quelconque des revendications 1 à 7, ou l'évaluation de l'activité d'un polypeptide selon l'une quelconque des revendications 1 à 7, dans un tissu provenant dudit patient et la comparaison dudit niveau d'expression ou de ladite activité à un niveau témoin, dans lequel un niveau qui est différent dudit niveau témoin est indicateur de maladie.

16. Procédé selon la revendication 15, qui comprend les étapes de : (a) mise en contact d'un ligand selon la revendication 13 avec un échantillon biologique dans des conditions convenables pour former un complexe ligand-polypeptide ; et (b) détection dudit complexe.

17. Procédé selon la revendication 15, comprenant les étapes de :
a) mise en contact d'un échantillon de tissu provenant du patient avec une sonde d'acide nucléique dans des conditions stringentes qui permettent la formation d'un complexe hybride entre une molécule d'acide nucléique selon l'une quelconque des revendications 8 à 10 et la sonde ;
b) mise en contact d'un échantillon témoin avec ladite sonde dans les mêmes conditions que celles utilisées dans l'étape a) ; et
c) détection de la présence de complexes hybrides dans lesdits échantillons ; dans lequel la détection de niveaux du complexe hybride dans l'échantillon du patient qui diffèrent des niveaux du complexe hybride dans l'échantillon témoin est indicatrice de maladie.

18. Procédé selon la revendication 15, comprenant :
a) la mise en contact d'un échantillon d'acide nucléique provenant d'un tissu du patient avec une amorce d'acide nucléique dans des conditions stringentes qui permettent de former un complexe hybride entre une molécule d'acide nucléique selon l'une quelconque des revendications 8 à 10 et l'amorce ;
b) la mise en contact d'un échantillon témoin avec ladite amorce dans les mêmes conditions que celles utilisées dans l'étape a) ;
c) l'amplification de l'acide nucléique échantillonné ; et
d) la détection du niveau d'acide nucléique amplifié provenant à la fois des échantillons du patient et du témoin ; dans lequel la détection de niveaux de l'acide nucléique amplifié dans l'échantillon du patient qui diffèrent significativement des niveaux de l'acide nucléique amplifié dans l'échantillon témoin est indicatrice de maladie.

19. Procédé selon l'une quelconque des revendications 15 à 18, dans lequel ladite maladie inclut les maladies intestinales inflammatoires, l'arthrite, le psoriasis, le rejet des greffes d'organes et la maladie de Crohn.

20. Procédé selon l'une quelconque des revendications 15 à 18, dans lequel ladite maladie est le lupus.

21. Utilisation d'un polypeptide selon l'une quelconque des revendications 1 à 7 à titre de protéine contenant un domaine MAM et/ou de protéine contenant un domaine LDL.

22. Composition pharmaceutique comprenant un polypeptide selon l'une quelconque des revendications 1 à 7, une molécule d'acide nucléique selon l'une quelconque des revendications 8 à 10, un vecteur selon la revendication 11, une cellule-hôte isolée selon la revendication 12, ou un ligand selon la revendication 13.

23. Composition pharmaceutique selon la revendication 22 comprenant, en outre, un ou plusieurs des ingrédients suivants : cyclophosphamide (CTX), CTLA4-Ig, rapamycine, acide mycophénolique, méthyl-prednisone, FTY720, agent de blocage LFA-1, ICOS-Ig, agent anti-apoptique, antagoniste de la fonction cellulaire T cytotoxique, TACI-Ig ou méthotréxate.

24. Composition vaccinale comprenant un polypeptide selon l'une quelconque des revendications 1 à 7 ou une molécule d'acide nucléique selon l'une quelconque des revendications 8 à 10.

25. Polypeptide selon l'une quelconque des revendications 1 à 7, molécule d'acide nucléique selon l'une quelconque des revendications 8 à 10, vecteur selon la revendication 11, cellule-hôte selon la revendication 12, ligand selon la revendication 13, ou composition pharmaceutique selon la revendication 22 ou la revendication 23, utilisable dans la fabrication d'un médicament destiné à traiter les maladies intestinales inflammatoires, l'arthrite, le psoriasis, le rejet des greffes d'organes et la maladie de Crohn.

26. Polypeptide selon l'une quelconque des revendications 1 à 7, molécule d'acide nucléique selon l'une quelconque des revendications 8 à 10, vecteur selon la revendication 11, cellule-hôte selon la revendication 12, ligand selon la revendication 13, ou composition pharmaceutique selon la revendication 22 ou la revendication 23, utilisable dans la fabrication d'un médicament destiné à traiter le lupus.

27. Procédé pour surveiller le traitement thérapeutique d'une maladie chez un patient, comprenant la surveillance sur une certaine période de temps du niveau d'expression ou d'activité d'un polypeptide selon l'une quelconque de revendications 1 à 7, ou le niveau d'expression d'une molécule d'acide nucléique selon l'une quelconque des revendications 8 à 10 dans le tissu provenant dudit patient, dans lequel la variation dudit niveau d'expression ou d'activité sur ladite période de temps en direction du niveau témoin est indicatrice de régression de ladite maladie.

28. Procédé d'identification d'un composé qui est efficace pour traiter et/ou diagnostiquer une maladie, comprenant la mise en contact d'un polypeptide selon l'une quelconque de revendications 1 à 7, ou d'une molécule d'acide nucléique selon l'une quelconque des revendications 8 à 10 avec un ou plusieurs composés suspectés de posséder une affinité de liaison pour ledit polypeptide ou ladite molécule d'acide nucléique, et la sélection d'un composé qui se lie spécifiquement à ladite molécule d'acide nucléique ou audit polypeptide.

29. Trousse utile pour diagnostiquer une maladie comprenant un premier récipient contenant une sonde d'acide nucléique qui s'hybride dans des conditions stringentes avec une molécule d'acide nucléique selon l'une quelconque des revendications 8 à 10 ; un second récipient contenant des amorces utiles pour amplifier ladite molécule d'acide nucléique ; et des instructions pour utiliser la sonde et les amorces pour faciliter le diagnostic de la maladie.

30. Trousse selon la revendication 29 comprenant, en outre, un troisième récipient renfermant un agent pour digérer l'ARN non hybridé.

31. Trousse comprenant une puce à molécules d'acides nucléiques, dont une au moins est une molécule d'acide nucléique selon l'une quelconque des revendications 8 à 10.

32. Trousse comprenant un ou plusieurs anticorps qui se lient à un polypeptide tel qu'indiqué dans l'une quelconque des revendications 1 à 7 ; et un réactif utile pour détecter une réaction de liaison entre ledit anticorps et ledit polypeptide.

33. Animal transgénique ou knock-out non humain qui a été transformé pour exprimer des niveaux supérieurs, inférieurs ou nuls d'un polypeptide selon l'une quelconque de revendications 1 à 7.

34. Procédé de criblage pour identifier un composé efficace pour traiter une maladie, par mise en contact d'un animal transgénique non humain selon la revendication 33 avec un composé candidat et détermination de l'effet du composé sur la maladie de l'animal.

35. Procédé pour sélectionner des composés biologiquement actifs comprenant :
(i) la mise en contact d'un composé candidat avec des cellules-hôtes recombinantes selon la revendication 12 ; et
(ii) la sélection de composés qui se lient au polypeptide exprimé selon l'une quelconque de revendications 1 à 7, à la surface desdites cellules et/ou qui modulent l'activité dudit polypeptide.
